# EUROPEAN PATENT SPECIFICATION

(11) **EP 1 745 151 B1**
(45) Date of publication and mention of the grant of the patent: **02.12.2009**
(21) Application number: 05740635.7
(22) Date of filing: 08.05.2005
(51) Int. Cl.: C12Q 1/68, G01N 33/68, A61K 48/00

(54) **METHODS RELATING TO DISORDERS ASSOCIATED WITH ABNORMAL PHOSPHATE METABOLISM**
VERFAHREN IN ZUSAMMENHANG MIT ANOMALEN PHOSPHATMETABOLISMUS ASSOZIIERTEN ERKRANKUNGEN
METHODES CONCERNANT LES TROUBLES ASSOCIES AVEC UN METABOLISME DU PHOSPHATE ABNORMAL

(30) Priority: 09.05.2004 IL 16188604; 18.06.2004 US 870030
(43) Date of publication of application: 24.01.2007
(73) Proprietor: TECHNION RESEARCH AND DEVELOPMENT FOUNDATION, LTD., Haifa 32000 (IL)
(72) Inventor: SPRECHER, Eli, 36056 Kiryat Tivon (IL); BERGMAN, Reuven, 34406 Haifa (IL)
(74) Representative: Machtalère, Georges
(86) International application number: PCT/IL2005/000484
(87) International publication number: WO 2005/108607

(56) References cited:
- US-A1- 2003 181 379
- "SMART PCR CDNA SYNTHESIS KIT/SMART CDNA LIBRARY CONSTRUCTION KIT" CLONTECH, 2000, page 50, XP002250735
- WHITE K E ET AL: "Molecular cloning of a novel human UDP-GalNAc:polypeptide N-acetylgalactosaminyltransferase, GalNAc-T8, and analysis as a candidate autosomal dominant hypophosphatemic rickets (ADHR) gene" GENE, ELSEVIER BIOMEDICAL PRESS. AMSTERDAM, NL, vol. 246, no. 1-2, April 2000 (2000-04), pages 347-356, XP004195510 ISSN: 0378-1119
- TOPAZ ORIT ET AL: "Mutations in GALNT3, encoding a protein involved in O-linked glycosylation, cause familial tumoral calcinosis" NATURE GENETICS, vol. 36, no. 6, June 2004 (2004-06), pages 579-581, XP002340867 ISSN: 1061-4036
- FRISHBERG YAACOV ET AL: "Identification of a recurrent mutation inGALNT3 demonstrates that hyperostosis-hyperphosphatemia syndrome and familial tumoral calcinosis are allelic disorders (vol 83, pg 33, 2005)" JOURNAL OF MOLECULAR MEDICINE (BERLIN), vol. 83, no. 3, March 2005 (2005-03), page 240, XP002340866 ISSN: 0946-2716(print) 1432-1440(ele
- FUCHS S: "[Peri-articular calcinosis. Tumor simulating calcinosis and differential diagnosis]" DER UNFALLCHIRURG. SEP 2001, vol. 104, no. 9, September 2001 (2001-09), pages 886-888, XP002341251 ISSN: 0177-5537
- DOSAKA-AKITA H ET AL: "N-acetylgalactosaminyl transferase-3 is a potential new marker for non-small cell lung cancers." BRITISH JOURNAL OF CANCER. 23 SEP 2002, vol. 87, no. 7, 23 September 2002 (2002-09-23), pages 751-755, XP002341252 ISSN: 0007-0920
- NOMOTO M ET AL: "Structural basis for the regulation of UDP-N-acetyl-alpha-D-galactosa mine: polypeptide N-acetylgalactosaminyl transferase-3 gene expression in adenocarcinoma cells." CANCER RESEARCH. 15 DEC 1999, vol. 59, no. 24, 15 December 1999 (1999-12-15), pages 6214-6222, XP002341253 ISSN: 0008-5472
- KRISTIANSEN O P ET AL: "IDDM7 links to insulin-dependent diabetes mellitus in Danish multiplex families but linkage is not explained by novel polymorphisms in the candidate gene GALNT3. The Danish Study Group of Diabetes in Childhood and The Danish IDDM Epidemiology and Genetics Group." HUMAN MUTATION. MAR 2000, vol. 15, no. 3, March 2000 (2000-03), pages 295-296, XP002341254 ISSN: 1098-1004

## Description

The present invention, in some embodiments thereof concerns to the use of *GALNT3* polynucleotides and polypeptides for the manufacture of a medicament identified for the treatment of a disorder associated with hyperphosphatemia such as familial tumoral calcinosis (FTC); to methods of diagnosing familial tumoral calcinosis by identifying in *GALNT3* mRNA, *GALNT3* genomic sequence or GalNAc-T3 polypeptide nucleic acid or amino acid substitutions resulting in downregulation of an expression level and/or activity of CalNAc-T3; and to a method of identifying agents suitable for treating a disorder associated with hyperphosphatemia.

The present invention relates to methods of treating disorders associated with abnormal phosphate metabolism, and more particularly to the use of regulators of GalNAc-T3 in the treatment of disorders associated with hyperphosphatemia such as Familial tumoral calcinosis (FTC), hyperphosphatemic calcinosis, hemodialysis, and chronic renal failure, as well as disorders associated with hypophosphatemia, e.g., X-linked vitamin D resistant hypophosphatemic rickets (HYP), hereditary hypercalciuria with hypophosphatemic rickets (HHRH), oncogenic hypophosphatemic osteomalacia (OHO), and X-linked hypophosphatemic rickets (PHEX).

Phosphate-related abnormalities (*i.e*., hypophosphatemia or hyperphosphatemia) characterize a class of metabolic disorders manifesting with hyper - and dys - lipidemia, rickets and/or serious metastatic calcification, which can eventually lead to death. These disorders result from disrupted phosphate metabolism. Inorganic phosphate is absorbed in the intestinal tract in a process regulated by 1α, 25-dihydroxyvitamin D3 (vitamin D3). On the other hand, phosphate excretion, which is regulated by the parathyroid hormone, takes place in both kidney and intestinal tract (*i.e*., fecal excretion). Moreover, the liver, skin and kidney are involved in the conversion of vitamin D3 to its active metabolite, calcitriol, which plays an active role in maintaining phosphate balance and bone mineralization.

Under normal conditions, a decrease in plasma phosphate level stimulates the production of vitamin D3 in the renal proximal tubule, resulting in increases absorption of calcium and phosphate, The increase in calcium level leads to a secondary suppression of the parathyroid hormone (PTH), which results in upregulation of the sodium-dependent phosphate transport in the renal proximal tubule.

Hyperparathyroidism, a condition characterized by overproduction of PTH in the parathyroid glands, results in hypophosphatemia and increased phosphate excretion due to inhibition of sodium-dependent phosphate transport in the kidney.

Other conditions which involve hypophosphatemia include vitamin D deficiency, which causes rickets in children and osteomalacia in adults, X-linked vitamin D resistant hypophosphatemic rickets (HYP), hereditary hypercalciuria with hypophosphatemic rickets (HHRH), Dent's disease including certain types of renal Fanconi syndrome, renal I alpha-hydroxylase deficiency (VDDR 1), defects in 1,25-dihydroxy vitamin D3 receptor (end organ resistance, VDDR II), oncogenic hypophosphatemic osteomalacia (OHO), and X-linked hypophosphatemic rickets (PHEX) [Francis, Nat. Genet. (1995), 11: 130-136; Rowe, Hum. Genet. (1996), 97: 345-352; Rowe, Hum. Mol. Genet. (1997), 6: 539-549).

On the other hand, hyperphosphatemia, *i.e.,* increased plasma level of PO₄, is often a result of renal insufficiency. End-stage renal insufficiency, a condition affecting approximately 250,000 individuals in the USA, can lead to metastatic calcification, *i.e.,* the deposition of calcium phosphate in previously healthy connective tissues and solid organs. Thus, advanced renal failure, *i.e.,* a glomerular filtration rate of less than 20 mL/min, causes a decrease in PO₄ excretion and an increase of plasma PO₄. However, other conditions may also decrease PO₄ excretion. These include pseudohypoparathyroidism or hypoparathyroidism. Hyperphosphatemia can also result from excess administration of oral PO₄, or from overuse of enema containing phosphate salts. Furthermore, hyperphosphatemia may result from migration of intracellular PO₄ to the cell exterior. Such migration frequently occurs in diabetic ketoacidosis (regardless of systemic PO₄ loss), bruise, non-traumatic rhabdomyolysis, systemic infection and tumor lysis syndrome. Moreover, hyperphosphatemia plays a critical role in the onset of secondary hyperparathyroidism, and the onset of renal osteodystrophy in patients under dialysis treatment for a long period.

Familial tumoral calcinosis (FTC; MIM211900) is a severe autosomal recessive metabolic disorder manifesting with hyperphosphatemia and massive calcium deposits in the skin and subcutaneous tissues, especially of the hips and knees. Hyperphosphatemia, secondary to increased renal phosphate retention, is the major metabolic abnormality associated with familial tumoral calcinosis (FTC) and is accompanied by inappropriately normal or elevated levels of PTH and 1,25-dihydroxyvitamin D3, two essential regulators of phosphate metabolism [Steinherz, 1985 (Supra)]. While hyperphosphatemia appears in FTC patients as early as 21 months of age, the calcium deposits are noted later in childhood. Thus, all FTC patients have elevated serum phosphorus levels, and in some patients, elevated levels of 1,25-vitamin D are also detected. FTC represents the metabolic mirror image of hypophosphatemic rickets caused by mutations in PHEX (MIM307800) and in FGF23 (MIM193100) genes [Schiavi, S.C. and Kumar, R. Kidney Int. 65, 1-14 (2004); Quarles, L.D. Am. J. Physiol. Endocrinol. Metab. 285, 1-9 (2003)] and which is characterized by decreased phosphate levels, decreased renal tubular phosphate reabsorption and inappropriately normal or decreased levels of 1,25-dihydroxyvitamin D3 [Prince M.J. et al. Ann Intern Med. 96, 586-591 (1982)].

Current treatment regimens of hypophosphatemia related disorders [e.g., hypophosphatemic rickets (PHEX)] include active vitamin D analogues (e.g., calcitriol) and oral phosphate supplementation. However, both of these nutrition supplements often fail to normalize serum phosphate level and in many cases, the patients fail to reach normal adult height. The recent use of recombinant human growth hormone (rhGH) was reported to benefit children with PHEX, however, is often associated with disproportional growth of the trunk (Reviewed in Reusz G, 2001, Orv Hetil. 142: 2659-65; Wilson DM 2000, J. Pediatr. Endocrinol. Metab. Suppl 2: 993-8).

Treatment of hyperphosphatemia involves the use of aluminum- or calcium-based phosphate-binding agents, which effectively lower serum phosphorus levels. However, while the use of aluminum-based agents can be associated with bone toxicity, renal osteodystrophy and encephalopathy, the use of calcium-based agents is often associated with hypercalcaemia and cardiovascular calcification. To overcome these limitations, non-calcium-, non-aluminium-based alternative agents were developed. These include the sevelamer hydrochloride and lanthanum carbonate (Hutchison AJ, 2004, Nephrol Dial Transplant. 19 Suppl 1:i19-24; Chertow GM., 2003, J Am Soc Nephrol. 14: S310-4). However, while lanthanum was found to be effective and well-tolerated (Joy MS et al., 2003, Am. J. Kidney Dis. 42: 96-107), sevelamer was found to be less effective than aluminum (Cizman B. 2003, Nephrol. Dial. Transplant. 18 Suppl 5:v47-9) and the use of both of these agents is limited by their high cost. Moreover, both of these agents treat only the symptoms by suppressing phosphate re-absorption in the intestinal track and not the causes which lead to hyperphosphatemia.

Thus, there is a need to develop pharmaceutical compositions and methods of treating disorders associated with phosphate metabolism devoid of the above limitations.

Phosphatonin is a novel circulating phosphaturic factor, postulated to be primarily responsible for modulating urinary phosphate excretion in a variety of hypophosphatemic disorders.

Quarles, 2003 (J Clin Invest 112: 642-646), suggested that phosphatonin is a circulating protein that inhibits sodium-dependent phosphate reabsorption in the renal proximal tubule via mechanisms which are distinct from PTH, and vitamin D3. The fibroblast growth factor 23 (FGF23), secreted frizzled-related protein 4 (SFRP4) and matrix extracellular phosphoglycoprotein (MEPE) [Schiavi, S.C. and Kumar, R. Kidney Int 65, 1-1.4 (2004); Quarles, L.D. Am. J. Physiol. Endocrinol. Metab. 285, 1-9 (2003)] were suggested as the putative phosphatonin proteins since they modulate circulating phosphate levels [Shiroada,T. et al.. Proc Natl Acad Sci. 98, 6500-6505 (2001); Bowe A.E. et al. Bioch Biophys Res Comm. 284, 977-981 (2001); Rowe P-S.N. et al. Bone:34, 303-319 (2003); Berndt, T. et al. J. Clin. Invest. 112, 785-794 (2003)].

Clontec 2000, page 50, XP002250735 relates to smart PCR CDNA synthesis kit/smart CDNA library construction kit.

Fuchs S. discloses peri-articular calcinosis. Tumor simulating calcinosis and differential diagnosis, Der Unfallchirurg, Sept. 2001, col. 104, no. 9, September 2001 (2001-09), pages 886-888, XP002341251 ISSN 0177-5537.

US 20031181379 describes novel nucleic acids encoding a fibroblast growth factor-23(FGF23) and proteins encoded thereby, mutations in which are associated with autosomal dominant rickets (ADHR). The invention further relates to methods of diagnosing and treating hypophosphatemic and hyperphosphatemic, disorders comprising inhibiting or stimulating, respectively, the biological activity of FGF23 in a patient. The invention also relates to methods of treating osteoporosis, dermatomyositis, and coronary artery disease comprising stimulating the biological activity of FGF23 in a patient.

Dosaka-Akita H. et al describes n-acetylgalactosaminyl transferase-3 as a potential new marker for non-small cell lung cancers, British Journal of Cancer, 23 Sep 2002, vol. 87; 751-755, XP002341252 ISSN: 0007-0920.

Kristiansen O.P. et al describes IDDM7 linkage to insulin-dependent diabetes mellitus in Danish multiplex families but not to the novel polymorphisms in the candidate gene GALNT3. The Danish group of diabetes in childhood and the Danish IDDM epidemiology and genetics group, human Mutation, march 2000, vol. 15, no. 3, pages 295-296.

Thus, FGF23 and other phosphatonin genes have been considered as prime candidates for the FTC gene [Jan De Beur, S.M., and Levine, M.A. (2002), J. Clin. Endocrinol. Metab. 87: 2467-2473],

While reducing the present invention to practice, the present inventors have uncovered that mutations in *GALNT3* gene encoding UDP-N-acetyl-alpha-D-galactosamine:polypeptide N-acctylgalactosaminyltransferase (GalNAc-T3) cause FTC. Thus, inducers of GalNAc-T3 can be used to treat hyperphosphatemia related disorders such as FTC, and on the other hand, inhibitors of GalNAc-T3 can be used to treat disorders associated with hypophosphatemia, such as hypophosphatemic rickets.

The use of an exogenous polynucleotide of the present invention is defined in claim 1 and claim 2, the method of diagnosing familial tumoral calcinosis is defined in claim 4 and claim 5, the method of identifying an agent is defined in claim 9.

According to another aspect of the present invention there is provided a use of an agent capable of regulating an expression level and/or activity of GalNAc-T3 for the treatment of a disorder associated with abnormal phosphate metabolism.

According to yet another aspect of the present invention there is provided a use of an agent capable of regulating an expression level and/or activity of GalNAc-T3 for the manufacture of a medicament identified for the treatment of a disorder associated with abnormal phosphate metabolism.

According to still another aspect of the present invention there is provided method of diagnosing familial tumoral calcinosis in an individual, the method comprising identifying in a polynucleotide sequence of the individual at least one nucleic acid substitution resulting in downregulation of an expression level and/or activity of GalNAc-T3, thereby diagnosing familial tumoral calcinosis in the individual.

According to an additional aspect of the present invention there is provided a kit for diagnosing familial tumorale calcinosis in an individual, the kit comprising a packaging material packaging at least one reagent, the at least one reagent for identifying in a polynucleotide sequence of the individual at least one nucleic acid substitution resulting in downregulation of an expression level and/or activity of GalNAc-T3, thereby diagnosing familial tumoral calcinosis in the individual.

According to yet an additional aspect of the present invention there is provided a method of diagnosing familial tumoral calcinosis in an individual, the method comprising identifying in a polypeptide sequence of the individual at least one amino acid substitution capable of downregulating expression level and/or activity of GalNAc-T3, thereby diagnosing familial tumoral calcinosis in the individual.

According to yet another aspect of the present invention there is provided a method of identifying an agent suitable for treating a disorder associated with abnormal phosphate metabolism, comprising exposing GalNAc-T3 or cells expressing GalNAc-T3 to a plurality of molecules and selecting from the plurality of molecules at least one molecule capable of regulating the expression level and/or the activity of the GalNAc-T3, the at least one molecule being the agent suitable for treating the disorder associated with abnormal phosphate metabolism.

According to still an additional aspect of the present invention there is provided a kit for diagnosing familial tumoral calcinosis in an individual, the kit comprising a packaging material packaging at least one reagent, the at least one reagent for identifying in a polypeptide sequence of the individual at least one amino acid substitution capable of downregulating expression level and/or activity of GalNAc-T3, thereby diagnosing familial tumoral calcinosis in the individual.

According to a further aspect of the present invention there is provided a method of identifying an agent suitable for treating a disorder associated with abnormal phosphate metabolism, comprising exposing GalNAc-T3 or cells expressing GalNAc-T3 to a plurality of molecules and selecting from the plurality of molecules at least one molecule capable of regulating the expression level and/or the activity of the GalNAc-T3, the at least one molecule being the agent suitable for treating the disorder associated with abnormal phosphate metabolism.

According to yet a further aspect of the present invention there is provided a kit for identifying an agent suitable for treating a disorder associated with abnormal phosphate metabolism, the kit comprising a packaging material packaging at least one reagent, the at least one reagent include GalNAc-T3 and/or cells expressing GalNAc-T3 and a plurality of molecules, wherein at least one molecule of the plurality of molecules is capable of regulating the expression level and/or activity of the GalNAC-T3, thereby identifying the agent suitable for treating a disorder associated with abnormal phosphate metabolism.

According to further features in preferred embodiments of the invention described below, the disorder is associated with hyperphosphatemia.

According to still further features in the described preferred embodiments the disorder associated with the hyperphosphatemia is selected from the group consisting of Familial tumoral calcinosis (FTC), hyperphosphatemic calcinosis, hemodialysis, and chronic renal failure.

According to still further features in the described preferred embodiments regulating is upregulating the expression level and/or activity of the GalNAc-T3.

According to still further features in the described preferred embodiments, upregulating the expression level and/or activity of the GalNAc-T3 is effected by an agent selected from the group consisting of:
(a) an exogenous polynucleotide encoding at least a functional portion of GALNT3;
(b) an agent capable of increasing expression of endogenous GalNAc-T3 in an individual;
(c) an agent capable of increasing endogenous GalNAc-T3 activity in an individual;
(d) an exogenous polypeptide including at least a functional portion of GalNAc-T3;
(e) a GalNAc-T3 substrate; and
(f) a GalNAc-T3-expressing cell.

According to still further features in the described preferred embodiments the exogenous polynucleotide encoding at least a functional portion of GALNT3 is set forth in SEQ ID NO:29.

According to still further features in the described preferred embodiments the GalNAc-T3 is set forth in SEQ ID NO:28.

According to still further features in the described preferred embodiments the disorder is associated with hypophosphatemia.

According to still further features in the described preferred embodiments the disorder associated with the hypophosphatemia is selected from the group consisting of X-linked vitamin D resistant hypophosphatemic rickets (HYP), hereditary hypercalciuria with hypophosphatemic rickets (HHRH), oncogenic hypophosphatemic osteomalacia (OHO), and X-linked hypophosphatemic rickets (PHEX).

According to still further features in the described preferred embodiments regulating is downregulating the expression level and/or the activity of the GalNAc-T3.

According to still further features in the described preferred embodiments downregulating the expression level and/or the activity of the GalNAc-T3 is effected by an agent selected from the group consisting of:
(a) a molecule which binds the GalNAc-T3;
(b) an enzyme which cleaves the GalNAc-T3;
(c) an antisense polynucleotide capable of specifically hybridizing with at least part of an mRNA transcript encoding GALNT3;
(d) a ribozyme which specifically cleaves at least part of an mRNA transcript encoding GALNT3;
(e) a small interfering RNA (siRNA) molecule which specifically cleaves at least part of a transcript encoding GALNT3;
(f) a non-functional analogue of at least a catalytic or binding portion of the GalNAc-T3;
(g) a molecule which prevents GalNAc-T3 activation or substrate binding.

According to still further features in the described preferred embodiments the mRNA transcript encoding GALNT3 is set forth in SEQ ID NO:29.

According to still further features in the described preferred embodiments the agent is formulated for systemic administration.

According to still further features in the described preferred embodiments the polynucleotide sequence is an mRNA sequence encoding GALNT3 or a genomic sequence region including the GALNT3 gene.

According to still further features in the described preferred embodiments the polynucleotide sequence is set forth by SEQ ID NO:29 or 33.

According to still further features in the described preferred embodiments identifying at least one nucleic acid substitution in the GALNT3 gene is effected using a method selected from the group consisting of DNA sequencing, restriction fragment length polymorphism (RFLP analysis), allele specific oligonucleotide (ASO) analysis, Denaturing/Temperature Gradient Gel Electrophoresis (DGGE/TGGE), Single-Strand Conformation Polymorphism (SSCP) analysis, Dideoxy fingerprinting (ddF), pyrosequencing analysis, acycloprime analysis, Reverse dot blot, GeneChip microarrays, Dynamic allele-specific hybridization (DASH), Peptide nucleic acid (PNA) and locked nucleic acids (LNA) probes, TaqMan, Molecular Beacons, Intercalating dye, FRET primers, AlphaScreen, SNPstream, genetic bit analysis (GBA), Multiplex minisequencing, SNaPshot, MassEXTEND, MassArray, GOOD assay, Microarray miniseq, arrayed primer extension (APEX), Microarray primer extension, Tag arrays, Coded microspheres; Template-directed incorporation (TDI), fluorescence polarization, Colorimetric oligonucleotide ligation assay (OLA), Sequence-coded OLA, Microarray ligation, Ligase chain reaction, Padlock probes, Rolling circle amplification, and Invader assay.

According to still further features in the described preferred embodiments identifying the at least one nucleic acid substitution in the mRNA sequence encoding the GALNT3 is effected using DNA sequencing of a GLANT3 RT-PCR product.

According to still further features in the described preferred embodiments the polypeptide sequence is set forth in SEQ ID NO:28.

According to still further features in the described preferred embodiments identifying the at least one amino acid substitution is effected using an antibody capable of differentially binding to at least one polymorph of the GaLNAc-T3 the at least one polymorph includes the amino acid substitution capable of downregulating the expression level and/or the activity of GaINAc-T3.

According to still further features in the described preferred embodiments the GalNAc-T3 includes at least a catalytic or binding portion of the GaINAc-T3.

According to still further features in the described preferred embodiments the GalNAe-T3 is set forth in SEQ ID NO:28.

According to still further features in the described preferred embodiments the cells expressing GaINAc-T3 are selected from the group consisting of kidney cells, fibroblasts, epithelial cells, lymphocytes, bone marrow cells, lung cells, liver cells and brain cells.

According to still further features in the described preferred embodiments the expression level is detected using an immunological detection method and/or an RNA detection method.

According to still further features in the described preferred embodiments the immunological detection method is selected from the group consisting of a radio-immunoassay (RIA), an enzyme linked immunosorbent assay (ELISA), a western blot, an immunohistochemical analysis, and a fluorescence activated cell sorting (FACS).

According to still further features in the described preferred embodiments the RNA detection method is selected from the group consisting of Northern Blot, RT-PCR, RNA in situ hybridization, and in situ RT-PCR.

According to still further features in the described preferred embodiments the activity is determined using an activity assay selected from the group consisting of in situ activity assay and in vitro activity assays.

According to still further features in the described preferred embodiments the activity assay is effected using a substrate selected from the group consisting of HIVH1Bgp120, Fibronectin, Prion-a, CD59, Mucla, Muc2, EA2, Muc7.

The present invention successfully addresses the shortcomings of the presently known configurations by providing methods of treating disorders associate with abnormal phosphate metabolism.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

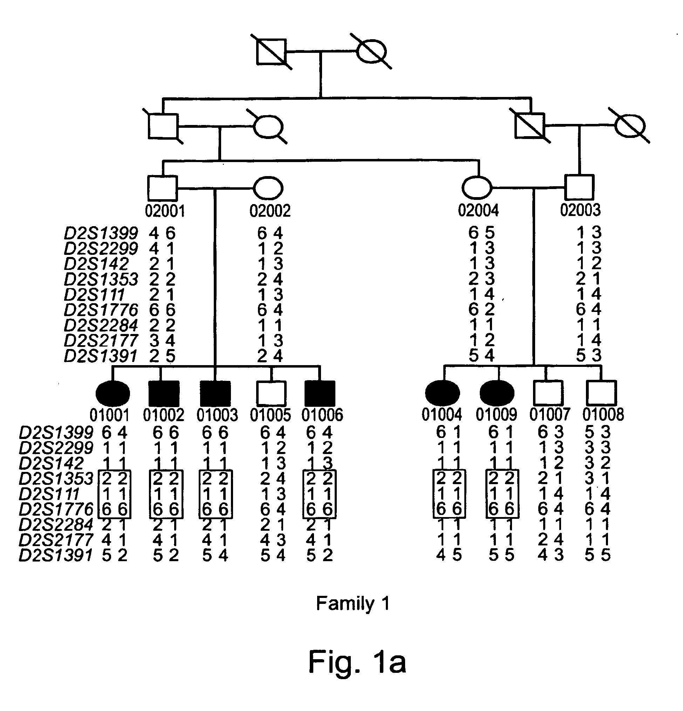

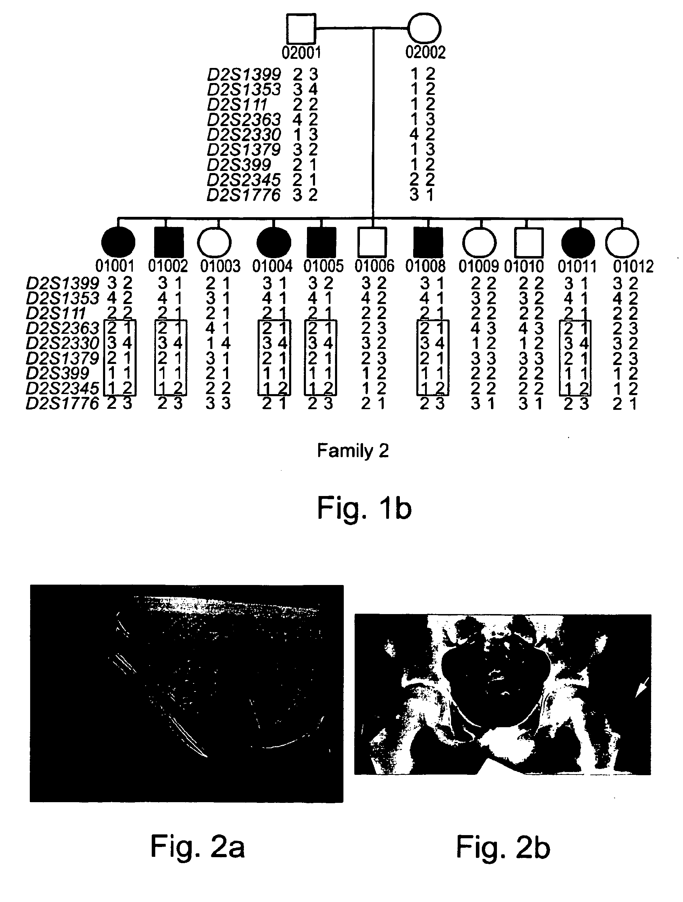

FIGs. 1a-b are diagrams illustrating mapping of the familial tumoral calcinosis (FTC) critical region. Shown are haplotype analyses of a consanguinity FTC family (Family 1, Figure 1a) and a non-consanguinity FTC family (Family 2, Figure 1b) using polymorphic microsatellite markers on chromosome 2q24-q31.1. The shared disease-associated haplotypes of all participating individuals are indicated by boxes. The noted DNA markers correspond to the following GenBank Accession numbers: G08198 (D2S1399), G07884 (D2S1353); Z16431 (D2S111), Z54008 (D2S2330), G08153 (D2S1379), Z24635 (D2S399), Z50933 (D2S2345), G08180 (D2S1776), Z53666 (D2S2299), Z16821 (D2S142), Z53511 (D2S2284), Z52323 (D2S2177), and G08168 (D2S1391).
FIGs. 2a-b are photographs illustrating the clinical features of FTC. Figure 2a - a large subcutaneous tumor over the left outer thigh of patient No. 01001 of family 1. Figure 2b - a periarticular calcified mass over the left acetabulum of patient No. 01006 of family 1.

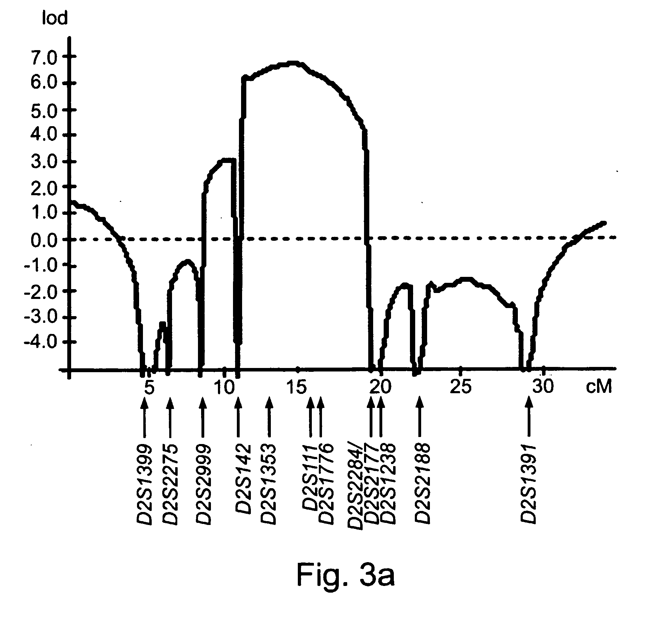

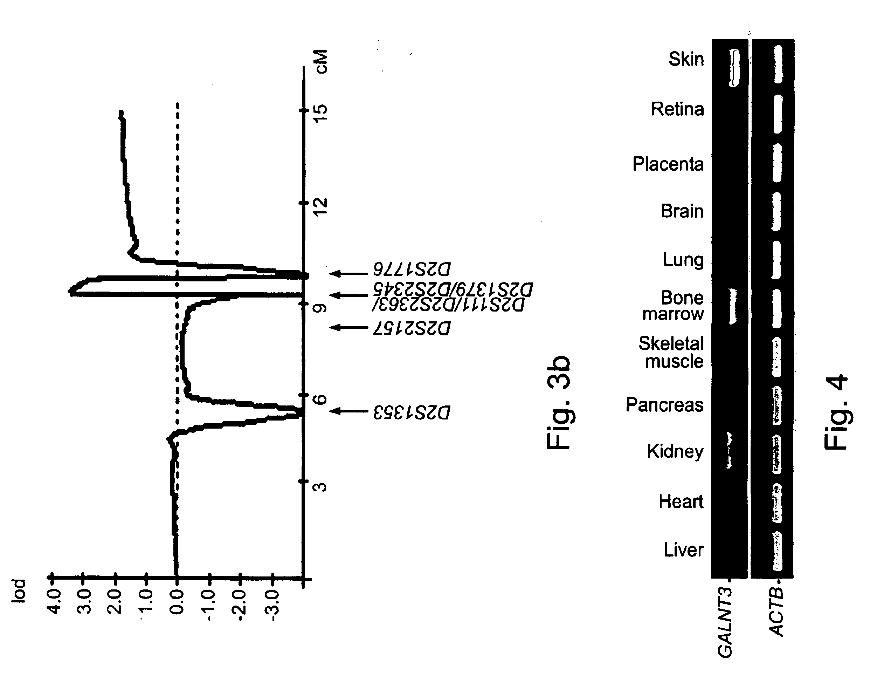

FIGs. 3a-b are multipoint LOD score analyses of a consanguinity FTC family (Family 1, Figure 3a) and a non-consanguinity FTC family (Family 2, Figure 3b). Figure 3a - homozygosity mapping for 12 informative markers spanning 18.1 cM. Note the maximum multipoint LOD score of 6.7 at D2S111 (HOMOZ). Figure 3b - multipoint linkage map using 7 microsatellite markers. Note the peak LOD of 3.4 for markers D2S2363 and D2S1379 (GeneHunter).
FIG. 4 illustrates RT-PCR determination of *GALNT3* gene expression. RNA samples were extracted from various human tissues and the RT-PCR reaction was performed using the GALex6F (SEQ ID NO:23 and GALex9R (SEQ ID NO:25) primers followed by nested PCR using the GALex6F and GALex8R (SEQ ID NO:24) primers which are specific for *GALNT3* transcript. Note the high-intensity bands obtained in RNA samples obtained from the skin, bone marrow and kidney. The RT-PCR product of the β-actin transcript (obtained using the Actin 5' and Actin 3' primers, SEQ ID NOs:26 and 27) was used as a measure of the level of RNA in each sample.

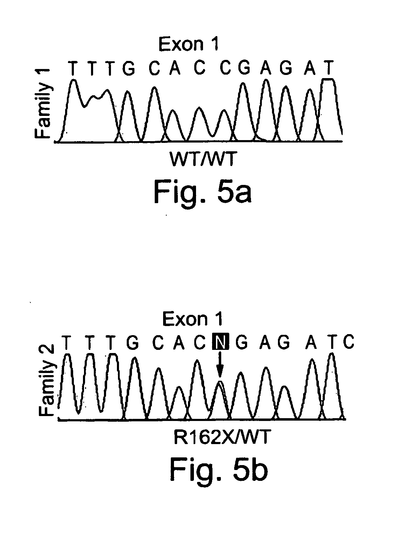

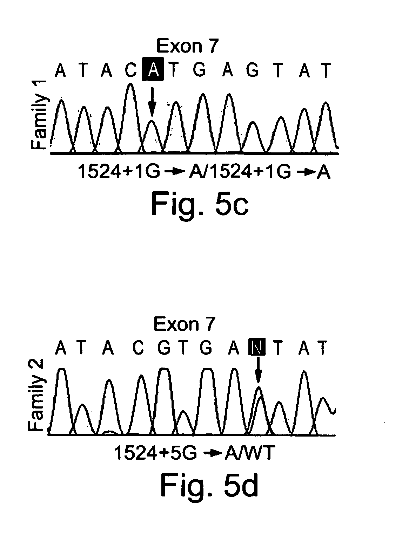

FIGs. 5a-d illustrate *GALNT3* mutation analysis in FTC families. Shown are DNA sequencing chromatograms depicting the genomic sequence of exon 1 (Figures 5a-b) and exon 7 (Figure 5c-d) of the *GALNT3* gene in two FTC families. Figure 5a - the wildtype sequence of exon 1 in family 1; Figure 5b - the presence of a heterozygous C→T transition at position 484 of the *GALNT3* mRNA sequence (GenBank Accession No. NM_004482, SEQ ID NO:29) creating a TGA termination codon (R162X) in exon 1 of affected individuals of family 2; Figure 5c - the presence of a homozygous G→A transition at position 1524+1 (*GALNT3* genomic contig GenBank Accession No. NT_005403) in affected individuals of family 1, Figure 5d - the presence of a heterozygous G→A transition at position 1524+5 (GenBank Accession No. NT_005403) in all affected individuals of family 2.

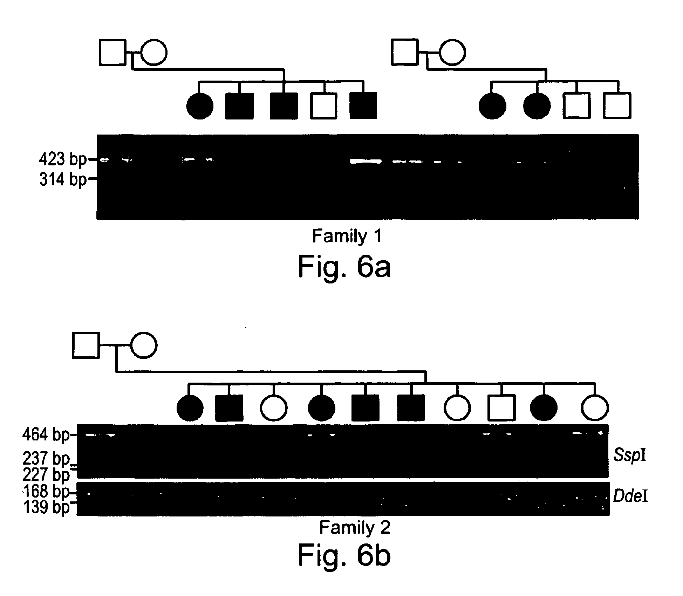

FIGs. 6a-b are RFLP analyses depicting the segregation of the pathogenic mutations in families 1 and 2. Figure 6a - segregation of the 1524+1 G→A mutation in family 1. The 1524+1G→A nucleic acid change abolishes a recognition site for BsaAI; consequently, digestion of a PCR amplicon encompassing exon 7 generates an homozygous (uncut) 423 bp product in affected individuals, while heterozygous carriers of the mutation display an additional fragment of 314 bp. Figure 6b - segregation of the 1524+5G→A and R162X mutations in family 2. The 1524+5G→A and R162X nucleic acid changes create novel recognition sites for endonucleases *Ssp*I (upper gel) and *Dde*I (lower gel) respectively; hence, affected individuals in family 2 display an additional fragment upon digestion of the relevant PCR amplicons (exons 7 and 1, respectively) with the corresponding restriction enzymes.

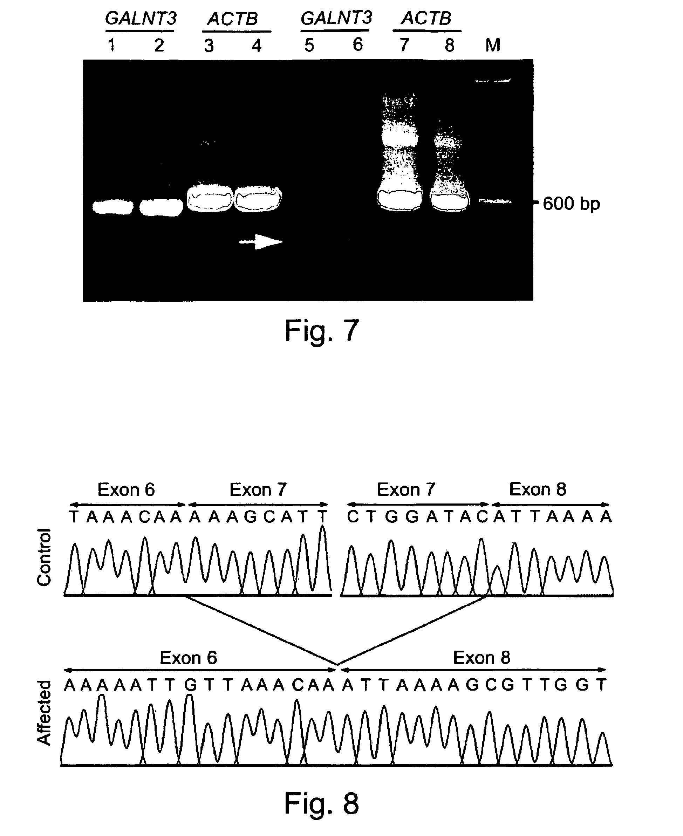

FIG. 7 is an RT-PCR analysis depicting the expression of *GALNT3.* RNA samples extracted from skin (lanes 1, 3, 5, 7) or blood lymphocytes (lanes 2, 4, 6, 8) of a healthy individual (lanes 1-4) and an FTC affected individual (patient No. 01001 of family 1, lanes 5-8) were subjected to RT-PCR analysis using the *GALNT3* (lanes 1-2, 5-6) or β-actin (ACTB, lanes 3-4, 7-8) PCR primers as described in Figure 4 hereinabove. Note the aberrant low-intensity, low-molecular weight *GALNT3* RT-PCR products obtained using RNA from skin (lane 5, arrow) and blood lymphocytes (lane 6) of the FTC affected individual as compared with the high-intensity *GALNT3* RT-PCR products obtained using RNA from skin (lane 1) and blood lymphocytes (lane 2) of the healthy individual. Also note the similar intensity bands of β-actin RT-PCR products in both affected and healthy individuals (compare lanes 3-4 to lanes 7-8).
FIG. 8 is a DNA sequencing chromatogram depicting the absence of exon 7 in a blood RNA sample of an FTC affected individual. Sequence analysis was performed on the aberrant low-molecular weight RT-PCR product observed in Figure 7 lane 6 using the GALex6F (SEQ ID NO:23) and GALex8R (SEQID NO:24) nested PCR primers. Note the absence of exon 7 sequence in the aberrant low-molecular weight splice product from the blood sample of the affected individual.

### DESCRIPTION OF THE PREFERRED EMBODIMENTS

The present invention is of upregulators and downregulators of GalNAc-T3 which can be used in treating disorders associated with abnormal phosphate levels. Specifically, the present invention can be used to treat familial tumoral calcinosis and other disorders associated with hyperphosphatemia, as well as disorders associated with hypophosphatemia.

The principles and operation of the methods of treating disorders associated with abnormal phosphate metabolism according to the present invention may be better understood with reference to the drawings and accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

Familial tumoral calcinosis (FTC; MIM211900) is a severe autosomal recessive metabolic disorder manifesting with hyperphosphatemia and massive calcium deposits in the skin and subcutaneous tissues. Current methods of treating patients affected with this disease and other hyperphosphatemia - related disorders (e.g., end-stage renal failure, pseudohypoparathyroidism, hypoparathyroidism) include the use of aluminum- or calcium-based phosphate-binding agents. However, these phosphate-binders can cause bone toxicity, renal osteodystrophy, encephalopathy, hypercalcaemia and cardiovascular calcification. To overcome these limitations, aluminum and calcium free agents such as sevelamer hydrochloride and lanthanum carbonate were suggested for treatment of hyperphosphatemia (FOSRENOL®, AnorMED Inc.; Hutchison AJ, 2004, Nephrol Dial Transplant. 19 Supp1 1:i19-24; Chertow GM., 2003, J Am Soc Nephrol. 14: S310-4). These agents suppress phosphate re-absorption in the intestinal track. Although safer than aluminum, sevelamer was found to be less effective than the aluminum-based phosphate binder (Cizman B. 2003, Nephrol. Dial. Transplant. 18 Suppl 5:v47-9). On the other hand, lanthanum was found to be effective and well-tolerated (Joy MS et al., 2003, Am. J. Kidney Dis. 42: 96-107). However, both of these expensive agents are used for treating the symptoms and not the cause of hyperphosphatemia.

While reducing the present invention to practice, the present inventors have uncovered deleterious mutations in the *GALNT3* gene encoding GalNAc-T3 in individuals affected with familial tumoral calcinosis (FTC). Moreover, the present inventors have uncovered that inducers of GalNAc-T3 can be used in treating FTC and other hyperphosphatemia - related disorders, and that inhibitors of GalNAc-T3 can be used to treat hypophosphatemia - related disorders.

Thus, according to one aspect of the present invention there is provided a method of treating a disorder associated with abnormal phosphate metabolism in an individual.

As used herein, the term "individual" refers to a human being suffering from a disease associated with abnormal phosphate level, *i.e.,* hyperphosphatemia or hypophosphatemia due to a genetic disease (e.g., an individual having familial tumoral calcinosis), hormonal imbalance (e.g., hyperparathyroidism), renal disease (as a result of e.g., hemodialysis) and the like.

The phrase "treating" refers to inhibiting or arresting the development of a disease, disorder or condition and/or causing the reduction, remission, or regression of a disease, disorder or condition in an individual suffering from, or diagnosed with, the disease, disorder or condition. Those of skill in the art will be aware of various methodologies and assays which can be used to assess the development of a disease, disorder or condition, and similarly, various methodologies and assays which can be used to assess the reduction, remission or regression of a disease, disorder or condition.

As used herein "abnormal phosphate metabolism" refers to abnormal levels of serum phosphate, which may reflect abnormal absorption of phosphate in the intestinal tract or abnormal phosphate excretion in both the kidney and intestinal tract (*i.e.,* via fecal excretion). Thus, any deviation from the normal range of phosphate in the plasma (*i.e.,* 2.5-4.5 mg/dl), can be referred to as being abnormal.

The phrase "disorder associated with abnormal phosphate metabolism" refers to hyper - or hypophosphatemia - related disorders, *i.e.,* the presence of excess of phosphate in the serum (*i.e.,* above 4.5 mg/dl) or insufficient levels of phosphate (*i.e.,* under 2.5 mg/dl), respectively.

The method according to this aspect of the present invention is effected by providing to an individual in need thereof an agent capable of regulating an expression level and/or activity of GalNAc-T3.

The term "GalNAc-T3" as used herein refers to the UDP-N-acetyl-alpha-D-galactosamine:polypeptide N-acetylgalactosaminyltransferase 3, which a member of the GalNAc-transferases family. Protein members of this family (e.g., GalNAc-T2, - T4, -T6, -T8) transfer N-acetyl galactosamine to the hydroxyl group of a serine or threonine residue in the first step of O-linked oligosaccharide biosynthesis. Individual GalNAc-transferase proteins have distinct activities and initiation of O-glycosylation is regulated by a repertoire of GalNAc-transferases. It will be appreciated that although these proteins share high sequence homology, each of the GalNAc-transferase proteins exhibits different substrate specificities. For example, the GalNAc-T3 exhibits substrate specificity towards HIV_{HIB}gp120, Fibronectin, Prion-a, CD59, Muc1a, Muc2, EA2, and Muc7.

The term "regulating" as used herein refers to upregulating (*i.e.,* increasing) or downregulating (*i.e.,* inhibiting or decreasing) of the expression and/or activity of GalNAc-T3.

According to preferred embodiments of the present invention the disorder associated with abnormal phosphate metabolism is hyperphosphatemia and thus the method of the present invention is effected by upregulating expression or activity of GalNAc-T3. Non-limiting examples of hyperphosphatemia - related disorders which can be treated according to this aspect of the present invention include familial tumoral calcinosis (FTC), hyperphosphatemic calcinosis, hemodialysis, chronic renal failure and end-stage renal failure.

Upregulation of GalNAc-T3 can be effected at the genomic level (*ie.,* activation of transcription via promoters, enhancers, regulatory elements), at the transcript level (*i.e.,* correct splicing, polyadenylation, activation of translation) or at the protein level (*i.e.,* post-translational modifications, interaction with substrates and the like).

Following is a list of agents capable of upregulating the expression level and/or activity of GalNAc-T3.

An agent capable of upregulating expression level of a GalNAc-T3 may be an exogenous polynucleotide sequence designed and constructed to express at least a functional portion of the GalNAc-T3 protein. Accordingly, the exogenous polynucleotide sequence may be a DNA or RNA sequence encoding a GalNAc-T3 molecule, capable of modulating phosphate metabolism.

The phrase "functional portion" as used herein refers to part of the GaINAc-T3 protein (*i.e.,* a polypeptide) which exhibits functional properties of the enzyme such as binding to a substrate. According to preferred embodiments of the present invention the functional portion of GalNAc-T3 is a polypeptide sequence including amino acids 188-374 (region of glycosyl transferase) and/or 507-629 (region of ricin-type beta trefoil) as set forth in SEQ ID NO:28. Preferably, the functional portion of GalNAc-T3 is a polypeptide sequence including amino acids 13-633, more preferably, amino acids 1-633 as set forth in SEQ ID NO:28.

GalNAc-T3 has been cloned from human and mouse sources. Thus, coding sequences information for GalNAc-T3 is available from several databases including the GenBank database available through http://www.ncbi.nlm.nih.gov/.

To express exogenous GalNAc-T3 in mammalian cells, a polynucleotide sequence encoding a GalNAc-T3 (GenBank Accession number NM_004482, SEQ ID NO:29) is preferably ligated into a nucleic acid construct suitable for mammalian cell expression. Such a nucleic acid construct includes a promoter sequence for directing transcription of the polynucleotide sequence in the cell in a constitutive or inducible manner.

It will be appreciated that the nucleic acid construct of the present invention can also utilize GalNAc-T3 homologues which exhibit the desired activity (i.e., transferring N-acetyl galactosamine to the hydroxyl group of a serine or threonine residue in the first step of O-linked oligosaccharide biosynthesis). Such homologues can be, for example, at least 80 %, at least 81 %, at least 82 %, at least 83 %, at least 84 %, at least 85 %, at least 86 %, at least 87 %, at least 88 %, at least 89 %, at least 90 %, at least 91 %, at least 92 %, at least 93 %, at least 94 %, at least 95 %, at least 96 %, at least 97 %, at least 98 %, at least 99 % or 100 % identical to SEQ ID NO:29, as determined using the BestFit software of the Wisconsin sequence analysis package, utilizing the Smith and Waterman algorithm, where gap weight equals 50, length weight equals 3, average match equals 10 and average mismatch equals -9.

Constitutive promoters suitable for use with the present invention are promoter sequences which are active under most environmental conditions and most types of cells such as the cytomegalovirus (CMV) and Rous sarcoma virus (RSV). Inducible promoters suitable for use with the present invention include for example the inducible promoter of the alkaline phosphates gene (Dollard MA and Billard P J. 2003. Whole-cell bacterial sensors for the monitoring of phosphate bioavailability. Microbiol. Methods, 55: 221-9) and the tetracycline-inducible promoter (Zabala M, et al., Cancer Res. 2004, 64(8): 2799-804).

The nucleic acid construct (also referred to herein as an "expression vector") of the present invention includes additional sequences which render this vector suitable for replication and integration in prokaryotes, eukaryotes, or preferably both (e.g., shuttle vectors). In addition, a typical cloning vectors may also contain a transcription and translation initiation sequence, transcription and translation terminator and a polyadenylation signal.

Eukaryotic promoters typically contain two types of recognition sequences, the TATA box and upstream promoter elements. The TATA box, located 25-30 base pairs upstream of the transcription initiation site, is thought to be involved in directing RNA polymerase to begin RNA synthesis. The other upstream promoter elements determine the rate at which transcription is initiated.

Enhancer elements can stimulate transcription up to 1,000 fold from linked homologous or heterologous promoters. Enhancers are active when placed downstream or upstream from the transcription initiation site. Many enhancer elements derived from viruses have a broad host range and are active in a variety of tissues. For example, the SV40 early gene enhancer is suitable for many cell types. Other enhancer/promoter combinations that are suitable for the present invention include those derived from polyoma virus, human or murine cytomegalovirus (CMV), the long term repeat from various retroviruses such as murine leukemia virus, murine or Rous sarcoma virus and HIV. See, Enhancers and Eukaryotic Expression, Cold Spring Harbor Press, Cold Spring Harbor, N.Y. 1983, which is incorporated herein by reference.

In the construction of the expression vector, the promoter is preferably positioned approximately the same distance from the heterologous transcription start site as it is from the transcription start site in its natural setting. As is known in the art, however, some variation in this distance can be accommodated without loss of promoter function.

Polyadenylation sequences can also be added to the expression vector in order to increase the efficiency of GaINAC-T3 mRNA translation. Two distinct sequence elements are required for accurate and efficient polyadenylation: GU or U rich sequences located downstream from the polyadenylation site and a highly conserved sequence of six nucleotides, AAUAAA, located 11-30 nucleotides upstream. Termination and polyadenylation signals that are suitable for the present invention include those derived from SV40.

In addition to the elements already described, the expression vector of the present invention may typically contain other specialized elements intended to increase the level of expression of cloned nucleic acids or to facilitate the identification of cells that carry the recombinant DNA. For example, a number of animal viruses contain DNA sequences that promote the extra chromosomal replication of the viral genome in permissive cell types. Plasmids bearing these viral replicons are replicated episomally as long as the appropriate factors are provided by genes either carried on the plasmid or with the genome of the host cell.

The vector may or may not include a eukaryotic replicon. If a eukaryotic replicon is present, then the vector is amplifiable in eukaryotic cells using the appropriate selectable marker. If the vector does not comprise a eukaryotic replicon, no episomal amplification is possible. Instead, the recombinant DNA integrates into the genome of the engineered cell, where the promoter directs expression of the desired nucleic acid.

The expression vector of the present invention can further include additional polynucleotide sequences that allow, for example, the translation of several proteins from a single mRNA such as an internal ribosome entry site (IRES) and sequences for genomic integration of the promoter-chimeric polypeptide.

Examples for mammalian expression vectors include, but are not limited to, pcDNA3, pcDNA3.1(+/-), pGL3, pZeoSV2(+/-), pSecTag2, pDisplay, pEF/myc/cyto, pCMV/myc/cyto, per3.1, pSinRep5, DH26S, DHBB, pNMT1, pNMT41, pNMT81, which are available from Invitrogen, pCI which is available from Promega, pMbac, pPbac, pBK-RSV and pBK-CMV which are available from Strategene, pTRES which is available from Clontech, and their derivatives.

Expression vectors containing regulatory elements from eukaryotic viruses such as retroviruses can be also used. SV40 vectors include pSVT7 and pMT2. Vectors derived from bovine papilloma virus include pBV-1MTHA, and vectors derived from Epstein Bar virus include pHEBO, and p205. Other exemplary vectors include pMSG, pAV009/A⁺, pMTO10/A⁺, pMAMneo-5, baculovirus pDSVE, and any other vector allowing expression of proteins under the direction of the SV-40 early promoter, SV-40 later promoter, metallothionein promoter, murine mammary tumor virus promoter, Rous sarcoma virus promoter, polyhedrin promoter, or other promoters shown effective for expression in eukaryotic cells.

As described above, viruses are very specialized infectious agents that have evolved, in many cases, to elude host defense mechanisms. Typically, viruses infect and propagate in specific, cell types. The targeting specificity of viral vectors utilizes its natural specificity to specifically target predetermined cell types and thereby introduce a recombinant gene into the infected cell. Thus, the type of vector used by the present invention will depend on the cell type transformed. The ability to select suitable vectors according to the cell type transformed is well within the capabilities of the ordinary skilled artisan and as such no general description of selection consideration is provided herein. For example, bone marrow cells can be targeted using the human T cell leukemia virus type I (HTLV-I) and kidney cells may be targeted using the heterologous promoter present in the baculovirus Autographa california nucleopolyhedrovirus (AcMNPV) as described in Liang CY et al., 2004 (Arch Virol. 149: 51-60).

Recombinant viral vectors are useful for *in vivo* expression of GaINAC-T3 since they offer advantages such as lateral infection and targeting specificity. Lateral infection is inherent in the life cycle of, for example, retrovirus and is the process by which a single infected cell produces many progeny virions that bud off and infect neighboring cells. The result is that a large area becomes rapidly infected, most of which was not initially infected by the original viral particles. This is in contrast to vertical-type of infection in which the infectious agent spreads only through daughter progeny. Viral vectors can also be produced that are unable to spread laterally. This characteristic can be useful if the desired purpose is to introduce a specified gene into only a localized number of targeted cells.

Various methods can be used to introduce the expression vector of the present invention into stem cells. Such methods are generally described in Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Md. (1989), Chang et al., Somatic Gene Therapy, CRC Press, Ann Arbor, Mich. (1995), Vega et al., Gene Targeting, CRC Press, Ann Arbor Mich. (1995), Vectors: A Survey of Molecular Cloning Vectors and Their Uses, Butterworths, Boston Mass. (1988) and Gilboa et at. [Biotechniques 4 (6): 504-512, 1986] and include, for example, stable or transient transfection, lipofection, electroporation and infection with recombinant viral vectors. In addition, see U.S. Pat. Nos. 5,464,764 and 5,487,992 for positive-negative selection methods.

Introduction of nucleic acids by viral infection offers several advantages over other methods such as lipofection and electroporation, since higher transfection efficiency can be obtained due to the infectious nature of viruses.

It will be appreciated that upregulation of GalNAc-T3 can be also effected by administration of GalNAc-T3-expressing cells into the individual.

GalNAc-T3-expressing cells can be any suitable cells, such as kidney, bone marrow, keratinocyte and lymphocyte cells which are derived from the individuals and are transfected *ex vivo* with an expression vector containing the polynucleotide designed to express GalNAc-T3 as described hereinabove.

Administration of the GalNAc-T3-expressing cells of the present invention can be effected using any suitable route such as intravenous, intra peritoneal, intra kidney, intra gastrointestinal track, subcutaneous, transcutaneous, intramuscular, intracutaneous, intrathecal, epidural and rectal. According to presently preferred embodiments, the GalNAc-T3-expressing cells of the present invention are introduced to the individual using intravenous, intra kidney, intra gastrointestinal track and/or intra peritoneal administrations.

GalNAc-T3-expressing cells of the present invention can be derived from either autologous sources such as self bone marrow cells or from allogeneic sources such as bone marrow or other cells derived from non-autologous sources. Since non-autologous cells are likely to induce an immune reaction when administered to the body several approaches have been developed to reduce the likelihood of rejection of non-autologous cells. These include either suppressing the recipient immune system or encapsulating the non-autologous cells or tissues in immunoisolating, semipermeable membranes before transplantation.

Encapsulation techniques are generally classified as microencapsulation, involving small spherical vehicles and macroencapsulation, involving larger flat-sheet and hollow-fiber membranes (Uludag, H. et al. Technology of mammalian cell encapsulation. Adv Drug Deliv Rev. 2000; 42: 29-64).

Methods of preparing microcapsules are known in the arts and include for example those disclosed by Lu MZ, et al., Cell encapsulation with alginate and alpha-phenoxycinnamylidene-acetylated poly(allylamine). Biotechnol Bioeng. 2000, 70: 479-83, Chang TM and Prakash S. Procedures for microencapsulation of enzymes, cells and genetically engineered microorganisms. Mol Biotechnol. 2001, 17: 249-60, and Lu MZ, et al., A novel cell encapsulation method using photosensitive poly(allylamine alpha-cyanocinnamylideneacetate). J Microencapsul. 2000, 17: 245-51.

For example, microcapsules are prepared by complexing modified collagen with a ter-polymer shell of 2-hydroxyethyl methylacrylate (HEMA), methacrylic acid (MAA) and methyl methacrylate (MMA), resulting in a capsule thickness of 2-5 µm. Such microcapsules can be further encapsulated with additional 2-5 µm ter-polymer shells in order to impart a negatively charged smooth surface and to minimize plasma protein absorption (Chia, S.M. et al. Multi-layered microcapsules for cell encapsulation Biomaterials. 2002 23: 849-56).

Other microcapsules are based on alginate, a marine polysaccharide (Sambanis, A. Encapsulated islets in diabetes treatment. Diabetes Thechnol. Ther. 2003, 5: 665-8) or its derivatives. For example, microcapsules can be prepared by the polyelectrolyte complexation between the polyanions sodium alginate and sodium cellulose sulphate with the polycation poly(methylene-co-guanidine) hydrochloride in the presence of calcium chloride.

It will be appreciated that cell encapsulation is improved when smaller capsules are used. Thus, the quality control, mechanical stability, diffusion properties, and *in vitro* activities of encapsulated cells improved when the capsule size was reduced from 1 mm to 400 µm (Canaple L. et al., Improving cell encapsulation through size control. J Biomater Sci Polym Ed. 2002; 13: 783-96). Moreover, nanoporous biocapsules with well-controlled pore size as small as 7 nm, tailored surface chemistries and precise microarchitectures were found to successfully immunoisolate microenvironments for cells (Williams D. Small is beautiful: microparticle and nanoparticle technology in medical devices. Med Device Technol. 1999, 10: 6-9; Desai, T.A. Microfabrication technology for pancreatic cell encapsulation. Expert Opin Biol Ther. 2002, 2: 633-46).

An agent capable of upregulating a GalNAc-T3 may also be any compound which is capable of increasing the transcription and/or translation of an endogenous DNA or mRNA encoding the GalNAc-T3 and thus increasing endogenous GalNAc-T3 activity.

An agent capable of upregulating a GalNAc-T3 may also be an exogenous polypeptide including at least a functional portion (as described hereinabove) of the GalNAc-T3.

Upregulation of GalNAc-T3 can be also achieved by introducing at least one GalNAc-T3 substrate. Non-limiting examples of such agents include HIV_{HIB}gp120, Fibronectin, Prion-a, CD59, Muc1a, Muc2, EA2, Muc7.

It will be appreciated that since GalNAc-T3 participates in phosphate metabolism downregulation thereof can be utilized to treat disorders which involve in hypophosphatemia, *i.e.,* reduced levels of plasma phosphate.

Thus, according to another preferred embodiments of the present invention the disorder associated with abnormal phosphate metabolism is hypophosphatemia and the method of the present invention is effected by downregulating GalNAc-T3 expression or activity. Non-limiting examples of hypophosphatemia - related disorders which can be treated according to the method of the present invention include X-linked vitamin D resistant hypophosphatemic rickets (HYP), hereditary hypercalciuria with hypophosphatemic rickets (HHRH), oncogenic hypophosphatemic osteomalacia (OHO), X-linked hypophosphatemic rickets (PHEX) and hyperparathyroidism.

Downregulation of GalNAc-T3 can be effected on the genomic and/or the transcript level using a variety of molecules which interfere with transcription and/or translation (e.g., antisense, siRNA, Ribozyme, DNAzyme), or on the protein level using e.g., antagonists, enzymes that cleave the polypeptide and the like.

Following is a list of agents capable of downregulating expression level and/or activity of GalNAc-T3.

One example, of an agent capable of downregulating a GalNAc-T3 is an antibody or antibody fragment capable of specifically binding GalNAc-T3. Preferably, the antibody specifically binds at least one epitope of a GalNAc-T3. As used herein, the term "epitope" refers to any antigenic determinant on an antigen to which the paratope of an antibody binds.

Epitopic determinants usually consist of chemically active surface groupings of molecules such as amino acids or carbohydrate side chains and usually have specific three dimensional structural characteristics, as well as specific charge characteristics.

The term "antibody" as used in this invention includes intact molecules as well as functional fragments thereof, such as Fab, F(ab')2, and Fv that are capable of binding to macrophages. These functional antibody fragments are defined as follows: (1) Fab, the fragment which contains a monovalent antigen-binding fragment of an antibody molecule, can be produced by digestion of whole antibody with the enzyme papain to yield an intact light chain and a portion of one heavy chain; (2) Fab', the fragment of an antibody molecule that can be obtained by treating whole antibody with pepsin, followed by reduction, to yield an intact light chain and a portion of the heavy chain; two Fab' fragments are obtained per antibody molecule; (3) (Fab')2, the fragment of the antibody that can be obtained by treating whole antibody with the enzyme pepsin without subsequent reduction; F(ab')2 is a dimer of two Fab' fragments held together by two disulfide bonds; (4) Fv, defined as a genetically engineered fragment containing the variable region of the light chain and the variable region of the heavy chain expressed as two chains; and (5) Single chain antibody ("SCA"), a genetically engineered molecule containing the variable region of the light chain and the variable region of the heavy chain, linked by a suitable polypeptide linker as a genetically fused single chain molecule.

Methods of producing polyclonal and monoclonal antibodies as well as fragments thereof are well known in the art (See for example, Harlow and Lane, Antibodies: A Laboratory Manual, Cold Spring Harbor Laboratory, New York, 1988, incorporated herein by reference).

Antibody fragments according to the present invention can be prepared by proteolytic hydrolysis of the antibody or by expression in E. coli or mammalian cells (e.g. Chinese hamster ovary cell culture or other protein expression systems) of DNA encoding the fragment. Antibody fragments can be obtained by pepsin or papain digestion of whole antibodies by conventional methods. For example, antibody fragments can be produced by enzymatic cleavage of antibodies with pepsin to provide a 5S fragment denoted F(ab')2. This fragment can be further cleaved using a thiol reducing agent, and optionally a blocking group for the sulfhydryl groups resulting from cleavage of disulfide linkages, to produce 3.5S Fab' monovalent fragments. Alternatively, an enzymatic cleavage using pepsin produces two monovalent Fab' fragments and an Fc fragment directly. These methods are described, for example, by Goldenberg, U.S. Pat. Nos. 4,036,945 and 4,331,647, and references contained therein, which patents are hereby incorporated by reference in their entirety. See also Porter, R. R. [Biochem. J. 73: 119-126 (1959)). Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody.

Fv fragments comprise an association of VH and VL chains. This association may be noncovalent, as described in Inbar et al. [Proc. Nat'l Acad. Sci. USA 69:2659-62 (19720]. Alternatively, the variable chains can be linked by an intermolecular disulfide bond or cross-linked by chemicals such as glutaraldehyde. Preferably, the Fv fragments comprise VH and VL chains connected by a peptide linker. These single-chain antigen binding proteins (sFv) are prepared by constructing a structural gene comprising DNA sequences encoding the VH and VL domains connected by an oligonucleotide. The structural gene is inserted into an expression vector, which is subsequently introduced into a host cell such as E. coli. The recombinant host cells synthesize a single polypeptide chain with a linker peptide bridging the two V domains. Methods for producing sFvs are described, for example, by [Whitlow and Filpula, Methods 2: 97-105 (1991); Bird et al., Science 242:423-426 (1988); Pack et al., Bio/Technology 11:1271-77 (1993); and U.S. Pat. No. 4,946,778, which is hereby incorporated by reference in its entirety.

Another form of an antibody fragment is a peptide coding for a single complementarity-determining region (CDR). CDR peptides ("minimal recognition units") can be obtained by constructing genes encoding the CDR of an antibody of interest. Such genes are prepared, for example, by using the polymerase chain reaction to synthesize the variable region from RNA of antibody-producing cells. See, for example, Larrick and Fry [Methods, 2: 106-10 (1991)].

Humanized forms of non-human (e.g., murine) antibodies are chimeric molecules of immunoglobulins, immunoglobulin chains or fragments thereof (such as Fv, Fab, Fab', F(ab').sub.2 or other antigen-binding subsequences of antibodies) which contain minimal sequence derived from non-human immunoglobulin. Humanized antibodies include human immunoglobulins (recipient antibody) in which residues form a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as import residues, which are typically taken from an import variable domain. Humanization can be essentially performed following the method of Winter and co-workers [Jones et al., Nature, 321:522-525 (1986); Riechmann et al., Nature 332:323-327 (1988); Verhoeyen et al., Science, 239:1534-1536 (1988)], by substituting rodent CDRs or CDR sequences for the corresponding sequences of a human antibody. Accordingly, such humanized antibodies are chimeric antibodies (U.S. Pat. No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole *et al*. and Boerner *et al.* are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introduction of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Marks et al., Bio/Technology 10,: 779-783 (1992); Lonberg et al., Nature 368: 856-859 (1994); Morrison, Nature 368 812-13 (1994); Fishwild et al., Nature Biotechnology 14, 845-51 (1996); Neuberger, Nature Biotechnology 14: 826 (1996); and Lonberg and Huszar, Intern. Rev. Immunol. 13, 65-93 (1995).

Another agent capable of downregulating a GalNAc-T3 is a small interfering RNA (siRNA) molecule. RNA interference is a two step process. The first step, which is termed as the initiation step, input dsRNA is digested into 21-23 nucleotide (nt) small interfering RNAs (siRNA), probably by the action of Dicer, a member of the RNase III family of dsRNA-specific ribonucleases, which processes (cleaves) dsRNA (introduced directly or via a transgene or a virus) in an ATP-dependent manner. Successive cleavage events degrade the RNA to 19-21 bp duplexes (siRNA), each with 2-nucleotide 3' overhangs [Hutvagner and Zamore Curr. Opin. Genetics and Development 12:225-232 (2002); and Bernstein Nature 409:363-366 (2001)].

In the effector step, the siRNA duplexes bind to a nuclease complex to from the RNA-induced silencing complex (RISC). An ATP-dependent unwinding of the siRNA duplex is required for activation of the RISC. The active RISC then targets the homologous transcript by base pairing interactions and cleaves the mRNA into 12 nucleotide fragments from the 3' terminus of the siRNA [Hutvagner and Zamore Curr. Opin. Genetics and Development 12:225-232 (2002); Hammond et al. (2001) Nat. Rev. Gen. 2:110-119 (2001); and Sharp Genes. Dev. 15:485-90 (2001)]. Although the mechanism of cleavage is still to be elucidated, research indicates that each RISC contains a single siRNA and an RNase [Hutvagner and Zamore Curr. Opin. Genetics and Development 12:225-232 (2002)].

Because of the remarkable potency of RNAi, an amplification step within the RNAi pathway has been suggested. Amplification could occur by copying of the input dsRNAs which would generate more siRNAs, or by replication of the siRNAs formed. Alternatively or additionally, amplification could be effected by multiple turnover events of the RISC [Hammond et al. Nat. Rev. Gen. 2:110-119 (2001), Sharp Genes. Dev. 15:485-90 (2001); Hutvagner and Zamore Curr. Opin. Genetics and Development 12:225-232 (2002)]. For more information on RNAi see the following reviews Tuschl ChemBiochem. 2:239-245 (2001); Cullen Nat. Immunol. 3:597-599 (2002); and Brantl Biochem. Biophys. Act. 1575:15-25 (2002).

Synthesis of RNAi molecules suitable for use with the present invention can be effected as follows. First, the GalNAc-T3 mRNA sequence is scanned downstream of the AUG start codon for AA dinucleotide sequences. Occurrence of each AA and the 3' adjacent 19 nucleotides is recorded as potential siRNA target sites. Preferably, siRNA target sites are selected from the open reading frame, as untranslated regions (UTRs) are richer in regulatory protein binding sites. UTR-binding proteins and/or translation initiation complexes may interfere with binding of the siRNA endonuclease complex [Tuschl, T. 2001, ChemBiochem. 2:239-245]. It will be appreciated though, that siRNAs directed at untranslated regions may also be effective, as demonstrated for GAPDH wherein siRNA directed at the 5' UTR mediated about 90 % decrease in cellular GAPDH mRNA and completely abolished protein level (www.ambion.com/techlib/tn/91/912.html).

Second, potential target sites are compared to an appropriate genomic database (e.g., human, mouse, rat etc.) using any sequence alignment software, such as the BLAST software available from the NCBI server (www.ncbi.nlm.nih.gov/BLAST/). Putative target sites which exhibit significant homology to other coding sequences are filtered out.

Qualifying target sequences are selected as template for siRNA synthesis. Preferred sequences are those including low G/C content as these have proven to be more effective in mediating gene silencing as compared to those with G/C content higher than 55 %. Several target sites are preferably selected along the length of the target gene for evaluation. For better evaluation of the selected siRNAs, a negative control is preferably used in conjunction. Negative control siRNA preferably include the same nucleotide composition as the siRNAs but lack significant homology to the genome. Thus, a scrambled nucleotide sequence of the siRNA is preferably used, provided it does not display any significant homology to any other gene.

For example, a suitable GalNAc-T3 siRNA can be the siRNA ID 14834 (Ambion Inc., Austin, TX).

Another agent capable of downregulating a GalNAc-T3 is a DNAzyme molecule capable of specifically cleaving an mRNA transcript or DNA sequence of the GalNAc-T3. DNAzymes are single-stranded polynucleotides which are capable of cleaving both single and double stranded target sequences (Breaker, R.R. and Joyce, G. Chemistry and Biology 1995;2:655; Santoro, S.W. & Joyce, G.F. Proc. Natl, Acad. Sci. USA 1997;943:4262). A general model (the "10-23" model) for the DNAzyme has been proposed. "10-23" DNAzymes have a catalytic domain of 15 deoxyribonucleotides, flanked by two substrate-recognition domains of seven to nine deoxyribonucleotides each. This type of DNAzyme can effectively cleave its substrate RNA at purine:pyrimidine junctions (Santoro, S.W. & Joyce, G.F. Proc. Natl, Acad. Sci. USA 199; for rev of DNAzymes see Khachigian, LM [Curr Opin Mol Ther 4:119-21 (2002)].

Examples of construction and amplification of synthetic, engineered DNAzymes recognizing single and double-stranded target cleavage sites have been disclosed in U.S. Pat. No. 6,326,174 to Joyce et al*.* DNAzymes of similar design directed against the human Urokinase receptor were recently observed to inhibit Urokinase receptor expression, and successfully inhibit colon cancer cell metastasis in vivo (Itoh *et al,* 20002, Abstract 409, Ann Meeting Am Soc Gen Ther. www.asgt.org). In another application, DNAzymes complementary to bcr-ab1 oncogenes were successful in inhibiting the oncogenes expression in leukemia cells, and lessening relapse rates in autologous bone marrow transplant in cases of CML and ALL.

Downregulation of a GalNAc-T3 can also be effected by using an antisense polynucleotide capable of specifically hybridizing with an mRNA transcript encoding the GalNAc-T3.

Design of antisense molecules which can be used to efficiently downregulate a GalNAc-T3 must be effected while considering two aspects important to the antisense approach. The first aspect is delivery of the oligonucleotide into the cytoplasm of the appropriate cells, while the second aspect is design of an oligonucleotide which specifically binds the designated mRNA within cells in a way which inhibits translation thereof.

The prior art teaches of a number of delivery strategies which can be used to efficiently deliver oligonucleotides into a wide variety of cell types [see, for example, Luft J Mol Med 76: 75-6 (1998); Kronenwett et al. Blood 91: 852-62 (1998); Rajur et al. Bioconjug Chem 8: 935-40 (1997); Lavigne et al. Biochem Biophys Res Commun 237: 566-71 (1997) and Aoki et al. (1997) Biochem Biophys Res Commun 231: 540-5 (1997)].

In addition, algorithms for identifying those sequences with the highest predicted binding affinity for their target mRNA based on a thermodynamic cycle that accounts for the energetics of structural alterations in both the target mRNA and the oligonucleotide are also available [see, for example, Walton et al. Biotechnol Bioeng 65: 1-9 (1999)].

Such algorithms have been successfully used to implement an antisense approach in cells. For example, the algorithm developed by Walton et al. enabled scientists to successfully design antisense oligonucleotides for rabbit beta-globin (RBG) and mouse tumor necrosis factor-alpha (TNF alpha) transcripts. The same research group has more recently reported that the antisense activity of rationally selected oligonucleotides against three model target mRNAs (human lactate dehydrogenase A and B and rat gp130) in cell culture as evaluated by a kinetic PCR technique proved effective in almost all cases, including tests against three different targets in two cell types with phosphodiester and phosphorothioate oligonucleotide chemistries.

In addition, several approaches for designing and predicting efficiency of specific oligonucleotides using an *in vitro* system were also published (Matveeva et al., Nature Biotechnology 16: 1374 - 1375 (1998)].

For example, a suitable antisense oligonucleotides targeted against the GALNT3 mRNA (which is coding for the GalNAc-T3 protein) would be of the following sequences: CTGGCACATACACCTCTGG (SEQ ID NO:30).

Several clinical trials have demonstrated safety, feasibility and activity of antisense oligonucleotides. For example, antisense oligonucleotides suitable for the treatment of cancer have been successfully used [Holmund et al., Curr Opin Mol Ther 1:372-85 (1999)], while treatment of hematological malignancies via antisense oligonucleotides targeting c-myb gene, p53 and Bcl-2 had entered clinical trials and had been shown to be tolerated by patients [Gerwitz Curr Opin Mol Ther 1:297-306 (1999)].

More recently, antisense-mediated suppression of human heparanase gene expression has been reported to inhibit pleural dissemination of human cancer cells in a mouse model [Uno et al., Cancer Res 61:7855-60 (2001)].

Thus, the current consensus is that recent developments in the field of antisense technology which, as described above, have led to the generation of highly accurate antisense design algorithms and a wide variety of oligonucleotide delivery systems, enable an ordinarily skilled artisan to design and implement antisense approaches suitable for downregulating expression of known sequences without having to resort to undue trial and error experimentation.

Another agent capable of downregulating a GalNAc-T3 is a ribozyme molecule capable of specifically cleaving an mRNA transcript encoding a GalNAc-T3. Ribozymes are being increasingly used for the sequence-specific inhibition of gene expression by the cleavage of mRNAs encoding proteins of interest [Welch et al., Curr Opin Biotechnol. 9:486-96 (1998)]. The possibility of designing ribozymes to cleave any specific target RNA has rendered them valuable tools in both basic research and therapeutic applications. In the therapeutics area, ribozymes have been exploited to target viral RNAs in infectious diseases, dominant oncogenes in cancers and specific somatic mutations in genetic disorders [Welch et al., Clin Diagn Virol. 10:163-71 (1998)]. Most notably, several ribozyme gene therapy protocols for HIV patients are already in Phase 1 trials. More recently, ribozymes have been used for transgenic animal research, gene target validation and pathway elucidation. Several ribozymes are in various stages of clinical trials. ANGIOZYME was the first chemically synthesized ribozyme to be studied in human clinical trials. ANGIOZYME specifically inhibits formation of the VEGF-r (Vascular Endothelial Growth Factor receptor), a key component in the angiogenesis pathway. Ribozyme Pharmaceuticals, Inc., as well as other firms have demonstrated the importance of anti-angiogenesis therapeutics in animal models. HEPTAZYME, a ribozyme designed to selectively destroy Hepatitis C Virus (HCV) RNA, was found effective in decreasing Hepatitis C viral RNA in cell culture assays (Ribozyme Pharmaceuticals, Incorporated - WEB home page).

Another agent capable of downregulating GalNAc-T3 would be any molecule which binds to and/or cleaves GalNAc-T3. Such molecules can be GalNAc-T3 antagonists, or GalNAc-T3 inhibitory peptide.

It will be appreciated that a non-functional analogue of at least a catalytic or binding portion of GalNAc-T3 can be also used as an agent which downregulates GalNAc-T3.

Another agent which can be used along with the present invention to downregulate GalNAc-T3 is a molecule which prevents GalNAc-T3 activation or substrate binding.

Each of the upregulating or downregulating agents described hereinabove or the expression vector encoding GalNAc-T3 can be administered to the individual per se or as part of a pharmaceutical composition which also includes a physiologically acceptable carrier. The purpose of a pharmaceutical composition is to facilitate administration of the active ingredient to an organism.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the upregulating or downregulating agent or the expression vector encoding GalNAc-T3 which are accountable for the biological effect.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition, which is incorporated herein by reference.

Suitable routes of administration may, for example, include oral, rectal, transmucosal, especially transnasal, intestinal or parenteral delivery, including intramuscular, subcutaneous and intramedullary injections as well as intrathecal, direct intraventricular, intravenous, inrtaperitoneal, intranasal, or intraocular injections.

Alternately, one may administer the pharmaceutical composition in a local rather than systemic manner, for example, via injection of the pharmaceutical composition directly into a tissue region of a patient.

Pharmaceutical compositions of the present invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethylcellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to the present invention are conveniently delivered in the form of an aerosol spray presentation from a pressurized pack or a nebulizer with the use of a suitable propellant, e.g., dichlorodifluoromethane, trichlorofluoromethane, dichlorotetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The pharmaceutical composition described herein may be formulated for parenteral administration, e.g., by bolus injection or continuous infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The pharmaceutical composition of the present invention may also be formulated in rectal compositions such as suppositories or retention enemas, using, e.g., conventional suppository bases such as cocoa butter or other glycerides.

Pharmaceutical compositions suitable for use in context of the present invention include compositions wherein the active ingredients are contained in an amount effective to achieve the intended purpose. More specifically, a therapeutically effective amount means an amount of active ingredients (the upregulating or downregulating agent or the expression vector encoding GalNAc-T3) effective to prevent, alleviate or ameliorate symptoms of a disorder (e.g., hyperphosphatemia or hypophosphatemia) or prolong the survival of the subject being treated.

Determination of a therapeutically effective amount is well within the capability of those skilled in the art, especially in light of the detailed disclosure provided herein.

For any preparation used in the methods of the invention, the therapeutically effective amount or dose can be estimated initially from *in vitro* and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures *in vitro,* in cell cultures or experimental animals. The data obtained from these *in vitro* and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition (See e.g., Fingl, *et al.,* 1975, in "The Pharmacological Basis of Therapeutics", Ch. 1 p.1).

Dosage amount and interval may be adjusted individually to provide plasma levels of the active ingredient are sufficient to prevent hyperphosphatemia or hypophosphatemia (minimal effective concentration, MEC). The MEC will vary for each preparation, but can be estimated from *in vitro* data. Dosages necessary to achieve the MEC will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

Compositions of the present invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation of the invention formulated in a compatible pharmaceutical carrier may also be prepared, placed in an appropriate container, and labeled for treatment of an indicated condition, as if further detailed above.

Thus, the teachings of the present invention can be used to treat individuals suffering from familial tumoral calcinosis (*i.e*., an hyperphosphatemia disorder). For example, an expression vector (e.g., a viral vector) including a polynucleotide sequence encoding the GALNT3 mRNA (SEQ ID NO:29) and the suitable promoter sequences to enable expression in kidney and skin cells is introduced into the individual via intravenous administration. Expression of such a vector in kidney and/or skin is expected to upregulate the expression level arid/or activity of GalNAc-T3 in those tissues and thus to correct the hyperphosphatemia. Dosage of such an expression vector should be calibrated using cell culture experiments and animal models. Success of treatment is preferably evaluated by determining plasma phosphate levels and the individual general health status.

It will be appreciated, that if such a treatment is employed early in childhood, *i.e.,* prior to the appearance of calcium deposits, or even prior to the detection of hyperphosphatemia (generally at 21 months of age), it may prevent the complications associated with such a disease (*i.e.,* massive calcium deposits in the skin and subcutaneous tissues). In addition, since the expression vector is targeted to somatic cells which exhibit limited half-life (depending upon the cell line transduced), such a treatment is expected to be repeated periodically in order to prevent hyperphosphatemia.

As is shown in Figure 5c and Example 2 of the Examples section which follows, the present inventors have uncovered that a 1524+1G→A mutation in the *GALNT3* gene, in a homozygous form, causes FTC in a consanguinity family (family 1). In addition, as is shown in Figures 5b and d and Example 2 of the Examples section which follows, FTC affected members of the non-consanguinity family (family 2) are double heterozygous for the R162X and 1524+5G→A mutations in the GALNT3 gene. Thus, the presence of deleterious mutations in the *GALNT3* sequence encoding GalNAc-T3 can be used in diagnosing FTC in an individual.

Thus, according to another aspect of the present invention there is provided a method of diagnosing familial tumoral calcinosis in an individual.

The method is effected by identifying in a polynucleotide sequence of the individual at least one nucleic acid substitution resulting in downregulation of an expression level and/or activity of GalNAc-T3.

As used herein the "polynucleotide sequence" refers to any DNA or RNA sequence which is derived from cells of the individual. DNA and RNA samples can be obtained from any source of cells. Preferably, the DNA is derived from peripheral blood cells (obtained using a syringe), skin cells (obtained from a skin biopsy) or mouth epithelial cells (obtained from a mouth wash), and the RNA is derived from blood or skin cells. Methods of extracting such DNA and RNA samples are well known in the art.

As is mentioned before, deleterious mutations in the *GALNT3* gene cause FTC. Thus, according to preferred embodiments of the present invention the polynucleotide sequence is an mRNA sequence encoding *GALNT3* (e.g., GenBank Accession No. NM_004482, SEQ ID NO:29) or a genomic sequence region including the *GALNT3* gene (e.g., SEQ ID NO:33).

As used herein, the phrase "nucleic acid substitution" refers to any mutation in the DNA sequence of an individual which can result in downregulation of the expression level and/or activity of GalNAc-T3. Non-limiting examples of such nucleic acid changes include a missense mutation (*i.e.,* a mutation which changes an amino acid residue in the protein with another amino acid residue), a nonsense mutation (*i.e.,* a mutation which introduces a stop codon in a protein), a frameshift mutation (*i.e.,* a mutation, usually, deletion or insertion of nucleic acids which changes the reading frame of the protein, and may result in an early termination or in a longer amino acid sequence), a readthrough mutation (*i.e.,* a mutation which results in an elongated protein due to a change in a coding frame or a modified stop codon), a promoter mutation (*i.e.,* a mutation in a promoter sequence, usually 5' to the transcription start site of a gene, which result in up-regulation or down-regulation of a specific gene product), a regulatory mutation (*i.e.,* a mutation in a region upstream or downstream, or within a gene, which affects the expression of the gene product), a deletion (*i.e.,* a mutation which deletes coding or non-coding nucleic acids in a gene sequence), an insertion (*i.e.,* a mutation which inserts coding or non-coding nucleic acids into a gene sequence), an inversion (*i.e.,* a mutation which results in an inverted coding or non-coding sequence), a splice mutation (i.e., a mutation which results in abnormal splicing or poor splicing) and a duplication (*i.e.,* a mutation which results in a duplicated coding or non-coding sequence).

It will be appreciated that certain nucleic acid substitutions can be present in non-affected individuals [e.g., single nucleotide polymorphism (SNP)]. Such an SNP can cause, for example, a missense mutation in the GalNAc-T3, which, when present in the heterozygous form can be harmless, however, when present together with another, deleterious mutation such as a nonsense mutation, can lead to FTC at various degrees. Thus, according to preferred embodiments of the present invention the nucleic acid substitution is an SNP.

The nucleic acid substitution of the present invention can be identified using a variety of approaches suitable for identifying sequence alterations. One option is to determine the entire gene sequence of a PCR reaction product. Alternatively, a given segment of nucleic acid may be characterized on several other levels. At the lowest resolution, the size of the molecule can be determined by electrophoresis by comparison to a known standard run on the same gel. A more detailed picture of the molecule may be achieved by cleavage with combinations of restriction enzymes prior to electrophoresis, to allow construction of an ordered map. The presence of specific sequences within the fragment can be detected by hybridization of a labeled probe, or the precise nucleotide sequence can be determined by partial chemical degradation or by primer extension in the presence of chain-terminating nucleotide analogs.

Following is a non-limiting list of methods which can be used to identify the nucleic acid substitution and/or SNPs in the *GALNT33* gene.

***Direct sequencing of a PCR product:*** This method is based on the amplification of a genomic sequence using specific PCR primers in a PCR reaction following by a sequencing reaction utilizing the sequence of one of the PCR primers as a sequencing primer. Sequencing reaction can be performed using, for example, the Applied Biosystems (Foster City, CA) ABI PRISM® BigDye™ Primer or BigDye™ Terminator Cycle Sequencing Kits.

***Restriction fragment length polymorphism (RFLP):*** This method uses a change in a single nucleotide (the SNP nucleotide) which modifies a recognition site for a restriction enzyme resulting in the creation or destruction of an RFLP.

For example, RFLP can be used to detect the R162X mutation (C→T substitution at nucleotide 484 as set forth in SEQ ID NO:29) in a genomic DNA of an individual. Briefly, genomic DNA is amplified using the GALe1bR (SEQ ID NO:1) and GALe1bF (SEQ ID NO:2) PCR primers, and the resultant PCR product is subjected to digestion using a restriction enzyme such as *Bst*DEI*, Dde*I (see e.g., Figure 6b) or *Tsp*RI which are capable of differentially digesting a PCR product containing the T allele (and not the C allele) at position 484 of SEQ ID NO:29.

Single nucleotide mismatches in DNA heteroduplexes are also recognized and cleaved by some chemicals, providing an alternative strategy to detect single base substitutions, generically named the "Mismatch Chemical Cleavage" (MCC) (Gogos et al., Nucl. Acids Res., 18:6807-6817, 1990). However, this method requires the use of osmium tetroxide and piperidine, two highly noxious chemicals which are not suited for use in a clinical laboratory.

***Allele specific oligonucleotide (ASO):*** In this method, an allele-specific oligonucleotide (ASO) is designed to hybridize in proximity to the polymorphic nucleotide, such that a primer extension or ligation event can be used as the indicator of a match or a mis-match. Hybridization with radioactively labeled allelic specific oligonucleotides (ASO) also has been applied to the detection of specific SNPs (Conner et al., Proc. Natl. Acad. Sci., 80:278-282, 1983). The method is based on the differences in the melting temperature of short DNA fragments differing by a single nucleotide. Stringent hybridization and washing conditions can differentiate between mutant and wild-type alleles.

It will be appreciated that ASO can be applied on a PCR product generated from genomic DNA. For example, to detect the R162X mutation, genomic DNA is amplified using the GALe1bR (SEQ ID NO:1) and the GALe1bF (SEQ ID NO:2) PCR primers, and the resultant PCR product is subjected to an ASO hybridization using the following oligonucleotide probe: 5'- CTTTGCACtGAGATCTTGG (SEQ ID NO:31) which is capable of hybridizing to the thymidine nucleotide at position 484 of SEQ ID NO:29. As a control for the hybridization, the CTTTGCACCGAGATCTTGG (SEQ ID NO:32) oligonucleotide probe is applied to detect the presence of the wildtype allele.

***Denaturing*/*Temperature Gradient Gel Electrophoresis (DGGE*/*TGGE):*** Two other methods rely on detecting changes in electrophoretic mobility in response to minor sequence changes. One of these methods, termed "Denaturing Gradient Gel Electrophoresis" (DGGE) is based on the observation that slightly different sequences will display different patterns of local melting when electrophoretically resolved on a gradient gel. In this manner, variants can be distinguished, as differences in melting properties of homoduplexes versus heteroduplexes differing in a single nucleotide can detect the presence of SNPs in the target sequences because of the corresponding changes in their electrophoretic mobilities. The fragments to be analyzed, usually PCR products, are "clamped" at one end by a long stretch of G-C base pairs (30-80) to allow complete denaturation of the sequence of interest without complete dissociation of the strands. The attachment of a GC "clamp" to the DNA fragments increases the fraction of mutations that can be recognized by DGGE (Abrams et al., Genomics 7:463-475, 1990). Attaching a GC clamp to one primer is critical to ensure that the amplified sequence has a low dissociation temperature (Sheffield et al., Proc. Natl. Acad. Sci., 86:232-236, 1989; and Lerman and Silverstein, Meth. Enzymol., 155:482-501, 1987). Modifications of the technique have been developed, using temperature gradients (Wartell et al., Nucl. Acids Res., 18:2699-2701, 1990), and the method can be also applied to RNA:RNA duplexes (Smith et al., Genomics 3:217-223, 1988).

Limitations on the utility of DGGE include the requirement that the denaturing conditions must be optimized for each type of DNA to be tested. Furthermore, the method requires specialized equipment to prepare the gels and maintain the needed high temperatures during electrophoresis. The expense associated with the synthesis of the clamping tail on one oligonucleotide for each sequence to be tested is also a major consideration. In addition, long running times are required for DGGE. The long running time of DGGE was shortened in a modification of DGGE called constant denaturant gel electrophoresis (CDGE) (Borrensen et al., Proc. Natl. Acad. Sci. USA 88:8405, 1991). CDGE requires that gels be performed under different denaturant conditions in order to reach high efficiency for the detection of SNPs.

A technique analogous to DGGE, termed temperature gradient gel electrophoresis (TGGE), uses a thermal gradient rather than a chemical denaturant gradient (Scholz, et al., Hum. Mol. Genet. 2:2155, 1993). TGGE requires the use of specialized equipment which can generate a temperature gradient perpendicularly oriented relative to the electrical field. TGGE can detect mutations in relatively small fragments of DNA therefore scanning of large gene segments requires the use of multiple PCR products prior to running the gel.

***Single-Strand Conformation Polymorphism (SSCP):*** Another common method, called "Single-Strand Conformation Polymorphism" (SSCP) was developed by Hayashi, Sekya and colleagues (reviewed by Hayashi, PCR Meth. Appl., 1:34-38, 1991) and is based on the observation that single strands of nucleic acid can take on characteristic conformations in non-denaturing conditions, and these conformations influence electrophoretic mobility. The complementary strands assume sufficiently different structures that one strand may be resolved from the other. Changes in sequences within the fragment will also change the conformation, consequently altering the mobility and allowing this to be used as an assay for sequence variations (Orita, et al., Genomics 5:874-879, 1989).

The SSCP process involves denaturing a DNA segment (e.g., a PCR product) that is labeled on both strands, followed by slow electrophoretic separation on a non-denaturing polyacrylamide gel, so that intra-molecular interactions can form and not be disturbed during the run. This technique is extremely sensitive to variations in gel composition and temperature. A serious limitation of this method is the relative difficulty encountered in comparing data generated in different laboratories, under apparently similar conditions.

***Dideoxy fingerprinting (ddF):*** The dideoxy fingerprinting (ddF) is another technique developed to scan genes for the presence of mutations (Liu and Sommer, PCR Methods Appli., 4:97, 1994). The ddF technique combines components of Sanger dideoxy sequencing with SSCP. A dideoxy sequencing reaction is performed using one dideoxy terminator and then the reaction products are electrophoresed on nondenaturing polyacrylamide gels to detect alterations in mobility of the termination segments as in SSCP analysis. While ddF is an improvement over SSCP in terms of increased sensitivity, ddF requires the use of expensive dideoxynucleotides and this technique is still limited to the analysis of fragments of the size suitable for SSCP (*i.e.,* fragments of 200-300 bases for optimal detection of mutations).

In addition to the above limitations, all of these methods are limited as to the size of the nucleic acid fragment that can be analyzed. For the direct sequencing approach, sequences of greater than 600 base pairs require cloning, with the consequent delays and expense of either deletion sub-cloning or primer walking, in order to cover the entire fragment. SSCP and DGGE have even more severe size limitations. Because of reduced sensitivity to sequence changes, these methods are not considered suitable for larger fragments. Although SSCP is reportedly able to detect 90 % of single-base substitutions within a 200 base-pair fragment, the detection drops to less than 50 % for 400 base pair fragments. Similarly, the sensitivity of DGGE decreases as the length of the fragment reaches 500 base-pairs. The ddF technique, as a combination of direct sequencing and SSCP, is also limited by the relatively small size of the DNA that can be screened.

***Pyrosequencing^{™} analysis (Pyrosequencing, Inc. Westborough, MA, USA):*** This technique is based on the hybridization of a sequencing primer to a single stranded, PCR-amplified, DNA template in the presence of DNA polymerase, ATP sulfurylase, luciferase and apyrase enzymes and the adenosine 5' phosphosulfate (APS) and luciferin substrates. In the second step the first of four deoxynucleotide triphosphates (dNTP) is added to the reaction and the DNA polymerase catalyzes the incorporation of the deoxynucleotide triphosphate into the DNA strand, if it is complementary to the base in the template strand. Each incorporation event is accompanied by release of pyrophosphate (PPi) in a quantity equimolar to the amount of incorporated nucleotide. In the last step the ATP sulfurylase quantitatively converts PPi to ATP in the presence of adenosine 5' phosphosulfate. This ATP drives the luciferase-mediated conversion of luciferin to oxyluciferin that generates visible light in amounts that are proportional to the amount of ATP. The light produced in the luciferase-catalyzed reaction is detected by a charge coupled device (CCD) camera and seen as a peak in a pyrogram^{™}. Each light signal is proportional to the number of nucleotides incorporated.

***Acycloprime^{™} analysis (Perkin Elmer, Boston, Massachusetts, USA):*** This technique is based on fluorescent polarization (FP) detection. Following PCR amplification of the sequence containing the SNP of interest, excess primer and dNTPs are removed through incubation with shrimp alkaline phosphatase (SAP) and exonuclease I. Once the enzymes are heat inactivated, the Acycloprime-FP process uses a thermostable polymerase to add one of two fluorescent terminators to a primer that ends immediately upstream of the SNP site. The terminator(s) added are identified by their increased FP and represent the allele(s) present in the original DNA sample. The Acycloprime process uses AcycloPol^{™}, a novel mutant thermostable polymerase from the Archeon family, and a pair of AcycloTerminators^{™} labeled with R110 and TAMRA, representing the possible alleles for the SNP of interest. AcycloTerrninator^{™} non-nucleotide analogs are biologically active with a variety of DNA polymerases. Similarly to 2', 3'-dideoxynucleotide-5'-triphosphates, the acyclic analogs function as chain terminators. The analog is incorporated by the DNA polymerase in a base-specific manner onto the 3'-end of the DNA chain, and since there is no 3'-hydroxyl, is unable to function in further chain elongation. It has been found that AcycloPol has a higher affinity and specificity for derivatized AcycloTerminators than various Taq mutant have for derivatized 2', 3'-dideoxynucleotide terminators.

***Reverse dot blot:*** This technique uses labeled sequence specific oligonucleotide probes and unlabeled nucleic acid samples. Activated primary amine-conjugated oligonucleotides are covalently attached to carboxylated nylon membranes. After hybridization and washing, the labeled probe, or a labeled fragment of the probe, can be released using oligomer restriction, *i.e*., the digestion of the duplex hybrid with a restriction enzyme. Circular spots or lines are visualized colorimetrically after hybridization through the use of streptavidin horseradish peroxidase incubation followed by development using tetramethylbenzidine and hydrogen peroxide, or via chemiluminescence after incubation with avidin alkaline phosphatase conjugate and a luminous substrate susceptible to enzyme activation, such as CSPD, followed by exposure to x-ray film.

It will be appreciated that advances in the field of SNP detection have provided additional accurate, easy, and inexpensive large-scale SNP genotyping techniques, such as dynamic allele-specific hybridization (DASH, Howell, W.M. et al., 1999. Dynamic allele-specific hybridization (DASH). Nat. Biotechnol. 17: 87-8), microplate array diagonal gel electrophoresis [MADGE, Day, I.N. et al., 1995. High-throughput genotyping using horizontal polyacrylamide gels with wells arranged for microplate array diagonal gel electrophoresis (MADGE). Biotechniques. 19: 830-5], the TaqMan system (Holland, P.M. et al., 1991. Detection of specific polymerase chain reaction product by utilizing the 5'→3' exonuclease activity of Thermus aquaticus DNA polymerase. Proc Natl Acad Sci USA. 88: 7276-80), as well as various DNA "chip" technologies such as the GeneChip microarrays (e.g., Affymetrix SNP chips) which are disclosed in U.S. Pat. Appl. No. 6,300,063 to Lipshutz, et al. 2001, which is fully incorporated herein by reference, Genetic Bit Analysis (GBA^{™}) which is described by Goelet, P. et al. (PCT Appl. No. 92/15712), peptide nucleic acid (PNA, Ren B, et al., 2004. Nucleic Acids Res. 32: e42) and locked nucleic acids (LNA, Latorra D, et al., 2003. Hum. Mutat. 22: 79-85) probes, Molecular Beacons (Abravaya K, et al., 2003. Clin Chem Lab Med. 41: 468-74), intercalating dye [Germer, S. and Higuchi, R. Single-tube genotyping without oligonucleotide probes. Genome Res. 9:72-78 (1999)], FRET primers (Solinas A et al., 2001. Nucleic Acids Res. 29: E96), AlphaScreen (Beaudet L, et al., Genome Res. 2001, 11(4): 600-8), SNPstream (Bell PA, et al., 2002. Biotechniques. Suppl.: 70-2, 74, 76-7), Multiplex minisequencing (Curcio M, et al., 2002. Electrophoresis. 23: 1467-72), SnaPshot (Turner D, et al., 2002. Hum Immunol. 63: 508-13), MassEXTEND (Cashman JR, et al., 2001. Drug Metab Dispos. 29: 1629-37), GOOD assay (Sauer S, and Gut IG. 2003. Rapid Commun. Mass. Spectrom. 17: 1265-72), Microarray minisequencing (Liljedahl U, et al., 2003. Pharmacogenetics. 13: 7-17), arrayed primer extension (APEX) (Tonisson N, et al., 2000. Clin. Chem. Lab. Med. 38: 165-70), Microarray primer extension (O'Meara D, et al., 2002. Nucleic Acids Res. 30: e75), Tag arrays (Fan JB, et al., 2000. Genome Res. 10: 853-60), Template-directed incorporation (TDI) (Akula N, et al., 2002. Biotechniques. 32: 1072-8), fluorescence polarization (Hsu TM, et al., 2001. Biotechniques. 31: 560, 562, 564-8), Colorimetric oligonucleotide ligation assay (OLA, Nickerson DA, et al., 1990. Proc. Natl. Acad. Sci. USA. 87: 8923-7), Sequence-coded OLA (Gasparini P, et al., 1999. J. Med. Screen. 6: 67-9), Microarray ligation, Ligase chain reaction, Padlock probes, Rolling circle amplification, Invader assay (reviewed in Shi MM. 2001. Enabling large-scale pharmacogenetic studies by high-throughput mutation detection and genotyping technologies. Clin Chem. 47: 164-72), coded microspheres (Rao KV et al., 2003. Nucleic Acids Res. 31: e66) and MassArray (Leushner J, Chiu NH, 2000. Mol Diagn. 5: 341-80).

It will be appreciated that nucleic acid substitutions can be also identified in mRNA molecules of the individual. Such mRNA molecules are first subjected to an RT-PCR reaction following which they are either directly sequenced or be subjected to any of the SNP detection methods described hereinabove.

As is shown in Figure 8 and Example 2 of the Examples section which follows, sequencing of the RT-PCR product generated using the GALex6F (SEQ ID NO:23) and the GALex8R (SEQ ID NO:24) primers revealed the absence of exon 7 from the mature *GALNT3* mRNA.

Thus, according to preferred embodiments of the present invention, identifying at least one nucleic acid substitution in the mRNA sequence encoding *GALNT3* is effected using DNA sequencing of a *GLANT3* RT-PCR product.

Downregulation or upregulation of the expression level and/or activity of GalNAc-T3 can be determined using molecular and immunological methods known in the art.

Following is a list of methods useful for detecting *GALNT3* RNA level in cells of the individual.

***Northern Blot analysis:*** This method involves the detection of a particular RNA in a mixture of RNAs. An RNA sample is denatured by treatment with an agent (e.g., formaldehyde) that prevents hydrogen bonding between base pairs, ensuring that all the RNA molecules have an unfolded, linear conformation. The individual RNA molecules are then separated according to size by gel electrophoresis and transferred to a nitrocellulose or a nylon-based membrane to which the denatured RNAs adhere. The membrane is then exposed to labeled DNA probes. Probes may be labeled using radio-isotopes or enzyme linked nucleotides. Detection may be using autoradiography, colorimetric reaction or chemiluminescence. This method allows both quantitation of an amount of particular RNA molecules and determination of its identity by a relative position on the membrane which is indicative of a migration distance in the gel during electrophoresis.

***RT-PCR analysis:*** This method uses PCR amplification of relatively rare RNAs molecules. First, RNA molecules are purified from the cells and converted into complementary DNA (cDNA) using a reverse transcriptase enzyme (such as an MMLV-RT) and primers such as, oligo dT, random hexamers or gene specific primers. Then by applying gene specific primers and Taq DNA polymerase, a PCR amplification reaction is carried out in a PCR machine. Those of skills in the art are capable of selecting the length and sequence of the gene specific primers and the PCR conditions (*i.e*., annealing temperatures, number of cycles and the like) which are suitable for detecting specific RNA molecules. It will be appreciated that a semiquantitative RT-PCR reaction can be employed by adjusting the number of PCR cycles and comparing the amplification product to known controls.

***RNA in situ hybridization stain:*** In this method DNA or RNA probes are attached to the RNA molecules present in the cells. Generally, the cells are first fixed to microscopic slides to preserve the cellular structure and to prevent the RNA molecules from being degraded and then are subjected to hybridization buffer containing the labeled probe. The hybridization buffer includes reagents such as formamide and salts (e.g., sodium chloride and sodium citrate) which enable specific hybridization of the DNA or RNA probes with their target mRNA molecules *in situ* while avoiding non-specific binding of probe. Those of skills in the art are capable of adjusting the hybridization conditions (*i.e.*, temperature, concentration of salts and formamide and the like) to specific probes and types of cells. Following hybridization, any unbound probe is washed off and the slide is subjected to either a photographic emulsion which reveals signals generated using radio-labeled probes or to a colorimetric reaction which reveals signals generated using enzyme-linked labeled probes.

***In situ RT-PCR stain:*** This method is described in Nuovo GJ, et al. [Intracellular localization of polymerase chain reaction (PCR)-amplified hepatitis C cDNA. Am J Surg Pathol. 1993, 17: 683-90] and Komminoth P, et al. [Evaluation of methods for hepatitis C virus detection in archival liver biopsies. Comparison of histology, immunohistochemistry, in situ hybridization, reverse transcriptase polymerase chain reaction (RT-PCR) and in situ RT-PCR. Pathol Res Pract. 1994, 190: 1017-25]. Briefly, the RT-PCR reaction is performed on fixed cells by incorporating labeled nucleotides to the PCR reaction. The reaction is carried on using a specific *in situ* RT-PCR apparatus such as the laser-capture microdissection PixCell I LCM system available from Arcturus Engineering (Mountainview, CA).

Following is a list of immunological detection methods which can be used to detect the level of GalNAc-T3 protein in cells of the individual.

***Enzyme linked immunosorbent assay (ELISA):*** This method involves fixation of a sample (e.g., fixed cells or a proteinaceous solution) containing a protein substrate to a surface such as a well of a microtiter plate. A substrate specific antibody coupled to an enzyme is applied and allowed to bind to the substrate. Presence of the antibody is then detected and quantitated by a colorimetric reaction employing the enzyme coupled to the antibody. Enzymes commonly employed in this method include horseradish peroxidase and alkaline phosphatase. If well calibrated and within the linear range of response, the amount of substrate present in the sample is proportional to the amount of color produced. A substrate standard is generally employed to improve quantitative accuracy.

***Western blot:*** This method involves separation of a substrate from other protein by means of an acrylamide gel followed by transfer of the substrate to a membrane (e.g., nylon or PVDF). Presence of the substrate is then detected by antibodies specific to the substrate, which are in turn detected by antibody binding reagents. Antibody binding reagents may be, for example, protein A, or other antibodies. Antibody binding reagents may be radiolabeled or enzyme linked as described hereinabove. Detection may be by autoradiography, colorimetric reaction or chemiluminescence. This method allows both quantitation of an amount of substrate and determination of its identity by a relative position on the membrane which is indicative of a migration distance in the acrylamide gel during electrophoresis.

***Radio-immunoassay (RIA):*** In one version, this method involves precipitation of the desired protein (*i.e*., the substrate) with a specific antibody and radiolabeled antibody binding protein (e.g., protein A labeled with I¹²⁵) immobilized on a precipitable carrier such as agarose beads. The number of counts in the precipitated pellet is proportional to the amount of substrate.

In an alternate version of the RIA, a labeled substrate and an unlabelled antibody binding protein are employed. A sample containing an unknown amount of substrate is added in varying amounts. The decrease in precipitated counts from the labeled substrate is proportional to the amount of substrate in the added sample.

***Fluorescence activated cell sorting (FACS):*** This method involves detection of a substrate *in situ* in cells by substrate specific antibodies. The substrate specific antibodies are linked to fluorophores. Detection is by means of a cell sorting machine which reads the wavelength of light emitted from each cell as it passes through a light beam. This method may employ two or more antibodies simultaneously.

***Immunohistochemical analysis:*** This method involves detection of a substrate *in situ* in fixed cells by substrate specific antibodies. The substrate specific antibodies may be enzyme linked or linked to fluorophores. Detection is by microscopy and subjective or automatic evaluation. If enzyme linked antibodies are employed, a colorimetric reaction may be required. It will be appreciated that immunohistochemistry is often followed by counterstaining of the cell nuclei using for example Hematoxyline or Giemsa stain.

Following is a list of enzymatic activity assays which can be used to determine GalNAc-T3 activity in cells of the individual.

***In situ activity assay:*** According to this method, a chromogenic substrate is applied on the cells containing an active enzyme and the enzyme catalyzes a reaction in which the substrate is decomposed to produce a chromogenic product visible by a light or a fluorescent microscope.

***In vitro activity assays:*** In these methods the activity of a particular enzyme is measured in a protein mixture extracted from the cells. The activity can be measured in a spectrophotometer well using colorimetric methods or can be measured in a non-denaturing acrylamide gel (*i.e*., activity gel). Following electrophoresis the gel is soaked in a solution containing a substrate and colorimetric reagents. The resulting stained band corresponds to the enzymatic activity of the protein of interest. If well calibrated and within the linear range of response, the amount of enzyme present in the sample is proportional to the amount of color produced. An enzyme standard is generally employed to improve quantitative accuracy.

The activity assays described hereinabove can use any of the GalNAc-T3 specific substrates, including, but not limited to, HIV_{H1B}gp120, Fibronectin, Prion-a, CD59, Mucla, Muc2, EA2, Muc7.

It will be appreciated that mutations which downregulate the expression level and/or activity level of GalNAc-T3 can be also identified in a polypeptide sequence of the individual.

Thus, according to another aspect of the present invention there is provided a method of diagnosing familial tumoral calcinosis in an individual.

The method is effected by identifying in a polypeptide sequence of the individual at least one amino acid substitution capable of downregulating the expression level and/or activity of GalNAc-T3.

As used herein, the phrase "one amino acid substitution" refers to any change in a polypeptide sequence (e.g., substitution, deletion, duplication, methylation, acetylation, glycosylation, phosphorylation of an amino acid) which can cause downregulation of the expression level and/or activity of the GalNAc-T3.

According to preferred embodiments of the present invention the amino acid substitution is identified in the GalNAc-T3 protein as set forth in SEQ ID NO:28.

Amino acid substitutions can be identified using any method known in the art, preferably, using an antibody which is capable of identifying between two polymorphs of the same protein.

The term "polymorph" as used herein refers to one form of a protein which is different in at least one amino acid or a modification on at least one amino acid from another form of the same protein. For example, the substitution of a methionine residue with a valine residue of a certain protein. In this case one protein polymorph contains the methionine and the other protein polymorph contains the valine. Other protein polymorphs can be identified by the presence or absence of specific post-translational modifications such as the phosphorylation of a serine, threonine or tyrosine residue in a protein.

Determination of at least one amino acid substitution between two polymorphs of the GalNAc-T3 can be accomplished directly, by analyzing the GalNAc-T3 protein, or portions thereof. Such a direct analysis is often accomplished using an immunological detection method as described hereinabove.

As is mentioned before, agents which upregulate or downregulate GalNAc-T3 expression level and/or activity can be used to treat hyperphosphatemia or hypophosphatemia, respectively. Additional examples of such agents can be identified via *in vitro* and/or *ex vivo* assays.

Thus, according to another aspect of the present invention there is provided a method of identifying an agent suitable for treating a disorder associated with abnormal phosphate metabolism.

The method is effected by exposing GalNAc-T3 or cells expressing GalNAc-T3 to a plurality of molecules and selecting from them at least one molecule capable of regulating the expression level and/or the activity of the GalNAc-T3, such a molecule being the agent suitable for treating the disorder associated with abnormal phosphate metabolism.

As used herein, "exposing GalNAc-T3" refers to subjecting a GalNAc-T3 protein preparation to various test molecules. A GalNAc-T3 protein preparation can be obtained by extracting proteins from cells or tissues exhibiting high expression level of GalNAc-T3 (e.g., skin, kidney, blood) or by purifying a protein extract of eukaryotic cells which over-express the GalNAc-T3 protein. Preferably, the GalNAc-T3 protein includes at least a catalytic (*i.e.,* the region in the protein which is responsible for the catalytic activity of the protein) or binding (*i.e.,* the region in the protein which is responsible for binding a substrate, receptor and the like) portion of the GalNAc-T3. For example, the catalytic domain of the GalNAc-T3, which includes the amino acids at positions 566-1122 of the GalNAc-T3 protein (GenBank Accession No. NP_004473, SEQ ID NO:28) can be used along with the present invention.

According to preferred embodiments of the present invention the GalNAc-T3 protein is set forth in SEQ ID NO:28.

The phrase "cells expressing GalNAc-T3" refers to eukaryotic cells, preferably mammalian cells, more preferably, human cells, which were transfected with an expression vector containing the GALNT3 polynucleotide (e.g., GenBank Accession No. NM_004482, SEQ ID NO:29) and are cultured in a tissue culture flask. Non-limiting examples of such cells include bone marrow cell, kidney cells, fibroblasts, epithelial cells and lymphoblastoid cells.

Once the GalNAc-T3 or cells expressing GalNAc-T3 are obtained, the test molecules (e.g., drugs, minerals, vitamins, and the like) are applied on the GalNAc-T3 or cells expressing GalNAc-T3 and the expression level and/or activity of GalNAc-T3 is detected using the molecular, immunological and biochemical methods which are fully described hereinabove. Molecules which exert significant modulations of GalNAc-T3 activity and/or expression level (*i.e*., upregulation or downregulation) become candidates for additional evaluations as suitable for treating disorders associated with abnormal phosphate metabolism.

The agents of the present invention which are described hereinabove may be included in a diagnostic kit/article of manufacture preferably along with appropriate instructions for use and labels indicating FDA approval for use in diagnosing familial tumoral calcinosis and/or identifying agents suitable for treating a disorder associated with abnormal phosphate metabolism.

Such a kit can include, for example, at least one container including at least one of the above desribed diagnostic agents (e.g., for identifying nucleic acid substitution) and an imaging reagent packed in another container (e.g., enzymes, buffers, chromogenic substrates, fluorogenic material). The kit may also include appropriate buffers and preservatives for improving the shelf-life of the kit.

As used herein the term "about" refers to ± 10 %.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., Ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (Eds.) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., Ed. (1994); "Culture of Animal Cells - A Manual of Basic Technique" by Freshney, Wiley-Liss, N. Y. (1994), Third Edition; "Current Protocols in Immunology" Volumes I-III Coligan J. E., Ed. (1994); Stites et al. (Eds.), "Basic and Clinical Immunology" (8th Edition), Appleton & Lange, Norwalk, CT (1994); Mishell and Shiigi (Eds.), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., Ed. (1984); "Nucleic Acid Hybridization" Hames, B. D., and Higgins S. J., Eds. (1985); "Transcription and Translation" Hames, B. D., and Higgins S. J., Eds. (1984); "Animal Cell Culture" Freshney, R. I., Ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### EXAMPLE 1

### CHARACTERIZATION OFA FAMILIAL TUMORAL CALCINOSIS (FTC) CRITICAL REGION

Familial tumoral calcinosis (FTC; MIM211900) is a severe autosomal recessive metabolic disorder manifesting with hyperphosphatemia and massive calcium deposits in the skin and subcutaneous tissues. To identify the genetic basis of FTC, linkage analysis was performed using DNA from informative FTC families, as follows.

### Materials and Experimental Methods

***FTC affected individuals** -* A total of 12 FTC affected individuals belonging to two large kindred of Druze and African American origin (Figures 1a-b), which have been extensively described in the literature (Steinherz, R. et al. 1985, Am. J. Dis. Child. 139: 816-819; Slavin, R.E., et al., 1993, Am. J. Surg. Path. 17: 788-802) were included in the study.

***Linkage analysis** -* A genome wide scan using 362 microsatellite markers (In Vitrogen, Cat. No. 20508, Huntsville, AL, USA) was employed in a consanguinity FTC family (family 1, Figure.1a).

***RT-PCR analysis** -* RNA was extracted using the QIAGEN RNeasy kit (QIAGEN Inc., CA USA) from the following human cells or tissues: liver, heart, kidney, pancreas, skeletal muscle, bone marrow, lung, brain, placenta, retina, and skin. RT-PCR reactions were performed using the TITAN One Tube RT-PCR kit (Roche Molecular Biochemicals, Mannheim, Germany) and the GALex6F (SEQ ID NO:23) and GALex9R primers, following by a nested PCR reaction using the GALex6F and GALex8R (SEQ ID NO:24) primers (see Table 1, hereinbelow).

**Table 1: RT-PCR primers and conditions**

| ***Primer (SEQ ID NO:)*** | ***Sequence 5→3'*** | ***Anneal. Temp.*** ***(°C)*** |
|---|---|---|
| GALex6F (SEQ ID NO:23) | GGCAGTTGGAGATTATGCCT7G | 60 °C |
| GALex8R (SEQ ID NO:24) | CAACATCCAGACATAGAGGCTG | 60 °C |
| GALex9R (SEQ ID NO:25) | CTGCTCTCCAGTGACAACTGTC | 60 °C |
| Beta Actin 5' (SEQ ID NO:26) | CGACGAGGCCCAGAGCAAGAGA | 60 °C |
| Beta Actin 3' (SEQ ID NO:27) | TCCAGGGCGACGTAGCACAGCTT | 60 °C |

### Experimental Results

***Clinical characteristics of FTC affected individuals** -* All twelve FTC affected individuals reported recurrent painful, calcified subcutaneous masses of up to 1 kg (Figure 2a), often resulting in secondary infection and incapacitating mutilation. Three patients developed deep periarticular tumors (Figure 2b) and one patient succumbed to the disease. All patients displayed hyperphosphatemia [range (mg/dl): family 1: 6.2-8.5; family 2: 5.2-6.6] but normal levels of calcium, parathyroid hormone (PTH) and 1,25-dihydroxyvitamin D3 (Vitamin D3).

***Mapping of the FTC gene to chromosome 2q24-q31** -* Consanguinity in family 1 enabled the application of homozygosity mapping to identify a 15 Mb segment identical by descent in all affected individuals. This region is flanked by D2S142 and D2S2284/D2S2177 markers on 2q24-q31 (Figure 1a). As is shown in Figure 3a, a maximum multipoint LOD score of 6.7 was observed for the 15 Mb region using the MAPMAKER/HOMOZ (Kruglyak L, et al., 1995. Rapid multipoint linkage analysis of recessive traits in nuclear families, including homozygosity mapping. Am. J. Hum. Genet. 56: 519-27). Multipoint linkage analysis in family 2 using 7 markers in this critical region further reduced the interval to 3 Mb, flanked by D2S111 and D2S1776 (Figure 1b) and yielded a maximum multipoint LOD score of 3.4 (Figure 3b) using the GeneHunter (Kruglyak, L., et al., 1996, Am. J. Hum. Genet. 58: 1347-1363).

***Analysis of FTC putative genes in the FTC critical regions** -* Using the Mapviewer tool available via the NCBI web site (www.ncbi.nlm.nih.gov/), 11 genes were identified within the FTC region, among which B3GALT1, SCN7A, SCN9A, SCN1A, STK39 are thought to play a role in neural or neuroendocrine tissues, while the function of TAIP-2, CMYA3, FLJ11457, LOC90643, LOC253782 is mostly unknown. The last positional candidate gene, GALNT3, encodes the UDP-N-acetyl-alpha-D-galactosamine:polypeptide N-acetylgalactosaminyltransferase 3 (GalNAc-T3) (Bennett, E.P., et al., 1996, Biol. Chem. 271: 17006-17012). The GalNAc-T3 protein belongs to a large family of Golgi-associated biosynthetic enzymes that transfer GalNac from the sugar donor UDP-GalNac to serine and threonine residues, and thereby are responsible for initiating O-glycan synthesis, a prevalent form of post-translational modification (Ten Hagen, K.G., et al., 2003. Glycobiology 13: 1R-16R).

***Tissue specific expression of the GALNT3 gene in skin and kidney** -* To determine if the *GALNT3* gene is a putative FTC candidate gene, the expression pattern of the gene was determined using RT-PCR analysis. As is shown in Figure 4, high level of expression was detected in RNA from both kidney and skin, two tissues of functional relevance to the pathogenesis of FTC [Steinherz, 1985 (Supra); Slavin, 1993 (Supra)].

These results demonstrate the identification of a 3 MB FTC critical region on chromosome 2q24-q31. Furthermore, the high expression level of *GALNT3* in both kidney and skin suggest its involvement in the pathogenesis of FTC.

### EXAMPLE 2

### BIALLELIC DELETERIOUS MUTATIONS IN GALNT3, ENCODING A PROTEIN INVOL VED IN O-LINKED GLYCOSYLATION, CA USE FAMILIAL TUMORAL CALCINOSIS

To test whether mutations in the *GALNT3* gene underlie the molecular basis of FTC, genomic DNA from FTC cases was subjected to sequence analysis of the *GALNT3* coding sequence.

### Materials and Experimental Methods

***Sequencing analysis** -* Genomic DNA was amplified using PCR primers designed specific for the amplification of the *GALNT3* coding exons. PCR primers and conditions are listed in Table 2, hereinbelow.

**Table 2: PCR primers and conditions**

| ***Primer (SEQ lD NO:)*** | ***Sequence 5'→3'*** | ***Anneal. Temp-*** ***(°C)*** |
|---|---|---|
| GALe1bR (SEQ ID NO:1) | GCTCACCCCTCTCTCCCCTG | 60 °C |
| GALe1bF (SEQ ID NO:2) | CATTGATGCTGGTGAGAG | |
| GALe1aR (SEQ ID NO: 3) | CTGAGGTGGACGGTCAAGGACAG | 60 °C |
| GALe1Af (SEQ ID NO:4) | GTAGGACTGAATAGCTACTAATAC | |
| GALe2R (SEQ ID NO:5) | CTGAGATGGCATACAGAGAGTAC | 60 °C |
| GALe2F (SEQ ID NO:6) | CTCTGGGTGAGTGATTTGCTTG | |
| GALe3R (SEQ ID NO:7) | CACAGAGCTGTTACCTGCTTGG | 60 °C |
| GALe3F (SEQ ID NO:8) | GCTCTGTGGTTTCATTAGCTTTC | |
| GALe4R (SEQ ID NO:9) | GCTATAAAGCAAACAGTGTGTAC | 60 °C |
| GALe4F (SEQ ID NO:10) | CAATAAATCTGAGGAAGAAAGAAATC | |
| GALe5R (SEQ ID NO: 11) | GTGCACACATCTGTAATCATATG | 60 °C |
| GALe5F (SEQ ID NO: 12) | CAATGGGAGAGGACACGAAGTAC | |
| GALe6R (SEQ ID NO:13) | GAATCGACGCAAAAGGACGTG | 60 °C |
| GALe6F (SEQ ID NO:14) | GAAATGGCAGGGGACAGAGAC | |
| GALe7R (SEQ ID NO: 15) | GTAAAATCTCAAAAGCAATAAAGAAAG 60 °C | 60 °C |
| GALe7F (SEQ ID NO:: 16) | CAGAAATGAACAGGCAGGCATG | |
| GALe8R (SEQ ID NO: 17) | GCAACATCTCACTTGTGCTTG | 60 °C |
| GALe8F (SEQ ID NO:18) | GATACGTGAGTATTTTCCTGTTCC | |
| GALe9R (SEQ ID NO:19) | GATATATTCTCTTATCACATGGG | 60 °C |
| GALe9F (SEQ ID NO:20) | GGCTATTGTATCGTCTATCAC | |
| GALe10R (SEQ ID NO:21) | CTACAGTGTATGCCTAGTCACAG | 60 °C |
| GALe10F (SEQ ID NO:22) | CTGTCTGCCTCTCTTCATTATG | |

### Experimental Results

***FTC patients of family 2 are compound heterozygous of a nonsense mutation in exon 1 and a splice mutation in intron 7 of GALNT3** -* As is shown in Figures 5a-b, affected individuals of family 2 were found to be carriers of a nonsense mutation in exon 1, in which a C→T substitution resulted in a change of the CGA codon of Arginine at position 162 of the *GALNT3* protein (GenBank Accession No. NP_004473 SEQ ID NO:28) with a termination codon (*i.e.,* TGA). Moreover, as is shown in figure 5c-d, the same affected individuals of family 2 were carriers of a splice mutation in intron 7 at position 1524+5G→A from the ATG translation start site (*GLANT3* mRNA sequence - GenBank Accession No. NM_004482, SEQ ID NO:29; *GLANT3* genomic contig - GenBank Accession No.NT_005403).

***Homozygous splice mutation in intron 7 of the consanguinity Druze FTC family 1** -* Sequence analysis of all 10 coding exons of the *GALNT3* gene from the Druze family revealed the presence of a homozygous G→A transition at position 1524+1 (from the ATG translation start site GenBank Accession No. NM_004482, SEQ ID NO: 29), resulting in the disruption of the intron 7 donor splice site consensus sequence (Figures 5c-d).

Noteworthy, all three mutations (*i.e*., 1524+1G-A; 1524+5G→A, R162X) were excluded from a panel of at least 290 chromosomes derived from healthy unrelated individuals.

Furthermore, as can be seen in Figures 6a-b, a complete co-segregation of the mutations with the disease phenotype was confirmed by PCR-RFLP in both FTC families.

***Analysis and Discussion** -* The nonsense R162X mutation is expected to result in a non-functional null allele due to premature termination of protein translation. Mutations 1524+1G→A and 1 524+5G→A alter the same splice donor site in intron 7. In contrast to the normal splicing score of 0.93 obtained for the intron 7 splice donor site predicted by the Splice Site Prediction by Neural Network software (http://www.fruitfly.org/seq_tools/splice.html), the calculated score of this sequence carrying a G→A mutation at position 1524+1 or 1524+5 was 0.00.

***The 1524+1G*→*****A splice mutation results in an absence of exon 7 from the GALNT3 mRNA transcript** -* To further assess the consequences of the 1524+1G→A splice site mutation, the expression pattern of *GALNT3* was assessed using RT-PCR. As shown in Figure 7, while a normal band of approximately 600 bp was detected in skin and blood samples of a healthy individual, no wildtype *GALNT3* transcript was detected in RNA from affected individuals. In addition, low amounts of an aberrant splice variant were detected in RNA. derived from both skin and blood samples. Sequence analysis of the aberrant shorter transcript revealed the presence of a shorter *GALNT3* transcript lacking exon 7 nucleotide sequence (Figure 8). This shorter mRNA transcripts results in an in-frame deletion of 44 amino acid residues in the mature polypeptide, destroying most of the linker region located between the catalytic domain and the ricin-like domain of the glycosyltransferase.

Altogether, these results demonstrate that deleterious mutations in the *GALNT3* sequence underlie the molecular basis of FTC.

***Analysis and Discussion** -* Since the original description of FTC more than a century ago by Giard, J.M, 1898 (Sur la calcification hibernale. Compes. Rend. Seanes. Soc. Biol. So. 1013-1015), the pathogenesis of this disease has stirred up a large number of investigations but has remained mostly elusive.

The results presented here suggest a role for GalNAc-T3-mediated glycosylation in the control of phosphatonin activity. Although the NetOGlyc 3.0 software (http://www.cbs.dtu.dk/ services/NetOGlyc) identified potential O-glycosylation sites in FGF23 (setting O-glycosylation score significance at >0.5), this molecule is unlikely to mediate the deleterious effects of *GALNT3* mutations in FTC. Indeed, impaired FGF23 activity in a murine model was recently shown to lead to prominent bone tissue abnormalities [Shimada, T et al. J Clin Invest. 113, 561-568 (2004)], which are absent in FTC patients. Of note, FGF23 circulating levels measured by ELISA (Immutopics, CA) were significantly elevated in 6 FTC patients (1710 + 864 RU/ml) as compared with 6 healthy controls (56 + 38 RU/ml), possibly reflecting a compensatory response to hyperphosphatemia. Thus GalNAc-T3 may affect phosphate homeostasis by modulating the activity of another phosphatonin or PHEX [Jan De Beur, 2002 (Supra)]. Alternatively, it may directly regulate non-circulating elements within tissues where *GALNT3* is expressed such as the skin, where calcium deposition occurs [Steinherz, 1985 (Supra); Slavin, 1993 (Supra)], the bone, where candidate phosphatonins are expressed [Schiavi, 2004 (Supra); Quarles, 2003. (Supra)], and/or the kidneys and gastrointestinal tract, where phosphate transport occurs [Jan De Beur, 2002, (supra)]. Given the existence of more than 20 ppGaNTase isoforms [Ten Hagen, 2003 (Supra)] substrate specificity and/or functional redundancy may account for the restricted nature of the FTC phenotype despite *GALNT3* widespread tissue expression.

GalNAc-T3 may not be the sole regulator of phosphate homeostasis in peripheral tissues. Prince et al [Prince M.J. et al. Ann Intern Med. 96, 586-591 (1982)] established 2 decades ago that FTC can also present with normal phosphate levels. Using haplotype analysis in four families with normophosphatemic FTC, this FTC variant was excluded from linkage to 2q24-q31 (not shown), suggesting that normophosphatemic and hyperphosphatemic FTC are non-allelic disorders.

In summary, these results establish autosomal recessive mutations in *GALNT3* as the molecular cause of hyperphosphatemic FTC and demonstrate the pathological consequences of a genetic defect in a mucin-type O-glycosylation pathway. The identification of the FTC gene should not only benefit the affected families, to which molecular testing can now be offered, but may also shed new light on the mechanisms regulating phosphate metabolism in health and disease, with obvious implications for the treatment of acquired disorders manifesting with hyperphosphatemic calcinosis, such as chronic renal failure.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art.

## Claims

1. Use of an exogenous polynucleotide encoding at least a functional portion of *GALNT3* and/or an exogenous polypeptide including at least a functional portion of GalNAe-T3 for the manufacture of a medicament for the treatment of a disorder associated with hyperphosphatemia, wherein said functional portion of GalNAc-T3 comprises a polypeptide sequence including amino acids 188-374 of SEQ ID NO: 28 and/or amino acids 507-629 of SEQ ID NO:28.

2. Use of an exogenous polynucleotide encoding at least a functional portion of *GALNT3* and/or an exogenous polypeptide including at least a functional portion of GalNAc-T3 for the manufacture of a medicament for the treatment of familial tumoral calcinosis (FTC), wherein said functional portion of GalNAc-T3 comprises a polypeptide sequence including amino acids 188-374 of SEQ ID NO: 28 and/or amino acids 507-629 of SEQ ID NO:28.

3. The use of claim 1, wherein said disorder associated with hyperphosphatemia is selected from the group consisting of hyperphosphatemic calcinosis, hemodialysis and chronic renal failure.

4. A method of diagnosing familial tumoral calcinosis (FTC) in an individual, comprising identifying in an mRNA sequence encoding *GALNT3* or a genomic sequence region including said *GALNT3* gene of the individual at least one nucleic acid substitution resulting in downregulation of an expression level and/or activity of GalNAc-T3, thereby diagnosing familial tumoral calcinosis in the individual.

5. The method of claim 4, wherein said mRNA sequence encoding said *GALNT3* is set forth by SEQ ID NO:29, and whereas said genomic sequence region including said *GALNT3* gene is set forth by SEQ ID NO:33.

6. A method of diagnosing familial tumoral calcinosis (FTC) in an individual, comprising identifying in a GalNAc-T3 polypeptide sequence of the individual at least one amino acid substitution capable of downregulating expression level and/or activity of said GalNAe-T3, thereby diagnosing familial tumorale calcinosis in the individual.

7. The method of claim 6, wherein said GalNAC-T3 polypeptide sequence is set forth in SEQ ID NO:28.

8. The method of claim 6, wherein said identifying said at least one amino acid substitution is effected by an antibody capable of differentially binding to at least one polymo^{y}h of said GalNAc-T3, said at least one polymorph includes said amino acid substitution capable of downregulating said expression level and/or said activity of GalNAc-T3.

9. A method of identifying an agent suitable for treating a disorder associated with hyperphosphatemia, comprising exposing a GalNAc-T3 polypeptide or cells expressing said GalNAc-T3 to a plurality of molecules and selecting from said plurality of molecules at least one molecule capable of upregulating the expression level and/or the activity of said GalNAc-T3, said at least one molecule being the agent suitable for treating the disorder associated with hyperphosphatemia.

10. The method of claim 9, wherein said disorder associated with hyperphosphatemia is selected from the group consisting of familial tumoral calcinosis (FTC), hyperphosphatemic calcinosis, hemodialysis and chronic renal failure.

## Patentansprüche

1. Verwendung eines exogenen Polynucleotids, das wenigstens einen funktionellen Teil von *GALNT3* codiert, und/oder eines exogenen Polynucleotids, das wenigstens einen funktionellen Teil von GalNAc-T3 umfasst, zur Herstellung eines Arzneimittels zur Behandlung einer Erkrankung, die mit Hyperphosphatämie verbunden ist, wobei der funktionelle Teil von GalNAc-T3 eine Polypeptidsequenz umfasst, die Aminosäuren 188-374 von SEQ ID NO:28 und/oder Aminosäuren 507-629 von SEQ ID NO:28 umfasst.

2. Verwendung eines exogenen Polynucleotids, das wenigstens einen funktionellen Teil von *GALNT3* codiert, und/oder eines exogenen Polynucleotids, das wenigstens einen funktionellen Teil von GalNAc-T3 umfasst, zur Herstellung eines Arzneimittels zur Behandlung von familiärer tumoröser Kalzinose (FTC), wobei der funktionelle Teil von GalNAc-T3 eine Polypeptidsequenz umfasst, die Aminosäuren 188-374 von SEQ ID NO:28 und/oder Aminosäuren 507-629 von SEQ ID NO:28 umfasst.

3. Verwendung nach Anspruch 1, wobei die mit Hyperphosphatämie verbundene Erkrankung aus der Gruppe bestehend aus hyperphosphatämischer Kalzinose, Hämodialyse chronischem Nierenversagen ausgewählt ist.

4. Verfahren zum Diagnostizieren von familiärer tumoröser Kalzinose (FTC) bei einem Individuum, umfassend ein Identifizieren in einer mRNA-Sequenz, die *GALNT3* codiert, oder einem Genomsequenzbereich, der das *GLANT3-*Gen des Individuums umfasst, wenigstens einer Nucleinsäurensubstitution, die zur Downregulation einer Expressionsstärke und/oder einer Aktivität von GalNAc-T3 führt, um **dadurch** familiäre tumoröse Kalzinose beim Individuum zu diagnostizieren.

5. Verfahren nach Anspruch 4, wobei die mRNA-Sequenz, die *GALNT3* codiert, durch SEQ ID NO:29 dargelegt ist, und wohingegen der Genomsequenzbereich, der das *GALNT3*-Gen umfasst, durch SEQ ID NO:33 dargelegt ist.

6. Verfahren zum Diagnostizieren von familiärer tumoröser Kalzinose (FTC) bei einem Individuum, umfassend ein identifizieren in einer GalNAc-T3-Polypeptidsequenz des Individuums wenigstens einer Aminosäurensubstitution, die zum Downregulieren von Expressionsstärke und/oder Aktivität des GalNAc-T3 imstande ist, um **dadurch** familiäre tumoröse Kalzinose beim Individuum zu diagnostizieren.

7. Verfahren nach Anspruch 6, wobei die GalNAc-T3-Polypeptidsequenz in SEQ ID NO:28 dargelegt ist,

8. Verfahren nach Anspruch 6, wobei das Identifizieren der wenigstens einen Aminosäurensubstitution durch einen Antikörper erfolgt, der zum differenziellen Binden an wenigstens einen polymorphkernigen Leukozyten des GalNAc-T3 imstande ist, wobei der wenigstens eine polymorphkernige Leukozyt die Aminosäurensubstitution umfasst, die zum Downregulieren der Expressionsstärke und/oder der Aktivität von GalNAc-T3 imstande ist.

9. Verfahren zum Identifizieren eines Wirkstoffs, der zur Behandlung einer Erkrankung geeignet ist, die mit Hyperphosphatämie verbunden ist, umfassend ein Aussetzen eines GalNAc-T3-Polypeptids oder von Zellen, die das GalNAc-T3 exprimieren, einer Mehrzahl von Molekülen und Auswählen aus der Mehrzahl von Molekülen wenigstens eines Moleküls, das zum Upregulieren der Expressionsstärke und/oder der Aktivität des GalNAc-T3 imstande ist, wobei es sich bei dem wenigstens einen Molekül um den Wirkstoff handelt, der zur Behandlung der mit Hyperphosphatämie verbundenen Erkrankung geeignet ist.

10. Verfahren nach Anspruch 9, wobei die mit Hyperphosphatämle verbundene Erkrankung aus der Gruppe bestehend aus familiärer tumoröser Kalzinose (FTC), hyperphosphatämischer Kalzinose, Hämodialyse und chronischem Nierenversagen ausgewählt ist.

## Revendications

1. Utilisation d'un polynucléotide exogène codant pour au moins une partie fonctionnelle de *GALNT3* et/ou d'un polypeptide exogène comprenant au moins une partie fonctionnelle de GalNAc-T3 pour la fabrication d'un médicament destiné au traitement d'un trouble associé à une hyperphosphatémie, où la dite partie fonctionnelle de GalNAc-T3 comprend une séquence polypeptidique comprenant les acides aminés 188-374 de SEQ ID NO: 28 et/ou les acides aminés 507-629 de SEQ ID NO: 28.

2. Utilisation d'un polynucléotide exogène codant pour au moins une partie fonctionnelle de *GALNT3* et/ou d'un polypeptide exogène comprenant au moins une partie fonctionnelle de GalNAc-T3 pour la fabrication d'un médicament destiné au traitement de la calcinose tumorale familiale (CTF), où ladite partie fonctionnelle de GalNAc-T3 comprend une séquence polypeptidique comprenant les acides aminés 188-374 de SEQ ID NO: 28 et/ou les acides aminés 507-629 de SEQ ID NO: 28.

3. Utilisation selon la revendication 1, où ledit trouble associé à une hyperphosphatémie est sélectionné dans le groupe consistant en la calcinose hyperphosphatémique, une hémodialyse et l'insuffisance rénale chronique.

4. Procédé pour diagnostiquer une calcinose tumorale familiale (CTF) chez un individu, consistant à identifier dans une séquence d'ARNm codant pour *GALNT3,* ou une région de séquence génomique comprenant ledit gène *GALNT3* de l'individu, au moins une substitution d'acide nucléique entraînant une régulation négative d'un taux d'expression et/ou d'une activité de GalNAc-T3, ce qui permet ainsi de diagnostiquer une calcinose tumorale familiale chez l'individu.

5. Procédé selon la revendication 4, où ladite séquence d'ARNm codant pour ledit *GALNT3* est décrite par SEQ ID NO: 29, et où ladite région de séquence génomique comprenant ledit gène *GALNT3* est décrite par SEQ ID NO: 33.

6. Procédé pour diagnostiquer une calcinose tumorale familiale (CTF) chez un individu, consistant à identifier dans une séquence polypeptidique de GalNAc-T3 de l'individu au moins une substitution d'acide aminé capable de négativement réguler le taux d'expression et/ou une activité de ladite GalNAc-T3, ce qui permet ainsi de diagnostiquer une calcinose tumorale familiale chez l'individu.

7. Procédé selon la revendication 6, où ladite séquence polypeptidique de GalNAc-T3 est décrite dans SEQ ID NO: 28.

8. Procédé selon la revendication 6, où ladite identification de ladite au moins une substitution d'acide aminé est réalisée par un anticorps capable de se lier de manière différentielle à au moins un polymorphe de ladite GalNAc-T3, ledit au moins un polymorphe comprend ladite substitution d'acide aminé capable de négativement réguler ledit taux d'expression et/ou ladite activité de GalNAc-T3.

9. Procédé pour identifier un agent approprié pour traiter un trouble associé à une hyperphosphatémie, consistant à exposer un polypeptide GalNAc-T3 ou des cellules exprimant ladite GalNAc-T3 à une pluralité de molécules, et à sélectionner à partir de ladite pluralité de molécules au moins une molécule capable de positivement réguler le taux d'expression et/ou l'activité de ladite GalNAc-T3, ladite au moins une molécule étant l'agent approprié pour traiter le trouble associé à une hyperphosphatémie.

10. Procédé selon la revendication 9, où ledit trouble associé à une hyperphosphatémie est sélectionné dans le groupe consistant en la calcinose tumorale familiale (CTF), la calcinose hyperphosphatémique, une hémodialyse et l'insuffisance rénale chronique.


### SEQUENCE LISTING

<110> Sprecher, Eli
   Bergman, Reuven
<120> COMPOSITIONS AND METHODS FOR TREATING DISORDERS ASSOCIATED WITH ABNORMAL PHOSPHATE METABOLISM
<130> 29405
<160> 33
<170> PatentIn version 3.2
<210> 1
   <211> 20
   <212> DNA
   <213> Artificial sequence
<220>
   <223> Single strand DNA oligonucleotide
<400> 1
   gctcacccct ctctcccctg 20
<210> 2
   <211> 18
   <212> DNA
   <213> Artificial sequence
<220>
   <223> Single strand DNA oligonucleotide
<400> 2
   cattgatgct ggtgagag 18
<210> 3
   <211> 23
   <212> DNA
   <213> Artificial sequence
<220>
   <223> Single strand DNA oligonucleotide
<400> 3
   ctgaggtgga cggtcaagga cag 23
<210> 4
   <211> 24
   <212> DNA
   <213> Artificial sequence
<220>
   <223> Single strand DNA oligonucleotide
<400> 4
   gtaggactga atagctacta atac 24
<210> 5
   <211> 23
   <212> DNA
   <213> Artificial sequence
<220>
   <223> Single strand DNA oligonucleotide
<400> 5
   ctgagatggc atacagagag tac 23
<210> 6
   <211> 22
   <212> DNA
   <213> Artificial sequence
<220>
   <223> Single strand DNA oligonucleotide
<400> 6
   ctctgggtga gtgatttgct tg 22
<210> 7
   <211> 22
   <212> DNA
   <213> Artificial sequence
<220>
   <223> Single strand DNA oligonucleotide
<400> 7
   cacagagctg ttacctgctt gg 22
<210> 8
   <211> 23
   <212> DNA
   <213> Artificial sequence
<220>
   <223> Single strand DNA oligonucleotide
<400> 8
   gctctgtggt ttcattagct ttc 23
<210> 9
   <211> 23
   <212> DNA
   <213> Artificial sequence
<220>
   <223> Single strand DNA oligonucleotide
<400> 9
   gctataaagc aaacagtgtg tac 23
<210> 10
   <211> 26
   <212> DNA
   <213> Artificial sequence
<220>
   <223> Single strand DNA oligonucleotide
<400> 10
   caataaatct gaggaagaaa gaaatc 26
<210> 11
   <211> 23
   <212> DNA
   <213> Artificial sequence
<220>
   <223> Single strand DNA oligonucleotide
<400> 11
   gtgcacacat ctgtaatcat atg 23
<210> 12
   <211> 23
   <212> DNA
   <213> Artificial sequence
<220>
   <223> Single strand DNA oligonucleotide
<400> 12
   caatgggaga ggacacgaag tac 23
<210> 13
   <211> 21
   <212> DNA
   <213> Artificial sequence
<220>
   <223> Single strand DNA oligonucleotide
<400> 13
   gaatcgacgc aaaaggacgt g 21
<210> 14
   <211> 21
   <212> DNA
   <213> Artificial sequence
<220>
   <223> Single strand DNA oligonucleotide
<400> 14
   gaaatggcag gggacagaga c 21
<210> 15
   <211> 27
   <212> DNA
   <213> Artificial sequence
<220>
   <223> Single strand DNA oligonucleotide
<400> 15
   gtaaaatctc aaaagcaata aagaaag 27
<210> 16
   <211> 22
   <212> DNA
   <213> Artificial sequence
<220>
   <223> Single strand DNA oligonucleotide
<400> 16
   cagaaatgaa caggcaggca tg 22
<210> 17
   <211> 21
   <212> DNA
   <213> Artificial sequence
<220>
   <223> Single strand DNA oligonucleotide
<400> 17
   gcaacatctc acttgtgctt g 21
<210> 18
   <211> 24
   <212> DNA
   <213> Artificial sequence
<220>
   <223> Single strand DNA oligonucleotide
<400> 18
   gatacgtgag tattttcctg ttcc 24
<210> 19
   <211> 23 <212> DNA
   <213> Artificial sequence
<220>
   <223> Single strand DNA oligonucleotide
<400> 19
   gatatattct cttatcacat ggg 23
<210> 20
   <211> 21
   <212> DNA
   <213> Artificial sequence
<220>
   <223> Single strand DNA oligonucleotide
<400> 20
   ggctattgta tcgtctatca c 21
<210> 21
   <211> 23
   <212> DNA
   <213> Artificial sequence
<220>
   <223> Single strand DNA oligonucleotide
<400> 21
   ctacagtgta tgcctagtca cag 23
<210> 22
   <211> 22
   <212> DNA
   <213> Artificial sequence
<220>
   <223> Single strand DNA oligonucleotide
<400> 22
   ctgtctgcct ctcttcatta tg 22
<210> 23
   <211> 22
   <212> DNA
   <213> Artificial sequence
<220>
   <223> Single strand DNA oligonucleotide
<400> 23
   ggcagttgga gattatgcct tg 22
<210> 24
   <211> 22
   <212> DNA
   <213> Artificial sequence
<220>
   <223> Single strand DNA oligonucleotide
<400> 24
   caacatccag acatagaggc tg 22
<210> 25
   <211> 22
   <212> DNA
   <213> Artificial sequence
<220>
   <223> Single strand DNA oligonucleotide
<400> 25
   ctgctctcca gtgacaactg tc 22
<210> 26
   <211> 22
   <212> DNA
   <213> Artificial sequence
<220>
   <223> Single strand DNA oligonucleotide
<400> 26
   cgacgaggcc cagagcaaga ga 22
<210> 27
   <211> 23
   <212> DNA
   <213> Artificial sequence
<220>
   <223> Single strand DNA oligonucleotide
<400> 27
   tccagggcga cgtagcacag ctt 23
<210> 28
   <211> 633
   <212> PRT
   <213> Artificial sequence
<220>
   <223> Single strand DNA oligonucleotide
<400> 28
   Met Ala His Leu Lys Arg Leu Val Lys Leu His Ile Lys Arg His Tyr
   1               5                   10                  15
   His Lys Lys Phe Trp Lys Leu Gly Ala Val Ile Phe Phe Phe Ile Ile
               20                  25                  30
   Val Leu Val Leu Met Gln Arg Glu Val Ser Val Gln Tyr Ser Lys Glu
           35                  40                  45
   Glu Ser Arg Met Glu Arg Asn Met Lys Asn Lys Asn Lys Met Leu Asp
       50                  55                  60
   Leu Met Leu Glu Ala Val Asn Asn Ile Lys Asp Ala Met Pro Lys Met
   65                  70                  75                  80
   Gln Ile Gly Ala Pro Val Arg Gln Asn Ile Asp Ala Gly Glu Arg Pro
                   85                  90                  95
   Cys Leu Gln Gly Tyr Tyr Thr Ala Ala Glu Leu Lys Pro Val Leu Asp
               100                 105                 110
   Arg Pro Pro Gln Asp Ser Asn Ala Pro Gly Ala Ser Gly Lys Ala Phe
           115                 120                 125
   Lys Thr Thr Asn Leu Ser Val Glu Glu Gln Lys Glu Lys Glu Arg Gly
       130                 135                 140
   Glu Ala Lys His Cys Phe Asn Ala Phe Ala Ser Asp Arg Ile Ser Leu
   145                 150                 155                 160
   His Arg Asp Leu Gly Pro Asp Thr Arg Pro Pro Glu Cys Ile Glu Gln
                   165                 170                 175
   Lys Phe Lys Arg Cys Pro Pro Leu Pro Thr Thr Ser Val Ile Ile Val
               180                 185                 190
   Phe His Asn Glu Ala Trp Ser Thr Leu Leu Arg Thr Val His Ser Val
           195                 200                 205
   Leu Tyr Ser Ser Pro Ala Ile Leu Leu Lys Glu Ile Ile Leu Val Asp
       210                 215                 220
   Asp Ala Ser Val Asp Glu Tyr Leu His Asp Lys Leu Asp Glu Tyr Val
   225                 230                 235                 240
   Lys Gln Phe Ser Ile Val Lys Ile Val Arg Gln Arg Glu Arg Lys Gly
                   245                 250                 255
   Leu Ile Thr Ala Arg Leu Leu Gly Ala Thr Val Ala Thr Ala Glu Thr
               260                 265                 270
   Leu Thr Phe Leu Asp Ala His Cys Glu Cys Phe Tyr Gly Trp Leu Glu
           275                 280                 285
   Pro Leu Leu Ala Arg Ile Ala Glu Asn Tyr Thr Ala Val Val Ser Pro
       290                 295                 300
   Asp Ile Ala Ser Ile Asp Leu Asn Thr Phe Glu Phe Asn Lys Pro Ser
   305                 310                 315                 320
   Pro Tyr Gly Ser Asn His Asn Arg Gly Asn Phe Asp Trp Ser Leu Ser
                   325                 330                 335
   Phe Gly Trp Glu Ser Leu Pro Asp His Glu Lys Gln Arg Arg Lys Asp
               340                 345                 350
   Glu Thr Tyr Pro Ile Lys Thr Pro Thr Phe Ala Gly Gly Leu Phe Ser
           355                 360                 365
   Ile Ser Lys Glu Tyr Phe Glu Tyr Ile Gly Ser Tyr Asp Glu Glu Met
       370                 375                 380
   Glu Ile Trp Gly Gly Glu Asn Ile Glu Met Ser Phe Arg Val Trp Gln
   385                 390                 395                 400
   Cys Gly Gly Gln Leu Glu Ile Met Pro Cys Ser Val Val Gly His Val
                   405                 410                 415
   Phe Arg Ser Lys Ser Pro His Ser Phe Pro Lys Gly Thr Gln Val Ile
               420                 425                 430
   Ala Arg Asn Gln Val Arg Leu Ala Glu Val Trp Met Asp Glu Tyr Lys
           435                 440                 445
   Glu Ile Phe Tyr Arg Arg Asn Thr Asp Ala Ala Lys Ile Val Lys Gln
       450                 455                 460
   Lys Ala Phe Gly Asp Leu Ser Lys Arg Phe Glu Ile Lys His Arg Leu
   465                 470                 475                 480
   Arg Cys Lys Asn Phe Thr Trp Tyr Leu Asn Asn Ile Tyr Pro Glu Val
                   485                 490                 495
   Tyr Val Pro Asp Leu Asn Pro Val Ile Ser Gly Tyr Ile Lys Ser Val
               500                 505                 510
   Gly Gln Pro Leu Cys Leu Asp Val Gly Glu Asn Asn Gln Gly Gly Lys
           515                 520                 525
   Pro Leu Ile Met Tyr Thr Cys His Gly Leu Gly Gly Asn Gln Tyr Phe
       530                 535                 540
   Glu Tyr Ser Ala Gln His Glu Ile Arg His Asn Ile Gln Lys Glu Leu
   545                 550                 555                 560
   Cys Leu His Ala Ala Gln Gly Leu Val Gln Leu Lys Ala Cys Thr Tyr
                   565                 570                 575
   Lys Gly His Lys Thr Val Val Thr Gly Glu Gln Ile Trp Glu Ile Gln
               580                 585                 590
   Lys Asp Gln Leu Leu Tyr Asn Pro Phe Leu Lys Met Cys Leu Ser Ala
           595                 600                 605
   Asn Gly Glu His Pro Ser Leu Val Ser Cys Asn Pro Ser Asp Pro Leu
       610                 615                 620
   Gln Lys Trp Ile Leu Ser Gln Asn Asp
   625                 630
<210> 29
   <211> 3874
   <212> DNA
   <213> Artificial sequence
<220>
   <223> Single strand DNA oligonucleotide
<400> 29
   atggctcacc taaagcgact agtaaaatta cacattaaaa gacattacca taaaaagttc 60
   tggaagcttg gtgcagtaat ttttttcttt ataatagttt tggttttaat gcaaagagaa    120
   gtaagtgttc aatattccaa agaggaatca aggatggaaa ggaacatgaa aaacaaaaac    180
   aagatgttgg atttaatgct agaagctgta aacaatatta aggatgccat gccaaaaatg    240
   caaataggag cacctgtcag gcaaaacatt gatgctggtg agagaccttg tttgcaagga    300
   tattatacag cagcagaatt gaagcctgtc cttgaccgtc cacctcagga ttcaaatgca    360
   cctggtgctt ctggtaaagc attcaagaca accaatttaa gtgttgaaga gcaaaaggaa    420
   aaggaacgtg gggaagctaa acactgcttt aatgctttcg caagtgacag gatttctttg    480
   caccgagatc ttggaccaga cactcgacct cctgaatgta ttgaacaaaa atttaagcgc    540
   tgccctcccc tgcccaccac cagtgtcata atagtttttc ataatgaagc gtggtccacg    600
   ttgcttagaa ctgtccacag tgtgctctat tcttcacctg caatactgct gaaggaaatc    660
   attttggtgg atgatgctag tgtagatgag tacttacatg ataaactaga tgaatatgta    720
   aaacaatttt ctatagtaaa aatagtcaga caaagagaaa gaaaaggtct gatcactgct    780
   cggttgctag gagcaacagt cgcaacagct gaaacgctca catttttaga tgctcactgt    840
   gagtgtttct atggttggct agaacctctg ttggccagaa tagctgagaa ctacacggct    900
   gtcgtaagtc cagatattgc atccatagat ctgaacacgt ttgaattcaa caaaccttct    960
   ccttatggaa gtaaccataa ccgtggaaat tttgactgga gtctttcatt tggctgggag   1020
   tcgcttcctg atcatgagaa gcaaagaagg aaagatgaaa cctacccaat taaaacaccc   1080
   acttttgcag gaggactttt ttccatatca aaagaatatt ttgagtatat tggaagctat   1140
   gatgaagaaa tggaaatctg gggaggtgaa aatatagaaa tgtctttcag agtatggcaa    1200
   tgtggtgggc agttggagat tatgccttgc tctgttgttg gacatgtttt tcgcagcaaa   1260
   agccctcata gctttccaaa aggcactcag gtgattgcta gaaaccaagt tcgccttgca   1320
   gaagtctgga tggatgaata caaggaaata ttttatagga gaaatacaga tgcagcaaaa   1380
   attgttaaac aaaaagcatt tggtgatctt tcaaaaagat ttgaaataaa acaccgtctt   1440
   cggtgtaaaa attttacatg gtatctgaac aacatttatc cagaggtgta tgtgccagac   1500
   cttaatcctg ttatatctgg atacattaaa agcgttggtc agcctctatg tctggatgtt   1560
   ggagaaaaca atcaaggagg caaaccatta attatgtata catgtcatgg acttggggga   1620
   aaccagtact ttgaatactc tgctcaacat gaaattcggc acaacatcca gaaggaatta   1680
   tgtcttcatg ctgctcaagg tctcgttcag ctgaaggcat gtacctacaa aggtcacaag   1740
   acagttgtca ctggagagca gatatgggag atccagaagg atcaacttct atacaatcca   1800
   ttcttaaaaa tgtgcctttc agcaaatgga gagcatccaa gtttagtgtc atgcaaccca   1860
   tcagatccac tccaaaaatg gatacttagc caaaatgatt aagtgttcct taaaattaag   1920
   ttgaaaaagg aaatattctt tctcataaaa ctgtgactag gcatacactg tagtttttga   1980
   aaattatgca aaagcagcta aatgtaactt attccaagtg catttttctt atttatatct  2040
   ttatgtagca ctactacaga aattctgcaa gtttctgttt caaagcacaa taactagtaa   2100
   taccaaagac tatttcaaaa tgtccagatg taggggaaga gatgtttaca gtatgatgaa   2160
   aataattttc caagtaaagt gatgtttgtg tgttttgtac acttagggat atatatatat   2220
   agctacattc acacactcac aatttaaaat atttccccta gttttttggg gggataggaa   2280
   gaaagatttg ttactgtatt tttttaacta cataaaaata gatcaataaa tgtcagcatt   2340
   ggcctctgtg tacaaaccaa gagcttttac agatccagaa tttattagtt taaaatgcag   2400
   gtgaactttt ttttgcgttt ggtttacttg tctgtcaaat gtttccttaa acatgaaact   2460
   gaataaggag aagagtattt ttaacactta aatttcttgg caaattttaa aacatttttt   2520
   agtctgtaat acactccact tgaagcactt aagtcttcct taaatgactt ttcttaagta   2580
   atgatactgt gtgttttccc aaagcacttt taaaaaaatt tttataaatt actatctgtt   2640
   gaaaaggtgt ccttttcctt tcttctagta ttttttttct taccaaaatt cactaatctt   2700
   gaatgtttgt gatattaaat ttcaaatgca gaatacttga ctcatttaaa gctaaatttt   2760
   gttactgatt caattataat tgtaatggat ttttgacttt gtaatggatt cttttcatca   2820
   aaaagcctta ttatttttta tctatgtgga aaacacaata aaaaatcctc aacactattg   2880
   taatcatttg gttaagtgct tattcctctt ttgggtaaaa tctgtaattg ataataggtg   2940
   ggggaaaatg aattttgtat gctgaatttc taagcgccta ttgtttgtaa aaccatcaga   3000
   tatttcttat ggcacaaaaa atgaggaata gcaaaattcc tgtgttcaat atttagaaaa   3060
   ttttgtatta atttctgata aagttcctta agcatctgat agaatgatgt tttaaaaaaa   3120
   tttgacgctt gcttaggaga tttaccactt tttttttttg tttttcgtca ttttatattt   3180
   agatctcctg tattcttgtt cccgaagtaa aatacgatcg gtttcatatt ttaaatctgg   3240
   cagagcctca gctgtacgaa aaagagcata tactggttat tgaccctatc ttctcattgt   3300
   ttgtttgtaa gtttgaattt gtattaaaaa gcctgcattc tgagctggac atggtggctc   3360
   agcttctaat cccagcactt tggtaggcaa aggtgggagg atcatttgag ctcaggagtt   3420
   ccagaccagc ctgggcaaca tagcaaaatc tcatctctac aaaaagtaaa aattaaaaaa   3480
   tgaaattaaa aataaaatta cctaggtgtg gtggcacgca tctgtagttc cagctataca   3540
   ggaaggtgag gcagaagcat tgcttgagct tgggagatcg aggctacagt gagctatgat   3600
   tacaccactg cacttcagtc tgtgtgactg agcaagactc tttcaaaaaa aaaaaaaagc   3660
   ctacattctc cagttgatta tttccaacta atgtgtatta tgtgcctaat tttctatcag   3720
   aagttgtatt aagcccgttt tcacactgct gttaaagaca tacctgagac tgggtaattt   3780
   ataaagaaaa ataggttcaa tggacccaca ggtccgcgtg gctggggaag cttcacaatc   3840
   atggcggaag gtgaaagcat gtcttacgtg gaag                                3874
<210> 30
   <211> 19
   <212> DNA
   <213> Artificial sequence
<220>
   <223> Antisense oligonucleotide targeted against the GALNT3 mRNA
<400> 30
   ctggcacata cacctctgg 19
<210> 31
   <211> 19
   <212> DNA
   <213> Artificial sequence
<220>
   <223> Allele specific probe
<400> 31
   ctttgcactg agatcttgg 19
<210> 32
   <211> 19
   <212> DNA
   <213> Artificial sequence
<220>
   <223> Allele specific probe
<400> 32
   ctttgcaccg agatcttgg 19
<210> 33
   <211> 157775
   <212> DNA
   <213> Homo sapiens
<400> 33
   ttaaaaagat ctttaactat tataaggttc atataattaa tatagaggaa tcatccaatg     60
   atacttatga agggaaaatg acctattttc cttcaccact ctgaggacct aactcagtaa    120
   atgcagagga ggctgaggaa acatggaaga gacacgacct cagcaaccag ccttttctcc     180
   agcgtgatca atccatctct acaaaatcag tgtataatga gatttccact tagtgactag    240
   ctgattgaag gggggcctct acccaaatac tcaactaggc ctttcttttc tgaagcattt    300
   tgatttgtct tgcctgggac aactagcaga acagtccaga atgcattgca tactttctaa    360
   ttacctcaca ttctcttatt aaagagaaca gatagaaata aaacatgcac tccccacctt    420
   tagattacct gcacttggtt cgtcggagga cttctaggat cccgtttccc tgtaaattaa    480
   tttaggtaac taaatagtgt aattctgcct ttttttttcc aatgcaatat gactatgcta    540
   aacctgtttc atatttttga ccttctattg ttatatcact tcacatgttt tataactatt    600
   tgaaatgcgg acatgacttg ctattcagtg actgcatcgc tacttatttt ttatgtcttt    660
   tagacttggg tgtttagaat tatggatata aatatattgt agatctcaat ttctaaaaaa    720
   aatattttat ttaattgaac ttaaaaatgc ttgatctaga gaaagtcctc cagtggaaaa    780
   gtttctacta aactttgttt tggggaaaaa atgtttactt ggcagctctt ccactgtgca    840
   gttggtttct gccccttctc ttccccccta cattgggcca aatttataac tcttaatatt    900
   aacattaaaa gagctttggg tttgtcttta tgtaggttgc agtaatgaga gcagggttgt    960
   tccttttgaa tgattggccc tttgggctaa tcagaatgag gatttgttga aatctcaaat   1020
   atacatatat atatatatat atatatatat atatatatac acacacacac acacatacac   1080
   atatatatac acatatatgt atgtgtgtgt gtgtgtgtgt gtgtgtgtat atatatatat   1140
   cccatgttgt ctgcaaagtg acagttttta gtgcttaatc tccaaaattt tccccctact  1200
   gatacaatct aaagagcaca ggcccaaaga gaaaatgaca agaggctatt gttccttcat   1260
   tcaagcacat gaaaggatca tgtgtactgt atttgctgga ccattacaag ctcttgaagg   1320
   atctgcttgt gaagtactct gaggttattc ttggaacaca gagtgtggga gagtggggaa   1380
   gagccagctc cagcctgagc aaagctgaag ggtagaatga cagtccagtt tctgaaaaga   1440
   gatattttaa ttttagttca gctttatttt cctaagacga tatgaagcct acttttttat  1500
   ttttggctag gtctttgttt tctagctttg tttccaaaag gatgcttaag aaaacagagt 1560
   ttttttcccc tatccattta tctagggtag aaagtcactg ccctgtgaga agaattaccc 1620
   ttccaatcat gacagcttct attccaactt ttcatatacc tgactaacct gtaaacatat 1680
   cccacaaacc acatacacag gctacctatt aaaactctcc catataccta acacatacac 1740
   atgcacacac atacaaaata tatacatatc acacacttag agaatgacag atttcaggca 1800
   atggccaata gtcagtattg agtttttcta accccagaat ctggttgggc tttatgatct 1860
   aaaaaaatgt ggtttagaaa agaggataat tggtagagct gtagtttgaa aatatcaggt 1920
   caaagttgca aacatactta tgatggaatc atttcatttt tagttccttt cccagaaaag 1980
   ctgagagagc acactgttcc agctgacttt tttttccatt aatagccgtt ctattcaaca 2040
   aagagctgcc cacatgccag gaaagtgcta cagtccttct aagcttttaa aaatggggat 2100
   caagctacat gcagccagtt ggaatactta agatctttat gccaagacac aataacctga 2160
   cctgcagctg ttgtattgcc tccatagtat atctagccaa actgtagtct ttgacacaca 2220
   actttttagt cattcactat caattgcaaa gcttcatgct ccagccctgg ctattctagt 2280
   accagtccta tacctttaaa tgggacttaa ttcccagtta taatcctggc cgaattgatt 2340
   tgaaagtcaa gtggctgaag cagcttgcat ctcctttgtc attcccaatt tcctttggga 2400
   gaagtcaatg aaagagattt acttaagttt ttcctcctta tctcttgatc attaattatt 2460
   aagacatggg taaaattcat ttgtctcatg tcttttggtt gattgaataa tatagttact 2520
   ttttgaaagc ccatcatttc tttgtgataa aaccattttc tcaatgtgac agccacatgt 2580
   aaaatgtgaa actaatgttt cttagtatgt ttaaaatctt cacaatcata tatggccctg 2640
   caatactccc tgaattcctt ccctccaaaa aaaaactatg agttgcaaaa acactagtta 2700
   cgaattaatc aagaagcaaa gagagtatgt taagtggcta tgttctttaa aattttacat 2760
   tctaatagat gacttctagg caaaattttg gtatgattac cattaaacag gaaaacatac 2820
   catgatttac tttctataac ttgctaatca ctgtttcttt tatttcaatt tttgatattg 2880
   ttgcacaagt ttatgactct ttggtcttta tattcaaaat ttattaagta tcaattcttt 2940
   ctttcaacaa atatgtactg aacacatgct gtgttagaac aactgtcttg ggtagtaaaa 3000
   agaggggaac actacaaatt ataatggata atattaatga agggtggcat gaaataatca 3060
   agctttctca aatcctctta atatgtgatt ttatcaagtg acattagaaa aactgacatc 3120
   agttcttgtc taattatatg tgagctgcaa ctaaaagatg cttcctttac ctaaattata 3180
   gaactcactg gatcgccctt catctctctc cctccatctg aaatgtatga attaaatcag 3240
   gactcagcta ggactgaatc aaatgaaaaa gaaattttcg attaattgct tcttcaatgt 3300
   tagacctata taagtgatgc taaatttaca ccagcaaact cctatcagac agtttcaagg 3360
   aggatttgac tcatgcataa cccaggaaac atttcaggaa acttttaaga caacagtgtg 3420
   gaacacaatc actcttactt ctatttataa aaagcctatc aaaacagcaa aatcctgttg 3480
   caaattccaa agacatcact ttgcagcagg tggaaaacag agaggttgtt agactccatc 3540
   actttcagct ggagttacaa accatcaaat tagcaggtgg aaaatttata ttctcagaag 3600
   ccaacaggag actgcctttt taaaaggtgt catgaagcct tctatttctc caaacaactc 3660
   aatatttctc tttaaaatgg catctctgtt ccttattctc ttgatgacag tttgcagtgc 3720
   cattatagcc agtgtattaa ataccaggct ttaatgggaa gcacctttgt gctttcaaag 3780
   gcaagacttg tctgtaattt taaattagaa ctgttggttg tattcttacc acaaccaaaa 3840
   cccaaagttt aaaggcttga ttactctata ttaggtctaa tatgaatttt caccttctga 3900
   ttgcattact ttttctgaaa tctgctaact agtgctggaa taacaaaaat gcctaagcca 3960
   aaatgctgta gctctgcacc aacagcacag ctcatcagat gttttctata gtagtaaaga 4020
   atttgattga cttaattgaa tatcagcaat tttaataccc actagaatta tggaagtatc 4080
   agagtggaag tgaggacgca ataaaagctt aataagtggt ggtgtcttct aggaattatg 4140
   aaaaaaaaag ctcagcagag ctaagccaga tcttattaca tcataaagac tagagttaca 4200
   aatggcagcc ccaaacctag aaggggcagt tacaatgtgg acaccctttc agcccagttg 4260
   gtgctcacat ctgctgacca acattcatat caattacatt tttacagtct gtaagtcatt 4320
   tgatgcttta gaaaataaaa acacacacct acagcattct aaagaaacta ttttgttaaa 4380
   aaatttaaac acattttata ttaaaataag tgatcaatga ctttcatgat tgtcaaaagt 4440
   aaaaagtgca gacatttaaa aaagctcttc tttcacattt tacctaccaa atctagatat 4500
   tcaacgtgag tccttaaaac agtcacttct aaattttatt ttgactcctt tcttggcttt 4560
   ttcaaactgg accatgagac ccccttccta aacaatgcag ttgttttgtt ttattctttt 4620
   tttccagaaa ttcaagctga aacatttgat atgacctgtg ttttacctgg tgatcagttc 4680
   tcaggctagt ctaagtctgt gctgtcttgg catgccttgg gtttcctatc ctgagagtgg 4740
   taatgccagt agaatgatgg gaaccaggag agtgaatacg accagattca aagcctaaaa 4800
   caatatgttt cagtttactt ttatatgtag attttttaaa tattcacatc tagcctggaa 4860
   ttttagtaaa tacagatact tgttgtatcc atgggaaatg aggtcatttc aaagatgtaa 4920
   gtcacgttgg tgactacttt cttttacttg tgataaatat atcactatac agggtaattg 4980
   cttttataaa tttgatcctt tgcattggct tataattcaa gaaatgtcaa cttcactgtg 5040
   tcactttatg tcatctctca taaaagtttc tggcaggact aaagtttcaa aggaatgtga 5100
   tagaatttat tttggataac atttgtattc agcatgctaa tgatgtaact cctctcttta 5160
   tcattaccac ggtgatagtt aagttcattc gttaactcat tttagtgaca tgtgaggcca 5220
   tttaaatatt tttaagataa tgtatattga ttagaaaggg ttagaaggag acaaatttat 5280
   ggtagaaatt agccttgcca ttgtctaagt taatttatgt gacctgattg aacagtttta 5340
   gttgtaactt tagagtagct tctttgttag aggatctctt tcctttctct tttggttttc 5400
   tcccaatatt tttatctgtc ttgggattat cttaaaaggt atttattaat gagtatttgg 5460
   aagaaaattg acctagaaaa tgtctctttt aaatttatct aggtacttgt atcaccagag 5520
   accttcatca agtcctactg ccacactgga taaggccact acccccgacc cttcttgtca 5580
   gggacatggc ttcctaataa tggttttgct gttgtctcta cataggggac ctatggaaaa 5640
   ggagtttgaa atttctacct ttgtagtaca ttgtatccaa ttttgttttg ctcctccttt 5700
   tcatgtggtc accaagcttt tttccttagc cattaaaaaa gccacccctc tttaaaacaa 5760
   atttaaagat ctatatacat ttttgcaact ttatttaata aaaaagagaa gaaaaaataa 5820
   aatctaaatc tcaattaccc taaagtggaa aaacctaagt atctaaccta ggaacttgtt 5880
   gcctggtggt ggttgttgtt gttgttgttt tttaatttat ttagtgttga tccttgagtt 5940
   atttaatggt atccaaatga tatctgaaag tgattgattt taagatgtaa tagttggcaa   6000
   agtcatttca tttgtgtctc aaacaaataa agcccactgc catgggttac catctgaacc   6060
   ctgattcatt aggcactagt catgccaaat ggggatctca gaatagttta atcttttcaa   6120
   agatactcac aatggccaac tgtgtgtgaa tactggaaag tttgagattc ttctatattc   6180
   ttcaaattta tggtcctgag actaggaggg atctccagag gtcatatggc tcatcgatct   6240
   accaccaggg aagactgtca cttaggcatg cccaacagga gagatcgcca tctattctta   6300
   acatctctag ggaaggcagt ttccttggca acacaattca gctctcttag gttttctcac   6360
   aataagaaaa tatcttggcc agtctttgag ggttaggcac ctggcaacag ttgtggtgat   6420
   ttgggccatt gtactagggg attcaaggga agttgggttt ccccctacat ttggtgatta   6480
   ttacattaat atatgatcat tccataagtc tcctaagaaa tcagattttt aaagaaagac   6540
   acggattcag agacaatggt ctgtaacatg accaactttt gacaaatatg gctgtactag   6600
   gttttcattt ttgctgttgt tgttgtttgt ttttgtgatt gctcttatgt cggtaggtaa   6660
   tgtcaggaaa agtgactgtt tcagatcccc agtacaaaca tcagaactag gatcaaatga   6720
   cttgactttt aagctgttcc attttctaag aagttacaat tacaaaaagt aagcattttc   6780
   tacattctta cttctaagta atgaccttct cattaatctt tccttggctt aaaatgacaa   6840
   ataagaatcc aaagtttttc cactcctcct gtgggagata ggaatagttg tgaagagtct   6900
   gatggaataa aatgactcca gagatttaca cagccaccat ttgatcccag cctttgtcct   6960
   tgtgttattt aaacaatggc ttcctgcttt ctcaggggtc aggaaaatga tttaaaaatt   7020
   tgtcttcttg acaaggcctg aaaacgaagg gaggtgtcgt ggagaaatgg tgatttttct   7080
   ggttacatca cttgattcaa gcactccctg gagctttcaa agcctttttt atctgctcag   7140
   agttgacacc caactcagag tcagaactca ccagaagatg aggaacaccc ccgaaatcaa   7200
   tgtttccttt ctccatggga tttttttcaa tcttggtaca gctgcatatc tcccaaactc   7260
   ggatgctctt gtgaaatgac agccacatca gtgtggttac tccccacaaa taacactagg   7320
   gggaaatgac ctttttattt taaaaaaaaa aaaaaggcta atgctataga atgatcatgt   7380
   aaagaatgtt gaataaaatt tggcccatgt tttaattgac tctaggcacc tctattgaaa   7440
   taagtctgag gcatagtttg tgaaacatgt ttcaaaagtg tttgatgtat aataaatgct   7500
   agaaatttac agtacagaaa tagtaaaaaa ataaaataaa aataaacctg tagattacag   7560
   ctctgttcct aagaacctca gcctttattg tgtcttagta aagttgatct gctttatttt   7620
   tagtttattg aatttctggt gtactagcct cttaagatag aacttttaat acatatgtac   7680
   tggttaatgc ctgtttacgc agaaaatggc actttgttct ttcagtgtgt ttccctcagt   7740
   gtcacagggt atatcaattt gatagatcat ttgacatttg tcaaaaatac gtttcccatt   7800
   ttaaaaatat gtataattgg ttgcatagac atatgtcact ttttgcagtg tcttcatctt   7860
   gatcatagtt ttagacatca actatgtctc actttccaac tgccatagaa atttggtaca   7920
   tttgcaaatt ctggtggaat ctagtgagct gaatgtattg gtagatcaaa tgatccaaag   7980
   gacttgggtg ttatccttca gtctgtgttg aaccattcag atattgggaa tggtccttac   8040
   acaagggaag tcttttgaga atatttgttc tcttattttt tgtttgtttg ttgctggcaa   8100
   aaaaaaaagt aactttaaaa actcagaaaa gccactgaac tgtattttgt gaccttatga   8160
   ttagttttat ctttcaactg atttttattg ttacttttac tcaatatcaa cagtacttca   8220
   atagatattt attagttatg ttcaatctaa tgacttaaaa cagttgaaaa ggaggaaaat   8280
   caactccagt ccacacatac agtcatgtta cattaagatg tcatgttgta tgtcacacag   8340
   tttctagatt gatttctctt gtgtataagt tataacatca aaaatgccaa agggtatata   8400
   aaataagcta caataatatt caacagaact taacctggac cttatgttgt aataatttat   8460
   tcatattagc tgtagtcttc tgtatttata ttttcagtat ttattatgaa tgacatggaa   8520
   attcgtgtat ttattgtggc tttttattgc agtgtgtata tatatataac aaataagcat   8580
   ttttaagtct tatccttaag tttcttttat tagtggcact tgtattatgc tgtacttttt   8640
   attacatatt gtacaatatc ttgtccattt gtttgcagca gagtaaaacc ggtttctaag   8700
   ttgtatctac tgttgcattt ctgttgcctg ttgtgtaaaa cctttgttct tacatcctga   8760
   actgcaatcc gttacgctgt cccagcacac acaaggatac ctcccttcca ctgtgtaaca   8820
   gataatattc ttgaaaagtt gtgtgtgaag tcagttcctg tgaaatggtt cattacattg   8880
   tcatttcaaa tatgtgttaa ctttattggg atttatccct aagtgaagat gcttatagca   8940
   ctttactttc tcagctattc aaccccttca tcaagtgatg atgtgtaaac aaacacaagg   9000
   gccagaggag aactctttat gtaaatgaaa tttggaggct atttttacaa ggacaaattc   9060
   aatctcttgt aagtagtatc ctttcactta actgcttagt agtatttttg ttttgctttg   9120
   cttaaagcag aatctgtgtg atttgatgtt tagaatatat gcgtgtgcat ggtacgtata   9180
   tgtgcccaac tgtttaaagc tactggctta aggtaactaa aacttaactt ttattggcag   9240
   taggatgaag gaaaaaaaat tgcatttttt taaatctcct atcatatcca agacactttc   9300
   ttccaacaac ttgttttata tttagagact ggatctatat ttttaaaagc aaataataat   9360
   atgtgatttt gtgatcatcc taaaactcaa atttgaacat gttattcact gaattaaaca   9420
   tattttgtgg tgttccctcc tcactgccaa caaaacaaaa ttcagactcc ttggaatgcc   9480
   tcacaaggtc gcctgagatc tgggtacaag ctctcccttt acttcccacg tcttctgctg   9540
   cccacaagag cggttcctta gatttcacct gctagtccat tttccctgct tctgttcagc   9600
   ttgtctcttc tgccaagaat atacacccca taaacctatc ccgacagcac aattctagtg   9660
   accatctatt tatttatcaa gagaaaacac aaatattctt tcctttatgt tgcccttcca   9720
   atactcctga aaatcgtttg cctgttgttt tatgcaaagc agacatgtat tagagcccct   9780
   tcgagtctca ctgcactagc ttatttatgc atctactccg cctcagtact tcctgagaac   9840
   agaaaacact tctcattact ctgtcttctc agcattcagt ttagtgttta aaacatggta   9900
   gagacttaaa atacatttac tgatgtatgc aaagtaaaaa attgagtgat gcaaagtcca   9960
   ctcctgtata ggagttcaga tgagggaaag atgattggct gaaacagtgg aaaaggtgtc  10020
   agattcagag agagggagaa tttcagctag cagggttacc cccatagtcc acattatgga  10080
   acaatttgag gaaaggctga tcagaacaac atcactcttc tggtgggggg cagaaggacc  10140
   tggttaagaa cagataattt actatcaaga acaaatgaca aatcaggtta ggagagtgca  10200
   tttagacagt gcaaagcttt gaagaccaac ccaaggaatc tggctcatat ccttagggaa  10260
   ataaggatat ttccttcagg aaatcctgaa gattttagaa aaggaaaatc aatcaaataa  10320
   gagtgatttg cagtatggaa tatcagtgtg gctacagtgc acagacagtt tgggaggatg  10380
   gaaatgccag aggaaaaatc tttgcagtag ctattgcagt cagtattcat tagaaaaaat 10440
   aagagttaag aacctactct gatttaggat actgtgcaag aatgtgctac aatgaggagg 10500
   gtgtggggga aaagacaggc agagtccctc tagacaagca agtagaaaga tgtgggtaat 10560
   tacagcagag aaaaagtcag ctatttgaaa gcctattacg atgtgagaga ttaaaggtaa 10620
   caaaataatt atgagcctaa aataccatag gtggaatatg gggaaggaca taagaagata 10680
   aatatcatca acataaatta gaatgttata aggaggaaga gatgtgttca ttttggacag 10740
   cttgaattga tgctgggata ctcagtgaaa ataacaaatg gacggaagga aatgtaagtc 10800
   tagagcttag acaaagacgt aagatttgga gcgaatgaga attttaagaa gagaatatac 10860
   atatatatat atagaaggaa ctaaataaca gacaggactg aagctttaga aaggctagtg 10920
   tttcaggttg tgtttgctgt aagcacacaa ttctgagatg gagtttagca tgcaggattt 10980
   ccatgaggaa tcaattcttg tgggagctag cgggaggaag cagattagga aaagacagaa 11040
   atcaggctgt gatgcggacc gaccccactg gagtgctcta gaggataagg gatcctctga 11100
   gtcatcccct cgcgggctga aatggctggg cctttatagc cacgccacag tcagtctctg 11160
   gatgtcttcc accccagaaa gggagtgctc tcagatgagt tggcaccctg cagtgaggag 11220
   aaagcctgaa aagagctgag agctggagga tgtttttctg acagcgctct taggacctgg 11280
   gacaagtcct tccttgaaag ggaatttttg aaggtaaaac agctctgtgt cttctacatc 11340
   catccctcag gtcctcagat tcatgtcatc acttaaattt cagggaacag ctccttcagg 11400
   attctggtgg gcctctctcc ctgggggaaa acttaaagga gcaaggttag taaaataaat 11460
   tacagttcct gccactctgg ctggtcctaa agccacaact aaaactgacc atctccctct 11520
   ctcactatta attctacatt cttctcagct tcatcaaaca cagttgctag cctcaatggc 11580
   ttacttggtg gtgtgaccca gagccaaaag gcacctaagt gaatattact tcctgctcct 11640
   attatgaaac agtaggccca tctcttcctg aggatcaagg tcaaatacct ctaccaagat 11700
   ggtgaggtat tttttctcct tgcctaaaaa tcctaggatg tagcaatagc ttaagtccaa 11760
   tgggactctt tagtcccctg ataaaaatgt gacctctttg gggactagga cctttaagac 11820
   tgcaaagaca tttaacccta tagagttgtg gaataggaag cacagttttc tccagtgggt 11880
   catgttaagt agagacattc ctgcttccac ccagtagagt ttgcacccat gtgttcttcc 11940
   tatgggggac atattattat gtaaaggttt tttattcaat ctatatactg gatcctggag 12000
   gatagtaacg aattcatgcg gaacatcatc tccaaacttg cacttcaact gcaccttcta 12060
   caggccagtc aacgctctgt caggccggga gctttgaagt ctatgatatg atcagtgggt 12120
   ctcatggtca tgtgcccatg ctctgatatg atgttatgtg ggatcccatg tgagcagatc 12180
   aaatatactg ttagtccttg aataacggtg tttgccaagg ccctgcaggt aagaatggca 12240
   aacttatact tggaatatat gtctattttt atttaaaaca aacaacaaat aaaaacacaa 12300
   aaaatctctg gcccttttaa gatggaaggg gcccaatgta atcaatctgc caacaagtgt 12360
   ctggttaggc tccagaagaa tagtgccata tggagggctc agcatcagtc tctgttgctg 12420
   gcagactgta catttggcag cagcaggagc tttatcagtc ttggtaagtg gcaggccatg 12480
   ctcatggacc caagaagagc cttcgtggct cccactgcca cccaatccat gtacctatca 12540
   cagcagccct ggagtagcca ataactgagg ctggctgctt gctgccaacc ggccaagtta 12600
   ttctgtctat atcaatacta tcaaaaaata tatataatgt gagctacaaa tgtgagctgc 12660
   atatgtaatt ttaagtgttc tcatagccaa cttaaaaaga gttaacaaaa agataaaatt 12720
   aatttaatga tatatttcac ttaacccaat atataaaaaa tttcaacatg taatcaatat 12780
   aaaaattatt agtaatattt tacatacttt ttggaagact aagtctttga aatccagtgt 12840
   gtattttgca attacagcac atttcaattt ggagtagcca cgtgtggcta gtgcatacaa 12900
   tattggacaa cacaggtcca caccattgtt taatatgtct tctgcagtgg gtgctctcta 12960
   gttgataggt gttaacatgt gacacaaaga tcttcacact ttgtgtccac tctcacatgt 13020
   cattcacatg cttatactcc aagactccta ttccccaatc ttctgatatc tctgctttca 13080
   gcatcctgag ccaggctatt cacttctacc caccagtcca tatatattct aacagagctc 13140
   ctatttattc ttccacacaa agagaataac cgggtggacc ttaaagctct acctatttta 13200
   aggatttttc tctagcccca tgagctactc ctgaacaagg ctgcagtgta gctgccatgc 13260
   atttttggct tgtttggcat ccacctacta aaccagccca tctataaacc aaatctgaag 13320
   gctttttcct cctccagcag tgagccatgt ggcatcctcc atatggagtg agggatgagc 13380
   tgtggaagag gttcagggac aacagtggca aatgacatgg aagatgggac tgtcttgtca 13440
   tgcagctttt tttgtgtctt ccagtcctat ttatgctcaa cccaagtttt ctacttctag 13500
   aatccatctc ttaaagttct gctagccctg ccttaagata cgtggttcta caggtgcagt 13560
   gccttggtgt cccatagcag gcacataatc tccatgaggc ttcagtaata tatcaggagt 13620
   tgtttctcaa aaggcataca aataactgct acagatggca aagctttaat cccatagcct 13680
   atgttgggat tctaccactg gcacttgaca taacctccac acagcatctt ttcccaccac 13740
   tgctaccact aatatcacgg tgtctgctgg cctacatgac ccaacaacag ggcttggtgg 13800
   actgcaacca atacctccta ctgagatttt tcctgctcta cgtctcactc aaagctggca 13860
   gccttttaca ttgcacaaca tacgggtcag agcagtattc ctgaatgaga aatacgttat 13920
   caaatctaga acctgaagaa gcttaccaag tgttgtgctt ctcttttcat ggtgggagtt 13980
   gcagcatgta ataatgtatc tttactatgg aagagacaac ctgaactccc cacaccacag 14040
   aatccctgaa gactatatta gtgtggcagc ctccgacctc ttcaaagggt ttatctccta 14100
   tcctccattt tgtgtgtgtt tcaccaatgc caccggcatg ctgacaagtt attgctcatc 14160
   tggcctaatt agcatagtat cataaaggag tggtattctg ttggaaatct agagagttga 14220
   gatctctttg tgctatatta tgacagaata caaaactaaa tatatattgt tgcctattcc 14280
   acgcacatgc aaacaatttc tgatcctctt tttctaattg gaatagaaag aaagacattt 14340
   gccattcaca tgacctcatc catgttactg aggttggtta acccactcta gcaaagatac 14400
   tacatctggc acagcaactg cagttgaggc taccaattaa ttgaatttgc aataatctac 14460
   tctcatttcc caggatctct gtgttctgca agagcaagtc actgcattaa gacaacttaa 14520
   gtggggatat gatagcaacc accaccctta tatcttttag gttcctaaag gtactaataa 14580
   tttccactat tccccatgcg atacaacttg tattttattt actaccttga tttgggtggg 14640
   ggatatgaac agtttcagaa acatccactt ggctctcccc acttctatag cctctgtccc 14700
   actcactaaa ggttcagtgt tgggattgca tgtattccaa acatgagaac ttcaattacg 14760
   tacttggaac cagggaaaat gaccactgtg tgggtcctca gacctagtgg acttactgca 14820
   agccagatct ctgccaggag tctgtttttt tacctggttc ttatatgctc ccactttaga 14880
   acaggagggc cccatggtaa ggcttcagtt ctccaggtat caatgtccac tcagacccag 14940
   tgtcaaacag tcctaaaatg ttttgagtgt ttccctttcc cttgtacaaa gtcacccaag 15000
   taaacagttg taggtacttt taaagatgga ctgggggaat catcatcata atacttgcac 15060
   tgtgtagctg ggtctttctt cctagggacc tggccatttc tttagtcagt gggtcctggg 15120
   tctgaaaact aactcagatc tggaaagtgg gcaaaagact acaacatctt attggcatga 15180
   ctgccttatt ggagtgagtt ccttcaataa ttagggcttc taattattta ccctaatttc 15240
   tcgtgatggt ataaaccaaa gtatccttgt tggctgccca tctatttagt ccctagggtt 15300
   atgagtttct attaaccatc tccatgactg tctgtgggtc aggccccatt ggctgccact 15360
   ctggctctgc tgctcattat gataattgca tccacctgct tttcagcagt tatgtggcac 15420
   ctcctgttct ctgttgtttc agggtctttt catttccatt ggattatttg tgcctcagta 15480
   ccatcagtga tggagttcac attactagcc aagtgacaaa tgataaagct tcaaaattta 15540
   tcttatcaac ttcatttttg gactattctg ataccaaatg tcagattggg tttttaaaga 15600
   ctctaaaatg gagttggact tgcaagatat ttatgaaggt tcaacagctg tggaagccag 15660
   gaggaggaag caatcaaaca gagagacaag atgaattatg aagcaggcgt gacagagcct 15720
   cagcccaacc ctcagagaac tccagagcca tatgaccctt tggagttgtc ccctagtgag 15780
   ccaaaatttt cagatcttta tatgtcctcc atggtcagtc attggatatg gatagctaag 15840
   gaaggttggg cttttgaaca aggcatcact ctgcaactga ggtcatctct gaaaaagaag 15900
   taacaagtca gccctgaaag aaacctgagt ggtgcatctc catatccatc acagccagat 15960
   ttgggattca aaagaagaaa ataaagacat gattttgtat ccaagagaat ccagagtagg 16020
   agttaagaga gcaatggaaa gtcttgagct cgggttttaa aaaggaacat gtgtatttac 16080
   cagatcagga attttccttc ctgaaaatca gatcacacat ggaataccaa gttcaattat 16140
   ggctactgca gtttaaaagg aatatccact tactgaaatg tgttcagagg agaatgatca 16200
   aaacagtaaa ggagctaaaa aacacaacaa agggctagtt aaaggaatga atgcttacac 16260
   agaatacaag ttttctttag acagctaaag gggcttgaaa ttattccctg tggaatcaag 16320
   agacaaaagt ctaggaaaac agaagttaga agtctaggaa gacagaatat agggaagata 16380
   attttaatat taaaaccaaa agaaaaagta ctgtagatat aaattagcca gatgaagggg 16440
   aaaagaggca ttttggatca aagcagccat cacaggcaac cgtgaaaagt tcagactggc 16500
   caaatataag accaatagaa agtggccgga agggaaaatg actatgaagt caatgtcaga 16560
   tcatcaagtt ccttgtatct catgcttagg actttggcta ttatgcagtg aagtagtgcc 16620
   tggcttggca cttcaaaagg tgaatctgac agcaaggaag ataatggatt agggagaggc 16680
   atgagataga aagcaggtaa accaattaag ggtagctatg atcaatagaa ttcatgtctg 16740
   tctttctgct cattgtattt gcagtacctg gcattgaacc ttcatataaa agaccctcaa 16800
   taaatatcaa atagatgaat gttcgaggaa agtaccaacg gttgtctgaa ctaagccaat 16860
   ggctatggga atacagaggg gttaacattc aaaagatctg tagaaggtgg aatggaaatt 16920
   aaaggagagg aagaagtcaa ggataataga tttgttgatt tgttcctagt ggcatacctg 16980
   ctgtggacct gaaatactga aggaaaagtg gtcggtgggc aggtgaaggg aaggaaaaaa 17040
   agattgctaa aggtctcatt tatcatctat gattaggctc tatctgttgc tgaaatttct 17100
   caacaaagtc tctactaatg tgctttctca tcgatgtttt ctatgcacaa gttccccgtg 17160
   attaccagca ccctgtcagt atagaacagc ctgtttcttt gcttttctct ggtaaagcaa 17220
   agcagcaagc caattactga gatcattcag atgaaaattt tacttttgcc tgaattagcc 17280
   agagaattga agcataaatg gaccttttca cttgactgat tgagataatt gcctcacttt 17340
   tctaggggag acctatagct gggaggctgt atttggtcac caaaacatgt ttgtaatata 17400
   cattattgac tttcttgaaa caactctggt tggctaggtg ccacactcaa acacttgccc 17460
   tcatcttttt aggtatttga aacaatacag agaaatacca tgcctcggaa actcagtgtt 17520
   gaattatttc attcttgggc agcctttaaa cacatcagct caggcctcca gaagaggtaa 17580
   agtggcacct aggggagtct gggagaaggc ggcagggcag gagcttggtt cagtggataa 17640
   aatgcaagaa gtgagaatta gctccaggtg catgggaaca aatggctttt cctgaaagaa 17700
   ttgggtaggt agacttgaaa gaaacatgta atttacattt actattggga ggggtgagag 17760
   gggttaataa agagagctga gtaggggcag acaattaaag cagcagcagg gctcagatca 17820
   gagccttttg gcctggatct tgggtaatag aagcattttc taacagcatg agacatggga 17880
   aaagaaggct tctaaagaaa cagcacagca tctgagcttt tctgcagctt ttgctatctg 17940
   gtggcctgct atgacagcct gggtgtgtac agacaatgtg tggtcagtac tgacctacaa 18000
   aattcactga aaagcaagat gtgtattaga aaaactcgta caaacttcaa gtctatgagg 18060
   aaaggttgtc atgtaaatgt gtctgtaatt actttgtgat ggctcccctt actggccaac 18120
   gggcagaacg tcctagatcc tgaagagaag taggaggagg taacgctgag gcaacctggt 18180
   taatttctaa gtttcctgta ttaaaatata tatatatata tatatatatg tgtgtgtgtg 18240
   tgtgtgtgtg tgtgtgtgtg tgtgtgtgtt gctaaaataa attttattat aaacttattt 18300
   tattaaaata agttttattt tctgagtatc aaataaaaac agaaatccca gccatgatat 18360
   ttataaaatc caccagtatt atttttaaat tttttatatt tatttatctt cagtgggtgt 18420
   ttagagtctg cgtatggttt tgtgactata aaaaatatgc ctgctttaat ttttcaattt 18480
   ttaaactttt atattacata taatttcaaa agcaatttga ggtgatggat gagacatacc 18540
   cacacatcag gattgatata tccaaacaag ctgcattttg gggagagatt atatgcttac 18600
   aataaggagg ttttcattgt ttaaagcaac cttgattttt tttgcattgt ttcattgttt 18660
   ggaatattgc tttaactgtg gcaaatagat atacattaaa agtatattca acatcttgta 18720
   atatccatag atcattcaga atcaactatg ttgaactagt tgaactagaa aagttaaaat 18780
   gttgtatcca aaataataaa aatgatgcac ctatcttcta aacttttccc caggattccc 18840
   aaagaataat cttaaaaatg tcaaataatg ttcccacagg ctatgtatgt tgctgtttat 18900
   ttaaatataa gtactgggat attttatttt aaagatgttt tgtttgttaa tatctcttta 18960
   taggtgtcat aaatattttt atgtatataa aaattggtct aagaggacca attgaattgc 19020
   tagtatcaga ccaatttttt gctttaatat aaacccagac tgacatatct tgtttacaaa 19080
   caaataatat tggcatgaaa aaagtcacac cattacacag attttttatt taagctgatc 19140
   ctgcaataga taggatactc ttgaaattag cactttacat taaaataatt aattttcttc 19200
   agatttttaa aattctttct gtactttttt tcatatatgc acagtattag aagattctaa 19260
   atttgttgct gtagtaacaa aaacccctcc aaacctcaat ggcttaaaac atggtttcac 19320
   tccatgtctg tcttcagtgg gtgaggtacg ccatcatcct ttttcaggtg ctaagactga 19380
   tggagcctcc accatctgca acattgcagg caggggaggg aaatcagctt cttgtgccct 19440
   gcttcttaaa ggtttctaac tggaaataac acatgtcact tccgctcaca ttgcactggc 19500
   caaaataagt tacatggaca gaaatagaac tgcattttac ttccttttgt cttaattgat 19560
   cccatcaaag atgctttaat ctttcagaga aaaaaaaaga aatttgggtc atgctgtcct 19620
   acaatgtgtt tggaaggaga acagaaacat tgctgaacag caccaataac tcccacattc 19680
   accctaactg aggaacttta tcttaatgcc gggaaagggg acagtgcttt cacagctgag 19740
   aatccaaaat agtgacagga tgtagggaaa cttatatttt gatttctttt ttaacagaaa 19800
   gaggcctaac attatatttg ctggttaggt ttaatgtctg aacttctttt caagtcttac 19860
   tttttactac aataaattgt gcttaaattg agctctctgg ggattccaca gggattgatc 19920
   gatttgcctg gttagggctt ttcaggatgg atgtcaggag tggggcaatg gcatgagaac 19980
   agctgttttc agggctctgg cctcaatctt gagcgttgcc atagtgacag ctgcccgcag 20040
   gaacccaccg ggagcccagt aggagaactg gctgccagag ctttgtatca gtgtgccatc 20100
   cccagccttt cagaaacaag cgtaatgaac tctttagcca gggtgtttct gcctactaac 20160
   ctaagggaca gtttattctt gcttgaattg tcaactgcca gatgtcagtt caggcaaatc 20220
   aactctattc cctctttcct cctgtaccct ttctctcagc ttattttcat atcaaattcc 20280
   acttttcccc attttatagc tacataatta acagctatta atagagcaaa ttcaattcat 20340
   aaatttagat ataacaaaaa aatccaaaga tacaaagtcc tctaacacag cactgcccaa 20400
   tagaattttc tgtgatgata taaatgtttc atatctatgc tgttcaatac agtagccact 20460
   ggtcaccaag tggcaactca gcatttgaaa tgtggccagt gcaactgagt aaccaaatta 20520
   tttaactgat ttaatttgag ttcattttaa ttttaaatag ctacacgtgg ctaggactaa 20580
   tgtattgagc aacacggttc tagaatcgta ttcaaatcca gggagtggta ccagctagag 20640
   ctggccaggg aggagtcaca gttagcagca ttctctcatt caaagtagcg tgtttattat 20700
   ctgctctaga gcatattatt attattgctg tataaggtgt gccctggcct gccctaactg 20760
   gctgttgtaa atttaattat gtagctaaag aggaggaaac ggaggcatga ttatctgggc 20820
   cgttacaggc aatatcctct agaagcaaga ttgaaaggga aaaggtgtta ctatttctga 20880
   ataatatatc agccacatta cagtgattct acaaaagaac tggattctaa ctggaagtca 20940
   tcaattctag tcccaaattt tatgctagta aattgtatta cttaggaagg acactcagtg 21000
   ggaggcttgt gtgtgtgtgt gtgtgtgtgt gtgtgtgtgt gtgtggcttt atttatttat 21060
   ttttgcctaa attcctcctc tgtataaaag ggagcgtaat aatattctct gtgtgcttat 21120
   taatatgaca ggggctttat gtaagtctca tttattcctt ttaagaaatc cataaattag 21180
   ggatttgtat gcagagtata tagatgagga aactgaagct tagcagagtt aagtgcctaa 21240
   gatttcccag ctaataattg gaagaatcaa aattcattca aactcaagtc tgtctgtctc 21300
   taaaacccaa tttctttcta ctaaatccca ttagaatgtt tctaaaatcc cttctaactc 21360
   ttaatcatca ctaagtgagt gccatgtaat aaatatattg aatgagtttt gcgtacaacc 21420
   caaaatagga ttcttaactg tatttcattt tttaaaacat agtaacaact gtaactacta 21480
   gttatttcct aacctggaac cacttaggat ttcctaatca gtggctttct cccctggctt 21540
   tgcattagaa ttatttggag agttttttaa aaataccgat acgaggtatt cttctcaaat 21600
   aacttaaatc acaatctgca caatggggcc tagagacagt ttttttcttg gttgtttttg 21660
   gtttgtttgt ttttgaggag ttttttgttt tgttttgttt tgtttgagac agggtctcac 21720
   tccatcaccc aggttggagt gcagtggcat gatcatagct cactgcagcc tcagcctgct 21780
   gggctcaagc aatcctccca tgtcagcctc ccgagtagct aggactacag gcatgcacca 21840
   ccatgctcag ctaattttaa aatttgctag cgatggcttc tcactctgct gctaaggctg 21900
   gtctcaaact tctggcctcc agacatcctt ccaccttggc ttcccaaagt cctggaatta 21960
   caaatgcgag ccaccatatc cagccaggat aatttgtttt taagtctctt atatgatctc 22020
   aaagtgcagg ttgtgctgag aaccgtttcc ctaatgtaag gtgtagaaat ctacttttcc 22080
   attgagccac ctgctaggac ttcaggacct ggcctccagg aggaagcccc ctgatagctt 22140
   ggttgatttt atcttatcag tttatgttag gggaaactgc caaattgaaa taagaaagcc 22200
   atctcccgca agcaagcctc tcctcctaaa gactaatcat aatgcaagag aaattttcca 22260
   tttctctcag gaatcagaat cctttgctac atgcacaact ggaaggaaag tagtacaaat 22320
   aggaggatcc ttacctgatg ggagaaggga gatcttgcca ttcaggagtt tgctttgaat 22380
   ctctttccac gtgcatgggt gctgtcacag tacatgtgac aggctgcaac agggtttgag 22440
   agggttcctt gctggaaggc aatgtcatag gaaaacaggt tttatgaagc agtgattccc 22500
   ccaaggagca aaggagttga ataaatagga tagttcctag actagaagag caggcttgcc 22560
   tacactctca cacccaaacc aactgccttc tgttgttctt tttttgaccc aaattctatg 22620
   cagaatcttc tcttatcaaa ataaataaat aaataaataa tgactgcaaa caaaaaactc 22680
   aatagctcct agtattactt aacttttgca tgattttaac gtgtcaatgc ttatcttaaa 22740
   gcactgtttt actaatttca ttcctatact tatcctaaaa gatctttagt gacgtgcctt 22800
   ttctcacagc aggacgcttc aaacttgggg gagtttattt aaaatgcctt tttctggact 22860
   ccattcctgg aggtctagaa tgggatgcat tcataatata tatgttaagt gttccaggaa 22920
   atcaataggt gattgggata ggctaaataa tggtccccca aaaatgtcct cattgctgtc 22980
   cccagaacct gtgaacatgt taccttcaac gacaaaaggg actttgcaga tgtgatgaag 23040
   gatcttgaga aggagggtta ttttggttat gcaggtggtc acaatgttat cataaggacc 23100
   cttatgagag ggaagcaaga ggatcaaaat cagaaaaaga tgtgatgaca aagccagagg 23160
   taagactgat gcactttgaa gatagaggag agagccatta gtcaaaggat gcaggcaaca 23220
   tctagaagat agaaaagcaa ggagacaaat tctcagatgg tttctaacac cctcaaaaag 23280
   aatgctgctc tgcaacatct tgattttaga cttctgacca ccagaactat aaaataatac 23340
   atttgtgttg ttttaagtca ctaagttggt ggtaatttgt tacagtaata attggaaact 23400
   tgtatagtta ttcacgaaaa gagtattacc ccaaataaaa cactttctat agacatcaac 23460
   ttcaatatct cttattattg taaaggatcc tggagcacta ggagcgaagt aaattgttca 23520
   acttcactga gggacttagg gaaaatccag gacaacagtg gaattatctt agtttggctc 23580
   tatctcttta tcctggcgtt caaggtatct tcccctagtc taggccaacc tttcccacat 23640
   tatcccatta tttctatatc cttacttcta taagcaagca aaatagtgga tatattttcc 23700
   ccctgaataa gccccattat ttctgtttca ctaccttgat acatagactt ccctcttcct 23760
   gaagtccttc cattctcgaa tgttaggttt cactaaaggt cgggtcctgc ctcttcctta 23820
   gctatccagc attaggtgag tactgacctt ggaactctta tggtgtcagc atggttgctc 23880
   tttaaagatc atgatgaaga atccctagtc tatttcttcc tcagtgtgat gatctggttg 23940
   gctctgaggc ttgtggatac ttcctaaagc aatattgaag aaagaatgaa tgactaaaga 24000
   ccttaatcca agtcaagtgc ctagattttc aagttccctc tctttatgaa ctatatttca 24060
   cttatttcca caaagtttta aaaataaata cccaatgaac ttaaaaaaat ttgtagagat 24120
   gagtgtcttg ctttgttgcc caggctggtc ttgaactcat ggctgcaatc ctcccatctc 24180
   agcctcccaa agtgctggga ttatagccat aagccaccac gccagctgca aaaaatttca 24240
   attacatgtt ctgttagtat acatagatat cttcctagtt taacagtaaa aatcttctca 24300
   gttacttgac tgatagatga caactcaata tttacatctc attggggtaa cacaacattt 24360
   taaaacatat ttcttattgg catatacttc acataacata aaattcagtt ttaaagtgtg 24420
   gtattcagtg gtttttagta tattcacaga gttgtctgac cattaccatt atctaattcc 24480
   aaatatttta attaccccca taaagaaacc tgttaaccat tagtagacat tcaccattct 24540
   ccctcctttc tctaagccac tggcaactac taagccactt tctcactcta tggattttcc 24600
   ttttctgaac acttcatgtc aattaatcat acaataatca agtggtcttt tatggctggc 24660
   ttctttcact tcacataatg ttttcaaggt tcattaatgt tgtagtatgt atcagaatct 24720
   catttctttt tacggctgaa taacattaca ttgaatgaat ataccacatt tgtaatccct 24780
   tcatcattga cagacatttg ggttgtttcc accttttagc taatatgaat aacgttattt 24840
   gtgcaacaat ttttgctttc tgttggtatt tgtgaaaata tgttttcaat tctataaggc 24900
   atatatatct ctaggagtag aattgttagg tcataaatca tttgtataac tttttgaggt 24960
   actgccagat tgcttgccac cttttcagtg gctgcatcat ttaacattcc cacaagtaat 25020
   gtataaggat tgcaatttct ccacaacctt gccaacagtt gttattttcc atttttatta 25080
   tagttatgct tgtaggcata aagtaagata tctcattgtg gtttttcgtt ttctaatgga 25140
   taatgatgct taccatcttt attggccatg tgcatatctt ttttggaaaa aatggctatt 25200
   taaatctttt gcccattttt taatagtttt ttttaaattg tagagaacat agcctttaaa 25260
   attaaaataa gagtccagaa gttgggctat atttcctagg tgttagctag tatcttgtcc 25320
   ccttttcata gatttatttg tgtatctcag gttatcagtg ccactataga tctcctccct 25380
   cacagcaatc aaacttagga cttcatccag aaatgggtgg agagaccact ctcccagctc 25440
   caaaaatgct taccgggatg gtcaatgact gcctccttct tgttgctcag ccctctgaga 25500
   gtccttggct ggctgtgcag tttgctgcac ccagttactt tctttctttc tctctctctc 25560
   tctctctttg tttttgagac agaatctgat tctgtcatcc aggctggagt acagtggcac 25620
   aaacatggct cactgtacct tggacctccc aggctcaagc gaccctccca ccttagcccc 25680
   ccaaatagct gggactacag gcgcatcacc acacgtggct aattttggta ttttggaaga 25740
   gatggagttt caccatgttg cccaggctgg tcttgagctc ctgagctcaa gagatcctct 25800
   ggcgtgggcc tcccaaagtt ctgggattac aggtgttggc catggcaccc ggcccactct 25860
   gttactttct attgcattgc ctgttacctt attttatagc tttcataata ctcatcatct 25920
   gatatttctc atttgtttgt ttgctgatta tctcttcctt ttggaagcgt gaattccatg 25980
   aggccaagac attgcctgtt atgactgact gtgtgactga tgctgacaca gtgcctgtca 26040
   catggtagac tggggctgag gttcagttcc caggggtctg gctcctcagg aagtagcatg 26100
   tgtcaaggga agatgtgtga catggggaag aactttctag aggagttacc aggtcctcca 26160
   gtattagact cctttatttt actttttaaa aatgttttga agaaaatctg gcaaactaca 26220
   agcatactca tcttaatagt aattatcaga agcaagtcca gattccggaa actaatttaa 26280
   ctcaatgaat gtgttaaact agttcaagat taatcagaaa cgcttagatg tctttattct 26340
   ttctgcaatc ctaaatgaaa cataataaac actttaggct aaatttttgc atagttttaa 26400
   gctcatcatt tgcatagtag tctcattgct accacagata gaaaagataa ggtctgaatc 26460
   tgtattgctc cccaggaaac tacattttag cagcacttac cgagtagtca cacccatcta 26520
   aattcaaatc ctagctctgc cacccacctt caggaaaaac ttggccaaga tgtttgactt 26580
   atctgagtct gtcccatgtc tcactgaggc tttcccaagt ggggaaaatg gagccagtaa 26640
   tacaggccta cagagctgtt tgaagattat aaacaaaaca ttgcccacaa taagggatca 26700
   ataaatgtta gctgattttt ctctctttaa atttctgttt cagctacaac tctgttatgg 26760
   gtaggtgtga gggaaagaaa ttaggtaaaa aaaaatctaa agaaaaaaac attggggaaa 26820
   ataaaataag aattctcatt tagtttgttt catgcgcgtc cgtgtgaaga gaccaccaaa 26880
   caggctttgt gtgagcaata aagtttttaa tcacctgggt gcaggcgggc tgagtccgaa 26940
   aagagagtca gtgaagggag ataagcgtgg ggccatttta taggatttgg ggaaggtaaa 27000
   ggaaaattac agtcaaagag ggtttgttct ctggcgggca ggagtggggg tctcaaggtg 27060
   ctcagtgggc aggagtggga gtcacaaggt gctcagtggg ggtgcttttt gagccaggat 27120
   gagccaggaa aaggactttc acaagataat gtcatcagtt aaggcaagga ccggccattt 27180
   acacttcttt tgtggtggaa tgtcatcagt taaggtgggg cagggcatat tcacttcttt 27240
   tgtgattctt tagttacttc aggccacctg ggcatatacg tgcaggtcac aggggatgcg 27300
   atggcttggc ttgggctcag aggcctgaca ttcctgcctt cttaataaga aaaataaaac 27360
   aaaatagtgt tgaagtgttg gggcggcaaa aatttttggg gggtggtatg gagagagaat 27420
   ggacgatgtt tctcagggct gtttcaagcg ggattagggg cggtgtggga acctagagcg 27480
   ggagagatta agctgaaggg aggtcttgtg gtaaggggtg atattgtggg gatgttagaa 27540
   gaaacatttg tcgtatagaa tgattggtga tggcctggat acggttttgg atgaattgag 27600
   aaactaaacg gaagatacaa ggtccaaata aaagaaggag aaaaatgggt attaaaggac 27660
   taacaattgg gaggacccag gacatccaat tagagagtgc ccaagggggt tcagcgtaat 27720
   tacttgcttg gttggcaagt ttttgggctc tatccttgag ttttttaatg ttgtcataca 27780
   ccaggccaga ttgatttagg taaaaacaac actcctcatt taagaatatg cagagtcctc 27840
   ctttttcagc agtgagtaag tcaaggcctc ggcggttttg gaggacaact gcagctaaag 27900
   agtcaacttg ggcctggagg actgataaag tttgtgatat gtctgtgatg ctagcagaga 27960
   agtcattaga caggctacgg aaggtcatga cagacgttga aatgcctgct attccagtac 28020
   cgagagcaac agtggaggca gaaagtccta aaccgaccat caagggaatt agtggaataa 28080
   ctcttttttg ttgtgtcggt gtcatgaggg gaacagggag ctcttcggtc ccatttgcaa 28140
   attgaatttt gggggtaagg aagactagtg tacatgtgcc tgtccaattg gcaggtaggc 28200
   acatgtaggt agaggatcca cagaggaaga agagaccttg tgcgaggcaa aactggagat 28260
   gtaaagtaaa aaggtgagaa ggagtgctga aaggggtgtc ttgtacccag actcctaggg 28320
   atccagctag ggcggcagct gtcagaggtt gtaatgggga ctgatggggt aactgcgtag 28380
   aggggaaggt tcgattttca tggtgtatga gaaaacgtcg agtatctacg agcaatcttt 28440
   cactgttatt ttcggggctg ggtataagta aacaagaaga gggcctggga ggagagtctg 28500
   atgagcaagg ggaagatagc caaggatgga gtgaaataca gggcaagtgt cttcctaagc 28560
   aataattact gctaatgttt ttaagtttgt cagtattgat agagggcttg tctgtaatat 28620
   ggagctggaa ggctccaatt gtttcattga tgtttgtagt tggacttcgg agatgaagag 28680
   taaaggaaca tcgagaaggt gaaagattac ctaggggaat tccagtgggt ctttgccaag 28740
   agatacacaa aggagcggcc acaggaatag tagtttgtgt tgtgagaggt ccaaatatgg 28800
   ggggagtaga gttgatataa ggagaaaggt tttttaaata agtgcgaagg agggcggcag 28860
   cttgctgatg tgaaatgtct ggggaagtct tgctggacct gtctagaaag taaatgagtt 28920
   cttcaggagg gtaaaggtga gggctgttaa aggaagttcg gaggtgtagg gagatgggag 28980
   atgttgccca gtctgtctgt aaggcgggga cagctgtgta ggcactggaa gaaagggaaa 29040
   tgcaaagcca gcagttgttc actaaggagg gattagaagc ggctaggaga gaatgggtaa 29100
   ggttgatagt gtggtggaga tagctgggga gaggtagagg atgacataag aatgggaatg 29160
   agaataagag tgagtataaa agtaaagaat agaacttcat cagggtggaa gtattggagg 29220
   gtgccttgcc agcaaagatc atctatccac tctaagacgg agttaagagt ggcagtttgg 29280
   ggatagcacc aagagatatc agctgtgatg gcttgaagaa acagtgtaaa ccggcggtgt 29340
   aaacaagagt agggcattta taagtagttg agaatggaga ataggagtat gaccggacag 29400
   aagataatag ggatgactag tttttggggg cttggcctaa gtggtggggt gacttcgtaa 29460
   agccctgttg caaaaagtag ggtaaggatg aacagaccta atagaatgaa gggatgtatt 29520
   aggctcataa gggttattac tgttcttcag aaatatgagt gagtttaagg gaagtggggg 29580
   agagtacttg cgacttccag gaggaagagg agggattagg ctggctgtcc gatggacaca 29640
   gctttattct ggaatggtga acccagtggg gaggattctg caggcagacg gcagtcgggg 29700
   tactatagat gactaagtag ggtccggtcc atcgaggttg tagagtttga ggggtcagat 29760
   tcttaacaag aactgatcgt ccagctaggt tgtcttcata tggctgggga tctggagtag 29820
   gcaagagaag attagcagcc tggcgaattt cctgtctagc ctgctggagt actggaagat 29880
   agtcgcctag agggctggtg tctgggatga ggttggggcc aagcaagaaa gtgcgtccat 29940
   ataaaagttc aaatggactg tatcctgtag catctcgagg acaggctctg attttgagaa 30000
   gagcaagagg taaaagtgct gtccaatcct ttgtaagttg gaggctaaac ttggtgaggt 30060
   gtgcctttaa aagaccatta gtccgttcca cctttcctga agattgagga cggtaagggg 30120
   tatgaaggtt ccactgaata ccaagagcct gagaaactac ttgggtgatt tggctagtaa 30180
   aggctggtcc gttatcagac tgtatagagg tgggaaggcc aaaccgagga attatgtctg 30240
   acagaaggga agaaatgacc acagtggact tctcagaccc tgtggggaag gcctctaccc 30300
   atccagtgaa agtgtctacc cagactaaga gatattttag ttttctgact cgaggcatgt 30360
   gagtaaagtc aatttgccag tcctgggcag gggcaaatcc ccgagcttga tgtgtaggga 30420
   agggaggagg cctgaacaat ccctgagggg tagtagaata gcagatggaa cactgagaag 30480
   tgatctcctt gaggatagat ttccatgatg gaaaggaaat gagaggttct aagagacggg 30540
   ctagcggctt gtaacctaca tagaagaggt tatgaaatga tgacagaata gaatgggcct 30600
   gtgaggctgg aagaagatat tttccttggt ctaagaacca tttgccttgt gtgggaagag 30660
   attgataggt ggaagtttca gtgggggagt aggtgggagt gactgaagtg aaggagaaaa 30720
   actggccgtg agggacagaa gttggagagc tagctgcttg tctagccact ttatcaacat 30780
   aagcattgcc tagagcaatg ggatctgatg ccttttgatg ccccttgcag tgaatgaccc 30840
   cagcttcttt tggaagtaaa gcggctttga gcagagtttt tattaaagag gcattaatga 30900
   tggaggaccc ttgtgtagtg aggaaacctc tttcagccca tatgactgca tggtggtgca 30960
   ggatatggaa ggcatattta gaatcagtat agatattgac gcatagtcct tttgcaagag 31020
   tgagggcttg agttaaggca actagttcag cttgctgaga ggtagtggag ggaggcagag 31080
   cagtagcctc aatgatagat gtggaagata ctatagcata gcctgccttc gctggtgagt 31140
   ggcgattagg cctggtggaa ctgccatcaa taaaccaagt gtgatcaggg tgagaaacag 31200
   ggaagaagga aatgtgggga aatggggtga atgtcaggtg gatcagagag atgcagtcat 31260
   gagggtcagg tgtggtatcc ggaataatgt gggaggccgg attgaagtcc gggccaggaa 31320
   caatggtaat tgtgggagac tcaacaaagt gtaagtatag ctgaaggagt ccgggagcag 31380
   aaagtatatg tgtcaggtgt gaggaagaaa atagattttg gaaattatga gagctgtaga 31440
   gagtgagttg agcatagttt gtgattttga gggcctctaa aagtattagg gcagcagcag 31500
   cggctgcacg gagacatgat ggccagccta aaacagtaag atcaagttgt ttggacaaaa 31560
   aggctacagg acgcgatcct ggtccttgtg taagaattcc gactgcacag ccctgcactt 31620
   cggctgtgtg taatgaagca ggttgggatg agtcagggag agctagagtg ggggcagttt 31680
   ctaaagctgt cttcaaggaa cagaaagagg agtggggaaa ggatttagga tctatggggt 31740
   cagctaagtt tccttttgtg agtttatata atggttttgt taggatggca aaaccaggta 31800
   tccaaaggcg aaagtatcca accatgccca ggaaggaaag gagttgttgt tttgtagaag 31860
   gggttggggc ttgagagatt agtcagacac gatcggcagg gagagcacgt gtgttcttat 31920
   gaagaattat gccgaggtag gtaacggatg gagaagaaat ttgagctttg gagggggata 31980
   cccgatatcc tttggagaat aaatgctgaa ggagcagaag tgtgtcttat tgagaagatt 32040
   caaaggaggg gctacaaaga agaagctcat caatatattg aataaggtga gaagcggagg 32100
   ggtggaagga aagtagatca tgagaaagag cttggctgaa gtaacgaggg ctgtccctga 32160
   aaccttgcgg cagcacagtc caggtaagct gctgggactg atgggtgtca gggtcagtcc 32220
   aggtgaaagc aaagagaggc tgggacgagg agtgcaggag aatagtgaaa aaaccatctt 32280
   taagatcaag cacggaatag tgagttgtgg aggaaggtat tgaggacaaa agagtgtaca 32340
   ggttgggcac cacagggtgg ataggcaaaa caatttggtt gataagacgc agattctgaa 32400
   ctagtctgta agacttatcc ggtttttgga caggtaaaat gggggaattg aaaagagagt 32460
   ttataggttc tagaagccca tgctgtagca ggcgagtgat aacaggcttt aatcctttta 32520
   aagcatgctg tgggatggga tattggcatt gagcagggta agggtgatta ggttttaatg 32580
   ggatggtaat gggcatgtga tcagttgcca gggaaggagt agagatgtcc catacttgtg 32640
   ggttaaggtg gggggatatg agagaaagac gcgaaggagg ctttgggttg gggagaaggg 32700
   cggcaatgag atgtggctgt agtccaggaa tagtcaggga agcagataat ttagttaaag 32760
   tgtctcagcc taataaggga actgggcagg tggggataac taaaaaggag tgcttaaaag 32820
   agtattgtct aagttggcac cagagttggg gagttttaag aggtttagaa gcctggccgt 32880
   caatacccac aacagttatg gaggcaaggg aaacaggccc ttgaaaagaa ggtaatgtgg 32940
   agtgagtagt ctccgtattg attaagaagg ggatgggctt accttccact gtgagagtta 33000
   cctgaagctt gccgtccgtg atggtctagg gggcttccga ggcgatcggg cagtgtcagt 33060
   cttcagccgc taagccaaga aggagtcagt cagagagcct tgggccagag ttccaggagc 33120
   tctgggagtg gctgccaggt gagttgaaca gtctgatttt cagtggggtc ccgcacagat 33180
   gggacgcagc ttaggaggaa tcctgggctg cgtgagttcc ttggcccagt ggccagattt 33240
   ccggtatgtg tagcaagttc ctgggggagg aggttctgga ggaacgcctg gctgctacag 33300
   ttcaggcgtt tggaagttct tgtgtgctgg agatgtggct ggggtttgtc tcacagtgga 33360
   ggcaaggaat tgcaactttt ttctgttatt gcacaccttg aaggtgaggt taattaagtc 33420
   ctgttgtgtg atttgagggc cagatttcag tttttggagt tttatttaat gtcgggagca 33480
   gattgggtaa taaaatgtat attgagaata agatggcctt ttgacctttt agggtctagg 33540
   gctgtaaagt gtctcagggt tgctgccaaa tgagccatga actgggctgg gtttttatat 33600
   ttgatgaaaa agagcctaaa cgttatctga tttgggataa agaaaaagga gcattaacct 33660
   tgactatgcc tttggctcca gccacctttt taagagtaaa ttgctgggca ggtgggggca 33720
   ggctagtcat ggaatgaaac tgtaagccgg accaggtgtg aggaggggag gcgataaaaa 33780
   gattataggg tggaggagcg gaggctgagg aagaattggg acctagctcg gcctggcgac 33840
   gagcagcctg gggaggaggg gaaaggtcag atgggtctgt agaaaaggaa gaccggaaag 33900
   actcggcgac gcttggggtt gggactgagg ggacaggcgg gagggaaaga aggaggatct 33960
   gggaggcatc gcattgtgaa cagaggctag ggagggaacg aagtgtgaaa aatgcctgga 34020
   cataaggcac ctcagaccat ttgcccattt ttcgacaaaa attatttagg tcttgtagga 34080
   tggaaaaatc gaaagtgcca ttttctggcc atttagagcc attgtcaagt ttgtattggg 34140
   gccaagcagt gttgcagaag aaaataaggc atttaggttt taggtcaggt gtgagttgaa 34200
   gaggttttaa gttcttaagg acacaggcta aaggagaaga aggaggaatg gagggtggaa 34260
   ggttacccat agtgaaggag gcaagcccag agaaaagagt agagacacag agaaggggtg 34320
   gagggttctt gccctccaga aaagcagaga aggtgttggg gcacggaaat aagggattgg 34380
   ggcacagaga taagaggtca gggtgcgtaa ataagggatt ggtgcacaga gataagaggt 34440
   tggggtgtgg aaataagcaa ttggggggtt cttgccccct aggaaagcgg gacttgccac 34500
   taagggtgaa ggagaagggg ttgaggggta cttgcccctg ccccaggaaa gcgggacttg 34560
   ccactaaggg tgaaggacca aggcaggcgt ccctgcgtgg tctgacaccc ttgaaacgtg 34620
   agtgtagaat cagagaggcg tccctgcaat gatgaaacac caagggaagg ctgccttccc 34680
   agtctgtgac cggagccgga gttttgggtt cacggataaa acatgtctct tttgtcttta 34740
   ccagaaaatg aaaggaattg aaattaagag aagggagaga ttgaagtgtg gcaccaagat 34800
   tgaaaggaga aagaggttga gggatagtga gggaggttgg agaagagagt aaaaagaggc 34860
   cgcttaccgg atttgaaatt ggtgagatgt ttcttgggct ggtcggtctg aggacctgag 34920
   atcgtaggtg gatctttctc acggagcaaa gagcaggagg ataggggatt gatctcccaa 34980
   gggaggtccc ctgatccgag tcatggcacc aaatttcatg cgtgtctgtg cgaagagacc 35040
   accaaacagg gtttgtgtga gcaataaagc ttttaatcac ctgggtgcag gcgggctgag 35100
   tccgaaaaga gagtcagtga agggagataa gcgtggggcc attttatagg atttggggaa 35160
   ggtaaaggaa aattacagtc aaagagagtt tgttctctgg cgggcaggag tgggggttgc 35220
   aaggtgctca gtgggcagga gtgggggtcg caaggtgctc agtgggggtg ctttttgagc 35280
   caggatgagc caggaaaagg actttcacaa ggtaatgtca tcagttaagg caaggaccgg 35340
   ccatttacac ttcttttatg gtggaatgtc atcagttaag gtggggcagg gcatattcac 35400
   tttttttgtg attctttggt tacttcaggc catctgagca tataagtgca ggtcacaggg 35460
   gatgtgatgt cttggcttgg gctcagaggc ctgacagttg gataacagtt catgaaagtt 35520
   tttcactgca ttattgactt aaggattcag caccatttgg gatttcctct tggctagaag 35580
   ttggatttct atacaatttc ctttttctgt gggcagtgtt ccccaaactt agctggataa 35640
   agatgttatt tgagaataca gattctggaa tgcacttcag atctccaggt cagaaccttg 35700
   gaaactcggt cttcaaggat ctttgtgatt gcccaaatat agaaaaacaa tgctgtattt 35760
   tttttattct ttgccagaaa ttaggtaatt ttttatttct tttctacgaa gaccaaaaaa 35820
   tgaacatgaa agatagttag aatatatttg gcttgactac agagatagaa atgatgctaa 35880
   ataatttaac aggaaactta ggaccgtaag aataagctac taaagaacat tatgtcaagc 35940
   acaaacaagg taaggacttg cttgagatac atttggtcaa atgctgtagg cgtatgagag 36000
   atgaaactta agattctcaa tttcctcttg tgtcaagatc aactttcctc ttgtacggct 36060
   gtacctaatt tagtatgtgc actactgaaa gatgtgttga ttcaaagtga catgtgcaca 36120
   atgcatttct tagtttgcag tctttgttta taaaattatc tctaaagtag ttgcttagag 36180
   ccatataaat atattctagg agatgtagta agtcgttgaa atggttaata tgcttttaga 36240
   tttttatgat atgatttaaa aaatccattt gaaataacag caggacagtt caaatagtcc 36300
   tatcttaaat tctggaagta gaggccaggc ttggtggctc acccctgtaa tcccagcacg 36360
   atgggaggca gaggtgggcg gatcacgaag tcaagagatg gagaccatcc tggccaacat 36420
   ggtgaaacct catctctact aaaaatacaa aaattagccg ggcatggtgg cgggcgcctg 36480
   tagtcccagc tactcgggag gctgaggcag gagaatggca tgaacccggg aggtggaagt 36540
   tgcagtgagc cgagattgag ccactgcact ccagctcagt gacagagcaa gactccatct 36600
   taaaaaacaa aaaattctgg gaatagaaag tcatggggag aaggaaggaa gactgcgcca 36660
   aaagaagaaa caaacgggtc ctacatttcc tgagcgtaga gcagtctaag aaaaatgctt 36720
   ttgcacgttt cattgtcttt tttccaactc tcattgtttt ttccatcctt ctcctctgga 36780
   ctatagcacc ctcacagata tagtacaagt aggccagcca caccccaacc aaaactctcc 36840
   tccacttaac aaataaatct tttatttcct caaaacagtg caaaaatctc aactaagttt 36900
   agaaaaaagg ttgtatctca ctcataactt tctatcatct caatgatatc tatcttattt 36960
   tataatggaa gctataactc aagacacata taaaatttag ggaatgttag gcagtataaa 37020
   aaaactaaag aacaaatcaa gctatagata ggaatgccag agactctaat attatcccac 37080
   tttttaaaag aacttgaggg gcactacctc ttacagtatt catggttgtc tgaaacgtga 37140
   aattgaatat caagtcctaa aaaattcaaa ttccctttct tcatgcaatc ccaattcccc 37200
   taaataggaa gcacttaaaa ataatttgta accaaaaaaa tctcagtaat gcccaggttt 37260
   cagtaatatg ccaggtcaga gagaggtgga tccattcctt tatttaaatt aagttggtgt 37320
   catgatatct tcattatact aagggaaaag taaggtttaa aaatagaaat tcatggttgt 37380
   tttgtgttgt ttttagttgt agctgttgtt tgctctccat gttttatttt tacgtacttt 37440
   caaatttaca gaaaagttgt aagagtgctg ttatggatgg gatgtttgta tcccttccaa 37500
   attcatatgt tgaaatccta atccccaatg taatgtattg gggagtaggg atttggggag 37560
   gcattgaggt catcagggtg gagccctcat aagtaggatt agtgcagtta tatatgggac 37620
   cccagagagc tctcttgacc tcttttgcca agtgaggata caatgagaag atggcagcct 37680
   gcgccgtaga agaggatgct cgccacaacc caaccacgct ggcaccctga tcttaagctc 37740
   ccagactccg taactgtaag aaatacattt ctattattca taagccccct aatctatggt 37800
   gtgtgtatat gtattctttt aatatactag cccaaactga ctaagtagta cagaaaattt 37860
   tcaaaaaccc ttcactcaaa tcccccaaat attacatttt accttgtttg ctttatcttt 37920
   caaacatctt tttccctgaa tcatttaagc ctatggtctg gaaatgatgc ccttcaccac 37980
   taaatcctcc aaatcaccat ggatccaaaa cctgctagct gggtgagtct ccagaccctc 38040
   tgaaggataa aggcccataa catctctccc ttggctttca gatcaacatt tggttccagt 38100
   ttctaatagc tggaacatct ccttagaaat ttggagcagc ttctggactt ctctgaaagc 38160
   aagaacagag taactattgt ccactctacc agagcctttg ctttctaatg cttttcaaca 38220
   tttaaagcca ctccagtatt ttaaaaaata cagctcttct tttaccttag ctgctgacct 38280
   aatttagctg aacacatttc attaaatatt tattaaacat cagttcgtgg aagcactgta 38340
   tcaaatgggt acacaagggc aatgcccact gggcaggttt ccagctcatc aaaaagcaca 38400
   gctctgaatt aagagggtgg ctgaggctct gaaaggaata agggcaacag tgaacttgac 38460
   tatttgaaaa aatgttctgt gataatagta ggaaataaaa aaagatgagt taaagaagcg 38520
   gaaccaggaa catgataagc acagcataag atcagctata tttcatcttc ctgtgtaggt 38580
   actggaatat gatcttttca tttaatttgc aattgctcat tttcttgaca acaaaaggca 38640
   gatccaagtg ttgtggaggc tgaagattat ataatttggg agtttctcat taaaaaaaaa 38700
   atgagaacac aaaataaagt gtaaaaataa atacttaaaa tgatcatttt atctcaacaa 38760
   atacattttg aaatctgaaa aatgtcacaa aagttaataa aaataacaat ttgccagaca 38820
   cagtggctca tgcttataat cccatcactt tggtaggcac aggcaggagg atcacttgag 38880
   cccaggagtt caaaaccagc ctggtcaaca tagtgagacc ccatctctac aaaaataaag 38940
   caaaaattag ccaggcatag tgacatgtgc ctatagtccc agatacttgg gaggctgagg 39000
   caggaggatt gctaaagccc aggaggccga ggctgctgtg agccatgatt gcaccactat 39060
   acttcagctt ggccaacaca gcaagatcct gtctcaaaaa atgtaatagt aattttgtta 39120
   attaactgac atgtctctat aatatttttt tctgcattgt ttagctggat gctctcttca 39180
   tataacaagt actttccaca cagagaatat aaaaaataat tccatcttac ctctagcata 39240
   attggataca atttttatca ataataaata aattgactcc atcattagag ccaatttaaa 39300
   caattcttta aatttccccc tataattata tccttgatct tcttgacagt acaggcaaaa 39360
   ttttcctgta attaacaatg agagggtcaa gtcccctctt ggaggaattt atcagagtct 39420
   ttccaaacgt ggaacctttt gttagagtga gtgtaaaaaa aaagaacaag aggacctcag 39480
   gacatgggag agaaagaagg acaatgaaaa taaacaatag ccttaaaaat agaagcggaa 39540
   aacagagcta ggtgagaaga tgctgaaact atctttactg aacaccgact cttaaggctt 39600
   ttggtaactt tttctcttct cccatcacaa aataaatatg aatgccaaag ggcaggcaca 39660
   cttccactgt tgagagaaac agatctaaaa ataagtagga agcagacaag aacgagcaat 39720
   gaacatgatg agaaccatgg tgtctacttt taggttaact atggaggcat gtaggcacag 39780
   atggaggaag ttagtttatc tttgctgctg cacttaaaaa ataaaaacca aaaatataaa 39840
   tgccatcaaa acattgactc ttctctaacc aaaatttttg gttttatatt taaggccaag 39900
   cccatttaga agaaaagcac attcatttgg gttttgtggt ttggagaaag gactgccaga 39960
   gaggaggtga aagccacagt ggagcattcc tggacaatcc attgcaggct tgggagatca 40020
   ccatgcaggc agctcctgct ccataatagc caaaatagca acatgaccac aaataattca 40080
   aaggctgaaa cattttgttc ccaccagttc aaactctcct gcttctgtca aatggctggt 40140
   aatcctatct cctctactaa gcctttccta aatgaaacag tggcaactcc ttttgaattc 40200
   tactatttat tacataaaca cacaattccc gtggttccct tatcactcca ttaaatgcac 40260
   ttgccctctg attgaaacac aatgcaaatc cttttatgct acatttgttc aatgcttgtt 40320
   gccttgactg tatatagcct taatatccgg taggtgcttc taacttattg taagttccac 40380
   atctaattca caaagtattt tgcttagcaa tgcctcttat gatgcaaata aactagtatt 40440
   tatcacgagt gctttaggat acagacaaga agactatttt agagaaaata agaattaaat 40500
   aatggtaagg aaagcaaacc acccctgcga tgtgcatgct ctccaccacc accccttttc 40560
   cctccctgga tggtgtctca ctctccacct cctttttgtc acttgtcctt ggtcacacat 40620
   caatctgccc tcagccctcc agagaagact tattcttcct gcctgaaatg ttatttctcc 40680
   cttgttcatc taaccttgtt cctttttatt aagtgcttta tatatacgga agactacata 40740
   aattatgtct tcacgttaaa taatagtaat acaatgaaca cctgtgcatc cactttaaga 40800
   aatagaatgt atctttgaag tctttgtgtg tactgtcatg ctcgtgtcct tccacattcc 40860
   aaggtaacca tattcctagt ttagtgttaa tcactgtctt gtgggttagt ccaagtcttg 40920
   ctattcttta gagttttact acctatttct ccctaacaat acatatattt agtttgaaaa 40980
   aaaactcaga gcaatcgcaa gcctagtgga gtaagagaaa gatcaacatt gacactaaga 41040
   gaggacatgt cccagaagat gagctaacag aggctctccg ttacagcatg gcttcagttt 41100
   ccccaggagt ttacaaaagt catttgcagg gaagtcagaa ggagagagac tggggatgtc 41160
   gggctctcta agacagggca ttggggtggt ctgtgaatgt gagtctggag ctggaggtct 41220
   ccaaccataa ggacatgaga ggtggcagct ctgccttctc cacttctgag ttcaccacag 41280
   cccacctcct gctcttcaga gtaggtggaa gatcaccctc ttttgcccca tctgaagaag 41340
   aaactccaga ttactaatgg gaagctgaag ggcagtcagc caggctgact ctaagttgtc 41400
   cttcaaaatg tagctcaggg tttcctcttc cagcagaact tttctgacac ccacatgcaa 41460
   tgaccagggt caacctttta accccctgtg cactctgtgc taacccctat caacagctct 41520
   ccctcctact gctgtccctc ttccttatta aactggatgt tcttcgaggg aataaattat 41580
   cttatcaatt ttactaataa taacagtatc atctgatatt tgctttattt tataaatgtg 41640
   gacaaacctc attaacatta aataccaaga tttaaaaatt gtaaggcaga atatgtgttc 41700
   ttgttactcc gaatttattg gaaataaaga attttgagtg tctcttctat taaagaagtt 41760
   ctgtgagtgt tccttgcatt cttctttctt caggcatgcc tcatctcata acatttcagt 41820
   tactgtgtca tttgcacagt tgagaccttt ccacatggat acacgtgaaa taaaggtgtt 41880
   acaaaaagta gtatttttca ctttactttg tgcctaaaaa caaatacacg tgtatggtac 41940
   tacataaatt aaaaaatatg atttaattat cttctacttt ttataacacg cctggaaatt 42000
   gtttttactt ttataattag gaggtgaaca gcaggtctac aaaatattaa cagaaaaagc 42060
   taataccttt taattaaact tttctaacta taactttcaa gataagtacc tacactgttt 42120
   taaagtaagc tataaaatta aagttagtaa aaagaaaaaa aagatattaa tatatcagac 42180
   attacctcac catccagaat taaccaggtt ataatgctgc atatcctccc tgatatcatg 42240
   tatttttaaa taaaatcatt ctgaatattc tggtctttac ctgatttatt tcattcaaca 42300
   tatcccggtt atctccccat ttcaataaat gcacttctaa agccataatg tttaatgtct 42360
   gtatatgaat ctctaagcat gtagtaaaat cataatgcaa aataaataaa aataaacaca 42420
   aaattcagaa gactgggtgt ttttttaatg agagaattgg atttgttaag ggtttagcca 42480
   tgattccaaa cacacacata tttttcatta tttgtatacc gtacatgttt tatatctaaa 42540
   aatttccata tgaatattat attatttcat aatatatgaa aaaagcactc attccaataa 42600
   gtgtttgtat tttttcccat ctttcactaa acaaacccct gacaaatatt cttctattaa 42660
   aatgttgcaa atatcctctt tttaatccta ggataaattt ctagaaatag aattgctgag 42720
   caaaatggtt tgtacaattt taaggccatt aatatatatt actaaactgc cacccagcaa 42780
   agtaatgtca atttctattc ccatcagcaa tgaatgagag tcattttctc tcattctttg 42840
   ccaacactga acataatgaa gaagaaaaaa atctctgcca atttgatcag tgaaaatttg 42900
   tgtttcatat tttctctaac ttgcatttct ttaactacta gtgtgattaa acctttttaa 42960
   agtttttacc cattttttac tttttcttct attaattgct tattaatggt ttttattctg 43020
   ttagggtatt attattctta tgtatttata aggttatata attaaagatt tcgaactttt 43080
   gtctgtctaa tttgtggtag gcagaataag gcttccccca aatgtccaca tcttagtcct 43140
   cagaaagcct ccaaacatct tatgttacat gacaaaaggg aatggaggtt atagatggaa 43200
   ctgaggttgc taatcaactg acctaaaata gggagattat cttggattat ccagcggact 43260
   aaatggaatt acaaggttct tagaagtagg agagaggtgg aggagggatc agagtcagag 43320
   agaggcttga aagcgctgta ctgctggctt aaagatggag gaatggagcc acaaaccaag 43380
   aaatgctggg gggctctaga agctggaaat ggcaaagaaa tagatgcgct cttgaagctt 43440
   gcagaagaac acagctctgc cagcatcttg atttttgtca aatgagactc atttcagact 43500
   tctgatctcc agaactgtaa gataaataga tttgtgttgc tttaagccac ctattgtgtg 43560
   ataatttgtt gtaatggaaa tgggaaactg attcacaagt tgcaaatgtt ttcccgattt 43620
   ttatatacat aatctttaca aagcaatcaa aatgtccctt atatttagta ccattttaaa 43680
   atgtattcat tatacctctt ctatgttcaa ctacagtgaa gcagataaaa tttgaccaaa 43740
   aatacaattt tttttttctt taaaaaagga gggggtttcc tcttcaccct ttctttctcc 43800
   ttttcttttc cctttgcttg gaggtggctg ggagctgagg aagctagaag aacaaggaga 43860
   aaatggctga ggagatagtc ctggggtcag gagccaaaaa aagctgatga catagtcagt 43920
   agattggtta tatgcagggg gattaaggaa ataagcaaat attgaagata gtgagagcca 43980
   ggtttttcac tgtcagagaa gggatttata aaaatgcaaa tgagccagtg ctttggctca 44040
   tgcctataat cccagcactt cgggagggca aggcgggtgg atcacttgag gtcaggagtt 44100
   cgagaccagc ctggccaaca tagtgaaatg ccgtctctac taaaaataca aaaattagcc 44160
   tagtgtcatg gcacgtgcct gtaatcccag ctactcggga ggctgaggca ggaaaattgc 44220
   ttaaaccctg gaggcagagg ctacagtgag ccgagatctc attactgcac tccagcctgg 44280
   gtgacagagc aagactgtct cagaaaaaaa aaaaaaaaaa aaaaaaaaaa tatatatata 44340
   tatatatata tatatgaaaa tggagaaggc taaggtatta cgttgtcatt gagaaaaaga 44400
   aggagggagg gagagaggta cagatgaaac agatatatgt atatatatat acacacactt 44460
   ttataattag atggatatat atagagagat agatgatata taaatacagc tctgtcggcc 44520
   tggtagtaat gagtatacta agcacccaga tctggattta taaatgcctc taccaaaagg 44580
   aaccagactg cagagaaaaa tagttgattc taaggttgag acatggaaag tacaaaatta 44640
   gcccaaaagt atggaaatat tctaagaatg atggaaacat gcaaaataaa cccacggaac 44700
   ttagcttgaa gaggctctca tggccaaatc tgagacacat gaagtatcag aataaatgtt 44760
   gatagtaacc agattataac ccattgaata aagtaggaat ctatcagacc ataatgaatg 44820
   aataaatgaa taaaaggaaa aaattcttgc agtataatgc caacaaacta atgtggaaag 44880
   aatgatgaca catatcagag aggaagttga atcaggtagg agtcatcagt ggatgccaaa 44940
   gccagtgggt agaaatttga tgaataatta tatatttgca tagtcttgag tatctcccca 45000
   tgaaacactt ccactcacaa aggggaaaat ggtggaccta tggtccagga acctggcaga 45060
   caccaacttg aacaggttgt caatgttaac atctccaggg ttcagatgaa ttaacattga 45120
   aggattcctc atatactact ggaagtacaa tatcatttca gtgctattcc tgataagaat 45180
   gtatggtgtg agtgtgaaca tgaggaaata ttagatggtt ccaggttgat tgatgtggtt 45240
   tatactctaa aactgcaaat tacatgaatg tcaaggaaaa gactgtcaag ccgctccatg 45300
   ttgaaggaca ctaaaataca tgacaaccaa atgcaatgca tgatcctgga ttgagttcta 45360
   gaacaagaag gagaaagaaa cattatggga acaggaatat ttgaatgaga tctttggaat 45420
   aattggctgc tagtattata tcaatgttta ttacttgatt ttagatagtg gtatctctat 45480
   tacgtggtag agtgtttttt gtatgcaatg cactatgggg tgttaggaat cacgggacat 45540
   gctgcctaca actcctatga tcccaaaatg attcaggaaa aaaagaatga agtgtgcatg 45600
   tgtgtgtatg tgtgtgtgtg tgtatgtgtg tgaactttca tgccacaaat atttattcaa 45660
   gcctataaga gatgctaatc actgcaaact aaatattgat aatccaagaa tttaaaaaat 45720
   gtaatagtta atactttatc ttagtgggca attaaaaata gaaacaatgg ctagaaaaat 45780
   agttttacac aagaggtaat taaaatgggc ctagaagacc atagatttca aacattaagg 45840
   aaagtaataa gcaataggca aaatatggta gtgtaaaagt gaatggcatt tcagggaata 45900
   atgaggtcag tacgttagat gtaagtgacg agaaggaata tagcgagggg aagaaatccc 45960
   taaaaaaaaa gaagaaaaga aaaaaaccta gaggccaaat tatgacacta gagcctttgg 46020
   acagtatctg cccgaagatt agaagccgct gtagctgggc atggtggctc gtgtctgtaa 46080
   tcccagcacc ttgtgggcaa aggcaagagg attgcttgag accaggagtt tgagaccagc 46140
   ctgggccaca tagcgagacc tcatctctaa aaaaaaaaaa aaaaaaaaaa aaaaaaaaat 46200
   taaaaattag ccagatgtga tggtgtgcac ctgtggtccc agctacttgg gaagctgagg 46260
   tgggaggatg aggctgcagt gagccgtggt tgcatccatg cactccagca tggatgacag 46320
   agaaaaactt tgtctcaaaa aagagaaagt cattgtaggc ttttagtggg caaatgaagt 46380
   attcaagtct gtaagttatt aatcaaatgc agtttaaata taaaagccca tataaaaata 46440
   tagtttaaga aataagcacc agaacaaaca atttctgaaa cattttcagt ggattatttc 46500
   caatggaaat agataaaaat gtgtccaatt aaagaatcca ttataaaaat aggttataag 46560
   ataaaaatgt aaacattctt catactcagc atctccaagt actcaaagat cccagagtca 46620
   tagcacttta aaactaatgt gatttttata aaggagtgga aaatatgctt agcatgcagc 46680
   ataaaaaagt aaaaatatta atctgtgctt tttaatatgg cagggaacat ctattctttc 46740
   atttaaactg gagtctcgcc ttctccacaa agacacaggg agaggaaaat aattgacaag 46800
   aataggaagt gaagaattta tgattctcaa taaaagtatg tattgaaaca aaatcagtac 46860
   tacacgtctt ctaaaaattg ctgctggatg gcctaataaa gtctaggtgc atgcaagaca 46920
   gctgaaaaat gtacttgtta ccacaagagg gaaggtgttt ctccaagtgc tttgcagcaa 46980
   tctaccaaag ttttgaagtt ggggcagtag agttgactga cataccctga agccatctca 47040
   gtatcagtct agtggtaggg caggaaagct gagagaagtc cctctgatat gcattagaac 47100
   ttcaccagct gccaactgaa agctgagtga aaggccaaga gaagtatccc aagttgcaga 47160
   aaagtggggg ccatactaca gagaaaatat aactcccaag tgacctaaag agttggacag 47220
   tttagttata aagcagaaag acctcttcca tgatttgaaa aacaggtgtc tgtgccataa 47280
   agtataaaga gatctgaaat ctcactggtg tataatcctc aagctctgct gaagacccta 47340
   attctgccct cagaatactt ttatacaata gtgagctgaa tcttattaaa gttataataa 47400
   aattcaaatg ttgcttaaga acagattaaa ttgacccaat tcccaactcc agcagcttga 47460
   cagaggagca tgcccttttc tgtagataaa tattgtcagt ctctactgtt ttttcatata 47520
   caatgctcag tataagacca ataacaaaaa agacaagcag gaaagtgtta cccccatgac 47580
   atgcaattta ctcatgtaac aaatctgcac atgtacccac tgaatctaaa acaaaagttg 47640
   gaaaataaaa acaacaatag gatatttaga atatcctcaa atttggaaat taagcaatac 47700
   acttgtaaag agttcatgag ttgaaaagat aaactgaaaa ataatttgag ctgagtgata 47760
   atgaaaacac catatatcat aatttgtggt gtgcagcagc taaagtatta caggagaatt 47820
   tatagctttg aacaagaaaa agggtttaaa atcaattact caagcttcca ccttaaggca 47880
   cgcaccacca tgcctggcta atttttgtgg ggattttttt gaagagatgg agtcccacta 47940
   tattgttgag gctggtcttg aactcctggc ctcaagtgat gctcctgccc cagcctccca 48000
   aagtgttggg atttcaggca tgaaccactg catgagccat gaaattatat ttatattaca 48060
   cattatcttt cagaaaataa aggtggagaa aacaactttc caaatggttc tatgtccagc 48120
   ataaccttga taataaaacc tgacacatta taagaaaatt cccaaataat atctttcaca 48180
   aacataaaca caaaaatccc ccacaaaata ttaacaaatt aaatcaatca aaatatagaa 48240
   agtatttgta atgcaggttg tagtcagggc aactaagtga gaacagaaat aaaaggtatc 48300
   cagattagaa agaaagaagt aaaacgattt ctacacacag atagcgtgat ctcgtataca 48360
   gaaagcccta aggaatccac taactataaa aactaacaat tcagcaaggt ttcagactac 48420
   aatatcaata tataaaaatc aattttatgt ctatacactt tcaatgaatt caaaataaac 48480
   tcaaaaacaa aactaagaaa atttataatt gcagcaaaaa gacaggaata aatttaacaa 48540
   aagaagtgca aaattaatag tctgcaaact ataaaacatt attgaaagaa attaggtatt 48600
   acaataaatg gaaaaacatc ccatgttcat ggatcagaag acttaatatt gttaaaatgg 48660
   caatctctat tagaatccca gatatcattg tggaaattga caagctgata ctaaagttca 48720
   tataaaatcg caaagggtac ataagagcta aaacaatctt gaaaacacag aaagcaaagt 48780
   agaaaaaaat cacacctctc aatttcaaaa tttactccaa aatgatggca accaaaatag 48840
   tgtggtgcta gcacaaggac agaatatagg tgaaaggaac tgaattgaga gaccatgttt 48900
   ctatggtcaa ctgattttga caagaatgcc acaaccattc agtgaggaga agaatagtct 48960
   tttcaacaaa tggggctgga actattggat atccacatgc aaaataatga ggctgaaccc 49020
   ttacctcata ccatatacaa aaataaactt aaaatgtatc aaagacctaa atttaaaacg 49080
   gaaaaccata aaaactcatt gaagaaaaca tagacataaa tcttcatgac ctcagatttg 49140
   gcaaaggatt cttacatatg gcatgaaacg tatgaacaac aaaagaaaaa aaattgataa 49200
   tttgaacttg ataaaaatta aaaccttgtc cttcaaagga aaccaccaat aaagtgaaat 49260
   aacaatttca cagaatggga gaaaatattt gcaaatcata tctcttatat gggactttcc 49320
   aaaatatata aataacacaa cttactaaga aaaaagacag cccaaattaa aaataggcaa 49380
   accacaataa acatttcttc aaggaaaata tacaaatggc caataagcac atgaaagatg 49440
   tttaacgtaa ttatttatca gagaaatgca aataaaaacc caaatgagat accaattcaa 49500
   acacacaagg atggctagaa tcaaaaagtt agataataaa aagtgttggt aaggatgagg 49560
   agaaatcaga accctcatat gttgctggtg gagttgtaaa atggtgcagc cactttggaa 49620
   aacaataggt aattcctaaa gtgattaaac atagagagac aatatgaccc agcaattcca 49680
   ctcctagata tgtactcaag agaattaaaa acatgttttc agtcagatgt ggtagcgcat 49740
   gcctataatc ccagtacttt gggaggaaag gcaggtggat tgcttgagct caggctttag 49800
   agaccagcat ggacaacaaa gtgagactcc atctctaaac aaaatacaaa aattagctgg 49860
   gtgtggtggc atgtgcctgt agtcccagct actcaggagg ctaaggaggg agaatggcaa 49920
   ggctgcagtg agccaagacc atgccactgc accctagact gggcaacaga gccacaccct 49980
   gtctcaaaaa acaaaacaaa acaaaaacag gcattcaaac aaaaacttat gcacaaatgt 50040
   ttttgtgtat acacagcaga attattcata aaagtcagaa agtgtcaaac gtccaacaag 50100
   tgatgaatga ataaacacat atggcttatc catataatga aatattatct ggtcataaga 50160
   aaaaatgctg atacttgctg aagtatgcat gaatcttgaa aacattgtga ctaaattaaa 50220
   taagccagtc acagaaaacc acatattata tgactccatt tatacaaaat gtcaagcata 50280
   ggagtatctc tacagacaga caagtagatt gttctttttt taatcctatg ttataaaagg 50340
   aatactttgt tatgaccaag tgtaggttat cccaagtatg caacgttggt ttcacacatg 50400
   aaaagtacaa catgagtagg ataaaggata agatcctatc atggactcaa tagaaaacat 50460
   atttgacaaa attttacact tatgcatgat aaaaactctc aaactaggaa tagaaggaaa 50520
   tttcctgaat ttaataagtg caaaatctat agcaaagacc acacttaagg ataaaacagt 50580
   gaatattttc ctcttaagaa cagaaaaaaa atgcaaggat gttcattatc acttctattc 50640
   aacattgtat tacaagtccc aataaatgta ataaggcaag aaaaagaact acaaggcata 50700
   aacattgtgg ggggaaagta aattaacaag tagattccaa aatttacaga aaaaatgcaa 50760
   ataatctaaa atagtcaaaa taatgttgaa aacagaataa gtaggacaat acacaatcta 50820
   taaaactaca gtaatcaaga tagtgtgtgt tgccataagc aaagacatgt agaccaataa 50880
   aatagaagag tgagttcaga aattgacaca cacaagcata gcatccattg attttttttt 50940
   ttttaccatt attatttttt cttttttttt tattattata ctttaagttt tagggtacat 51000
   gtgcacattg tgcaggttag ttacatatgt atacatgtgc gatgctggtg cgctgcaccc 51060
   actaactcgt catctagcat taggtatatc tcccaatgct atccctcccc cctcccccca 51120
   ccccaccaca gtccccagag tgtgatattc cccttcctgt gtccatgtga tctcattgtt 51180
   caattcccac ctatgagtga gaatatgcgg tgtttggttt tttgttcttg caatagttta 51240
   ctgagaatga tgatttccaa tttcatccat gtccctacaa aggacatgaa ctcatcattt 51300
   tttatggctg catagtattc catggtgtat atgtgccaca ttttcttaat ccagtctatc 51360
   attgttggac atttgggttg gttccaagtc tttgctattg tgaataatgc cgcaataaac 51420
   atacgtgtgc atgtgtcttt atagcagcat gatttatagt cctttgggta tatacccagt 51480
   aatgggatgg ctgggtcaaa tggtatttct agttctagat ccctgaggaa tcgccacact 51540
   gacttccaca atggttgaac tagtttacag tcccaccaac agtgtaaaag tgttcctatt 51600
   tctccacatc ctctccagca cctgttgttt cctgactttt taatgattcc cattctaact 51660
   ggtgtgagat ggtatctcat tgtggttttg atttgcattt ctctgatggc cagtgatgat 51720
   gagcattttt tcatgtgttt tttggctgca taaatgtctt cttttgagaa gtgtctgttc 51780
   atgtccttcg cccacttttt gatggggttg tttttttttt cttgtaaatt tggttgagtt 51840
   cattgtagat tctggatatt agccctttgt cagatgagta ggttgcaaaa atttctcccc 51900
   atgttgtagg ttgcctgttc actctgatgg tagtttctct tgctgtgcag aagctcttta 51960
   gtttaattag atcccatttg tcaattttgg cttttgttgc cattgctttt ggtgttttgg 52020
   acatgaagtc cttgcccatg cctatgtcct gaatggtaat gcctaggttt tcttctaggg 52080
   tttttatggt tttaggtcta acgtttaaat ctttaatcca tcttgaattg atttttgtat 52140
   aaggtgtaag gaagggatcc agtttcagct ttctacatat ggctagccag ttttcccagc 52200
   accatttatt aaatagggaa tcctttcccc atttcttgtt tttctcagat ttgtcaaaga 52260
   tcagacagtt gtaggtatgt cgtgttattt ctgagggctc tgttctgttc cattgatcta 52320
   tatctctgtt ttggtaccag taccatgctg ttttggttac tgtagccttg tagtaaagtt 52380
   tgaagtcagg tagtgtgatg cctccagctt tgttcttttg gcttaggatt gacttggcga 52440
   tgcaggctct tttttggttc cacatgaact ttaaagtagt tttttccaat tctgtgaaga 52500
   aagtcattgg tagcttgatg gcgatggcat tgaatctgta aattaccttg ggcagtatgg 52560
   ccattttcac gatattgatt cttcctaccc atgagcatgg aatgttcttc catttgtttg 52620
   tatcctcttt tatttccttg agcagtggtt tgtagttctc ctcgaagagg tccttcacat 52680
   cccttgtaag ttggattcct aggtatttta ttctctttga agcaattgtg aatgggactt 52740
   cactcatgat ttggctctct gtttgtctgt tgttggtgta taagaatgct tgtgattttt 52800
   gtacattgat tttatatcct gagactttgc tgaagttgct tatcagctta aggagatttt 52860
   gggctgagac aatggggttt tctagatata caatcatgtc atctgcaaac agggacaatt 52920
   tgacttcctc ttttcctaat tgaataccct ttatttcctt ctcctgccta attgccctgg 52980
   ccagaacttc caacactatg ttgaatagga gtggtgagag agggcatccc tgtcttgtgc 53040
   cagttttcaa agggaatgct tccagttttt gcccattcag catgatattg gctgtgggtt 53100
   tgtcatagat agctcttatt attttgaaat acgtcccatc aatacctaag ttattgagag 53160
   tttttagcat gaagggttgt tgaattttgt caaagtcttt ttctgcatct attgagataa 53220
   tcatgtggtt tttgtctttg gctctgttta tatgctggat tacatttatt gatttgcgta 53280
   tattgaacca gccttgcatc ccagggatga agcccacttg atcatggtgg ataagctttt 53340
   tgatgtgctg ctggatttgg tttgccagta ttttattgag gatttttgca tcaatgttca 53400
   tcaaggatat tggtctaaaa ttctcttttt ttgttgtgtc tctgcctggc tttggtatca 53460
   gaatgatgct ggcctcataa aatgagttag ggaggattcc ctctttttct attgattgga 53520
   atagtttcag aaggaatggt accagttcct ccttgtacct ctggtagaat tcggctgtga 53580
   atccatctgg tcctggactc tttttggttg gtaagctatt gattattgcc acaatttcag 53640
   ctcctgttat tggtctatta agagattcaa cttcttcctg gtttagtctt gggagagtgt 53700
   atgtgtcgag gaatttatcc atttcttcta gattttctag tttatttgca tagaggtgtt 53760
   tgtagtattc tctgatggta gtttgtattt ctgtgggatc ggtggtgata tcccctttat 53820
   cattttttat tgtgtctatt tgattcttct ctcttttttt ctttattagt cttgctagcg 53880
   gtctatcaat tttgttgatc ctttcaaaaa accagctcct ggattcattg attttttgaa 53940
   gggttttttg tgtctctatt tccttcagtt ctgctctgat tttagttatt tcttgccttc 54000
   tgctagcttt tgaatgtgtt tgctcttgct tttctagttc ttttaattgt gatgttaggg 54060
   tgtcaatttt ggatctttcc tgctttctct tgtgggcatt tagtgctata aatttccctc 54120
   tacacactgc tttgaatgcg tcccagagat tctggtatgt tgtgtctttg ttcttgttgg 54180
   tttcaaagaa catctttatt tctgccttca ttttgttatg tacccagtag tcattcagga 54240
   gcaggttgtt cagtttccat gtagttgagc ggctttgagt gagtttctta atcctgagtt 54300
   ctagtttcat tgcactgtgg tctgagagat agtttgttat aatttctgtt cttttacatt 54360
   tgctgaggag agctttactt ccaactacgt ggtcaatttt ggaataggtg tggtgtggtg 54420
   ctgaaaaaaa tgtatattct gttgatttgg ggtggagagt tctgtagatg tctattaggt 54480
   ccacttggtg cagagctgag ttcaattcct gggtatcctt gttgactttc tgtctcgttg 54540
   atctgtctaa tgttgacagt ggggtgttaa agtctcccat tattattgtg tgggagtcta 54600
   agtctctttg taggtcactc aggacttgct ttatgaatct gggtgctcct gtattgggtg 54660
   catatatatt taggatagtt agctcttctt gttgaattga tccctttacc attatgtaat 54720
   ggccttcttt gtctcttttg atctttgttg gtttaaagtc tgttttatca gagactagga 54780
   ttgcaacccc tgcctttttt tgttttccat ttgcttggta gatcttcctc catcatttta 54840
   ttttgagcct atgtgtgtct ctgcacgtga gatgggtttc ctgaatacag cacactgatg 54900
   ggtcttgact ctttatccaa tttgccagtc tgtgtctttt aattggagaa tttagtccat 54960
   ttacatttaa agttaatatt gttatgtgtg aatttgatcc tgtcattatg atgttagctg 55020
   gtgattttgc tcgttagttg atgcagtttc ttcctagtct cgatggtctt tacattttgg 55080
   catgattttg cagcagctgg tactggttgt tcctttccat gtttagcgct tccttcagga 55140
   gctcttttag ggcaggcctg gtggtgacaa aatctctcag catttgcttt tctgtaaagt 55200
   attttatttc tccttcactt atgaagctta gcttggctgg atatgaaatt ctgggttgaa 55260
   aattcttttc tttaagaatg ttgaatattg gcccccactc tcttctggct tgtagggttt 55320
   ctgccgagag atccgctgtt agtctgatgg gcttcccttt gagggtaacc cgacctttct 55380
   ctctggctgc ccttaacatt ttttccttca tttcaacttt ggtgaatctg acaattatgt 55440
   gtcttggagt tgctcttctc aaggagtatc tttgtggcgt tctctgtatt tcctgaatct 55500
   gaacattggc ctgccttgct agattgggga agttctcctg gataatatcc tgcagagtgt 55560
   tttccaactt ggttccattc tccccatcac tttcaggtac accaatcaga ggtagatttg 55620
   gtcttttcac atagtcccat atttcttgga ggctttgctc atttcttttt attctttttt 55680
   ctctaaactt cccttctcac ttcatttcat tcatttcatc ttccattgct gacacccttt 55740
   cttccagttg atcgcatcag ctcctgaggc ttctgcattc ttcacgtagt tctcgagcct 55800
   tggttttcag ctccatcagc tcctttaagc acttctctgt attggttatt ctagttatac 55860
   attcttctaa atttttttcg aagttttcaa cttctttgcc tttggtttga atgtcctccc 55920
   gtagctcaga gtaatttgat cgtctgaagc cttcttctct cagctcgtca aagtcattct 55980
   ccatccagct ttgttctgtt gctggtgagg agctgcgttc ctttggagga ggagaggcgc 56040
   tctgattttt agagcttcca gtttttctgt tctgtttttt ccccatcttt gtggttttat 56100
   ctacttttgg tctttgatga tggtgatgta cagatgggtt tttggtgtgg atgtcctttc 56160
   tgtttgttag ttttccttct aacagacagg accctcagct gcaggtctgt tggaataccc 56220
   tgccatgtga ggtgtcagtg tgcccctgct ggggggtgcc tcccagttag gctgcttggg 56280
   ggtcaggggt cagggaccca cttgaagagg cagtctgccg gttctcagat ctccagctgc 56340
   gtgctgggag aaccactgct cccttcaaag ctgtcagaca gggacattta agtctgcaga 56400
   ggttactgct gtctttttgt ttgtctgtgc cctgccccca gaggtggagc ctacagaggc 56460
   aggcaggcct ccttgagctg tggtgggctc cacccagttc gagcttcctg gctgctttgt 56520
   ttacctaagc aagcctgggc aatggcgggc gcccctcccc cagcctcgct gccgccttgc 56580
   agtttgatct cagactgctg tgctagcaat cagcgagatt ccgtgggcgt aggaccctcc 56640
   gagccaggtg tgggatatag tctcgtggtg cgccgttttt taagccggtc tgaaaagcgc 56700
   aatgttcggg tgggagtgac ccgattttcc aggtgcgtcc atcacccctt tctttgactc 56760
   ggaaagggaa ctctctgacc ccttgcgctt cccaggtgag gcaatgcctc gccctgcttc 56820
   ggctcgcgca cggtgcgcgc acccactggc ctgcgcccac tgtctggcac tccctagtga 56880
   gatgaacctg gtacctcaga tggaaatgca gaaatcaccg gtcttctgca tcggtcacgc 56940
   tgggagctgt agaccggagc tgttcctatt cggccatctt ggctcctccc ccgcatccat 57000
   tgatttttta acaaaggtac cgacacaatt cctgtattag tccattctca cattgctata 57060
   aagaactgcc tgagattggg taatttataa aggaaagtag ttcaatcgca tggcagaagg 57120
   ggaagcaaag gcatctttct tcacatgatg acaggtagga gaagtgcaga gcaacagggg 57180
   aaaaaacccc ttataaaacc atcagatctc gtgagaatgc actcactatc ccgagaacag 57240
   gatggaggaa actgcccgca tgattcaatt atctccagga catgtggaga ttatgggaac 57300
   tcaaattcaa gatgagattt gggtagggac acagccaaac cataacaatt cctttgggaa 57360
   agcaagttcc caaatggtgt ttgaacaacg gaaaattaaa ttagtaaatc tcaacctcct 57420
   acatcactct atgtacaaaa attatttcaa gattaatcat tgacttaaaa taaaagttag 57480
   aaaaataaag catctagaaa aaacattgaa gtagatcttc atacgtttga gataggcaaa 57540
   gatttcttta gagcataaaa agcaccaaac ataaaacaaa acttgataaa ttacacaaca 57600
   tcaaaaataa gaacttctgc acattaaaaa atgttataag aaataaaatc aagcttcaga 57660
   cttggagaaa atatttacaa cacaattatt ttacaataca aatatctaac aaaggacttg 57720
   tatttagaat atataacgac ctgcaaatca ataatgaaga aatctactca ataaatatgc 57780
   atataatatg taaacatttt accaaaaaaa atgactgttc aacatgaaca taaaaaggtg 57840
   cttatcaggg aaataacaat taaaaccacc acaagataca tactagaatg tttaaaataa 57900
   aaaggactga aaataccaag ttttggcaag gaagtgaaac cactgggaac tctcatatat 57960
   tgctggtgga aatgtgaaat atcacaacca tttaaaaaaa cctgtggcag taccttttaa 58020
   agttggatat acatggtatc catgaatcca ttcctaggtg tctaccttaa aaaaaagtga 58080
   aaacattttt cactggaaaa tgttaaagat gtttcattgt agaacttttt ctccagtgaa 58140
   aaagtaaaac cttttaactt tttttttttt tttttttttt gagatggagt ctcgctctgt 58200
   cacccaggct ggagtgcagt ggcatgatct cggttcactg caagctctgc ctctcggatt 58260
   caccccattc tcctgcctca gtctcccgag tagctgggac tacaggagcc caccaccacg 58320
   cccggctaat tttttgtatt tttagcagag atgggttttc attgtgttag ccaggatggt 58380
   ctcgatctcc tgacctcgtc ctcgtgactt ccctgcctcg gcctcccaaa gtgctggaat 58440
   tataggcatg agcccggcca tgttttcact ttttttaaag ataaaaagac ttgtatatga 58500
   atgatcatag cccccttatt caaaatattc aaaaatttaa aatatctcaa atgtctgtca 58560
   tgaaagaatg gacaaagaaa ttgtggtata tctatacatg gaataatact cagcaattga 58620
   aaagacagtc tacaaatgga caaaacctca tggattcata tcaaaaaaca ttataatgaa 58680
   caaccaaaag cagatacaaa aatatgtaat gtattattcc atttagatga aacaaaagaa 58740
   taattgatgc taatgcatgc tgataaaaat caggatggtt gctttcaggg ttgaaagttt 58800
   gagttgaggg agatgcaaag cactttctgg tgtgaaggaa atattctaga tcttgttttg 58860
   ggcggcagtt ataaggatat ctacaagtca aaactcatta aactgaactc ttaaattctg 58920
   tgcattttat tatatacaaa ttattttgca ataagaatta atttttaaaa agcaataaaa 58980
   attgctgctc ggcgaaaaca aattatgaat atactatcta acacactttc tggtgtgaag 59040
   gaaatattct agatcttgtt ttgtgtggca gttacaagga tatctacaag tcaaaactca 59100
   ttaaactcaa cttttaaatt ctgtgcattt tattacatat aaattatttt gcaaaaataa 59160
   ttaattaaaa aacaataaaa attactgttc tgagaaaaca aattatgaat ataccatcta 59220
   actctacaga gtacagaaat aaaaaatata gtaatatctt tgacctattt gaaagtcaac 59280
   aaaagaaaag caagaaagtg cctacaatat gattaagtga atggcattta tatatatgta 59340
   taatcattta aatgtttaca gtttaaagga aaaaatagtg aggaaaagaa tgctatggtt 59400
   ttcaaatcct acggtttgta ctaaagttgt ttgtcttttt ttttttctta gctacctggc 59460
   atctgaactc cctttctgtg cctgtgaaat ttccaccttc aactgcagaa gctgcaaagc 59520
   cagatacttg ctttcatagc ctccattact ttgagggtag ggcacatgaa taggctcagt 59580
   tactctgact tcagcttgga tattgacttg ggaactagtg agaaaagaag cactcagtgg 59640
   agaattagtt ctggcagcaa tagtagcagt tacctcgagg atctggtcac agttatagca 59700
   ggggggtcat ccagtagcca atgtcagcag caaacaatgc caatgccaat gctagagaaa 59760
   tgagaggtac aaaccattca tctagcattt gctggcatca gaaaaaatat cctcactagt 59820
   caagttctgc agcataattt tagcttcaaa gctatgcact ttgttttatc ctgcctgccc 59880
   attttttaag ccttttctct agtgttccaa atgactctgt gaactactca atatcctttc 59940
   aacaaacttc ttttccactt gaatcatcca gagttaattt ctattgctgg caatactaac 60000
   tctgatggaa aaataaaata ataaatgcac tctaacacag aataacagaa ataatgctcc 60060
   acctagttaa caaggatttt acaaacaagt agaagttatt tggtcttctt tttaaaaata 60120
   cgttcttaaa aataacattt ttgacaccat gaattccacc tgtgttttca aactattttt 60180
   cactattaac attatgtttt aaaggagata cgttttcaaa agatccactg gaaaaaatta 60240
   gacttctcct ataataattt acacagtgag tggctgagat tctaaaggaa tatcataaaa 60300
   ttatgataat attaaataaa agcaaatact ctcataactt tcaaggaaac atgcgatgac 60360
   tgatgtagta tcaaagggat atgttttgga agatgctctc ttctatgtga ctgaaaatat 60420
   ggggacatgc tatacattct atttttcttt attgattgtg aatttgttag tcatcaattt 60480
   tgctgagtct attcaaaatt tccacttaca taaactcttt gagtaatgag tttcatagat 60540
   atattgtgtg ctatgcgaac tagtacctct atttcttggt tccattattc ttgtactata 60600
   ggatattgta aataaaccta tgttttctgt aacatatctt ttgaaaaaat aatgcatctt 60660
   ctcagaaaac ttttcctatc aagaattttt gttgttgctc ttcatcagtc taagaaaaaa 60720
   ctgtcttttt tgtccttttt tatttttgcc catattttcc tcttcatcct ccattcctat 60780
   gctcatagcc catcctcact ggcaccaatt ctgtgtttaa tgtaaacgtc cttgaaaaat 60840
   agtgttgttt catttcgagg atatactgaa gttacctaaa tttgaacatg atatggattt 60900
   cattatttct tatcttgctt ttgtgaagat gacattttat ggagacaaaa gataagttca 60960
   cacataaaat ttcaggtagt ggtaaatgta acaaggaaat aaacacggtg attcagtagt 61020
   gattgggtta ctttataaaa gatggtctct tctcagaatg ttttatttga gctgagaata 61080
   gaaacatata aaagatactg ttactagggg catccagacc aagaaaatgt tggctctaaa 61140
   gcaagaatca gtttgataca cgagacaaat ccaggaggca actactctgt ctgaagcaca 61200
   ctcagcacag aaaaggtggt cctagatgag gcaggagaaa ttggcaagga gtgggtcata 61260
   tagggtcttg caggctacaa ccagaagcaa gaattttact ctaagaacaa tttgagagcc 61320
   attggaaggt tacaaagcag aggagcaatg ttgtttttta tttaaaaaga taggttgctg 61380
   tgaaaaaatt ggaaatcatc gaagaattaa tgggatttct tgtcaagagt atcagattct 61440
   atcttcattt tcttctgctg tcttccaaaa tcccattaaa atgacagtaa agatataggg 61500
   aaaaaaatat atatccatgg ggagaagagt aggagaagtt taatgacaac actgttttga 61560
   agcctggaaa gcaaattaat tagtagttag tccaaagcta tcattggaga aagcagagta 61620
   gtaatgcatt tcatactaca gaaccacaga aagaatcaag aactggtgga accaagcgcc 61680
   tccaaaagtg agaaagatac agttggggag gagaagtagt tgacgctcca tttaccaatc 61740
   atttagactc ctcgttcctc tcctacaccc aaacagaagc ttagacattc attcactgga 61800
   gaggataaga tagagtgtct ctagaacagg ggctaccagc acagttaaga atcttatact 61860
   gaaaattcat ccccagtcct cttctcccaa tgattctcag gtttacacct tccagggtga 61920
   ttggaagatt cttctctggg gatctaactt gtcattttgt tttccagcac aatggccgca 61980
   cttgggtcag cctacaggaa agaccatagt caataagccc tcctcagact caagagcttt 62040
   ttaataagta ttcaatcctc tgttcaatat gaacaattaa aaatcaccag actttagagg 62100
   aaagcatctg ttatgaaatg cagagagtaa atagggggaa aaaaagcagc ttggaggaaa 62160
   tagactatgc aagtagatga aacctttaaa aaaagaacta ccattaatat ttttattgat 62220
   aatctcagaa agacaaaaga aaaaaatcac attcacaaaa taatagtggt attcaagaaa 62280
   aattcagaga acaagaatgt gcttttggaa attacaaatg acagaaaaga aaaactcaat 62340
   agaaggctta aaggagtaca ttaagaatac atcctggata tgcaaagata cggaatatag 62400
   atcagaaaag ctgccctcct atatagacta gagaatcaat atgttaatgg tctgatataa 62460
   taagattccc agagagaaac tgagaataca aaggataaaa ttaataaata tttggaaaag 62520
   atttcataat ttaattacgt gagtatttaa agagcacagt aaagcatact gtaatataag 62580
   acttacagtg aaatttaaca cattgtggca aataggatat ctataagtat tcagagtgaa 62640
   aataataaaa tcactaacaa agaaccaaga atcaaaaata gactggagtt atcaatactg 62700
   aaatttagaa aaacaaacaa acaaaacttt taaaatattg aaaaaaaatc catccaagaa 62760
   atctatactc agctaacctt ctaattgtga gggtagaata caggtatttt ggatataagg 62820
   gtctcaaaaa ctgtatgcat cctaaaccgt tccccagaat attactgtgt aatgtactcc 62880
   tccaaaatga ggaagttaac caagaaagag gaagttatag tatacacaaa acaggagacc 62940
   taacatatga aagagagaga ggatacagga tttccccaga aagatggtga atgtgcccag 63000
   cctgacaatt gtttggatgc agaagtagtg gtagcaaata ttttggttta tccagggttt 63060
   ggggttttct aggtaagcac gatggagaaa cagacacatg aattgacagt gaactctttc 63120
   actgtcattg ttacaatgag gaaatattaa aaccattctg gataagaata aaagtataga 63180
   ctagaggaga atgatagtga acaagtagca aaatcaatgg tttagaagtc ctgaagagat 63240
   agaaaaagtt tctagagagc aagaactagc ataaatgagc taggaaagga ggtggtagtc 63300
   agagacgtta aggtgatcaa gatgttagag aagatatagt ttctagtgat gaccagggaa 63360
   tgtgtggttg agggaaggaa atgtggttgt caaatacaga gagctcagaa tgctggagtt 63420
   atctgcacca ataatgaagt cacccatagg gtgacacaag tagaggtata aagaaagaaa 63480
   ggccaacagg aactaaagta ttcaattaat gcagggaatg aattaagata aatgacctat 63540
   aaattcatgg ttgacagcag cacgaagggg tagagcataa tatatccaca tgacacggct 63600
   tcaaataagc tggggcattt ttgaagaagg acacacctat cccaaatcct agccttgaag 63660
   tgcaagagga atgatagaaa acaaaaacaa aaaacaataa ccctcactag aattggaaga 63720
   tttatcctta attaatgtcc agttcagtta aggcaagaca atacgtagaa ttttcagaga 63780
   caaaattgct gatataatag agttttctaa tcaaagaagt ttgcagagta gaaggttatc 63840
   agaaggtggg ggatttatca acttagagat atacaaagga tgtaggatct gccagggaag 63900
   aataatgatg ataatggttg atagagaggc attgacttct ggtcgtacta gaagtaatta 63960
   tggatgtcaa gcatggtcaa agtcatcatg gtagtctcga agaaccctgg gcattggttc 64020
   actgggtagc ctttctattc aaagcgcggt accagggcca gccgctttgg tatcacctga 64080
   aagtttgtca gaaatacaaa attacaagct ctaccccagg cttaatcaat cagaatctta 64140
   ttcaatctga ttgattctta atcagaatct tattcaatct gattgattct taatgaatca 64200
   aaatcttatt caaactgatt gattcttaat caatcagaat cttattattg tgtgtacaaa 64260
   aaggtttaag aagcattact tcaattattt acctctaaaa tatggggtgc ctgttttctc 64320
   ctctagctcc tgtcagtgga ctcaccagaa gttgaatatg ttgacttcat catgggacta 64380
   tttctcttta aaacatcatt atgagaccac attggtgacc acattactaa actataactt 64440
   tctccgcctt cagatgggga ctattttgta tattattagt gcctggtgcc taataaaatc 64500
   ccaaaaacag acacaaaaaa tttcctaggt gttgtggaag cctagagagc gagagggact 64560
   aggcgtgcag gaggaatctc ttttgcagtt gggctggtga tgtggaaaaa gtaggactgt 64620
   aggactggaa aagggaagat tgggtaggtt gataatttgg aaaaggtaat taaaatataa 64680
   attcataaaa agatgtcaaa tattttaatt taaaatatac acactacaca cacacacata 64740
   cacactacac acacacacac acaatgccac atttgtatgt gtattaattc cttcacagat 64800
   attcttttgt agggcccaag gccttttatc tgagtatgaa ctggtttctg agagttctgc 64860
   aaacatcttc cttgcacaaa gcatactaaa aatgcattgt ctaaagatat ttatttctgc 64920
   tgtctttaaa gtgaattgtg aagcaatctg agtacagaat ctgcctagtt ttttgttttc 64980
   cctatctttt acaattctca tctacacata acagcatttt aaaaatgatt tttttctagt 65040
   ccttccgata aagtcaacct tgtccttttt gcacttatat tttgatggta tataaagtac 65100
   ttaactaaat tgtccaatac tagtctctgg taatataggg gcttaaaaaa tccatcatca 65160
   ttggttatag taatgggcaa ttttagtgtg ttgcttattt tctcctatag ttaactctgt 65220
   gatcccctct cagatttcag tgaacttgcc catcaggtta accctagtgg atctggcagt 65280
   gcagcttacc aaccgagagt tcttaggact tggtgctctg gaatgaaggc agcatgccag 65340
   gtcttctgta gcaaccagac catttgccat ggcagcttca aattgagcaa gaattccaca 65400
   atttcacact tgagtgcagt taagtctcag gtggatggca gccaggttcc ataaataact 65460
   ctcggttatc attgttccaa aactcaagac ttttaaaagg cactacctac cctaaaggtc 65520
   cagtccttaa tgacatgatt tgtatgacac ttgagcaata gcctgaggct gccaaatcag 65580
   gtaagaagga ctttcccata ataatatcaa cctactctaa gtagatttca gttggatcat 65640
   gatatatcaa catatgagtg atacatgcag ggcactcact ttggtttttt tgaaaatatg 65700
   tttaagttca tttatgaaat atttttgaga aataattttg tgtttagaga tataataagc 65760
   tctaagagaa atatatgctc atagatattc atgcctagtg gtacttacct tctactttaa 65820
   aattatttgc ctttctttct gacccatgag acatagctca aaagtaataa caagtcttac 65880
   tctgcctacc atcacttctg atatggtttg gctgtgttcc caccaaatgt catcttgaat 65940
   tgtagctccc ataaatctca cgtgttgtga gaggggccca gtagtagata attgaataat 66000
   gggggtggtt ttccccacac tgttcttgtg gtagtgagta agtctcacga gacctaacgg 66060
   ttttataagg ggaaaccact ttcacttggt tctcattctg tcttgtctcc ctccacgtaa 66120
   gacatgcctt tcaccttccg ccatgattgt gaagcctccc cagccacgcg gacctgtgag 66180
   tccatcgaac ctatttttct ttataaatta cccagtctca ggtatgtctt taacagcagt 66240
   gtgaaaacgg gctaatacaa cttctgatag aaaattaggc acataataca cattagttga 66300
   aataatcaac tggagaatgt aggctttttt tttttttttt gaaagagtct tgctcagtca 66360
   cacagactga agtgcagtgg tgtaatcata gctcactgta gcctcgatct cccaagctca 66420
   agcaatgctt ctgcctcacc ttcctgtata gctggaacta cagatgcgtg ccaccacacc 66480
   taggtaattt tatttttaat ttcatttttt aatttttact ttttgtagag atgagatttt 66540
   gctatgttgc ccaggctggt ctggaactcc tgagctcaaa tgatcctccc acctttgcct 66600
   accaaagtgc tgggattaga agctgagcca ccatgtccag ctcagaatgc aggcttttta 66660
   atacaaattc aaacttacaa acaaacaatg agaagatagg gtcaataacc agtatatgct 66720
   ctttttcgta cagctgaggc tctgccagat ttaaaatatg aaaccgatcg tattttactt 66780
   cgggaacaag aatacaggag atctaaatat aaaatgacga aaaacaaaaa aaaaaagtgg 66840
   taaatctcct aagcaagcgt caaatttttt taaaacatca ttctatcaga tgcttaagga 66900
   actttatcag aaattaatac aaaattttct aaatattgaa cacaggaatt ttgctattcc 66960
   tcattttttg tgccataaga aatatctgat ggttttacaa acaataggcg cttagaaatt 67020
   cagcatacaa aattcatttt cccccaccta ttatcaatta cagattttac ccaaaagagg 67080
   aaaaaaactt aaccaaatga ttacaatagt gttgaggatt ttttattgtg ttttccacat 67140
   agataaaaaa ataaggcttt ttgatgaaaa gaatccatta caaagtcaaa aatccattac 67200
   aattataatt gaatcagtaa caaaatttag ctttaaatga gtcaagtatt ctgcatttga 67260
   aatttaatat cacaaacatt caagattagt gaattttggt aagaaaaaaa tactagaaga 67320
   aaggaaaagg acaccttttc aacagatagt aatttataaa aattttttta aaagtgcttt 67380
   gggaaaacac acagtatcat tacttaagaa aagtcattta aggaagactt aagtgcttca 67440
   agtggagtgt attacagact aaaaaatgtt ttaaaatttg ccaagaaatt taagtgttaa 67500
   aaatactctt ctccttattc agtttcatgt ttaaggaaac atttgacaga caagtaaacc 67560
   aaacgcaaaa aaaagttcac ctgcatttta aactaataaa ttctggatct gtaaaagctc 67620
   ttggtttgta cacagaggcc aatgctgaca tttattgatc tatttttatg tagttaaaaa 67680
   aatacagtaa caaatctttc ttcctatccc cccaaaaaac taggggaaat attttaaatt 67740
   gtgagtgtgt gaatgtagct atatatatat atccctaagt gtacaaaaca cacaaacatc 67800
   actttacttg gaaaattatt ttcatcatac tgtaaacatc tcttccccta catctggaca 67860
   ttttgaaata gtctttggta ttactagtta ttgtgctttg aaacagaaac ttgcagaatt 67920
   tctgtagtag tgctacataa agatataaat aagaaaaatg cacttggaat aagttacatt 67980
   tagctgcttt tgcataattt tcaaaaacta cagtgtatgc ctagtcacag ttttatgaga 68040
   aagaatattt cctttttcaa cttaatttta aggaacactt aatcattttg gctaagtatc 68100
   catttttgga gtggatctga tgggttgcat gacactaaac ttggatgctc tccatttgct 68160
   gaaaggcaca tttttaagaa tggattgtat agaagttgat cctagaataa aaggaaacaa 68220
   cagacattaa attccaagac tcatagttaa gtgacaaata tgaaaaagat tttatggttt 68280
   cattgaagca aacctaatag caaatctttc taaggtgagc catgtctgat aaactctttt 68340
   tggcttctag tgtaaatgcc accttttaag ctactactct tcaattagta agatattaca 68400
   taatgaagag aggcagacag aacaaggttt gaatcacatt ttcaaacaag aagtattccc 68460
   ttgatatatc cataagtgac aaactcttaa aaacatcata aattataaaa gccagtgtta 68520
   acgttttcta gtactgcaca aaacaatgtt caggtaagaa ttgctatgat acagctaagc 68580
   cttaaacatg tttaaaaaca gaagagacct gttatgtatt ctctcaattc taggtttgtg 68640
   aaaaagtgct aaaacagggt agaaaaaaat taatcacttc aagtgagaag atataataat 68700
   ttttcttctc tctgacagaa aaaaaatatg actgttgcta aaagcattat aagaaaccct 68760
   aaatagtaaa aaacaagcga ctaatattaa taattcacag aagtgctcat ctgcatatat 68820
   cacggttcaa ccaagcaact taattttttg ttactaagag tacttaattc atagatatat 68880
   tctcttatca catgggtaga aacacataat aacaaagtta ataaagggag agaaattctg 68940
   atagataata ttaatgtacc atgttccact cattttccca gataagcaag tagagctaag 69000
   aaaaatagtt aaatagatga attaattgca ctgaggtacc ttctggatct cccatatctg 69060
   ctctccagtg acaactgtct tgtgaccttt gtaggtacat gccttcagct gaacgagacc 69120
   ttgagcagca tgaagacata attccttctg gatgttgtgc cgaatttcat gttgagcaga 69180
   gtattcaaag tactatggaa ggaatagcac tgttactgac aaaagcaacc catgtgctca 69240
   gttgcaaaat aaataaataa atcagcaact cgttaaataa taaagtcatt tctatttcaa 69300
   caaaataata caataaaaat agttattaga aacataagct gagtttttgt gatagacgat 69360
   acaatagcct tccttcatca acaggtggtt acttcattaa caaggtgctg tcttcctccc 69420
   cataccagaa gttggaagtt atcaaggaga gtggaggcta tcccaggcat tcctcagaaa 69480
   agggaatgaa gagttataac aagaacatta attgcctctc tcccacaagt gttccatctc 69540
   tcttcttcat ctcaatcaaa ggtgttttca ctaaactagg gacttcaggg tgttcaccat 69600
   gcctcagttt tctcactaga aaatgggtaa tttaatagtt cctaccccac agggttatga 69660
   agataagtgg gttaatacgc ataatacatt tagaacaatg actggcacac ggtgagtttt 69720
   catacatttt agctattgtt actactgatg cttctctttc tccacatatc caagtaattc 69780
   gtcccttagc tgcactattc ctctcagatg ttaacatccc atccattctc acttccgtgt 69840
   actcacttct gttactcaga ctctcagaat ttttactgaa acacctacag ctacttccaa 69900
   tttctgttcc tatcatccag caatccaatc tgctgccaga tagtctttta aagcattggt 69960
   ttgatccctg actgcccttg cttaagtgaa tcactactgt tgctaagata aagtccacac 70020
   tccttaattg gtacatgaaa tctttctttt tgatcttgca tcaatcattg ccccagttat 70080
   atattatgct tctttcacac aattctacca atcagaacat atgtatctat aactctaaga 70140
   ctttgcttta cagtctgtcc tctgcctgga atacactttt gacccttttc tgcctggcta 70200
   actccttttt aatccttcaa gagccaactc aaatactacc ttccttcttt ggctttgcct 70260
   gaatttgccc tgctgcacaa aattacaatc ttttcaaggt tcccatagta catcagagat 70320
   aacttttcta gcactctatt agaattgtgt gtggtgtgta gacctggctt ccccatagac 70380
   tgaagtcagg caatttatcg agtcataaca cccatcacaa atccaatatt tcataatatt 70440
   gacattataa agataaaata agttaacata attgattttc aataatgaaa taattagtag 70500
   gagcactaat atttgttaag tccttattac atgttaggca ggcactgtgt taaactcttt 70560
   atgtgtatta cagtaggcac tctacaaaca ttcgctgaat tcacagagcc atatatcttg 70620
   agtttcccta ctcttagtct catgcttaat ttatctccgc ccacaagcaa aacatcagtc 70680
   atttttagaa ctagtagttc ttcagatgga aacacaggaa gaaactgagt ttagatgatg 70740
   aaaatagcat tctagctata cttggctaag aaagataagc cttaagatct caaaaatggt 70800
   aacaaaggct gtcgagtatt ttacccatta ttaaaaagtc attaaaataa aaattacaaa 70860
   aaatggaaaa tattgaaagt actttggtaa caaaaaagag cacataattg gataactgtg 70920
   aatgttggag agacccatta cttagaacga gtagagaaga ggcaaagcag taaatataat 70980
   caagttataa tgactcaaac tgcattacac tagtaatatt ggccagctta gtcacataac 71040
   caaaatattt atttttattt ggcatacatg aagttttatg ctataaaaat attatctgat 71100
   caaaagtaga aggaaacatg aaaaaatgat caagttatat aatatctaaa aattttagca 71160
   ctcagggaac taaaatttgc aaaacggttt aagtgcaatg cttgattaaa aataaaatgt 71220
   caaggaaata aagcaaaata aaatcaatag ctgtgttagg gtaacaaaag ctgaagtttc 71280
   tatttagtat atacaccaga taatatttat tcttctgctc aaaactcttc aattgtttct 71340
   gtcaaagtca gaattaagtc caagtttctt aatggggcca gttaagccct acatgatcag 71400
   gtccctatca atgccccgac ctcatttcct agtcctcttt gctagaccac tctgctctag 71460
   gtacactggc tatctgcttt ctccatgtat ctgcctctgg gcctccactc ttggaaatcc 71520
   ttcctccaag atgagcatct tctcctggaa cactctctca cagtattcag gcctccctct 71580
   aagatcgctt ttccaaaaag gtcttccctc acctcttaaa ataaccaccc taccccgaaa 71640
   cacactattc ccttacctgc tatatttttc ttcatgccca aaactacata tttttttctt 71700
   tgttcctgtg gaacataagc tccacaagat caggagcttt gtctttttac tacggtaaca 71760
   gtcagtacaa gctactttat aggttctaaa caaacactta ttgaaagaat atgtgaatgt 71820
   tttcccatcc cccaatttct aaaagatagc tatattctat ttaaaatgta aacatatttt 71880
   tgatacagag ctatcataaa gctgttatta tctttatgaa aatgataaca gaaaaatgga 71940
   aaaaagcaat gtgtacaata tatcttcagc ttttaagata aaataaatgt aatgctagaa 72000
   gaagcctaag agaaatttat agggatttac atttactagt aagattttaa caaatgcaga 72060
   aatatttccc tttgctcaaa atcttaattt ttacaagcat agatcagtgt tatttccaaa 72120
   gaatgcaatt atttatgtaa ctatttttct tctaatatat ggagggtaag aaaattctta 72180
   aaaagatgca tatagattta tgcacataca catatatgta cagatatata tgtatataga 72240
   tataggtata gatgtatgtc tgtctatatg catccatata tatctccatc ctatttcaga 72300
   attacacctt aaccatccag caatagatcc agttcaccag ctctggcttc tgggcatctg 72360
   tatcatctgc cctcagtaga ttgattggtt taggttaata aggattagaa ctagattaag 72420
   ctgagcctaa tgggtaggaa gaaatataga agaggatgga ccaggagata aactggctct 72480
   tttttcctaa aactgacatc tcaaagtttc gtcccattaa aataggacat catggatatt 72540
   aattattgca aaaatcctcc acacacatct tggactctac accaaaatac ctgcaatgcc 72600
   cagaagagat actccaggct cttctcttca tttcactttc cttcacactc ttcttattct 72660
   gttgccattc cccacagcca cctcactagc tcctacctac tttttttttt tttaatgttt 72720
   tcaaatatca tctttctcag gaaaatgtgt ggacacccag tctcattcaa tgcccttttc 72780
   tataccccca gaacactctg aatgctttat cctaaggaac tgctagttta tagatttcct 72840
   aaaggagtta gagtctaaat agcttgaact catcagagta tctggcatgc agggctcact 72900
   caatgaattt gttgaatgaa caaatgaggg aaatagtcta atctttccag gagttaagct 72960
   gcaaaaggta aggaaaggaa gcttaaaaaa acagaaactt tgtatataac atgttctcta 73020
   cccataggca agtaaatagt aatatttgac atgtgaatct taatcagtaa aatagtcaaa 73080
   aggtagtaaa gcaggtatgt cactaatcaa ggagaatctt cctcttttgt tggagaagga 73140
   ggaacatatt gaaataatat tgtggcccac aaaattaatt tataatgctc tattttgtta 73200
   ttaacacttg atgtaatttg tgattcaatc cagttatatg tggcctttat gtgtaaactt 73260
   gcaaaatcac catgccaata gtcatgcaaa agttaaagca tcaaagagct acatagaagt 73320
   ttctttttaa aaaaaatttc aaatggcatt attctattat tttctcattt tccttttttt 73380
   ttttttaatt tgtttgtttg tttttgagac agagtctcac tctgtcaccc aggctggacc 73440
   gcaattgtgt gatctcggct cactgcaacc tccacctccg tggttcaagc aattctccag 73500
   cctcagcctc ccaagtagct gggaacacag gcatgcacca ccacgcccga caaatttttg 73560
   tatttttagt agagatgagg tttcaccata ttcgccaggc tggccttgaa ctcccggcct 73620
   caagtgatcc acccacctca gcctcccaaa gtgctgggat tacaggcgtg agccactcag 73680
   ctcggcctcc tttttttttt tttttttttt tttttaatgg ttaagctact caaattgttt 73740
   tcagagccag agtaaaaacc aaaaaaaaat actcatcaat atcaattgcc aaactcagtc 73800
   tgaaaacatt tcacacacag cttacccaag tataaagctg ctgtgggact tctgaaaaat 73860
   aggcaacatc tcacttgtgc ttgtaaatgc tttacaagtg aaggattttt aatgcagtgc 73920
   tcacctggtt tcccccaagt ccatgacatg tatacataat taatggtttg cctccttgat 73980
   tgttttctcc aacatccaga catagaggct gaccaacgct tttaatctaa aggaaaattt 74040
   tcaagttatg aaaagttttt taatccatgt gatttatata tttttaaaat aatttagaaa 74100
   gtaatgttaa tgattataat gtaaaatctc aaaagcaata aagaaagtat ttcagcaatt 74160
   ttcttagaac cacataaagg ttggtggcaa ggagtatatt aattaaaaaa ggaacaggaa 74220
   aatactcacg tatccagata taacaggatt aaggtctggc acatacacct ctggataaat 74280
   gttgttcaga taccatgtaa aatttttaca ctgaaggcgg tgttttattt caaatctttt 74340
   tgaaagatca ccaaatgctt tctgtagaaa catgagaaat gaagggaatg cttaattaaa 74400
   acaacattga taaagcactt aaaatactct tatttgtatt tctatttagg tatttttaaa 74460
   aatccatgct aaaaattcaa gaggcaattc aacagccttc atcattcatt caagtaaaaa 74520
   ccatgcctgc ctgttcattt ctggctttaa aagagagtca acaccacttc agtgctgatt 74580
   aaaatactaa aatgatgcta ttttattctt catttgtgcc acaaacattc cctacaagac 74640
   aaagatttct agtaaaatgc tgtgggcaaa ggaatggtgg taggagcatc caaaattggt 74700
   cccaacttta tacctaaaaa tgatctgcaa agtgaggtgt cagaaatcag gtatagacaa 74760
   acagtgccaa tacacaatgc aacttctatc tcttctccat caagatcaca gaaaaccacc 74820
   agcaagaaat aacccactgg gagtaatttt tctagtttca ggatgttgct tgttcttaac 74880
   aatatcccca actgaacctt tagtagaaaa aggcaatttt caagttaaat gaattttgct 74940
   ttccttcatt ttaacgtact aagaaaataa cttttttaaa aaaccattat taaacttcaa 75000
   ggtatttgac ctacaggcaa aatattagag aaaaagtaca tgtaatatct aaatgtatgc 75060
   aaaagtaaat actcaatctt atcagttcaa ctaaagaaaa tattttctta tgccaacttt 75120
   gatgtcttga acccaggcgt tttagcaaaa agtatgagtc tttaggactt ctggctagct 75180
   tattacattt accatgtaga tctatgtgtc ctgtggtaac caacaggtat ttgctgattt 75240
   atgtgcccct ttgtggggga caagcagagc ataagcttca tttagtgccc acttgcctgc 75300
   ttagccaagt actattgtac agtacacaat ctgcccaatt tgtcttcatg gccttgggta 75360
   aaaagatagc aatctaactc agtagtgctc ttaatttgca tcctaacagg gagatactgg 75420
   agaccagaga aaaagggaaa gaggtaacca aatcgttctt caccgcaaca gccaaaatat 75480
   tgataccctt gactcccaat aaatactcca ccttccctca ccctgcaaat tagcactatc 75540
   tatttcctac aaagagatga gtactataca ctgaagcatg gtcttaaact atctagttaa 75600
   tttaccttat ctaatattat taagactatt ggaactcact tctaatatat taggtttcat 75660
   gattctgcag agcagaatct atttatggca ggttaataac aagagtacca tatatttgtt 75720
   agcacttatc actttcttat ttcacagcta ctctgggaga gagacagcat gcgtatttca 75780
   tctcttaaaa cagggaagga gagaaaagaa atatgttaag catgcactcc atgtccagcc 75840
   accttgcagc cactctatga agtgtcttcc ctattttaca ttctcaaagg ctttgccaaa 75900
   gtaacatctc cagtaagtag tgaaactaga gcttgaaaaa aaaaggcttg gcactcctgg 75960
   tttattctca tgccacatta agacactgtc tctatctcca gtgcaaaaag tcacttagaa 76020
   cctttattta ggtgttatat ctcctaaaac cagtaaaaac agttctactt aattttcttc 76080
   gagtaaaata ttgaaccagg gcagcaacaa aaatgaacta tctttcatat taaaaacatc 76140
   aagtagaatt cctgattttt aaaaagcagt ttacttagtt tttttcttaa aaaaggaaac 76200
   ttggattcat aagtcatgta ctttaagagt taaaaaccta ctttcaaatt ttttgatgtt 76260
   ttgtacttga gatgaatcga cgcaaaagga cgtgtgaact tgttatagat tttattgcaa 76320
   tttaactact tagctttaac ttacttgttt aacaattttt gctgcatctg tatttctcct 76380
   ataaaatatt tccttgtatt catccatcca gacttctgca aggcgaactt ggtttctagc 76440
   aatcacctga gtgccttttg gaaagctatg agggcttttg ctgcgaaaaa catgtccaac 76500
   aacagagcaa ggcataatct ccaactgccc accacattgc catacctgat aattaatgat 76560
   atttgtttaa atttacagta ttttagaaaa atcaaagtgt tgcttttaag ttcaccttta 76620
   aggtattatg aaaaacaaaa ttagagaaga gattacaata ttacaaatag tctctgtccc 76680
   ctgccatttc aacttaagtt cttaagtgta aggaattatt ttattaaatg gcatagacac 76740
   taaagacaac agaagtgcat tttcagtaag ttaacaaaat ggtgatttca gatttttcat 76800
   aaaaatgaat tccaggcttt tgtttttgca aataattatg taagttctct aatatgcaca 76860
   gaaaactaaa ggctcttatg catttgactt ctgtctgaac tactgacata gtcacttcac 76920
   ttaaactaga gtttctcttc aaagtttgga tataagtgga ctcagaccct atttatctct 76980
   tgtatattca attttgtaac caagaaaaca attccagatg gctattatta ccaatgatca 77040
   ttctactgaa cttgcaaagt agaggatgat tatttaaaaa ttcatgccaa tttagagaga 77100
   aaacgtcaaa aaaaatcctt attggcatta atgctaaata aaatggcaaa tcatgaaatc 77160
   aatgcctttt cacttaaata tcttaataat tgactctgcc atagttacca agcaaagaat 77220
   cagaaaataa gtcatattta atactatgtg cttgtagtag agcaagtgat tacaaatctc 77280
   tttctctcct ttctttcttg aacaaatgag aagactcagt gatatgttca attcaacaaa 77340
   ctgtgcttgt gacccaacac gtgctagggg ttggaaatac tgaaatgaat aaacaagacc 77400
   ccgccataag cagttcaaac ttggctaaga tcatcactgc cagtatgtga tggagccaga 77460
   gccaaaactt aaatcttcta actcctagtt cagttctttt tccactacat tccagcctaa 77520
   gagatgaaaa atgaatgata aagttactgc agaaaatgaa actcatgaaa tcaaatacat 77580
   aaggtacata cggagtctac ccaaagcctt tttttatatt aacttataat atgtatagaa 77640
   aaaatcaagc tcattcattc caaacagtta agtccaatgt cctttaaaat tattcataaa 77700
   tgtttttcca ttctaactcc cttgatgatc aactcatttc ctgaagcact agatttaatt 77760
   atatttatct tggctggaat tcagtagcta caagtgcatt aattggtgtt actgcctgga 77820
   cacaggagtt taactgattc ctcttgttac actggcgaca atgaataaat atattaagtt 77880
   acatattaaa agaaaatatt cttattccaa aatttccagc aattaagttt cagaaagcac 77940
   aaaaagtaga gtgcacacat ctgtaatcat atgtaccagc cgattagaac acaatatatt 78000
   tcattgttta atatatctgc tatatttaat ttccataaac ttactctgaa agacatttct 78060
   atattttcac ctccccagat ttccatttct tcatcatagc ttccaatata ctcaaaatat 78120
   tcttttgata tggaaaaaag tcctcctgca aaagtgggtg ttctgaaaaa taatccattg 78180
   tcaaattttg ttataaaaac taaacaagat aaaacagcat attttaagaa ctgaagttaa 78240
   aaaattaaag ccatatcaca ttttcaaagc ttttattcag attaataaaa actagaaatg 78300
   ctatcttcaa agtcaagatt tttggggtcg gggggagtac ttcgtgtcct ctcccattgg 78360
   agtaaactta aatgtcttct cctaaccatg ctttacgatt tttaagtaat gattgatgag 78420
   gaagacataa gagctcactc actgctacct cttactcagc aacatcatgt ataagcaagt 78480
   acacaaatga atgactttta gattctcttt atatagaata tgtgtgttct ttatcaatta 78540
   cctcagaggc aattgctata aagcaaacag tgtgtacata ttcaatgtat ggtatagggt 78600
   gtaaatataa gactctgaga gcaatcatct tacttaattg ggtaggtttc atctttcctt 78660
   ctttgcttct catgatcagg aagcgactcc cagccaaatg aaagactcca gtcaaaattt 78720
   ccacggttat ggttacttcc ataaggagaa ggtttgttga attcaaacgt gttcagatct 78780
   atggatgcaa tatctggact tacgacagcc gtgtagttct cagctattct ggccaacaga 78840
   ggttctagcc aaccatagaa acactcacct ggagggaaca gaattttaat taattcaata 78900
   aaaacattga agtttttttc tttagttatt taggtgaagg tttacagaga aatgagaata 78960
   acagagtcaa tttgtgtttt gaacatgtat ataatggaga tttctttctt cctcagattt 79020
   attgagatat aacaaataaa aattctatac atttgcagtg tatatcatag tgtttatata 79080
   tgtatacatt gtaaaatgat tattgcaatc aagctaatta acatatccat caccttatcg 79140
   gcttatggag cgggtggtaa gaacatctaa gatctactct tagcaatttt tgagtagaca 79200
   atatattatt attaattaca gtcaccacgt tgtacaatag atctccagaa ggggagattt 79260
   tttaaaggtt tattgtgatg agatgagggt aggatgaaaa aggtgaaaaa agattcacta 79320
   cagttagaaa aattccccta cagatgatga ccttctttct tctacaaaag atgcaaaata 79380
   acaccagtgt atttaaagtt tagatgatgt gttaaaatgg cctatgagtc aaaggtagct 79440
   ttttgctgtt ccacattacc tggccctaaa agtgttctga tttatttaaa ctcgtatctc 79500
   cctgaataag gtacagtaat tgaaattttc atttatctga ctgggtctta ggagaaaata 79560
   aggagtcagt ataaattccg attttagcaa ctggagaaga aggaccattc aaagctgccc 79620
   cagacctaat ataaagggag ggccaaggag tttcatgtca tgataggtct aagccctggt 79680
   cacaggccag gctgttttat gtaacacaca gcaagttcag ccaatgcatg tgctgtccta 79740
   ttctgggctc cccacaaagc agtatttggc agtcatggtc cgggaggacc gtcagacata 79800
   tccacccagt gggtagtggg taagaatgtc cagtatgatc ttattgtaat tttgaacaac 79860
   gagaagaaat accagaggat ggtattttcc tttttaaagc acctctctag cattaccttt 79920
   agtgttctct tcattttcta aatgtctggt attaaaagag aactgaatgt gatggctcta 79980
   agcaacacaa gcacatccaa aagcccttct gtaataatcg gtgtgtgctc aaaactagta 80040
   attgggtgaa acataaccta agcactggcc aattcaaagc tagacactca tacaaagtat 80100
   agcccactaa actcagagct caagggacaa aatgacagtt tagtaataac tagcatgcat 80160
   atgacacatt gactctaaaa atgctttcta aatacttcaa aaactttgac tctgaaaata 80220
   attaaagtac aaagaaaaac caacagcagc aaagtcaaac aaaaattgac aatgtttatg 80280
   taagggatac caaagaagat gggatggggc acccctaacc tggcataaaa agtatatata 80340
   taaacatatt ttggttttgt ggtttttatt gaaaattcgc agactccttt ttatgccaag 80400
   gagtctgcaa atttaaaaaa aaaaacacac acacaaacta aaatataaaa aaagcagcag 80460
   tactaggacg aagtttaatg atagggtgga ttatattcac tcactcattc attcattcat 80520
   tcattttgaa acggagtgtc actctgctgc ccaggctaga gtgcagtggc acaatctcag 80580
   ctcactgcaa cctctgcctc ccaggttcaa gcgattccca ttcctcagtc tccctagtag 80640
   ctggaattat aggcacatgt caccatgcct ggctaatttt tgtattttta gtagagacag 80700
   ggtttcacct tgttggccag gctgatcttg aactcctgac ctcaaatgat tcaccaacgc 80760
   cggcctccca aagtgctggg attacaggaa tgagccacca cgcccggcta catttaaata 80820
   gtaaataagc tttcattcac tttaaagtgt gggaatcaag aaaacaaaca aacaaacaaa 80880
   caaacacttt tttttttaac agaaaagaaa catggaaaaa aaaaaaagag gcaaggataa 80940
   ctggctggaa cagctagtaa ctggagaaca ctgaagttga ggaaacttaa acacaaaagg 81000
   agataagaat tctgcataag tgaaaggaat tttctccaca gagctgttac ctgcttgggc 81060
   tgtcattgct ataatcgcca gttgaaaacg ctgttaaaga aaatgcaatt aggcatttag 81120
   aaaagaaaag acttccttac cttttaaatt agagggagag ggagggaaaa tgttacaacc 81180
   atatccatac atatatactt acagtgagca tctaaaaatg tgagcgtttc agctgttgcg 81240
   actgttgctc ctagcaaccg agcagtgatc agaccttttc tttctctttg tctgactatt 81300
   tttactatag aaaattgttt tacatattca tctagtttat catgtaagta ctctgtaagg 81360
   aaaaaaaatc agggttaatt ctttctaaat gcattttcat atatagctta tgaaagctaa 81420
   tgaaaccaca gagcaatgat ccttccaaat tcataatctt aacagttgca tgtgtccttc 81480
   cagcaaaatg aaaattttat ctttaactcc ttactgggga aaaaggctag ctatgcaaat 81540
   tatcactact gtaacacaaa acactgtaat gcatacttat tctgtacact tcctatcaat 81600
   aaatataatg tgtttggagg gaagtgagaa acttcaaatg gagtcaaccc tagccgttca 81660
   aaagactcat ttagaggtct gagaagactg actacaagag tgaaagatat ggaatttggc 81720
   caaaaagata ttcaatgctt tgaaattggt atttaaatct ggaaatctta aagaaaaatg 81780
   tcaaatatct aaagacctct gatatttaag caaggatcaa aataatttaa caccaaaatg 81840
   ttgagccacg tcagaaagtc cacagctata gcagtttggg acgattccta cacagagtca 81900
   gcagaaaaat ttgcgaaagc tttggagtct cctgacagtt ttatttagta aaatatgaaa 81960
   caggcaaatt ttagtattgg gtatatactt taagatattc aatataccaa attggataag 82020
   gtttgtaata agcaaaattt gagatcatag gttttaaaaa agttattaac aaatgtcaat 82080
   ccagtgttgt ttttttgtct ggttttgttt tgagacaagg tcttgctctg tcgcccaggg 82140
   tggagtgcag tggcacaact cactgcaacc tccgcctctg ggctgcaagc agttttcctc 82200
   agcttcccaa gtagctggga ccacaggcat gctccaacag gcccagctaa tttttctttt 82260
   cttttctttt tttttttttt tagagacggg gtttcatcat gttgcccagg ctggtctgga 82320
   actcctgaac tcaagctatt tgcaggcctc agcctcccaa aatgctggga ttacaggttg 82380
   agccactgcg cccagctgtt catattctga aacaacttct taagcctgtt tttctcaatt 82440
   aaatttttga tttcttattg agtacaaaat gcagttcttc aagcagcctt cttgggccca 82500
   aaacatagcc tcaaaaaaat ttgggaatgt taagaaaatc aataaatgtc ctggggcaaa 82560
   agtttgcttc tgccagattt tagaaggaaa tgcagatgac tttgacttta tctgtgataa 82620
   tataaccttt ttttcaattc tccagtggtt cagttaagag agctgcttat ttaaagctta 82680
   aacattcata agagaggaaa gtgcaggaac aacccttgta gaaaaataaa tctatctaaa 82740
   cactgttgtc tcagagtaat agattaactt tttaacacct tctaactacc tcaaaataat 82800
   tctcagatta agcttttaac accttctaac tacctcagaa ataattctca gaaagacaag 82860
   cagaattgta actcatctta aatatataat cataaacatt ttctgtttta agaggcttgt 82920
   aaaaatttca acattcttgg ttagaattat tttaaatgga gttactacag attgcttcaa 82980
   tataccctct cactgttgga atttgttaaa aaaaaattta cttacccaca acatatgaac 83040
   ttcccgtaaa tttatatctg caccctaaat ttagctccca tgcacaccct ttatttggga 83100
   ggttaagaac agaaagctac atgaaatcag atgccctgat ttcactttat ggcacaatta 83160
   cttttagctg tgtgaccttg ggcaaggaat tttacctttt ctgcgcctca gtttctttaa 83220
   gatgaggata atcggactca ccatcacaag acattgctgg gaggataaaa tgagatatct 83280
   gaccttcagg aatctaactc caaacttgga tttattaagg aataaatttt gtaatcattg 83340
   aaataatatg tatctgtttg acacaatatt taaaatactt atgatgtttg aaaatttaca 83400
   agttagagta tttgttaaat tagccttgtg attccaattt aaaatgtgta atttagtatt 83460
   tgacttccaa ttttaagtgg aaatgtacta aagaatttga ctaacattgt aagtaatatt 83520
   ggaatattta agaacaccga aataaaatag aacatggtga ctctttttaa ctattcattt 83580
   atattattta ttgatattta tattaactca ttcagtcttt aaaacaactc tagaaattat 83640
   gtactagttc tgtatttcct caattttaag ctacacaatt ggattcttct tataatcaat 83700
   ttcttataat caatggcatg ttaagtttaa ttagctgtgg gttttctcta gtgatttata 83760
   aattaacaac acataatata tttaatgaaa tatggcatta ttcccatttt gctgataaat 83820
   cacagcagag aggttgagta acttgcccaa gtcatatagt tggttaggaa aagagctggg 83880
   attcaaatca aggcagcctg actcaaaggc tcaagtctgt gctcttaacc tctatgcctt 83940
   actgtaacag tctcacattg aggactacat agttatgaat aatggtgttt catacatttc 84000
   tgcatttgaa aatatttgaa tatgtccatc cttggattct aatttcattc accataccca 84060
   ctatccaaac tattatattc aagctctgag atggcataca gagagtacca cataaccaag 84120
   tagactgtag ataccacaat atggatttgg gaaacaatct aatttgcatg tgctttctta 84180
   ccatctacac tagcatcatc caccaaaatg atttccttca gcagtattgc aggtgaagaa 84240
   tagagcacac tgtggacagt tctaagcaac gtggaccacg cttcattatg aaaaactatt 84300
   atgacactgg tggtgggcag gggagggcag cgcttaaatt tttgttcaat acatctagaa 84360
   gaagtcagag aagtagaaaa acaattacaa attttattat tttatttcac agctatacca 84420
   ttgctaacat tatcattatt gaatctttat cactgaaaaa ggatagtttt cttgtatgtg 84480
   atataatctc aatgctatat aaaatgtaaa taaatcaaaa caattttttt acaagcaaat 84540
   cactcaccca gagatgatgt ctaccaacag agatgacaac atatcaagga ttacaacatg 84600
   ttattatagc tttctcaaca atgtaatttt aaatgcaaat aacattttaa ataatgaaaa 84660
   taacctacaa tgaggcagca tgtaaaaatg acaaaccctt ataattgctc aaataaaaac 84720
   caaatttttc ttatttcatg cagaaatcat tcatttttca tatttgcaac tttaaaaatt 84780
   gtaccacttt attaattttt ttttttttat ggagtctctc tctgtcgccc aggctggagt 84840
   gcagtggcac gatctcggct cactgcaagc ttcacctccc aggttcacgc atttctcctg 84900
   cctcagctcc cgagtagctg ggactacagg cgcccaccac cacacccggc taatttttgg 84960
   tatttttagt agagacgggg ttttaccatg ttagccagga tggtctcgat cacctgacct 85020
   tgtgatccac ccgcctaggc ctcccaaagt gctgggatta cacacgtgtg ccactgcgcc 85080
   cacaatatta aatcattttt ataaagatta tctttaatta gatatttacc taagtatgta 85140
   atataggttt cttaagtatt tcctaggttt attgaatcaa tttacttaat ctatacacag 85200
   aatcaaaagg gaaaaaatag ctttattgta aaacctatgt tcttcttatt tttatttaaa 85260
   atagagacag tctgtgatgt tgcccaggct ggtcttgaac tcctgacctc aagcaatctt 85320
   cctgcctaag cctcccatag tggtgggatt ataggggtga gccatcatgt ccagcctaaa 85380
   cctatctttt tatgatgttc atcattcagc aaatacttat taagtactgc catttgccat 85440
   gtgttaaaga ttttgctcca ataatttgat ctactatcaa ataagactag cgtttaaaaa 85500
   aaccctttaa aaatacgtag aaataaagat ctaaataaag acctaaatcc atgttaaaag 85560
   tcctgtgctc atgtcaccat ctaaaacatt gattgggcac agggagagag accctaagtg 85620
   cccttgactg attagtggta ctactatcca ttagttaaaa acattactta taaattgatc 85680
   tcaaattcat ttgtatagga atgtctttcc tcaatgaaaa atatcttcat attaagccac 85740
   tggggtgcag gagagtctgc atagtctcca gcctttgagg ctagagatgt agagataacc 85800
   acagtgccaa gcctgctgaa tggaggtgag gattaaatga aagaacatat aggagagggc 85860
   ttcaagtcac tgggacataa aataaaactg tgtgaggttt atgggaaaga taagcaaata 85920
   aagcaactat atctttaagg tcacttaaaa accataaatt ggagataaaa gcccatcacc 85980
   cttcagttcc tctgccaggg ccaatgggga aactgtagcc tccagtgtga tattaggggc 86040
   ctgagaaaac tgccctaaaa aaagatacac aaattatccc attaattact ttcatagtca 86100
   tacctcaata ggaatgtctc tgataccctt ttttaacagc aagtctttag aatggattat 86160
   ttgtagtgat taaaaaaagg agttggattg gggtaaaata tcaagagaag atgtcaacaa 86220
   cagattagaa aaaggagagg atggattctg agtgaaagtg gcattgctct tcagtttctt 86280
   catgatttca aagcatgata caggtgcatg ggcggaaaaa ataagcacct tcttcatcat 86340
   tcataaataa aaacaaatta tttattctaa attgaatttt ggataaacta ccaattaata 86400
   aaaaagctca aacacaaact attaatatac acacttgaac cacaactaca cagaaataaa 86460
   tgactaaata taagcttata ttttgaaccc ataaaagttt ataaccaaca taaactagaa 86520
   gtaaaaatta tgaaaaattc catattgtgg tattttattt ttaaagaaaa aaaaaagagg 86580
   aaacctatga tttgttataa gcattttgag gagaaagtga aattctgagt ataagatttt 86640
   tttcctacct ttatttgcta tgtggttaat taatatagta ttaatcgata attataaaat 86700
   ggttttgaag atttaaagta taaaatttca caaaaattca tactagtaaa atgttatcac 86760
   tttaatcttg caagattttc cgtcagttta aactaggatt tatttgctta actgataatt 86820
   accaatatca agtacaaatg tcatatcaat acaagtaata aaatttcaca atataatttc 86880
   ataaacccca ttcattttga aacaacatgt taaatgtatg ctttgaaaat tgttttacat 86940
   atttaaaata gtgcaattaa aataacttgc caaagtgact gtatcattcc agtcaaacca 87000
   tttgaaaaac ctggcacata gaaacatgct taaattatca gtcacatgac catctggctt 87060
   tgggagaaac taggtctata tttctaatga gcatggaaaa cattagcagt tcttcagaaa 87120
   atgtgttatt tgctgatact ggttacattc aaagtaaaaa acaaatcttt tttttttttt 87180
   tttttttttt gagacagagt cttgctctgt caccaggctg gagtgcaatg gtgcaatctc 87240
   ggctcactgc aacctccgct tcccgggttc aagtgattct cctgcctcag cctcccaagt 87300
   agctgggact acaggcgcac atcaccatgc ccacctaatt tatgtatttt tagtagaaat 87360
   gaggtttcac catgttggcc aggccaggca cagtagctca tgcctttaat cccagcactt 87420
   tgggaggcca aggcgggcag atcacgaagt caaaaaacaa atctttacca cctgtacaaa 87480
   accctgtcca aagtaagaaa taattaaatc ttcactccaa ccaaaaaatg ttgttaatgt 87540
   gcttacttgt ttttcactta cttagcgatt aacattattg aaaattattg cagcttctta 87600
   atgctatatt gggagaaaaa agctcatttt gagctgtcac tgattgtcat gtcaaaagaa 87660
   taaaaatcat gattattgat ttttaatgta gattactttt caatcaaaac ctctcttcaa 87720
   gtcagtaaag tgcaatactg cattctaatc tattactgta tgaaaggaac tataatgtgc 87780
   cttgttatat cagctagaat attttataga tattagtttc tttacacatt catttaaaaa 87840
   ttatgagcaa cctaaacttg aatcaatcag attagaatag ctcaacagaa atctaagcta 87900
   cacagtttca atgttaaggg actcatatac ttggatccag tattgataat cctaaatcta 87960
   gaaaaacctg agtgaaacaa tagaccaaaa gttatcttca agttcactgc caaatgtaat 88020
   tctctaatat ccagaaagca aagcaaaata cacaggcatt gtttgcttcc agtttgctta 88080
   tcttgtactc tttttttttt ttttttttgg agatggagtt tcgttcttgt cgcccaggct 88140
   ggagtgcaac ggcacagatc tccgctcact tcaacctctg cccccaggtt caagcaattc 88200
   tccagcgtca ggctccccag tagctgggac tacaggcaca cggcaccctg tccggctaat 88260
   ttttgtattt ttaatagaga cggggtttca ccatgttggc caggctgctc tcaaactcct 88320
   gacctcagct gatctgccct cctcggcctc ccaaagtgct gggattacag gtgtgagcca 88380
   ccaaacctgg cctatcttgt acacttaaaa acttactgag gggctgggtg cagtggctca 88440
   cgcctgtaat cccagcactt tgggaggctg aggtgggcgg atcacttgag gtcaggagtt 88500
   cgagaccagc ctggccaaca tggtgaaacc ctgtctctat taaaaataca aaaattagcc 88560
   ggacgtggtg ccacatgcct gtagtcccag ctgctcggga gcctgacact ggagaatcgc 88620
   ttgaacccag gaggcagagg ttgcagtgag ccgagatcac actactgcac tccagcctca 88680
   gtgaaagaga aagactccat ctcaaaataa taataataat aataataata ataataataa 88740
   ataaataaat aaataatctg ttgagggtgg gccaggtgca atggctcatg cctgtactcc 88800
   cagcactttg ggaggccaag gcagatggat cacctgaggt caggagttca agaccagcct 88860
   gggcaacatg gtgaaacccc gtttctacta aaaatacaca aaattagcca gctgtggtgg 88920
   cgggcgcctg taatcccagc tacttgggat tgtttacaaa aaatagggca ttctgaaata 88980
   taattaggta ctcaaaaact ttgagtcaaa gtaatcacaa agtctgtgaa ttgctttaca 89040
   catatataaa atattatcca acagttccaa aatcttggca tgtttttaaa catttttatt 89100
   ctaaagaaat acagagaaat ggaaacaatt cctctacctt ctacttccta tacaaattgt 89160
   ccaataattc cccctaaata gatgagaaaa ccacagtatt taggaataaa gaatttcctt 89220
   gtggatagca gtcagatcta gaattcagtt ttctaatttc caagctaata acttttcata 89280
   tctgcactgc ctcaccaagc ataaataggg atatgtagcc tcaatcatta taagcaaaaa 89340
   ataaatacaa caggttaaat aaacagtatt tgctgatttt ctgccttaga gtagctaaaa 89400
   aacagataac ttccttagct cacccctctc tcccctgcaa agcctggtga taaagaataa 89460
   acaataaata ttcttcaaca tactcaggag gtcgagtgtc tggtccaaga tctcggtgca 89520
   aagaaatcct gtcacttgcg aaagcattaa agcagtgttt agcttcccca cgttcctttt 89580
   ccttttgctc ttcaacactt aaattggttg tcttgaatgc tttaccagaa gcaccaggtg 89640
   catttgaatc ctgaggtgga cggtcaagga caggcttcaa ttctgctgct gtataatatc 89700
   cttgcaaaca aggtctctca ccagcatcaa tgttttgcct gacaggtgct cctatttgca 89760
   tttttggcat ggcatcctta atattgttta cagcttctag cattaaatcc aacatcttgt 89820
   ttttgttttt catgttcctt tccatccttg attcctcttt ggaatattga acacttactt 89880
   ctctttgcat taaaaccaaa actattataa agaaaaaaat tactgcacca agcttccaga 89940
   actttttatg gtaatgtctt ttaatgtgta attttactag tcgctttagg tgagccattc 90000
   tgacattaaa agcttgtcac ttgacaaata acagttattt cttcttctgt tacttatatt 90060
   ttttatcata gatttgctga gaagaaggta tctttaatgg tagtacctat aaacagaaat 90120
   gatcgatggt attagtagct attcagtcct acaggcatgg ctcaattcat tcatttaacc 90180
   aacaactgaa cataagctac caatgtactc ttatggttaa tgagaataca aagatatctt 90240
   gtcctcaaga aaattacact tttggaagaa aataattgtt acaatttact acaatttatt 90300
   ggggttgcaa aaatattgat attcctctcc attaaaaaat acattctttt taaactctaa 90360
   tatccactat atactatgta taaaattaat tttataaata tattttgagt taaaaaagaa 90420
   tatcttcact tgataaccaa ggaatcaaag gacctcatta aataaattac aaaaaagaga 90480
   gctccataag gaagtaaggg tatactgata agtactatta ggcatccatc taaaatagtg 90540
   gccttcattt tatgtagttt accctcaaac tgtgatctgc tccagcttaa tgaatgataa 90600
   tacagcagtt tcatcagtaa gtaaactatt attctcccag attttccaac ccaatagtac 90660
   ttgtgactcc tgaaggaacc cctaaaaaca agaaaatcaa cagcatttaa tttgttgtta 90720
   tataacagct agttttaata gctcacagaa atacaagatc taatagtaaa aagagaattc 90780
   attagcttta cttgaagtgt ctgtagggag cctatagaag tcataaagtt aaaaaagcag 90840
   aatttggttt gataaaatat tgcttgcaat tgaccctaag aacaggacag caagtttatt 90900
   aaaccctaat atgggacaac aggagcaaag gaaaagaaaa ataggacttt ttaggaaacg 90960
   attacatact atgagccgcg atagccagtc aagaaatttg cagggacgaa ggggagggaa 91020
   aatgcctgtt cttcctaagg tctaaacata ttttaaagaa aatatcattt atagcaatga 91080
   aaattaactt ttgacacctt ctttaacttc tcttccaaag ttctccctaa atttaaaata 91140
   agaaaaccag gttctaagat ggattctctg gcttttggca ggaaatttta agctgaataa 91200
   acaaaatagg cagaacttag ttctatatac tatttctaat tggagtgctc tatattcttt 91260
   tcagtatgga gtggatactt ctgctccaaa agataacccc aattctgaca tatattgctc 91320
   ataaaagtga gattccaatc tttctgaggt ttggttgctt taagaacttc aggtaaaaaa 91380
   tgaaatgacc tcttaagctt ttgaaaagaa tataagaagt tatagaacca cactatgaca 91440
   gttgtgtaat taattatgtg tcaaatgttg tggtaggcta tggttaagaa attaaagata 91500
   gcccttacct tcaagaagca gaattgaaat ataggaagcc agaggacagt cacaaagcag 91560
   tgtgatatgt gctacgacag gagactaaga aagctacaca ggcttggaac agtggctcat 91620
   gcctataatc ccaacacttt aggaagctga ggccagagga tcacttgaga ccaggagttc 91680
   gagaccagcc tgggcaacat agcaagaccc catcctacaa aaaaatacaa aaattagtca 91740
   aatgtggtgg tgcacgcctg tagtcccagc tactcaggtg gctgaggtgg gaggatggct 91800
   tgagctcaag aggttaaggc tgcattgggc tatgattgtg ccactgcact ccagcctggg 91860
   tagcaggaca agactctgtc tcaaaaaaaa aaaaaaaaaa aaaaaaaggg aaaacaatgt 91920
   agaaggtcag gagaaatgtt aaggaaagtt tcctagagat gacactgttg agcaaaatct 91980
   tgaaatacaa gtacatttta ggcagttaga caggagggaa aatagggaat gtcagatgca 92040
   aaaccataaa agcaagaaaa ggcatggtca gtccttgagt acagcataag tggaaggcac 92100
   agggagagaa tgacaactga tgtggcttga aggtcagtta aagatcagtt cataaaaacc 92160
   cttgtgcatc tttcagtgaa tttggaaatt accaattttt tccaaaatag atggaatgga 92220
   gaggactgtt tttaagaaaa gaccacaatc aaatttatgt tttagaaaaa tcacaagttt 92280
   ttcagcccca gtaaaggtga tagatattaa tgagatgagg tgggtcaaag gatggagtgg 92340
   aatgatttca agagagtcca ggaagtggaa tcaaacaaca ggacttggtg agagaaaggg 92400
   aagaatggga gatgaatggt gatatggttt ggctgtgtcc ccacccaact ctcaccttaa 92460
   attgtaataa tccccatgtg tcaagggcag gggcagatgg agataatgaa tcatgggggt 92520
   ggtttccccc atactatttt tcatggtcat ggataagtct cacgagacct gatggtttta 92580
   taaatgggag ttcccctgca catgttctct tgcctgccat catgtaagac gtgcctttgc 92640
   tcttctttca ccttctgcca ttattgtgag gtctccccag ccatgttaaa ttgtgagtcc 92700
   attaaacctc tttttcttta taaattatcc agtctctggt aagtctttat tagcagcatg 92760
   agaacaaact aatacaaatg agtaaagtga taacattaac aaaggcaaga aacacaggaa 92820
   ggatagattc agggtaggaa ttaaggagat aatttgttca gttttggaca tagcaaatgt 92880
   aatatgctaa tggaacttcc aaagtgagtt tccaagagca aaaagatata tgggactgga 92940
   aattaggaaa aagttctgca gtagagatga aaatttgagt cagtcttggg gtaaatgtaa 93000
   aacaaagagg atagagaaat acctacattt aagagtagaa aaaaagagac tgactggaaa 93060
   gtccaatttt tccaatccta tgaaggaggg gtggtaaaaa aaaaaaaaag acaaatgaaa 93120
   acacagataa attatccacg tcaagtggaa gtaattttac aatcttatct cactcttgag 93180
   agatgttaaa tgtgcaacag ttggttgacc tttaaaactg aacttcatta gacctggcct 93240
   aatgggcttt agtgaaaaat gagaagagaa ccttggcaga gagagtagtt gtagtcatgc 93300
   cttcaagatc ctaaggctta ggattcaggg cacttccaga ataatagggc agcaacctca 93360
   cctatggggt tccggaagat aagaatggtc aaaatgttgg ggtctcacag gaaagtggtt 93420
   cccagttgat gaactacaac ctcatcccct ctaactgtat cttgacaaaa ggtagatatt 93480
   catcaacctg tgttcacctc ctttctgttc ctgaggggac taccaaaaat ccccttcccc 93540
   agggctatcg agtgacagtc aatttttctg cagctagaag taggctaaat taggaatttg 93600
   agaagaattg agctggactc cctattttga cagaacatgg aaaaagagtt cagagaaagc 93660
   acagaatagg aagatatgac agaactagat tcttctacag tagtgatagt tttcccaaaa 93720
   cattttatgt cagaaatcat acttgtggat agggtgggaa ttgaaaagaa ggatagaatt 93780
   aatgacgcag atagaagata gttcttttta gaataaatca taaactagag gaattgttca 93840
   tcagaatgac agtaatccta tcattgtgag tgcatgtgta tctatgtaaa gacccattca 93900
   attagtaggt tttagtttgg gatgcttctg ataaaggagt caaggaaagc tcatgagaga 93960
   catggatagc atcttattca tggctgtttt tctggccctt accacaatgt tgataacata 94020
   gtaggcaccc agaaaaattt attgactgga aaaaggaaga aagaggacca agatttcatc 94080
   agaaacaaca acaaaaacaa aacttaactt gtgttaaatg ttgactaggt ctctggtatt 94140
   aaataacatt taatacataa gttctcattc tcccagcaat attgtgaagt attcttcttc 94200
   ttctctcttt tttttttttt tttttttttg tagagatgag gtctcactat gctgcccagc 94260
   caggctgatc ttgaactccc aaactcaagg gatcctcctg tctcagcctc ccaacgtgct 94320
   gggattacag ccatgagcta ctgcgcctag cctttgtaat tccaatttta ctgacagaca 94380
   gggagagtca aagagattaa gtggcttggc catgccagtt cagtttaaga ccagcatttt 94440
   tctaactctg ttcaaactca agttcaatgt aaaatcctga gcttttaact gaaattatct 94500
   aaaataaaat gtttaagagt caagaactga tatcctaaat aaaaggcgat aagaaaaaaa 94560
   actgaggaaa aaccataaaa actacaaata gcttcctatc attgggtaat attttacaaa 94620
   taaatgattt ttaaaaactt ttcaaagcac catttcagta ataaagattg aaaataagat 94680
   tgtagatctt aaaatggaag attaagaggt tgagaacctt acagaggcag agcctagaag 94740
   taggaagatg atgttttgat cttttcagac tatttctgaa gggattaatc ttctcttatc 94800
   aaccctgatc acttaaagat tccaacattg atgggatttt cattatgact tttctgtact 94860
   ttgctgttaa tgtgcaatat taggcttcca ttaatgctga atgacactca acactaagca 94920
   tctccatcag ctgtcttcaa ttctggatat acattagtga aaagctctta aaaaaataaa 94980
   gtgccttagg cttcacctct gatttaattg atgtgggggc agggcatata ctctgctttt 95040
   gttgttactt taagctctcc agataattct aacaagcagc aagggttaag aattacttgt 95100
   ctagatcaca atattttggg caaatcaaaa ctagcatctg ataagggttg tatctgtcca 95160
   aaggcaacca cattactaat ttctaataag ccaaagatgg gtgtttcaaa atagattctt 95220
   gatgtaaatg aatagaagtt atttagacat ggagatacac gataaaatcc aaggcaagaa 95280
   tgacccacag ttttccaaaa agactcaggg atttacgctg atataaatga ggcactgagg 95340
   ttttactgca aattatgttt ctgtaaaata attttataaa tattttattc caatcataaa 95400
   aatgggaaaa ttgatacctg aacaataaaa gacataagcc aaaagataca caataaataa 95460
   atgagtaaaa tgaaaccagg atttttaatg cctttttccg ggaataatgc catgttcctt 95520
   gtcagtacta cctgccaaat tggaagccct acgtaaaaac agctcgtgaa acaaaacaaa 95580
   tctctcctac agtcaacttc cagttggact cagaagtttg agaattgata agaaaaaaat 95640
   cagcacaaca gcaaactcag gatataacat gtcatatttg cacattagaa atgattagca 95700
   gataaagggt ttgttcttct tgtactcttt ctaaaacctt taaacacacc tatccaagat 95760
   ggctatttgc taattaccaa agatcagatt cacagaggaa gagaagtcaa aaatcatgaa 95820
   atagttttta aaaactaaaa aaaaagaaaa aaaagaaaag aaaaattcag agatcttcta 95880
   aattctaaat tcttatgaat tcctaagagt gaaattcaga gacctggttt taaaaagtta 95940
   ttaatctgta acctggtata tatgtagcca ttgcaaaaag tcattaataa aaatattcat 96000
   aaagtgaaaa gctaaagcaa tatacacaat tttaactggt tgtgacactc gctatgttaa 96060
   aatcaatgga ttattctcag gggtaaaaat atgcttccat ctgaaatctt acaaatttaa 96120
   aataatctaa ctaacaagtg gtagcttcca gttatctaga ctacagcttc tcaaacttta 96180
   aagtgactat gaatcacctg gggatcttgt ttaaacacaa attgagaagg gaaaatatca 96240
   aggtaagatc catcatgatt gtccctctag gtgaagaagc ataggttgca gaaatattag 96300
   ttgttcattt aacctaaaaa ttccaactgt agcaattttc tgaagtagtg gaaagcagaa 96360
   cattataatt ttacatgaaa gtaacattta atgaaataag aagatagtgg ttcctctcac 96420
   ttcttgtttc catgtttgtg acaataaacc attacaactc cagaagttta aagtctggtt 96480
   cctgatgaat ttaattgctg tgatgtctaa tttggtatac aaaacttcct tactactgag 96540
   ttaaggcatg ctgtaaagag ttaggggtaa ccacactaag cagaaaaaaa acaaaattac 96600
   atcccaatat cctaatatag attatagatt aagaaaagta cactattgtg aattattata 96660
   aaactcattg gatctttgcc acacattatc tatcatctgt atttcctaat tgaattcaaa 96720
   acaaaacaaa gaaaaacaaa atttaaagac cttgccctga taataaacct ttaagtgctt 96780
   gggtagaagt tttattatgt gcccctcatc tactggttaa agtgttaggg aacagaggag 96840
   tggcagaaag tcatttctgc ttgcatttat tggtgggtca aatctcatgg tgttttgctg 96900
   ctgtagtttg ttagttttga ttatgtttaa gttcatgttt acattcttgg cagcatttct 96960
   tctgagcagg aagtggcatg tggatgtatg acaagagaca aacactgatg caaagcaaac 97020
   tgcatgaaga cagccacatt ctgtctcaga gggggcagca gtagacatac ctcaaagatc 97080
   tcagctaaag gcaaagtaga gttactctgg gtggctgggc aatgccacca gtggatgact 97140
   aaactacttg caattgttat aaactaagaa cagctttctg aaccaagata tctgcaggct 97200
   gtgagacaaa aagctcagtg acagagttaa ccatcagcaa ccgtgaggca caggtttcaa 97260
   tactgagaag gacagggtca gacatccaac ttctcagcta cccctggtca ggtagacatc 97320
   tccctcatcg tcatctctga atgtgaaagg ttgctgatag aatcttgatt tgtgagctag 97380
   ccaactctat cttgacagtt tgacacaaac aagtaattat gatactacat tatctgtcaa 97440
   gggagtcaca aactcagggg ctcacatgct ctttcctgcc acatctacac ctgtgaatga 97500
   cttttacatt acatatgaaa gatatgcatt gattacttat ggcatactac agagtagagg 97560
   aattctgagg agtgatttag gtggatccag agattattca tgtaactggt attccctgcc 97620
   ccccaggtag aggcactgca ttttaaaaca tttgtccatc tatttacctt gtaaacatag 97680
   ttgttggctt ttggattagt aaggagatag aaaccacatg ctaatttgaa cagagaaagt 97740
   ttaatacaat ctggtattaa aagaggattg gagtaacagg aaattgccta gtgggagtta 97800
   aggagaattc taaagaatac aggagttgta aatacagtat aaggggcaac cactccccct 97860
   atggctgaaa taagtgttca aggaagggct ctcctctcac cagggctgag atccagagat 97920
   gtctatggag atgccctagc tttgactacc tggtaaaaag tcatagaagt acttcattca 97980
   tgggacttac cagaaatctg cccctgaagt tccagaagag gccattcaca gccgggggac 98040
   agaaccctag aactagctag gtgctgagga aaactgctgg ctgctgaggg aatctggaat 98100
   cacagaggtt gtcccctgca ggagtttagt gaactttcat gtgccacaca ggtaacaacg 98160
   gctctgggct ggctgcctgg gacaaaggtt gggcagctgg ctccctagac acccagtctt 98220
   gtggggcccc gagtttggca tgtgtggatc tgaggagaga gtttggggag cagtgactta 98280
   ggtgacagag caggtccatg gtgtctgtgt ctcaagggta caccactgct gcctaaatca 98340
   agctctaaag aaagtgcagt gtgtggggaa caccaactct agaaaaagct gtgttctgca 98400
   gatacctagt actggagaaa atcatactgg ggagtgagca gtggaggaaa actatgctca 98460
   aaaggagctt agccagtgag catcacagaa accagaaaga gaagcctcag ccacttcagt 98520
   gtccctccag tgccctctac tgacaaggct taacattgta cgagccagca aaaaagaaat 98580
   ttacaaggcc cagctccatt attacagagc aggctaatac agtaaatttg gaactaagag 98640
   acagtaaact gataagcagc aaagtctaca cttttcacta ctcagcttcc acatgcaccc 98700
   tctcccacac actgaaactc ctgtacaata acaacacaat tctatatttc acctaacaaa 98760
   gtctaactgt ccttctttat aagtgaagaa gttctatctc ctcaaaaacg agagacatag 98820
   agtcctaaca gctattgtag tcattgctgt gactctgagt tactccttaa attctgtcac 98880
   agtcccacta aatattctgt tacctaaatt ggaaagttga tctccaacgg cttctattta 98940
   acatttttaa aatgaaaatg agagaagaaa attgttaaca tatacaaaaa aaacctagat 99000
   ataagaggca aagaaaaaat gtgcattgaa cctgagaaag ctgattgaaa aaaaattttt 99060
   aaagaaaaaa tacgcaaacc tacaataatc ctcacttaca taattggctg cgaggccata 99120
   atgaatatct atggcttctt tcttacacta actactactc tgtattcacc atgcccttaa 99180
   gcattctagg agtccacaga tgttggtgat gtcaaggaag ggcaaatcca tatgtagaat 99240
   acttgtctat tctagtgagg agaagtctct gccccctcaa tgaaggaaga aatccagtat 99300
   aatgaacctg ccacccagag gctggctgat ccccgcaggg gctcaatgct ggattctgca 99360
   gattaggaac tcagcagcag cgctatccca gtcaacttta caaaggggaa gccatgttgc 99420
   tgagcccatg catacacagc tccagtcttg ccaccaagac tcctgtttat gggcccactg 99480
   agacagctgg agagataggc tgactgacat gcagaatgtg tcattttgtc cacttgatta 99540
   ttaagagcct cctctgcaat ggatgtgctt ttgtgggcac tcagagggga tacaaattct 99600
   ttcagggtct tttcattttg aaaggttcat tcacatacct cttcctcaaa ctaactacct 99660
   tgtcaccaat cttccaatcc tgttccttcc aactctgacc ataaaaacca ttaacaacta 99720
   cccatcgatg accaatatag atctgtattt ctaggcatgt ctcattccca acatggtgga 99780
   caaccatatt tactgttcaa atctctgcct acagaaaagt tttccttatc cagtattatt 99840
   cacagttact cacgagtagg gctatagtgc tacacagtca ttaatcttgg gtagtactag 99900
   tgttttgttc agattcatct gcaaatcagg tttgaggggt ttttttgttg ttgttgttgt 99960
   tgttgttgtt gttgtttttt cttctaacaa atggactaag ggaatcgcca agaagccaca 100020
   ggcatgagtt gaaagagaca aggcaatgcc attagagttg ctaccaaaga agtctgagtc 100080
   cctggttcat gcaacttatt tgtcctatcc agatctgctc tagttctgtc tcatgtacta 100140
   tttccactca atgatatatt gctgttgtac atcgctaacc ttatggaaag ataggtcaga 100200
   ttttacccag ataatgatgg gaaacttggt ccacatgata acctgacact ccataattat 100260
   gcattcaata tctacctagt agcaagtcag aaggtgttta tcaaaaagta aacagttatc 100320
   tgaagaaaat atttatttca aaaccctaga tgtctgtatt gtgattctgc tattggcact 100380
   ttccagaggc tccatagagc attcctcttt ggcacagata ccaagagagt cgttgagttt 100440
   gctagatcat aaagctgaag tggtagcata gcctgtactg ctgggcttgc tgcagagcct 100500
   tcttctactt tgatcatcat taaaaactgg cagccttatg agttactctg taaacaggtt 100560
   gaggtagcat attcaaatgt tgtaaatgtt gcccccaaat tcaaaaatga ctattgaggc 100620
   tgggtgcagt ggctcatgcc tgtaattaca gcactactga ggtaggcgaa ttgcttgagc 100680
   ccaggagtgc aagaccagcc tgggtaatat ggcaaaaccc catctccaca aaaaaaatac 100740
   ataaattagc cgggtgtggt ggagctcacc tatggtccca gttactcaag aggctgaggt 100800
   aagaacattg cttgagccca ggaggcagag gttgcagtga cccccagatc ccaccactgc 100860
   gttccatcct gggcaacaga atgatactcc ctctcaaaaa agaaaaaaaa aaaaagccta 100920
   tcgagcatag tggctactga atgcccagaa gttttgctga atttttgtgg ggctgattta 100980
   tcaccttcta attcttatat gctctacaag gatatctaaa gtatttgcta ctttcttctc 101040
   atcagagaca atcagcatat tatcatcaat gtagtggatc agtgtgatga cttgtgaaat 101100
   gttacgatga ttaagattaa gaattgatct agccctgagg caagactgtg aagccatatt 101160
   gttggctctg ctagataaaa acaaattgtt tttggtggtc cttgtaaatt ggcagaaaaa 101220
   gatattaact aagtcaatag ctacatacca aatgccaggg gccatgtggt ttgataaaga 101280
   tattacatct gggtcagcaa ctacatttgg agtcacccaa taattaagtt taggggagtc 101340
   tacagccatt ctctaagatc catttaactt ctgccgaggc aaaataagtg agctaagtgg 101400
   ggatgtgata gatatcacaa gtcttatttc aaatctttga tagtgacatc aatcttgaga 101460
   gtttcctcag ggatatgtta ttacttctga tttactatct ttgaagaaag gggaaattct 101520
   aaagccttct actcagctct cctatcacat ggtcctctct ctatgggtta gcaggggtgt 101580
   ccaatctttg ggtttccctg ggccacatta gaagaagaag gattgtcttg ggccacacat 101640
   aaaacacact aacacgatag ctgatgagct gggggaaaaa aaagaattac aaaaaaaaaa 101700
   aaaatctcat aatgttttaa gacagtttac aaatttgcgt tgggccacat tcaaagccat 101760
   ccagggtcgc atgtggccca tgggccacag gttaaacaag cttggtttag agagtcaata 101820
   tagagattct gccagttacc cagtatgtct attccaatta ttcattaaag aactggggaa 101880
   ataagcacag gctagatctg ctgaccccct ggacccacta tgagtacaac tggagctaag 101940
   actccatttg ttacctgagc acttttaagt ctccactttg aacagttggc cacagtattt 102000
   ttgggtcccc ctaggaatta gtatgaaagg tctgattatt tccttttccc caaagcatag 102060
   tctctggtaa atggctacag gtccctttgg ggaaaagctc tgggagtgtg aattgactta 102120
   ggtctgaaaa ctgagtgaga tgctgtgact cttgattgca gcaactaaag ttaacttttt 102180
   tgctaccaga gctagagatt ttcctattat atagatgaag ggatgctata gtactgctta 102240
   cgtatttcat tcattagcta ccactaggca tataccaaag acattatctc agctgtcaga 102300
   aacatctgca agccaatgtt ttctgatacc tctatggccc agcttttcat tgtgggagat 102360
   gtatatactt tgtatttggt gtataagggc ttactgccac ttggcttttg ctactatgga 102420
   atttcatcat ctccagtaaa atcagagagc caatttcaat ggtctacaaa ggagagcagg 102480
   cacagatttt ttcatggatg cagggaatcc cttcactaat gtacttctta atactttagt 102540
   gaaaggagtg tctactgggc cttttcatgg catgtatttg gaggatgggc aggttgcatt 102600
   caattaattc aaccaaccat tccttttttg aaggagtcca aacacacaca agcagtaggc 102660
   aatttctctc cattcattct tcctttgagg ccccaaaaat attaaggaat agggggtgaa 102720
   ggaataaaga agattgcttg ctcaattgtt atcattttgc attcaccttt ctacaggttt 102780
   accaatgctg atgtgataga cactcctcct ccatctctca gcttccatta ccctctagaa 102840
   aaagtgtcaa caaacttttt ataaaggact agataacaaa tttttttttg ctttgctggc 102900
   catatggtct ctactgcaac tattcaattc tgttgtagta caaaaaagac aatttgtaaa 102960
   tgaataatca tagctgcatt ccaattaaac tttgttttca aaaacaggta gtggatcaaa 103020
   tttggtctaa gaactatagt ttactaaccc ctgctctaga aattctaaaa gacttcaatc 103080
   tcaatatttg taagtctccc catatttacc ccttccccat tagctgccac ctccccagat 103140
   ataaaacaga atttttttca agtcttccat aacatcattt tttattatac ataaaatctg 103200
   tgtctacctt taagattagg ctatatatgt gcctaatttg aagcgaacaa tatccctcca 103260
   cttctccaac taccatctga aattcctgca ttatccactc ttcatggtta aggcacaaga 103320
   aaagctcctt taatgtaaaa tttgggttgg aaataaaccg gtctcagcct aaactttaat 103380
   tcttctatca acagctggcc tcaagtaggg taattttacc cagaaccatg catagtcaat 103440
   taccatacac agtgaggcct actaaacaca cactggatgc caagctccaa ataggggagg 103500
   gtggggtgct taattcagtg ggatttatat ttaattcttt aggcaaaggg agaggccctg 103560
   atgagttttc agcaaggtga tgacctaatg agaacggaag tttagaatga ttgtcgttga 103620
   agttgtgctg aatatggaat ttttggggag gtagaaaaac ctgataggag gctaaaggca 103680
   attaggatac ccttaaaagt agtccaggca agagataaat ataaggggca tggaagctta 103740
   gaaacagatt ttggaagtag agttagctga actcagcaaa tgagtcaatg tggaggctac 103800
   agaggttgtt aagttgtgac gggtgttaag gatgtgttct ggacttgttg aaaggtgcta 103860
   gctagctagg tgcttgtaat cccagctact cgggaggctg aggcaagagg attgcttgag 103920
   cccaggagtt caagaccagc ctaggaaaca catcaagacc ccatcctgaa aaagaaaggt 103980
   gctagcagat ggaatgctct ctcttctgca ttatcttcag tggtagacct agaactcaaa 104040
   aatatgagac aagaggccag tgaaactaac agaggcaatg atagagggta gagtacaaga 104100
   aggagactga ggtaacaaca gaaacaagtt ttatccagtt caaaatgatg ggtcacatct 104160
   gaaaaaacaa aggtcatgtt caaaaagctc aaagaaaaca ataatttcta tgagaaaccc 104220
   tgtctgcttt attaattgtc ctctagaaat gagccccctc tgccattaag tatttactat 104280
   caggtgtttt tccccctttc tccttatctc atttgactca gaattatcaa acttgcaggg 104340
   ctgcttacta ctgtagtctc tataaaagca tcaatcagat gtggggatga cttctagaaa 104400
   tcccatagat acgctaatgc actagtgaaa tggcacacct gtaacttgca cattaaagca 104460
   ttgttctaat atcaaagggt tgaaacaacc gagaactcta ccaataggga aagagataaa 104520
   ttatgatgga gtaatatgat taaatattat gcaaagtaaa aaacaaggcc aatttatatg 104580
   ctgatatgaa atgatcccaa tatagttaaa tgggaacaaa aaaagcaaga attatttgta 104640
   ttttaaaaag agggaaaaaa catacatatt gtttttatat ttttaaaaaa tagcatcggt 104700
   tacctctggg gaagaaaact ggtgtcagag ggcaacagtg aaaggaagat ttatcatata 104760
   cctctcctaa cttttgaaaa ttgtgaactt caaacaactg gagttcacaa acaactgcaa 104820
   ctactaacac atttttaaga attaaataaa acagaaagga aaatgtatga aggtagaata 104880
   aagagtttta gacattcaat atctcacatt tcttcattcc atgcaccatt ttccagcaag 104940
   ctagtggtgg atatgtgttt catccaaatg agggggtgaa taaaaaacaa aagtcatagt 105000
   atctaaggaa cagaggctct atcagagatg tcagcaaaaa gaaattccga acaaccacca 105060
   cggaacaggc ctagagagca acagtccgaa caggggcaag aggacagatt gctccagtaa 105120
   gggacaaata aatctgttat gttagactac atggaaaacc aagctgaaaa gatgttagag 105180
   tacatagaaa ttgtactcca atagatatat ggaaacacta agccaagtgt tttcttaaat 105240
   gagttcatta ttacttctag gattaaaaaa aaattctaca agtaaggaat cctactcgta 105300
   atatgcttgc ttgactctgt agtaaacaat atctatgtaa tcacagtaaa taaatactaa 105360
   aatgcaaaca aacaatgtga gaattatgtt gggaggtcaa ggagggagag tagaggtggc 105420
   tgtgtatgaa agagctaaat atttatctac cataatagga agtcagtaga tattgccaaa 105480
   tattgataca ttaagaaaca gcagtataag catattattt gaaaaggtaa gggctagaag 105540
   aaacatttaa ataaatgaaa gtagttgctt ttaggcaaca ggactgggga gacgagaaag 105600
   gaggggcaga gttgactgga tttttattgt tgttggaagt cttttaataa ccctatttta 105660
   ctttatttac atgtaagtag gataaatgta cagtcatgtc ctggtatctg caggagattg 105720
   gttccacaac ccctgtggac accagcatct gcagatgttc atgtcgcttt tataaaatga 105780
   tgcagtactt ggatttaacc tatgcacatc atcccaatta ctttaaatca tctctagatt 105840
   acttttaatg cctaattcaa tgtaaatatt gtgtaaatag ttgttatact gtgctttttt 105900
   atattatttt ttattgttgt gttacttttt tcctgaatat tttcaatcta aggttgtttg 105960
   aatctatgga cacagaactc atggaaacag aaggcgaact gtgcaggatt tggaattgta 106020
   ctgcttcgac ttgagtcttg gctctaatct ttcactaacc ttgtaaatag aggcaaaata 106080
   cttaatctct caattttcct atctttaaaa tagggaggaa aatagtatgc acctcatagg 106140
   gttgctttga agactaaata agtaaactca tgtaaattgc ttaaaatatg cctgccatgt 106200
   aagtgttcaa tagtaaataa ggaccagtac ttattatgag aataaatata aactattatt 106260
   acttgataaa aatataaagg cattaataac taaaatctat ttttaaaaat atgggccata 106320
   tattaatgtc tgcctccttc gtggggaaac ttttgttttt ataagttaat gctcccaggt 106380
   actaaatagg actttatttc tttagcctac agccttcact ctaacccata gttaggggga 106440
   aggatgtggt gagactgttg aaaaattcca agttacagtt agtgatgaaa aagtgggagc 106500
   tcaaactgga atttgaagac tgcatgttaa gtagttatgc caaaatggga gggaagagcc 106560
   ttccaggcca ggaagcccac ttgcaaagac ctagaggcag aaggaacaga gcaatataaa 106620
   aagattggtg gagctggagc aatgatgagt gtgctatatg tttgatggga gccaaatcat 106680
   gccaggcctt tgggcatgtt aaggagtttg ccttatctta agggccatga agggctttat 106740
   gcagggtata acataaccac atattttgaa aagatcactc tgggtgcagg gtgaggaact 106800
   aacaagcaag aagccagaat gagctcagat agacaaacag cgaggccatg gcaaaagtcc 106860
   aggcaagaga tgatgataga gataggaatg aataaaagta gatggatttg aggaatattt 106920
   agaaggtaaa acttacagaa cttggcatag gactagccaa aaagagatga aaggactgct 106980
   aattccaaga aatcttttgc ctcattttat ttttgttttt ctttgtttgg tctagtctaa 107040
   ctcaattata ttttttaagt aaaggctgct ccctttaagc tcctcaacaa taataatata 107100
   ctcaattgtg gaatatgtat aagcaaaagc tgcaagacat ttcaattaca aagttaactt 107160
   ttcactttta aatgataaaa ccccaagatc ttggaacaga accaaaaggt cccaaaataa 107220
   aatagaattc aaagtaaaaa gatagaagta acagcaatcc aaattaaata gactaattaa 107280
   gtcagtgaaa caagttcgct actccccagt ttctttggtt acttcccatt aaaaaggcag 107340
   attgggccag gcgcggtggc tcacacctat aatcccagca ctttgggagg ccgaggcggg 107400
   agattcacaa ggtcaggaga tcaagaccaa cctggctaac acggtgaaac cccgtctcta 107460
   ttaaaaatac aaaaaaatta gccaggcacc tgtagtcccg gctacttggg aggctgaggc 107520
   aggagaatgg catgaaccta ggaggcagag cttgcagtga gccaagatca cgccactgca 107580
   ctccagcctg ggcgatagag caagacatca cctcaaaaaa aaaaaaaaaa aaaaaaggca 107640
   gattgactta tattgcctgc cgatgcctct cccctttctg tgacatttct accccaaaat 107700
   ttttattcca caaaaggctt aatggaaaga gaaattttgc atgttttgca aataatccaa 107760
   aagggtgtgt gtgtgtgtgt gtgtgtgtgt gtgtgtgtgt gtgtatacag acaagagcaa 107820
   aagcagacta tatttatatc gtatcaagca aataatagaa gtatacacag gacacctcac 107880
   cctgctcagg tatatgtgga ggtgtgggtg aggggacaca gtttcccaaa tgaagcaaga 107940
   ccttctttga gttttaagag ccattaagag ctatagatag atgtaaagat agaaggaaag 108000
   gcactctctg cagtgggaag tgcaaaagaa aagcacacaa gaaaaaaaaa acaataagaa 108060
   gtctattgct gggaaataaa atttcggctt atggagtatc actccagagt taagcaagtc 108120
   tcagatccca tgggaccatt ttaagggctg tgggttctat ccggtaagca acagaaaatt 108180
   gttgaaggat tttaagtgag agagagaatt tgcatttcta aaaagatcat cctcatcctc 108240
   cagatggtgg acttgaggtt gacaacacta tctgctgaga taccaggtag gaggcagcta 108300
   tgataccttg gttgagagat gttaagacct taaactagat cagtagaagt gaaagaattt 108360
   atggctggga gaaaggtata aaatgttaag tggattcagt gttctaatgg ttgttgtctt 108420
   agttcatgct ataacaaaat accttagact gggtaattta aacaacacaa atataccact 108480
   cacagccaga aagctgggaa gtccaatatc aagctgctgg cagattcaat gtctggcgag 108540
   ggcacatccc tcatagatgg cctcctaggt atcctcacat ggaggaaggg caaaaggcta 108600
   acaagctttc tcagtcctct tttataagga cattatcttt tacaaaaaca cacgaggcca 108660
   aggccctcgt gacctgatga cctcccaaag accctactcc taataccaat acattggatt 108720
   tgatttcaac atatgaattt tggaaaaaca caaacattca gaccatagca gttgtagagg 108780
   ctaagaaaaa gagtttaaaa ttacctctgg atttctggta tggcacctgg atggtagtac 108840
   acatttatga ttcactctcc caaatctctg cagcaaaata tatttcaaaa ttcaggattt 108900
   gacagcctgt agtctcagct acttggaagg ctgacatgga aggatcactt gagtccagga 108960
   gttcaagatc agcctgggca acatagtgag accttgtctc ataaaaaaaa aaaaaaatct 109020
   gatttttttg gatgttagaa aggtgatatg gtttacataa tcaatgtaaa agtgtacccc 109080
   agcagggcct gggcagtatt atgtaatcaa acagcactat ttctgcagct ggacataatc 109140
   cactaagttg aatacacaat cacaatagcc tcctgacagt tcactcaggc tttgctccaa 109200
   ctgagttcaa gttttgctaa caaatgagtt aaattttgtt ttgctttgtt ttcagaggat 109260
   gatttttgaa ttatagataa gaaatggtac aatgtactac tatctataaa tagaatacag 109320
   agagtgagca ggttgtttgt tcatttgttt tttgatgaga tgaaggttct ggtttgaaca 109380
   tatataatct ccagtgatga acaaatgtta aagatgtcca ataggcaatc atatagaaaa 109440
   gaaaattagt ttggacagtt tgaaactgaa acaattatag ggaaaaggtg tacttggggg 109500
   aatattctct ctttagtgcc aaaaataaga gcccaactct gagaatgacc caaggaagtg 109560
   tcatataatt gtcagtagtc tctaatgacc tagaattcag agtcctactt gtctttgctt 109620
   tgcaataagt gaatgtactt ttgtctcttg ccactgctgg aattgatttg aattcagttt 109680
   tcccccttag tgtacatgag ttgtgttaaa tttcacccaa attacatagt atgaattatt 109740
   ttgatactaa ctaaatgcat aattgaacag ctaactgaaa acaccttcct tttcaatagg 109800
   attttatatc tataaagtat ctatcattcc aaatgtattt ttgcacttga tggactaaag 109860
   aactgaatac atgtgaagat gattcaggca ctgatcattt ctaaaatcct aaatatgata 109920
   atattttcct taggagaaat atttcaccca ccaagaatgt aaaggcaggt taaaattgaa 109980
   agtgtctacg gaaggcttaa ttatccaatc agagcttcca agactacatt cctacaaatc 110040
   attgagggat ctattttaat ctctcaatgt agtctttttg tggagagggg gagtgtaatt 110100
   cctacaattt gtctcatact tgtttcatag taaaaaagaa aagcattaaa tattaacaga 110160
   atataaacca aataataaac tggctatgtt ttctctaata aattttcaga ctccacccag 110220
   aactctaact cagtcataaa actttaagag ttcagaatag agctggacag aaggtttctc 110280
   cctcaaaatt tcactgaatt cttatcatca aaatcccctc agaaaaaaat atttctagtt 110340
   tttcctgttc ccaaatacac tacagcatga tgagaaccca tttaacacaa aatatgtata 110400
   atgaacaagg tttcatgtag ttcttaatat ttgaaagatt taatctagca ccaaggtcct 110460
   ggagcctcaa gccctacaat acatgtttaa ttaggattac agtaaagttt ttaagaaata 110520
   atttattttc tttcatgtga gtgggtgtgt gcctgtgtgt gtgtgtgtgt gtgtgtgtat 110580
   acgcatgtat gagacagaga aaaagaaagc tggtagaaaa atcagccttt aaaagaacaa 110640
   aaattcaaca gaatagtgct attttcacat tgagggctcc agcactttcg tggtcataaa 110700
   gcagtctaat tgtttagacc aacgttttta aaaataaaat aataaagaac atacatgttg 110760
   taaggttaag cactgtttta tattaataaa acttgtttca tttatataca ctcacattgg 110820
   agtttacaga gttgaggcag agaataaatt agttattgag gattgcagta aaaggaaaaa 110880
   aaagtttaaa gaaacttagt gttaaggaag tgtgcttgaa ataaaaaatc tggagtgttg 110940
   caaatggatg aaaggcactg ggtagctgcc tcataaaata tattttaagc caatataata 111000
   aaaagatcag aagactaaat aatgttatcc acattgactt aaaaaattaa ctgtggcttg 111060
   gaaataccaa gaaggaacta aaagtctcaa ctgacatggc atcatcaata tgcatgctat 111120
   tccacaatga gaggagcatg atgccttcac tgggtagttt ccacgaagtc aggcaatatt 111180
   tggacaagta gaaatgaggg taaatcagag tgaagaaaga gcaatggaca attctgtaat 111240
   tacaaaggcc ttacaaatta tatgaaagaa aaaagataaa catcttatgg taagtgagga 111300
   tgtgggtggt gaaaagtaag atacaggagt catgagtcaa gccactggaa tgatcatgcc 111360
   atctctgagt gaaatgaagt tagaatacct ctcttggctt caatttttca taaatggatg 111420
   gtttgagtat tgaatctcca aagtgagaaa gggatctcga gaatgctagg gctggaagat 111480
   actcctgatt gtttttaagg ttttgttatc ttaatctagg aaagtattat ttttcctatt 111540
   taacagatag attaagttac actgttactc tagctaatga aaacatgtta catttctcac 111600
   tagaggttaa gaggaaagac agtctggtaa catctgccta gtaagtaatg aagactaata 111660
   ttcaaaattc agggaacaac tcctatatct catatttaaa cagtatttag cacatttgtt 111720
   tctctgtatg gagacaagat taagaggtca ggtgagatcc ttttaagaaa tgtagaaaag 111780
   tggtgattga attaataatt acatcatgtt atgctgtggt tggcttttag atttccatct 111840
   aatcttaaaa agaagaagaa aaataacccc actacatgtt gcatcaggca ttttaagtaa 111900
   aacgtgcaat ttaaaaaata tttttaaaat gtgtggagat agaacaaaac aatggaattg 111960
   ttattattat cagaaacttc tacgtaaaaa tgttggttaa agcaatatat aaagattgat 112020
   tctcggtaca aattgcttgg aaagtttaac atgtgcacta gatttcagat attaaaagtt 112080
   aaattaaaaa aaagaatcta atggcagcgt gtgactttaa cctcatggat tttagccaca 112140
   aacgcaaaga aaaatatata caataaaaga agcaacagat tttcccacaa gcagagattt 112200
   tctaattaaa atgctgtcac tttaatattt tttagctata gaatttgact gtaaatgtct 112260
   tcattatgta gtgtaacttc tatagcaagt tatgttaacg ttttcaagaa tcggaaatat 112320
   tggtaactcg atggggttct taaaacagaa atagtttctt tgatgagttg gaaagagaag 112380
   gaaataaaag tctaaggtaa gcgccagttt caccctcaat tcttaggctt cagaaaagcc 112440
   taattaggca gggaaaggaa tctgggcctg gcacacccaa gcttagacgt ggcaggatcc 112500
   tgaaacaatg tttttttgaa cctttgagtc atatgccaca ggcaaaccat agccgcgata 112560
   tgcaggtcct taagtcaaca ggtcctctaa cgagatggtg agtggttggt gcgtggcagt 112620
   tggatttctt cccgccacgg aagaaaagaa ctcttctcca ttgatcagga aagtgggccc 112680
   aactctggcc tggagagcgc acccggaaaa gtgcagagtc gaaacagaga gagaggggtg 112740
   tccgcgtccc ggccacagag ccctttccaa accactcgga aaccatttaa atggctcggc 112800
   ggccgcggtt tgcagcctag aactgtttgg ggtccccggc taggaaagtt gcctggcaaa 112860
   gccatgtctt tcgctgtcgc gggatgtcct ctcgggcaga gaggctggag gagacaggca 112920
   acctgctagc cggagtggaa gcgcgcccgc ctcccgccgg gccctccctc tcagggtccc 112980
   cacgctgcga ccctcccaag ggcaagtccg gggccaccag gtctttggtc tccgtccagc 113040
   tcctcagggg ccgccccata gggactggcg ggggcaccgt gggcgagagg gtgtgaccgg 113100
   gctccccccc gaggcgccac ctgccccttg gcccagggcg cccctttcgg gatctctcca 113160
   ggggcaaccg acacactgct ctcgagaccg tctccaaccg gccacctgga ccgtagccct 113220
   gtccccaaca cgtgaacccc tgcggccgtc ctcccgcctg ctctaacgca gcccaggggt 113280
   accgcgtctc cctccgcctg ccgccggctt acctggcggg tgggcagggc agggtggcgg 113340
   gaagcggcgg ccgggcaggc gctggacgtg ggctaggcgc caggtgcagg tggcggcggc 113400
   tgcgactccg gttgctgtcg ccacagttgc ggctcagtag agctcctcct ccgccgccgc 113460
   ctcctgcctt cccgctgggc ctcccgcgtt gcctggagag gcagaaccga ggctcggctt 113520
   ccacttggag tctcccaggt gagctccagc ctgcgacgtc ggcaggggcg aggccccact 113580
   tccgcgcctg cgcgccagcc tcccgccccg ccccagccct acctgagcgc tccaggtgag 113640
   aaccttggat cgcgcgcgca gggtgggggc gccgtccggg ccaagcctgg ctgtcgcgcg 113700
   gcttctctct gagtggtcgg cgaggctgct gctccgcgca agttgtggct cccggcccat 113760
   ctacattgga ggaatcctgc actgacctgg tggcagtgat caccttgtag ccagaacaca 113820
   gtctgctggg tccttgggga accagaagtt ctagatttcc cccacacggt tcctcccttc 113880
   ctcctcggtt cgccaaaatg aaggggtgcg ctgcctccga ggaccacttc gggagggcag 113940
   caactgctgg ctcatgtggt ttcttcgggc agggttcagc agcttctgtc accagttagg 114000
   tttcgtgagc ttccttcccg tgtagtcttg atttcatcac ttgactcact tcagcgcagc 114060
   tagtggtttc cgtttctgta cgcgggcgct ctggtagggg cgcctcacgt ccaaaggagg 114120
   agtcttcttc caaagaagag cctttggtat ttggcagagc ctcgccaatt tggggcgcac 114180
   ctccccttcc tgggatgctc tcagaaggca aaccaattcc cagaagagag ggaaaaacag 114240
   ccacaacaga gagaaccttt gctgtccctg tttggaaaga gattgggatt tcctggagag 114300
   ggcttgcttc tactgcaggt ttgtttaaaa gctcaacatc tattctggaa gagaacaacg 114360
   cagactccag gaagatggtg ggttttgctg tttttaagac aaaaaaaaat tgttaaacag 114420
   tatttgttcc atttatgaca caactttctg tttcagcctt ccttctgctg ctgcaacaaa 114480
   cattgacaca cccaaaacca ggacttaaaa tggcctttat acctttctat gaactcattt 114540
   ctttggccct atcccaaaga tatctaggga tataaataga aaactagcta aaaacttttt 114600
   ggtgtcgtgt ttaatcacat ggtggtaggt tttactatga agtttagttg tggccttcct 114660
   cctacatttt gacatgctct tatggtgctt attcactgtt ttattagtgc tatgcgtgac 114720
   ctaaaaataa ggtttaaaat acagtttagt atagtatcaa caagtgtatc tttagtattt 114780
   gactactcta agacattggt tttagcatcc atagtgctat tttatactag gacatctatg 114840
   agttagggtt taagacttta gtaaactagg tagttctttg cttgttttac atgtcatggt 114900
   tattttgcga gcaccaagga acctccatgt atttgcaaca ttttaatatc agtacctaca 114960
   agcaattgcc aagctccctt tccctccaaa aacaaacaaa caaaaaaata gttcaggaga 115020
   tacttgtgtg tattgatata ctaacttggc agtgtttttc cttaggtcca ggtccagaac 115080
   tgaaaatact aactactgaa attctcttct ccttctcctt cttcttcgtt gtgccacttt 115140
   gtcgtttacc cctaaccaga ggttaatgtt agtctttgga aaatttgtaa ggtatcctaa 115200
   atgtataacc aagaaacctg tacccttttg cacgcaccac ccatctctga atgaagagcc 115260
   aaagattgtc agagttttga tgtcccacca ggcgaagagc aacacttttc actgacaatc 115320
   aggacagagc agatggatat agaataacaa tctggaatcc aaggactgag atgcattgac 115380
   agcaagacta gtaacgtcaa ataacaactg aaaagagaag ctagggtaga cctattaaaa 115440
   gagagagcca gcaaacagga ccaaaatgct ggatgaggag tctaaaaaga acagaagaaa 115500
   caaaaaagat caggagttac taaaatttga ctgtcagttt tacaatggag acagacaatt 115560
   taaagaagga agcagttttt ctgagtgtgg cattacttgt gcacgttgag tgtcatatat 115620
   atagagagag agaaagagag agagtgagag agagtgagag agagagagag agagatatct 115680
   tggggatgag acctgtctaa acacaaaatt catttatgtt tcatatacac cttatacaca 115740
   tagccttaag gtaattttat aaaacattgt aaataatttt atgcatgaaa caaagtttgt 115800
   gtaaagtact tatgtgtgga attttcaact tgtggtgtca tgttggtgct caaaaagttt 115860
   tgaattttgg aacatttcag attttggatc tgtggattag ggatgctcca cctgtactat 115920
   tttacacaac gttgcttgta ctatttcaaa gcattctgaa agcaaagtct taatattaat 115980
   aaaaaatttt ctatgtgaca ataaaattaa aatgctgcag gcccaagaaa aaaatattat 116040
   catattgaaa tatttaatgc cccaaatcta atcagatgca ggtttttgaa agtaccatcc 116100
   tattcaaaat tatgaaataa agaatttgtt tcataaagag aaattagcat ttcatcataa 116160
   ataaaatcca ggaaccttag tgtagacatg cttcagaaat aagctaattt taatagtata 116220
   tgtttgacat ataatgacag ttaagataga aaactgggca gattttacac aggaaggaaa 116280
   caaacattct ctctctctct ctctcacaca cacacacgta cttattacac acacacacac 116340
   acacacacac acacacacac gactagccac aaaatgctca tttgcatgga tttgaaacaa 116400
   tactcactcc ttaatacttt ctgttaacat ttaaattaat gccaagacca gcacctggca 116460
   cttagtaggt acttaataaa tatgtgttaa atgaataaat tgacccatca agtaccataa 116520
   aaaaaatgcc tagtgtcttg agcaatttac aggtctaaag aatggtttta cccaacagta 116580
   atgacaaata atccaaggca gataagaaca ctagactggt aatctgaatt gcaaagttct 116640
   aagaacaata gctaccagct ttcacatacc tacatttcaa aagacactac gcaaaacaac 116700
   ttacactcct acaaattact ttacatctgg cttaaatatt ctcttccaca aaacaaaata 116760
   gtggtacaag atgatctaca ggcttcttta cattctgatt catataaact ctatgccaag 116820
   agaattgggc aaataaataa ataaataaat gaaaccttct ggatatagca ataataatta 116880
   atgggacagc aaagatgaag aaactgaaat tatttagctg agatcgaaat tttaattcat 116940
   aaaaaatagt tgtaatatat aatgtaacaa gtaaatgtgt aaagttacta agcctaactt 117000
   taaactattt tagtttgtta attgtttaat tgttggttac acaactttgc tttgaaacat 117060
   actctagaaa cagttttgcc tcatgaggga actagagctt ttagtgagga tctcaaagtc 117120
   agactgagag atttggctca aagactgaac agactggaat aatttcttct caaagcagta 117180
   aaagtgttga aatgctattg attcaagcag agaatagagg tggtatcaca gtgaagagcc 117240
   acaaacacgt gggtattttt taaataatga tccaacataa ttctacattg tgtttaaaat 117300
   atagcaagtt aaaacaggga aaatataaat aggttatttt agtatgaact tttttggaca 117360
   ttgttttata tgattcttcc ccctgtagaa tctcagaaca atcatctcag attttcaaag 117420
   tggttcacat actatgaggg gttgacttag attacaacaa aactatgtct tgctaatctt 117480
   gtattcttag tacttggcac atagtaggca ctcaataaat atttgatgaa atgtattgaa 117540
   tagagtgcat tgtttcaagc ttttaagcat tagcataaga gcaaaaagaa atgtcatgct 117600
   gagtcagaat aatattcaat ctaactggct ctaaattctg acagtggcac caaagaatat 117660
   tttgtggaaa actatggtga tagtcttgca tgatgttgac ctcagtgatg tactcgagta 117720
   tccctcactc actcttttcc taaattgatt tctctttttc tgtaacatgt ttctttaaat 117780
   tgaaaagaaa ttaatattca ttacagagaa atcagaaaat acacataact gcaaagaaac 117840
   tagctttgaa atactcagaa ttccttatac caagatgaat cagtgctatc agaccctttc 117900
   tatcatgtat ctttgcagat ttttttattt gaaaaagttg gcaggaggca cggcatggaa 117960
   tggggaacat agagggatac agagacaggt ttgtaaaata aaaactagat cattctgggg 118020
   acttgttttg tagccagagt tttctacttg acatataatt aaatgtcaat aaatttattt 118080
   tacagtatta ttttaatggc tgcctgatat ccctttgtaa ggatttaatc tgtcaattac 118140
   tgttggacaa tgaagattgc tcccaaattt tgctattgca accagtgcta taatgatcat 118200
   actcttcatc tgttgaaaca tttattacga ttttcttcta attgactttt attttgtttt 118260
   tgttttatat agagatgggg gtttcactat gttgcctagg ctgcaattga actcctgaac 118320
   tcaagcaatc ctcctgtctc ggcctcccaa agtgctggga ttacacgcat gagccaccag 118380
   gccagggata tttttgtctt ttgagacagg gtcttgttct gttgtccagg ctggatcaca 118440
   gtggtgcaat catggctcac tgcagcctca acctcccagg ctcaagtgat ccttccacct 118500
   cagcctcctg aatatctcgg actacaggca tgtttcatca tgtctggctt ttattttatt 118560
   ttattttatt ttttggagag atgaggtctt actatgttgc ctaggctgtt ctcgaactcc 118620
   tagactcaag caatcctcct tcttcagcct actgtagtgc tgggcttata ggcaggaacc 118680
   accatgccca gccctgactt ttattaagcc acattgtgtc aattatccag atcctaaact 118740
   atgacaatgt cattagaggc atcatatcac aaaaacaaga ggtagaatac ctattaacaa 118800
   tttacaattt tctattggta agaaaaataa taagaatatt aattctagct accagacctt 118860
   catacaaaga acgttctatt caatgaacac cttataattt tagaaatatg gaaatagaat 118920
   acgtcactta ttttaaaaaa aaactcagat ctcttatttt cctctgatca taccacagtc 118980
   agtttcccac ctgtgaaagt ctttgtatct tctaatatga ttttcttccc cctctccaaa 119040
   tcccctccag cccaactgaa gaccctagtc tcccatggag tcttgatatg gtttggcggt 119100
   gtccccaccc aaatctcacc ttgaattata ataatcccca catgtcaagg attggaccag 119160
   gtggagataa ttgaatcatg gtggtggttt tcctcatact gctcttgtgg tagtgaatac 119220
   gtctcatgag atctgatggt tttataaatt ggagttcccc tacacaagtt cttcttacct 119280
   gccaccatgt aagacgtgac tttgctcctc attcgccttc tgctataatt gtgaggcctc 119340
   cccagccatg tggaactgtg agtcaattaa acctttttcc tttataagtt acccagtctt 119400
   gggtatgtct ttattagcag cataagaaca agactaatac aagcctcctg gatcagttca 119460
   aatcaccttg atatttctta attccaatgc attggcataa agtaaatttc cttcctccct 119520
   tggccctcac ctctctcttg actccaataa gggagagttg tggagagatg gtacctgggt 119580
   ttgatataat ttgttgccag ataagttata ataatttgtt aaggaattca agccgtagag 119640
   gatgaggatg ctcttttagc atattaggtt tgaggtaaca ggccaaaagg aatagcattt 119700
   ggttattaag gtaaaaagag aaataatttt tatgcatgtt agaaggggaa ttttgaagga 119760
   aattacactt tttctgtata tttatatatt ttacttttta tttaccagga gaaaaggcat 119820
   ttgctgccca aggtagaatc ccatcaccat actcctttcc tacatccaat cacaaaatca 119880
   gaatatgcca attctctatt cccaagtaag tcaacagacc ctaagattac attccctttc 119940
   tcccctctgc aaagcttcag ggacaggagt ccctgacagg cagctgacat ttggggttgc 120000
   atacaaaaag tcaagtcatt gcaagagaag gaagtttgtt tctattggaa gagttagtgt 120060
   acaacatata aaaacggagt gaggatgggg atattgtagt agggaggggg ttaaagggct 120120
   tagaaaatga tttttttaaa ggtggacagg ggaagagtag aaggcaaaaa aatgtcatcc 120180
   atgtgggaca tcttctgaac gcccaaggag aatctttggg gatacattcc ccccaagatt 120240
   taaagaagtg gcttcactcc tgagaatgta cagggagcgg agaagctaaa ccaggagcaa 120300
   catccaattt ctttgtctct ccttctcttc tcactgcaaa ctctgtacag gtttggcata 120360
   actatctaaa ctttctagta aaaatcatag ccttcatgaa gatccattgt gtatttgatt 120420
   cacagaaagt tggtattgaa ttgtaaaaca cggctacaaa tttctccctc tccaacaggc 120480
   gtgtcatttt ggaatgtgtc ttgctgctct tctcatcaaa ggtaaagtcc tcttcttcac 120540
   cttttaacct agagtagctg tgcaactttc tttgaccaat caaatatggt ggaagtgatg 120600
   ttgcgtgact tccagatata agtcttaaag agatttttca ctttctgctt tcactctctt 120660
   ggaatgttgc cctgagatca ccacaccatg aagaagtctg gggcgaaagg ccatgtggag 120720
   tgagaggctc agcttcccag atgatctcac tgagcacagc ccccagccac ctgccagctc 120780
   aatgcaacct ggtgatggac ccaagcaaat accagcagaa gaatcacctg gtcaacccac 120840
   agaatcatga gaaatactaa tttgttgttg tcttaagcca gtaagttttg agagagtgtt 120900
   ttaactgttt cacctaagtt tggtctctta acatgaacat aagcactttt ttgcatttgt 120960
   acatcctttg tactcttcct gttcataccg agaaagcttt tgcaatgcaa aagctcaaat 121020
   gtattggaat tttagaagtt taagccttga aggactctgt acaaaaccat gccaaaaacc 121080
   atatattttg aagaatatgt tctcagtctg agccagtttt ctatttccac ctcaaatctt 121140
   gattggtgca gactggagaa aggagaagag agtcaggggt aagaccgtgg gtcggaggtg 121200
   gatatcgaca atgtacctga gtaaaagccc tgtagcctct caccatgttg gaataccata 121260
   tggcacaact acatggaccg ccatttacat agagtaggat gtttagtact gcgtcttgac 121320
   ctttgaaacc agactggtga gaggactcac caacaaaagt gaagctaggg tcataggccc 121380
   taccttggag attggtaaga ccccagagtt attccttgcc caatgttaag taagaacaga 121440
   aaacatttct aaagagggtc ctgaggcaga caggtcttaa gcagtcccac agccctcatg 121500
   tgccctagaa agatgcagag cgcccagaag cggtttttca gtgcaggtga gaatggttta 121560
   gatccacatg tttttgtaaa ttagttttta ttggaagaga ggcaaaccca tacatttacc 121620
   tgttgtctat ggctgctttg acactacaat agcagagttg ggtagttgtg acagagatca 121680
   tatggtacac aaagcctaaa acatttacta tctggtcctt tatttttaaa aagtccttat 121740
   attagatgaa acacaaagaa ctggtaaaac ttaagccttg tgtaataata caaaacaaaa 121800
   atataccatg tagaccataa tcaaagggcc cattgtcttc agccgctgac attgggacca 121860
   gggtgtagtg tcagaacatg aaagcctgga tacctacaat ggcctaattt tctcatacaa 121920
   atgcatccta ccactcccat cccatacctt atgctagata ggatccatgg aattagctgc 121980
   atctccagtg aaacaggaaa agtccctgat tttactgaga tttagctatt gtcacatggt 122040
   gtaacaggga tttaagagaa aaaataacct caggtataga cataggaaaa taatgttaga 122100
   ttaagtttct tatacacctg gttctgtctt cagaaactgg cacccactat aaagccttcc 122160
   atagtgataa ttatcgcaca aaatcgatac aaaggaaata cctgctgaat gttctgcctc 122220
   agtaggtttt cctttggtct tttaccttag aaaactgcat gatagcattt ttctattatt 122280
   ttgacatttc atgtaggaag catgttattc acaacttatg ttttataaaa tctttttctg 122340
   atgatgatga tgatgacact tgttaatttc catctccttc cttcactgta agtttttcta 122400
   ttccctgaga caactatttc tctcacttgt cctaaacccc aaggatacaa gagatgcata 122460
   ggaaaatatc tatgaagtgt gtgggaggag acaacagggc agggttccag aggtctgtgg 122520
   tttattgcac gtaaaacact ggaaagtgct ttggatgata aggaagtagc ttgttgtcct 122580
   ttgcaatggc ctgacattct tacactgaga aaatcttact ccccactttg agctgggatg 122640
   tgggagctgg gaggacattt actgagtatt aactctgtgc caggaattat tcctacatta 122700
   tctcatttag tctccggaaa accctacgta acaggtgaga agacctcagt gacaagctca 122760
   aagatgcata gctaaagagc tgaaatgtaa aagcaggtct gccagactca aaaacccatg 122820
   ctctataaac tacatcatag aagttgtaaa ataattttac cttgaatcca agggtgaata 122880
   atttgtcatg gttagcttgc cctttggcat tcataaacaa tttctactgt cccagttttt 122940
   gttccattca tataggagta acatgtggaa gtattaagca atagaaggct ttgttttcat 123000
   gtataaattt ttacgatatt ttcaaatata ataaagccaa aattgttaat ttcacttcat 123060
   gtagagattc cttcaaatat aagatgctat gaaacagtgt cataccaaaa agcaaacaca 123120
   ctgatttcta taatcaaaac attgaggaac acaggaaact ctagataact cagaggaaaa 123180
   agggagtaca aaatcgggtt gacacaatgc ttcatttaag aataggaaca ttgtttgttc 123240
   atttgggtac tttataactt taagtataat gaggtttctt taaaaaattg taacaatttt 123300
   ggtataattg tataataact tggtatgtgg tatttttatt tctatttatt tatttattta 123360
   ttttgagaca gagtctcact ctgttgccca ggctggagtg cagtgatgtg atcttggctc 123420
   acagcaacct ctgccttctg ggttcaagca attctcctgc ctcccaagtg gcctccacgc 123480
   ccagctaatt tttgtatttt tagtacagac agtgtttcta ctaaatgttg cccaggctag 123540
   tctcgaactc ctggactcaa gcaatctgct ggcctcggcc tcccaaagtg ctggaattac 123600
   atgcatgagc ctatcacact cagccgtagg tgttattttt aaaacatatc caaaatgatt 123660
   tggatcattt ttaaaatgat cctaagtaat tctttataat attaatagta taatttggtc 123720
   cattaaatta taactagaaa aaaataaata aaattcttcc aaattgatat attgatgtaa 123780
   aaataagggt ctctccttta agtctctcca taaactttag ttgagagtga aagacttttg 123840
   gaccaatgat tcttaactaa agcaatttct tgacagaagc aaaagtgtga aatgagaaga 123900
   tacaataaat ttaacatcaa aacaagaact gtaagtttta tcatttaatt gtattgaaat 123960
   acatacaaac cactagatat gtggaatttc agaggcattt aaagagtcat tctaaaagta 124020
   tatgaaatta tttcatgaaa aggtcgatca aaataagaag aggatcctca taaataaaaa 124080
   tgttttataa tatctcgatt ataaaagtaa tttcacagta acttttcctt tctgcatctt 124140
   gtgaaacaca atagacctga gacagcctgt cagtttgctt tatggaattt gactttttgc 124200
   agcctcacag accacgatga actggtatta ccagttttgc catcttatct ttttatttaa 124260
   acagcaatta aacactgcca taatatcata taaatcatta aataatgttc cagaccacac 124320
   acaagtggat gagttatgaa agactgaagg attatattac ttttaaaata ttttgcttag 124380
   atagaataat ggcaataaat attagcaccc tttgttttat acttttacca aatattgagt 124440
   agtttcaaag gaacacttac taaatacatg caatacataa ggaatcataa tacaataaac 124500
   aactatgtac ctactactta cttaaagaaa aataatattt ttgtatctgt catggaaccc 124560
   tctcagatcc tgtcccctcc taattttgaa ttttgtgttc aatcatttcc ttgtttttct 124620
   ttagaggttt actgcatatg cctaaacaaa acactggtta atttcatggt ttttacagct 124680
   ttgaacatta atgaagtgta tcgatattat tttgctactt gatttttttg cccaaaattg 124740
   gatacatgag attcgtacat gttgttgcat gtagctgtaa tcaattaact ttcacaccgt 124800
   attatgttaa tccattctgc tcttgatgaa catttgaaaa atctccaggt gcatgtttat 124860
   gtgtgagaga gattacaaat agtgctgcta agaacatttt tatactaccc tggttaaaga 124920
   gcatgtgaaa gatcttctct tggaaaaata actaacagtg aaattgctgg gtagtagcaa 124980
   atttaccaga taatgccaat tattttccga agtgcttata taacacaccc ttatggtttt 125040
   aatggcatgc ttctttacta atttatctct ctctcacaca cacacaggca cacacacata 125100
   cacgatatag agatatatcc ttttaatata cattactcct tttctgaaaa tgattttata 125160
   tactaaaaat ctaaataata gtattagtgc tggcttttca gaaaacctag aattgtgaaa 125220
   gatgtggttg attgggaatt tttgtcattt gttgccagga aacaaaatat taaggaaaaa 125280
   atgggaagaa tgttttagtt gtattttttt tttttttttt tttttttttt gggacagagt 125340
   ctcgctctgt agcccaggct ggagtgcagt ggcgtgatat cggctcactg caggctcctc 125400
   ctcccgggtt cacgccattc tcctgcctca gcctcccgag tagctgggac tacaggcgcc 125460
   tgccaccacg cccggctaat ttttttgtat ttttagtaca gacggggttt caccgtgtta 125520
   gccaggatgg tctcaatctc ctgacctcgt gatccgcccg tcttggcctc ccaaagtgct 125580
   gggattacag gcgtgagcca ccgcgcctgg ccagttttat cttaacagat ggaaaaacaa 125640
   atgtatttat ctttcatatg ccttctatta cacttctgat gtcttcattt atagggctaa 125700
   aatatgcatt cactctattt tcaatcttgg tctttggtct tttaaagaag cccaagggct 125760
   gactggtgac attcaaaata atgtgttatg caagaaacca attttaggga tgaacgatac 125820
   ttcttcagct ataactccta aaaatcatca gaattggaac actggaaaag aatttaagat 125880
   atttttgtct actctgtaac tctttttgta cattttttct cttataacaa gcatctactt 125940
   taagttgaag aggatccgtt aactatccaa ctaagtatat ttcaaatgct ctaaaagttc 126000
   agacttgact taactaaaat cctctaggta caatactacc agaagcacat gcttctcatt 126060
   tttatctaat aaagtttctt taatcagtga aggcctataa gaatgtgatg tcccttaaaa 126120
   gaaatgcatt gcaaaattcc aaaaaattat ttcttttata ctctctttgt gtaaatatcc 126180
   tacttttcaa aatttaccaa tgttataaga actccaaatt atttaaatta gaatcaagca 126240
   aatggtaata ttattttaaa atgttcctag agtggtaccc tcccagctct tgcactctgc 126300
   tgtttttaat gtgtgggccc ctgatggtct tccatgagaa ccagaaatct gtaagactct 126360
   atcttgcatt taagataaag tggtttaggt acttcattac gtgttcattt taatggtgca 126420
   gtgcactcat taatatgctt tatcaacact aaataagtaa cctagtgtat gtatgagcaa 126480
   tcattgatat tagcctaatg agaaattact gggcagtaag tactatgata agaaacatat 126540
   cataggttta tattacaaat taaatagaga attgcaaaaa ttttctgagt tttctaagca 126600
   aaatatattg ggccctaaat tatttggcta ttgttattgc tttgttgatg gagaaatact 126660
   cagcacagag atgcttttct aatctataat ccagtatcac aaaggaagca tccaccatta 126720
   aaaaaatcaa aaatcatacc tcaaataata ataaaaatat caatggaata ttaatgattg 126780
   cttattatat gtaccaataa ttttacatac tctaatttgt acaaaagctt ttcaggtagg 126840
   taatattatt atcatgttgc agatgaggag attgagtttt agaagatttg tgtgaattac 126900
   ccaaggcaac aaagctagtg agcagcagag tgaggattca gttcatgcca gctgatgtag 126960
   aagctatgtg cttactatac tattcaactc taataagata tgtctttcat tagtaccttt 127020
   cttctcatat gtatgaactc tgatggtcca aacatatata agcaccaggt acatacactc 127080
   atgcctgaat gaaatgcctg ctgtatacac tatgccatgg gaagtaaagt aaggactcat 127140
   tcaccctttc ctcaaggagc ttacaatgta gatcgagggg ataagacgtt aacaaagata 127200
   cgtataatca caagcaaaat atgtcaatag ccataagaaa catattctgg caatgcacgg 127260
   aagaaagaaa tcctgttggc taagtgggac aaaaagagtc tttataaaga aggtaaggtg 127320
   aatcttttgg ggaacagtga agtggaaaat caggattttg cacaaagcat gctttatgcc 127380
   aaaaagctta aggtacgttg taggcatgac ttgttttatt ctggctgggc tgggacaggg 127440
   catgtataaa aactgcctag agactgggca ctctttatac tctttgatga atttgtacca 127500
   aattattaaa cagtgggaaa ctattgaagt tttttttttt tttttttttt tttttttttt 127560
   ttttttttga gacggagtct cgctctgtcg cccaggccgg actgcggact gcagtggcgc 127620
   aatctcggct cactgcaagc tccgcttccc ggcttcacgc cattctcctg cctcagcctc 127680
   ccgagtagct gggactacag gcgcccgcca ccgcgcctgg ctaatttttt gtatttttag 127740
   tagagacggg gtttcacctt gttagccagg atggtctcga tctcctgacc tcatgatcca 127800
   cccgcctccg cctcccaaag tgctgggatt acaggcgtga gccaccgcgc ccggccacta 127860
   ttgaagtttt gaagtaagga atgatatcat tcattcaaaa actatttgtg gggtgtcagt 127920
   atgtgcctgg cactgtgaca gatgatggga atattgaggt gaacaagacc agtccatttc 127980
   ctgccctcat agatcctaca agttgatggg gagaaagagg ctaattttca ctatgactaa 128040
   gaatgtgatg gccctgcatg aaacagaagc aaagcctctg agtagaaaaa actttgatgg 128100
   tttggtttac atttcctcta ctaccttttc ttaaagtaaa tattttattt tagaataatt 128160
   ttagattcta ttactattag agtctaatat catttaaagg taatttagaa aatttatttc 128220
   aattactatt aaggttgcta tttgatcatt taatgcgtat ccttctatgt cataactctg 128280
   tgggacaaat ttcagaaaga ctaacacatt taaacatcag ttagttgcac aatctaaact 128340
   gctactgcaa ctaggtagat aaacatgtta aagaaatgtc tttggtacat ctgtacttgg 128400
   ctctgatccc tctgagcata gcaagttaca aagtgttgag ttggaaaact ttgtaatgac 128460
   caaagaggta ttacaaatac agaaaggcca attttaaaag aagtcaacga gaaaggtata 128520
   caaactgccc aactataatc aaacacttgc atgaaaaaaa aattgtaggt aagacctgct 128580
   tgtattaaag gtttttcaca gaaacataaa actcagagat ttatgaaact aaaaagtatg 128640
   gaagattact gaattaatcc tgaggtaaat aatgtcccag tagaaattag cagtaaggta 128700
   agcactccaa aacaatcaaa ttagcagggg gacataaact gatatgaggg taaagggacg 128760
   actagtgaga aataagaaat cagactacag ccaaattaaa aaaaaattta aatatattga 128820
   tgcttagttt cctttaagca ttgcaacata tgatattgac aaatgtggtg acagctggta 128880
   aaaactgtat atgttaattg ccaacatcac ataactaata tttactataa aaagtgtccc 128940
   taaaataact ttctttcatc atcagatagc catatccatc tagcattccc tgtttatgtt 129000
   ttgaaatgta tgtattacat tttttcttct ccttttagga ttaaaatata aagttttatg 129060
   ttccctaata tatctacttt aagaatattc taaatattga aaataaaaaa ttttttaata 129120
   aaataccact gacagctgtt ctctacttat catttcttag cagggtgaag actatatttg 129180
   ctcacataaa ttgtgtcctt ataacagaaa aagcttctta gggctggtgc ggtggcttat 129240
   tcctgtaatc ccagcacttt gggaggcgga cgcaagcaga tcacttgagt tcaggagttc 129300
   tagaccagcc tggcctacat ggtgaaaccc cgtctccact aaaaatacaa aaattagaca 129360
   ggcatggtgg tgcatgcctg taatcccagc tacctggaag gctgaggcag gaaaattgct 129420
   tgaacttggg aggcagaggc tgcagcgaac caagatggga ccactacact ccaaactggg 129480
   tgacacagtg agactccatc taaaaaacaa acaacaacaa caaaagaaaa agcatctcag 129540
   aaaaacgttc ccctttttca ttgattccat gcaaatgata cttcacccta ttgtttactt 129600
   gtcctgaaat ttcaaagcag cagaaattct gctatacatt atccttttat ctggtattta 129660
   ttgaaaatgt atttggctat gatttgaaat atataacata gtgtcaggtt tattttctca 129720
   tgtaacgagt caagaagtag gtggttgctg cctttctttc agttattcaa cactataatc 129780
   aatcactgtc ttgtcttttt atatttctgt tcgctatctt ctatgtgttg ggtttccttc 129840
   ccggtgggtg tcatatcagg gtcacaaaat ggctgctatt gcccagtaat actcgaatgc 129900
   tcgagcgagg gcaggatgga gggaggggct gctagctgta tctgtctcct tttgtcagaa 129960
   aagcaaaagc tttccgggaa gctctcctag cggcgactcc ttacatctca aggccacaaa 130020
   tttcaaagag tggaaaagca gaaaacaaga ttctcatttt tggcaccatt gtgagacatt 130080
   gcctaggaaa ggcatattga caattcaaac aaaatcttaa attctagtag caaaggacac 130140
   agaggaatga atatttggta cgggacagaa aggatctgcc taaatgccct agttcatttg 130200
   aatgtaaaat tcttaaaggc tgggattgtg cctgttctct tctttgtgta taggactcag 130260
   atcataagaa aaatctttga cactacaaat tctatacact aaggcagtat ccccaaattt 130320
   cattcaattt ttctaatatg ggggtgctac tgagcacaaa actagaaaga gattataaat 130380
   atacatattc ttggtcagtt tagactacta tagcaaaata tgatgcacta ggtggtttaa 130440
   atgatctcct cttagggccc ttgctgtgtc cttgtatggt ggagagacag ttctctggcc 130500
   tctcttctct tcttataagg ccactaattc catcatgggg acttcaccct cacgtcctga 130560
   tctaaacctg agcacccccc aaaggttcca cattcaaata ccatcacact agaggttagg 130620
   gcttcaacat ataaatttgg gggaagataa gcgtttagac cattatttat ttatatgtta 130680
   aaaataatat agaatattcc cagaagtcaa tttttgctcc atgtattaga atacttatta 130740
   aaccttccta tgcccagcaa aattgtagac actgaaaaaa taccaacaga aaatggtcca 130800
   gcttttaatg catttaagct gaaataagaa gtggtgaaca cagatccaaa ttgaactaaa 130860
   atgttatgta atctataata gcaacctttt atctggtgtc tattatgtga taatggtata 130920
   acgtacttta tctaatcttc acaacaacac tgctaggtag ttattagcct catcttaagt 130980
   gttagaaaac agagaacact gagaggctaa gaaattcatt caaggccacc cagggcataa 131040
   gcagtggagc tgaagtttgg acaagatttc tttttctccc caagccatgc ttgcacataa 131100
   tgcacatacc actacatgct gattaattca ttttatgaat aaaatcttcc ctcttataat 131160
   tctgctttgt ttttatgctt tttcattcct agctttctca ctggtgactt atgtggatga 131220
   atcaatggga acaaggtcag atcatggaca gatgagaaaa taatgagaca ttagattatt 131280
   aagagagatg aagttaagag tgagaggtga atgaaaggga agaaggggtg aaagctcagg 131340
   attatgaagg gaataaacag gaaagaggag agagaagaag agaatgaata ctacccagat 131400
   atttctctgg gcactgtgga tctgatggaa tggtggtatc ccaccctgta accctgtctt 131460
   ctctgatctc tggccaagtg tgtggcttgg cactgcacaa ctaacagtta gcagcattct 131520
   ggggagaggc tcatggtagt gaccaaattg cactttccct agctattctt gtgttctaat 131580
   cctcccctga aggtctttta tggttaatct caaaccatat aaatatttaa ctctcctttt 131640
   agttacattg cagaagcctg ttctcaaaca agtataagtt tcagtaacca caaattgata 131700
   ctaatctacc ctaatgtcac tgtgttcctc ttcttcaagt gagttgaaag catggatcca 131760
   tacaatttgt cttgttcagt ttgtacctac ttgtatcttc cattaacaga gcatagactc 131820
   tcaggtatgg agaacagatt aagatccaat tactcagcca aaatttcaat ccacctctct 131880
   ccaggacatt tttttgtttt tgtttttgtt tttctttaac atcctccaaa ctggtagtat 131940
   agctatatcg agtacttcca gtccatggaa cctaacactt cctcaagtag tcctttccat 132000
   gcttagacaa cttcgacttt agagtgtggt ttcttatttc accagtcagg atagctaagc 132060
   tgttctgcag taacaaacag ccctaaagtg tcagctctaa tacaacagag gctttctttt 132120
   tttttttttt tttttggcta acattatgtg cctatcaaat cagcttggct ctggtatata 132180
   ttgatatcca tctgggatcc tggctgaggg agaagcccct ctctgggaca tttctgatct 132240
   catggcagag gaaaagaggt cctggaggat cgtatactgg ccattagagc ttccacatgt 132300
   cattagccaa catgcatcac atggagtcat cccacagaga aggacagggc caggagcaag 132360
   acaacccaga gaacagccgt aaatatctgg aataacagtg caatgtacca catagttatt 132420
   gagtcaaatc cttttccaag tcatatccct ccttccccat tgccttttct ctttaccagc 132480
   tctacaaatg ttaggaatcc ttagccatgt tttcttcgag ctgtctttct tctagtctaa 132540
   ttggctccaa ttcctccact gacctgttcc ctgtatctag tggtcttatc tgacattctc 132600
   ttgagccttt tcagctgtct caagtcaact ggtgagagaa ttgttggtct attctccaaa 132660
   ggaagtggtc ccttgacata ctttccttta ggatgatggg tttggcgatg tggctgagcc 132720
   ttctagacaa aaaaagcttt ccttaaacaa tatctcaact aatcagtctc caaatatttt 132780
   agtagcttaa atctttaggc ttttcacaaa tgcccagaca ctttgcctgt tatatattag 132840
   aatgtagaga tgtttgagca cttccagagg gcaaatgtca catccctgac tatactttag 132900
   gtaacttagc aatatatata catggttgta ttgacaaaac tcctcaagcc tactattttt 132960
   tcataggact ggctcagaat gtggactttg gaaataacca gtcttggatc aaaccctgat 133020
   tccactcagt actacctata cgacctttga caagttaatt aatcctgagc tcttatttcc 133080
   tcatctaatg agatacagac aataagagtt atctttctca tgtggctgtt gagaggatta 133140
   aatgacaaca gtggggctca atcagtgtct tcttttgtta ccatactggg gtcctagcac 133200
   tcagagctat ttctggtctt actcaaggac caggtcctct agcatccctg ggtttaccat 133260
   ggttatgcat agctccctcc tctctcccag gtgtccttct ctcctcctga aatgtttcag 133320
   cctcaagtca gcatcatccg agcttgacca gtatctctca aggtgttgtc caagaacaag 133380
   ctgcactgga atcacctgga ggcccacgtg gctccatttc agactaactg acagagttag 133440
   aagatgaaac tagtaaatct gtattttaac aagcttcccc cagctagagg tgccagataa 133500
   attataggac atccagttaa atttgaattt cagataaaca atgaacaatt ttcagtataa 133560
   gtatgtctgt cccatgcaat atttggaaca tacttatact aaaaaaaaat ttattattta 133620
   tctgaaattc aaattaaatg gagtatcctg tacttttaat tgctaaatct ggcaaacaaa 133680
   tctcagggat tccaattctt actaaatttg aagaccactg gtttaagata tttggcaaaa 133740
   atctcaaatt caacttcatg agcttccaag gagggaggga gggaaagaac gaaggaagga 133800
   aggaaggaaa cgaaaggaaa gggaagggaa ggaaggaagg aaggaaggaa atgaaaggaa 133860
   agggaaggga agggaaggga gggaagggag ggaaggaagg gaagggaggg aagggaagga 133920
   agggaaggaa gggaaggaag ggaaggaagg gaaggaaggg aaggagagaa ggagggagga 133980
   aggagaagag aggaaagaaa gaaaggaaga aagagacaaa gagaggaaag aaaagcaaag 134040
   caagcgaagc ttatagacgg aacacagatg gcccatgcat ccacaaaaca aatatgcaca 134100
   agaccaagta ggttagcatt tgtttcagca gccacagtgg caaacataga caatccagca 134160
   gccagagaaa aatccacttc cttgtttctc tagtttggaa ttgggcttta ggtgtctctg 134220
   tctccttctg ctgctttctc cttttttttt tttttttttt tttttttttt tgtctaaaca 134280
   tctccatgct atgctagcca acacctactc atcctcattt ggtatttcct cttttgcttt 134340
   tttgctttcc tacaaagcat cttgggccca tcctcctctg tctgccccaa acatcagaat 134400
   acctaatcaa atagtcatat agccatcttt ctccctctaa tattaagtgt ggactccctc 134460
   aattttagtt ttctgatggg attttcctaa ttctcaaacc attctgtctt atgtgtacta 134520
   gagtaatgca ttgtgttttt ggttcgtgta catttacaga ggccttaaaa gagacattag 134580
   ctgatttttc acaataaccc cataatcgag gcagatgaaa aacggagccc ttttattcct 134640
   aggccaagac ccttcttact cagccattgt ctcatctcag gttattatac tcacatcatt 134700
   catttgtcat tttagaatag tgattaaagc acagctgtgt gccttggtta gttttatttt 134760
   tttcttaacc aatctaagct tccatttcct catttgtaaa cttaggttaa caatagtgcc 134820
   tacatcatga gcactgggtt tagtcagatg atcaaagtct tagcactgtg actgcaaatt 134880
   gtaagcactc aaaatattag ccattattaa aactatcttg tgggccaatt cttttccagg 134940
   cccagctttt ctcttgttgt ctttcagcaa atagagatat aaaatggaaa acttagacta 135000
   aaaccagcag aacaagaagt atcaatggct gcacaaggaa gtaatgcaga cctatgctaa 135060
   atttctcttt ggtaaacact tcctttttca tacccctctc ccttgaactt tttcacctcc 135120
   actcctacaa ttttatgtta tgcagagtca aaaatataat catcctaaac taggctcagc 135180
   agtgtggaca gtaactgatt gaaatatgag gttctttaat tagaagcagg aactagagta 135240
   tagtactaag aataatggta atataattat ttgcatcttg tctaacttcc tcatttacac 135300
   tggccttctt catttctatc tcattccctg ataaatatgg tatctcaaag ccaaatgttt 135360
   gggactccaa tattatcatt gtgttcacct tctttaccca ttagggcctt tatatgataa 135420
   taagtgtaat atgatatccc caatgaatga gatagagtgg cccatacagg agcgacctca 135480
   aagcactttt tctgtactgc agatgcagtg caaaattagg aatattagga aaattacgac 135540
   tgctttcttc agaaaatact cagggcaata ttgttgtgga taatgggaaa taactggtgg 135600
   tttttgaaca aggaaagtga atgtcggtta tagtattgaa tagaatagtg ttctagaaat 135660
   agacaatcta tgtagaccag taagtctgaa gtcagggtca ctgtgcagca acagaggaca 135720
   taatctgaga ggctaccttt tcatgactgt gtcagcaaat acaccaccat tgcttttcat 135780
   cttagaggca gcacaaaact atgcaaaaga aaaatcaatc aagaacatat ttaggacaca 135840
   gcagggaagg ctcctataaa catttttttg ggaagaacat agatatatga tgctttttta 135900
   aaaagttatc agtggaaaaa attggaattt cacaggactt tgttatattt gtttttggct 135960
   tagagtttgt tttgtttggt ttctttgttt aactatattt ttggaagggt agaaggtgga 136020
   tggacattgt aatcttcttt actatttaga aactctaaat gtcttaatct gattcagtcc 136080
   aggactgcat aggaattact tgatttttgc catcgacaca ggtatcataa tccctttact 136140
   gctctatccg gagaatgttg tgttttttcc cccctttaag atgaggaatc tgtcagtatg 136200
   gttgctccct tatgactaac aggttagatt atgcttaagt attctacgtg gtccctccca 136260
   taaaaactgc ctcagaaacc tccagtgcct caaatactct tgtccttgaa tggtacaagg 136320
   aagtagagga ttcctggggc atatatgata cttaatctct ctctctgcct actgacattc 136380
   taacattttg gtgagcccca ttttatttgc tatggaattt gacaaacagt tcaatcccac 136440
   aaacatcgag tgagcaagta ccatgtgcaa gcaattcatt caatgaatat tttcaactcc 136500
   tactacgtat catgcataag attcacccta atgaaggaga agaaggagga gaagaaggaa 136560
   ggaaggagga caaggaggaa gttgggagga atccctgttc tcaggagctt caaatcctgc 136620
   tggctagaag atatgtaaac ataagggcaa tagaaatctt atgctgcctc tgtggactga 136680
   gagtgacagg ggatagatcc ttgaagaaat agcagtgtgc ttgggggaaa cagatgctgg 136740
   gcaggcaaaa acttctcctg aaaaatacaa actgtgactt gagccatgcc tgagggagaa 136800
   gtagaacttt gattccagtt gaagcatagg cagtagaaaa tgtatggatt tgagagttaa 136860
   aaataatttt gaacccatct tcaccaatga gctgtggtga ctgtgaataa gttattttgc 136920
   ttctctaagc ctccactcta aaataggaat tgtgatgttt agcccaaagt gttgaggtat 136980
   acatctcctc aggcctcaat gcagtcttgt ataatgactt agagcatgga ctctgaagcc 137040
   aaactgcctc agtttgaagt ctggtgctaa cacttagcta ttaaacttgg gcaaattatt 137100
   gaatttatca gtacctcagt ttccacatct gaaaatagaa atgataatag caaactgacc 137160
   tctcagtagt tgtaaattaa acagacaggt taatgatata aagtacttta ataagtgact 137220
   acacatggta agccctataa ccatttgcta ttattaccaa gtgtttagtg atacaatata 137280
   agccattaaa ttaccatatt acctggttag tagatgctat tttttattat agtgcactgt 137340
   gataaacatt gtaagaaata taaatgttcc ttgtattttt agggaagaca tgaaagaaat 137400
   tatagcctag tcataagtga cactcacaag taatcataat aaagatggta tgtaataaat 137460
   attgcaagaa agatcagata tgtccaactg atagttcaga agaataaggg atcaggaaga 137520
   aaaaaaaact ataatagaga catggccttc tcatcatggc ctgggcatga tagcaagacc 137580
   ccatctcttc aaaaaatgaa gagatatccc ctaggagcat gcaatcctag aggaaggcat 137640
   gggtgtacct tccagattgg aaggagaggt aatgattgtt gagtcagcaa tgcaagatta 137700
   ggtagactat tgctgggtgg attagtgcag ggaatgcctt ccctgatgaa accaacaatg 137760
   gttgcaaagg catagaagca tgaaagggca aaaagtgtgt gtgtgtgtgc atgttacaga 137820
   aaaagcaaag cagtcaagag agatggaaga tgtgtacttg gggacatcac atgagcgatg 137880
   gagacacaaa gatgaataag acatgaagga aatatttttg gatgatgcat gctggagact 137940
   gtttttgttt tttgtgtcct tttgtcctta acttcctcct tccttttttc cttcaaaata 138000
   catttactga tacctacaat gtgctagaga aatatgactt tccttatatc acctgttata 138060
   taacagagtt ttgtcccatg ataagtgata taatagaaat cgcctcatat gaaacaaagt 138120
   tatgttatat cagaaatgtg ataaataatt ttccaagtta caccgaaact aggccccaaa 138180
   tagggctctt tcattaaact ctttgttcag ttgctttctc tcactcccat gtccctaaag 138240
   tacatggata gtagagaatc cttttttttt ttttcagttt atgtgggctt agcaaagagt 138300
   gggcaggcaa tagagacttg cagatctcaa ctgaattact ggagaattta attttaaatg 138360
   ctctagaagg ttatcttctt cacaacttgg tggcaaattc tttcaggaac tgaaagttgc 138420
   atgtgtttta aaggggcata agcttgtgga gaacctttct ctttggttta gctgcatcga 138480
   tatgctgatt ttttaaaatg cactgaacat gcaaaaatga gtatattatt gacctcaaat 138540
   caatgcccat cactgtttct atcttataca gtgagcttct tggcttttta atattttcat 138600
   tgtgaagtta ttccaatgaa gacaaaattt tgtcaaattt attacccagg gagctttcaa 138660
   aatgaagcag agagactttg accgagcaat ttcacttctg gaattttatt ctacttatgc 138720
   atacttatac atatgtgcaa agacatatat atctgaaagt ttattatttt ttcgaagcat 138780
   tgtttatagg gagaaaaagc aggtaacagc ccaactgtcc ttcaaaagag aactagttaa 138840
   tttatgatat atccatataa ttaatattat tcagtcatta aaaagaataa tatagatcta 138900
   tgtgagctta tttggaatga tctccaggac atgtttttaa attaaatcac aaatacaaac 138960
   aaaagaaaga gagaaagaaa agtacagaat aggtataagg aagatttcca tttgtgagac 139020
   ataaatattt atctgtgctt tgtatattta tttagtacac atgaatgcta tgtaggcatg 139080
   gaacatttct gaaaagatca gcaagaaagt acaaatagtg attatttctg gatgggaatc 139140
   tggactacgc agcaggcaca cttttcattg cctgtccttt gtattatgtg atttttacca 139200
   tgctaatata tttttattaa aaattaatta aagaaagaat aactactgga gataagccat 139260
   ttttggtcaa ttccatagtg gctagttaga ggctaagtaa tatgcaatca tgaaattaat 139320
   gctcaaatta ataaaacagt atttcccaat cttgatttta ttacaagttc atttctgagt 139380
   agttcaagaa cttgtagaat tattttaaag atctcaaaat aaaagaaagt ggtaatctaa 139440
   ttttgaaagg aaatatgttt cacacagttt gggaaagaga gaagtattgt tttcaaatag 139500
   ggagcagtgc cttagtgagt aatttcattt gcattcctgt gtatctttaa gctgagtaca 139560
   aactaagaaa aactgttctt gcagaataca ctgtaaaatt actttaatca gtaattactt 139620
   tgacctttcc ctatcaaagg tcaaaacacc ggacagcctg ttgctcttaa catcagctct 139680
   tctgcgtaat atttctcttt gctctggaac acaattagct cttgggaaaa gaggccaact 139740
   tttcagtttc tgcctttcat ggtttctgtc ttcccctctg cttcagtgca ggtcagtcac 139800
   acagataagc attatgccca gaataaaact ggctctgttt atctggacta ataaatgaat 139860
   ggaaccagtt attttcattg gcttggtctg gaaggaaacc aaactaattt ccacagacct 139920
   taaattggac ctaatccaag ctacttgtcc tcactaataa gagcaataac aattagtata 139980
   aaagcaaaaa tttttacaaa agcaatccat atttactgct gggcaagaaa attggataaa 140040
   actaggaact aacatgaggg actctgaaag ctctgagaaa tccacttcat ttgtatttca 140100
   atgagtctga gctttgcagg tgaatacaac ctttctttag gttctagata ctccagtttc 140160
   tctctggtca atttttctaa tcaagttgtg ctaacttccg ctgatgagca gaaagtatgt 140220
   gtatggtaca gtcaaaactt ttgcactttt gtcttactta agaaacttaa aactcacatt 140280
   tgtattccta gggtttctga caagtcggca tactttattt cacaatgtgt tcatggaaca 140340
   gtgagatttt gacttccatt gtgcacatgt aataaaatgt tctcattagt gatattcact 140400
   cagtgtatga aagcagttac tgcctctgcc caaaaggcat tgctgtttcc atttaagatt 140460
   tatggttggc cttttaatta tactcccaac actgccccta aatatatccc catggaaaaa 140520
   tatataaaag tatttcactt aattattatc ttgctcaaag atcagggcaa tatcttggag 140580
   ctgcttccaa gactattgct ttatgatttg aaagctgttt agctattttt accaaataat 140640
   taattcagat atatcaagaa tagggtaaac tctttattga aacgatttgt agggaactta 140700
   aagagtaatt aattttccat atgtattttt aaatgaaaat aacttattct ttctttctga 140760
   tttaaaaaaa tgcctacttg tagcaaaaca aaatagggga attcattaaa aaattgttta 140820
   aacccaatgc aacccaccta tatttctagg taccaagaat accactatga aaatgttcat 140880
   gaacatcctt tcagacattt atatggatag acattaggca tacacatttt ttaaaacaaa 140940
   tgagataata ctgaaggagt tcaggacatt ccaccccaaa atatgtccct ttggcgtatt 141000
   gattattttg atttaaagac acctggggct gggcatggtg gttcacacct ttaatcccag 141060
   cactctggga ggccaagata ggcgatgact tgaggtcagg agttcagatc agcctgacta 141120
   acatggtgaa accccatctc taccaaaaaa atacaaaaat tggccaggag tggtggcact 141180
   cacctgtagt cccagctact tgggaggctg aggctggaga attgcttgaa cccaggaggt 141240
   ggaggctcca gtgaactaag attacacctc tgcactccag cctgggtgac agagtgagat 141300
   cctgtctcaa aaaaaaaaaa aaaaaagaca cttagaagac aataggtatt agaagggctt 141360
   tctgaccacc ccttttctat ttaaaaacag tccataaaat ttcctatgaa aaagctgcct 141420
   tccttgtatc aaaaagtgaa gaacatcctt atcagcagag actgggaatc aacactaaaa 141480
   tgaatccata tacacaaatt tactaaaata actcttattg tccaccagtt ttccccactt 141540
   aatgttttat cacttccccg caattaactg cccctagccc aaaccacttt gtcttgtcat 141600
   ttcttcacaa attcatcact tctttgtcca aaaggtacat atgctttctg ctctggtcct 141660
   ttcttcatgg tttcattttc ctgtgaggat tcccatgcac atgtgccatt ctaataaaac 141720
   atatgtgctt ttctcctgtt catctatcta tgtcaatttg acttttaggt ccagtcagag 141780
   accacaagaa cgcagaagac acattttatt tcctctataa taccatactg ttttaaaatt 141840
   aacaatatat tctttaaaat atgtaaatat aagtagatga ttagatagag agatctccaa 141900
   ctcaccactt aaagtgctgc atcatattag attctgtttt catattttac caatccacta 141960
   ttgatgaaca tttaagtaat ttcaattcaa ttttaaataa cactatagca aataccaatg 142020
   tgtgtttttc tgattatctt tctaggtttt attcccaaag gttactgact atggatattt 142080
   aaaatgtttg aaatgtattc ttaacttccc cttcaacaat gttaaaacca atggttttaa 142140
   tatccttaaa ggtagtttct actttacaca aaagttattt ggaaggagat ttggggtaaa 142200
   ggaagtgatt aggaggaata gaggattaag agatgaggat gatactggat atatttctta 142260
   aaaaagtcta tgagaagata gaaaaatgag tacagcttag agatgctgaa atatcataac 142320
   agtaaaaaag gtatgtgtta catgaagtta ggcaccaatc caaagaagtt gctgagagaa 142380
   caatcttgtg aacctacaag ttaattgtag gagagaaact cagaagtttt agtatgaatg 142440
   agctggaagg ttactgtttc actcaggaga ttagaaagat aacaagagac attgcttaaa 142500
   tctattttca aatataatgc taataatttt ttcagtaggc tataaaaaca agtcttccct 142560
   ttcctactgc agttaaaaaa aatgcattgt ccttttcccc cctcacactt tgtttgtatc 142620
   aggaccttct tgtttagatc aggttactaa atatctttag aagacactac atttagaaaa 142680
   agaaattatt gttttgaagt ttttccaaag caaataaaga taattcttat acttctatag 142740
   atatttaaat ccaaatgtgt ctgttcaatg gataaaacca tagaggtctt taattttgca 142800
   aaacaagccc atggcaaacc catgaaatag aaaaatgtat tattttgaat actactatcc 142860
   aatcttcccc cacgctttct ctggtgccca gtttttccag gtgacactgc tatacctcag 142920
   cctgaagcct ctacccctct tgttcagctc tgtccctgca gacagaactt ctaggtgggt 142980
   ccccaaggac cagatttgcc tcactctata ccctctgaat gctttagcct ttgacttgtt 143040
   tcctcaaggc actcctaaat gatgctcttt taactgatga actattttga aaattagtga 143100
   ctgagggtag accagtgtgt tactactcca agtaacaaga gcaatttaaa agtgtccttc 143160
   agtgcttcag tggaaagaaa tctgttccat aaagggaatt caggcttcct gcaacaggat 143220
   gttttctgtt caatccatag tcataccaag cagggcaaca gctcttagtt tctagtcaca 143280
   atcatagata caggtaggag atactgataa atatttgata aatattattt cattggagct 143340
   tcaattctat ttaattttta attttatcag agtaatgcac agatacatgc ttttataaag 143400
   taaaatagca cttagagagc ttttaataaa attagcttgc ctgccgcatt gccccacttc 143460
   aaaagcagga tcttccacac ttgatttgtt tatgtgtact tctgaatttc taaactacct 143520
   gcccacttta ttgacttttc aattttagat atcattattg aatgatatcc ctgtaagaga 143580
   aatgaagatt aagcagtctg atgcacgctt ctcaccacac accacaaata catgtccctc 143640
   cttccatctt cttattctgt aacagacttt ggggttaaat taatatttaa tatttccatt 143700
   gatgtcactg tgtctatcac tcacatctga aagatgccat gttttgtaat ttacatttct 143760
   tttctcatat taattttcat tattcctaaa ctttaaaata gccatgcact gttttgtttt 143820
   cttcatttcc tatgtgccta tcatttacgc aatctcaaat tttctgatac tgctgctaaa 143880
   ctctcagtaa aatcagacac attaggtgaa ctatcagtag tacttctcct ttcatggaga 143940
   cattactcct ggggctgtct gtccttttgc ttctctggtc tattgcagat aatctgttat 144000
   tcctaactgt atctcttcat tcaaatgaca ataaagctca atatttagtg aagagcaatc 144060
   ttgacatgtg attcgaagac ataactttat tttcattttg attatttttc ctatgtctca 144120
   aataaggttt cttgtgtgag tgagggataa ccaggaatga gcaaatgttt ctgtgttttg 144180
   cctataaaac agacacagag tcccaagact gtctctctag aagaattttt ccagagattc 144240
   cttagttcat cctactctca aatggctaat tttattcccc ttgcaagtct ttaccaaaaa 144300
   tgattttgta gcctgaactg ctgtggtact agaaatttca tctgcttttt tctgagtatt 144360
   cccattcctt ctcattttta atcagccctg tatagtttag atttcatgtc ctcctctttt 144420
   ttatttaccc tctcattttg aagaagcaaa tagttcaaca gcctcttgag aaggaggact 144480
   tgagaggtaa atatattgag gtattgcatg tcaacaatat ctttattctg ttctcatgct 144540
   tgattgtggc ttggcacagt gccaaactca aggttggaaa tcattttccc tcagaatgtt 144600
   gaaggcagtg ctccgactgg aatggagccc atgctgctgt tgagaggttc agtggaatct 144660
   gatccggatt ctgaatcctt gtaatgagaa ctgatttttc tctctgggag acttttaagg 144720
   tctttctttt atctctggct tcctgatgat aagccttgct tcatgtgggt ctttcttcat 144780
   tcattgtgtt aggttcttaa tcagactttt caatctgaga atgtgtcctt cagtaccaaa 144840
   aatgtttatt ctatgatttc cttgataatg ctatcccctc tgttatatct ctttcctgtt 144900
   tgtgaaactc ctattaatcc atttatgcct agtgttccat tattggaaca ctaagcttgt 144960
   gagagttatt tatatcttac tgcccaaggt catcaccaag gtctgatttt tcacacaaaa 145020
   aaatttgcaa cctcctgcat aaatgggtta attggatgtg aaacctctga ttcttgtctt 145080
   ttctatactg tttttcatct ctttgatttt tatttcagtt tctgattttc tcaaattttg 145140
   tgtttaaatt cttctgtttt tatttgtgct actgtatttt tttttaattt aaagagcttt 145200
   taaaaaattt atttctttta gatagcattc tgttcttttt catgggtgca aaatctactt 145260
   ttgtttctgg ggatattgct tacagaaact tttcctgtta ctccatgaat ggtttccagt 145320
   atatggtgta ctggctggaa atttagactc tgcatccagc ttaaatgtca atcaacacca 145380
   ccattgactg tgcctatgac cttgggcaag ttatctaaac tctgctcatc agttgtctca 145440
   gtaaaataga aacaataaga gtatacagta acgcatggca ttattgtgag gtacaaatga 145500
   gagcaaaatg tattcggcac tcagaacagt aactggaaaa gaggaagccc atatgatatg 145560
   ttattatatc catttcctcc aagttccttt ttttcctgtt ttattttact ctttatcatt 145620
   tgtgttagac atttttctca aatgcctgat gatccttggc tgtgtgttca tatttaataa 145680
   tgaggaacta agaagtaaat tggaaacctt ctgtgtgcct ttgaactcct gaagagccat 145740
   gtgggaatat aacaccccac agcttcagtg ctggaaagac ctcatgatag agagtggtga 145800
   tcaaggagca ccagatgttg caatcactgc ctgcagccat atcagatacc ccaataagaa 145860
   ccgcctcgct gagtccagtc agcacccaaa accatgagaa gtcataataa caacaagtga 145920
   ttgctgttga tttgttccac caagtcatgt aacaatagac aaatgatgca gagaagaata 145980
   ggccctagaa tgagcaagag ccccacctag tatcagatct tgtgacttga aagtttccct 146040
   tcccatctag gaacagcctc tcaatctcta ttcctctaaa atctactaga caatatcctt 146100
   gtttgattct ctatagatat tttagtgtta atctttcctt gaagtttaag acaggtcatc 146160
   caaacacagc tgaaggtaaa tcggagtgtt ggagatgatg ccactctaag gcatagtaag 146220
   ccagttttct aagagttgag ataacacata atgataaatt acctacgaca aacacaatat 146280
   aactgttact tggattaata accctctttt tctcttcaca tctcatcagt tccctctgtc 146340
   atctaaatac attcctatgc caggaataga atttcagaaa acagtttact ttgcaaattc 146400
   aaaagtatct caaatttagg gaaaagaaag tgagatgaaa gtttcagtga agtcttctcc 146460
   aaagctaaaa caatcatttg aaaatagatg cccacaaatg ttgcagcttg atttatagag 146520
   gcaaataatt cagcattaaa tgaggagcaa catgattgct aagagtatat atgtttaaac 146580
   tgtgtcaaca gctattacac tgtgaagagg gatttggatt ttgaacatct tgtcaagtca 146640
   agagacgttg tttacatttc actgctcact ttatttctgc taccgagaat gagatttgac 146700
   ttcttccaaa gaactcattg ctacaaggaa gggtaaatac agagagtagt caaggtgatc 146760
   tcacagggga gaaggtaact aagcatccaa attgaaacac ttaaatttaa acagctgtaa 146820
   aacatgtaat cagccattca aaatgtaatt gtcaaaactg gctacactta ccgaacattt 146880
   cactctgcat ttgtttgaaa tgttttgaaa ctctaagttt aggttgaaca ttatatttgg 146940
   ttagcaggga aaattttgcc tattctgatt tacagtgaac tacaggaagc taataagttg 147000
   cctgggagat actgactgaa actatgatct gcataggtca cttagttact aattggttat 147060
   gggaaaggca tggctaagcc tactgcactg aagatttgaa actatgaaca ccacacacag 147120
   gaggaacctt aacccttagc agtcaaatgc tgcatgtaat aatatgcaga agaggaaaat 147180
   ctatccttta agtttgttaa gaaccagcag ttttaggagg aatcatttgc cattctctga 147240
   cagtttacat gtgtttccct gaaaaatgag ataaagtaaa ataaaacaaa aataagcaca 147300
   agtttactca gagatgatga aaccccaaac tagattggtg gttgagaaac atgatattca 147360
   tggccttcat agtaaaagca ttgttgttaa gcactctact ctcattacct cttttagccc 147420
   ttgtaactgc tgtgatggat atgtctatat ctccccctct atatttgagg aaactgatgc 147480
   ttaaagagac taagaaatag gttcagcaac catcctgatt tgcccgagac tgagaggttt 147540
   cccaggttgc agaattttca ctcttaacac caggagactc ctgggcaaac cagaatggtt 147600
   ggtcacctta tgcagcagca ggtcacttac ttactaactg gttatggaaa aggcatggct 147660
   aggcctactg cactaaagct ttgaaagcaa ctccacatgg cagcaagcat ttctttctgt 147720
   gttatagggt atcttacttt tttggagaca gggtcttgct ctgtcaccca ggctggagtg 147780
   cagtgataca actatagctc actgcagctt cgaactcccg ggctcaaagg agcctcccac 147840
   cttagcctcc caagtagcta ggatgacagg tgcctgccac catgtctggc taatttttta 147900
   atttaaaaaa ttttaaattt tggggtctca ctatgtttcc agggtgatct caaactcctg 147960
   gactcaggcg accctccttc ctcaccttcc caaagtgctg ggattacagg catgagttgc 148020
   catgtccagc cttatagggt atttttcatt gtactgattt agatattcaa ccctctccac 148080
   tcatccttag acctgagaga tactcactcc atatgatctg ctaaaccatc caaatgtgaa 148140
   catcctcccg tgcccctgag gctgaaatgg cagcattcac atgtatctga tacaaaatct 148200
   tatttttatg atagagaaaa ctaaagctca gagaggttta agtagttaac caaggtgcca 148260
   tttaacttga cttctgtaaa tcaccttgag acttaaagga acaaattgtt tttcctttaa 148320
   attagaaatc tttaatacct ggattaggtt caaagggtaa actgtcaagg gtaaatttca 148380
   agagagataa tatttcaaat tggtggtatc gaatgataaa aatacataat ctgtaatgtg 148440
   tatttgcaag taattaattc ggctgtagaa ggcaagaaaa caaacaggat gggtgaagct 148500
   cagaagatgc attaagcata gataagtaaa ttagtaatct gtaagacgtg ttcaatataa 148560
   ataaggtaag tggagcatat gcgtgtgaaa atagaaatgg ttgaaaggac gaagtgactt 148620
   taactattca tgatatacta attatatatc aagcatgtgt gttcccaact agtcacatta 148680
   catgaagtat gtaatgtaac gtgtaagtac tcagaagagg aactcttgaa ctatatatga 148740
   aaggtaagtt ttctagaaga ggtgatacct aaaccaaatt cttaaggctc tgagaaggta 148800
   agggtgggct ggggaggggc attcagatgg agacaaccat aggtagttgg gtatatggat 148860
   ggatacaggg taaggaatca ctgtaggtta attctatgaa aatgattgaa gtcaaaacgg 148920
   aagcacagtc taaaaagtat gcaggatgga actcaaggag ctcacagact gtcaaggaaa 148980
   gaggacgata tgtagcatct gacttactta tattgcaaat aactcaaagc atgtattaat 149040
   atctttagta ataatagatc caggttgggc cttgggaata ggtatagatt tggacttatc 149100
   atgtcaaaag acattaatgt tcaaaaattt tattaccatg tcatcaaaaa tgtctttact 149160
   taggaaccta agaggttttc ttttaatact aatacagcaa ctgaaaagag taaaaccttg 149220
   acatgcatct gttaaagtcc catctatagt catcattatc atcattgtta cctttattat 149280
   ttaagtaaat ctagactgtg ttaaataacc accacacatt atcttactta atatgtaata 149340
   ctataaagta ctatacaata tgctatacta atattataaa ataggtacaa tagtccccca 149400
   cttatccatg ggggataagt tccaagaccc cagtggatac ctgaaaccta gaagagtacc 149460
   acacctgatg gccatcaatt ggaatacact tctgttcatg tcttccattc acaagtttaa 149520
   tgccttttcc atttaaccaa gcacttagca tgcactgtgg ctgtaatttc tgcaatttgc 149580
   gatgtgacaa ccaaactggc agaaatttct tttccttctt cacaatttca cagatagaac 149640
   atcttagcaa tctcagcata tgattctttt ccttcctaaa gttgacaact tttatctttc 149700
   acttaaagga agcaactgac agcttctttt tggcatattc aaattgctag tatccttcct 149760
   cttgcacttt ggagccatta ttaagtaaaa taaaggttac atgagtccaa gcgctgggct 149820
   gccaggacag tcaatctgat aaccaagaca gatactaagt gactaatagg tgagtagtgt 149880
   gtacagcgtg caggctctgg acaaagggag gattcatgtc tcagggagga cagagcagga 149940
   cagcacgaga tttcatcatg ctactcagaa gggtacaaaa tttaaaactt aagaattgtt 150000
   tatttctgga attttccatt taatattatc aggttgacca gggctaactg aaactgcaga 150060
   ttgtgaaact gtggataatg ggagactact cttgacttaa atagacttta actgaaaatg 150120
   aggctctgag agttgaagtt tatacaatca agaagtggca aagttggcct aaaaagccta 150180
   atgtatatga ttccaaaaca ataaggtttg tatctagaat cagatacatt aggcttctta 150240
   ggccaacttt gccacttctt gattgcataa acattatcag aaatacattc tgataataaa 150300
   tattttaaca attagacttt agttaacata actcaagtca gtacaaattt caacaaagct 150360
   gatagtacca ggagtcgttc caagtgcttt tcatgattat ataacacttt tctattcttc 150420
   catagccatg agcaaaaagc aagaagcaca atctgagtgc caaccctgcc cacaatgttc 150480
   agtagacaaa aatttcccat gatactcaaa attgcctggt gcagtctcac ctaccaccaa 150540
   taccactgtg aggatttatt tagcagattc atgatctatg cattcttcac catcaacatg 150600
   aaaggctaaa tagcatcaca acattaacct ttctccagag taatttatat accattaaga 150660
   gatgcagtct gtcaaaggaa aaatgccact gatggtagaa ttttaatcat tgacaaacat 150720
   ctttaggaga cattttgtgg ttttcttttt tttagattag ggaagggaga tagagatagg 150780
   atgtcagggg gtatgataga gagtagtttg gggaaaagca aaccctctct tgccataggt 150840
   ccctcacttt atttgtaatg actctacctc tggatcataa atattgggaa aaaaatcatt 150900
   aatgagtgca aattgttgta aatgacaaaa tagaaagtta ttagtcttta gctacacata 150960
   tgtatgtgtg tgttatcaat acactgctct aaacactaca tggtgtgtta tgaatttacc 151020
   actaaaaaca ttatgcataa attggaacaa ttatctgtat cacgaggtaa gatctacctg 151080
   tcaaaacaag gggcttatag tgccaggaaa tggagtggga ggattttatg gcatacccca 151140
   ggagaggcac tcgtgattag cggtccactt cccataagac aaccaaagtc agttgactgg 151200
   tgaaaaccaa aattcagtgg cttccagggc cacttctact tagaatacca agaaaaatgc 151260
   ttctgctgtc cactcacctg caaagaatgg aaggaccttc tccaactcac aggaaatgca 151320
   tttctttcca acctgaagaa ttaagagttc gctagtcatt ctccctactt tttgttttct 151380
   ttcctgtatc ttggtcaaaa gtgtgggaaa atcgaggcat gctaaagata ttttgagctc 151440
   tcctaatcct cagttaaaat tgtctgctct gctgaggcag gagaatggcg tgaacccggc 151500
   aggcagagct tgcagtgagc cgagatcgcg ccactcttgg acgactgagt gagactctgt 151560
   ctcaaaaaaa aaaaaaaaaa aaaaagaagt ctgctctgga gaatggtaat gaaatgtaat 151620
   aagagctcta gaatgaaacc ccatacgtaa aaacttttca tcacaccaac tggttattct 151680
   ttctgagatg ggcctgaaat taaaccaatc tttaaagaca gaagttcata gcaatctcta 151740
   ggatgagaaa acctgggctc attcattcat tcatgcatcc attcattctt tctttcaaca 151800
   atatttacca agtgaattct aggtgatgct gcacttccat gtcttcttgt tgagcgttga 151860
   agcagaaaac gagtgggagc caaaccatga caattcaatg cctgacactg agaaactaga 151920
   aaattcaaat gcagatgggg cttcattttt gtctactgat aatgtttttg aaagccttga 151980
   tttcctcccc acaactgaat tcccaataaa ggtatggaaa gttcattgtg caatggccgg 152040
   ggaattcctg ggtcacttgc accgcttgaa gaagcgatta gggatggggt cactgtgttc 152100
   cccagtgact gtagtaaaga gctgggaagt caaggaccca gtcactttct gagcttttgt 152160
   ttcctcagat ttcaaatagg cattaaaaaa acaaaacaaa acaaacctat ttcaagaaga 152220
   attaaagaga gaacttccac aaagaacctg atcatggtat gtgagataac atacaaaatt 152280
   gctaagtcag gaacttgaac caaagacctt ctggttcaat ttgcaagaga tctacatagc 152340
   cacttgagag ggtcagacaa aaattttact attaaaggag ggcttgactg tttttctctg 152400
   gactcctgac cactcagggg ccatgtgatg tgttctgaga aatggtgtca cggcagtaaa 152460
   agatagtggc cttcttcaga gtcatgcctt acagagcctc cttagtgttt cacctggaca 152520
   aaaagaaaaa cagaatttca cagaaaatta aagccttact ctctttcttt gacaaagata 152580
   tttggcaaga ttcatcatac aattccacaa gcattagtca ttgaaggatc caaagcacgg 152640
   actgaagaac agaaaaacct gctgatacaa gtcccttctc aggaatggct tggactgacc 152700
   aggctggttc ttccacccaa tttggccaat ggatttcaaa gacaaagttt tggacttgaa 152760
   tttcggtaac tttaagtcta cggaagaaac ttatcgtttt gacaaacttg aaatatgatt 152820
   tgtgtgtact tgtctattct atctaatgag agatggcaaa ctccttaaga acaagaattg 152880
   tgttatgtct ggcacattgt aggtgctcaa gaaatatatt cactacatga ataaataaat 152940
   aaaggtcaac aaaagcctgc aaaatagttt gtgtgattat tttttggaaa gttttaatga 153000
   taaagagtgt ggtaaaaatt aaatagaata ataagaagaa atgaatccac gaattcatgt 153060
   tatttaaaaa aatttgaaat tttaaaaata acaaataaaa ataaaaataa aaaaataaaa 153120
   aataacttga aaagaaagcc aatatatcca gaaaagcaga gcctgagaaa ctgcagaaaa 153180
   atggagcaga agcctacttt atctctggac ttagtgttga gacaatatgt ttcctcattg 153240
   cttaagccag tttttctatt atttgcagtg aaaaagtgct ataattgatt caaaatcaat 153300
   gcatttgctt ccagtggggc atattctttc caataaccac aaggcattgg ttgcttcccc 153360
   tagacactcc ttcctctatt ggaagatctg ggaaaccaaa ttagttattg gaaatttctc 153420
   tgcctcaact cctcccttcc ctcctgaggg tgagttcttt aaaagagaat gggatgaatt 153480
   ttcagagagc ctgaaataca tttttagaaa tcctgttgaa gtactgtgac ccagaaacaa 153540
   ggtcccctct ccagtgactg agagattcaa gacactccac agtgagaact ttttttttct 153600
   tcttgaacaa tggtgaagac cctctgtcct tgtcttctcc tttgcttctc tttgtgtaga 153660
   ccatcattgt tcaaaaagtg tacatcacat gagcttctca tgcaaactac atatatgtaa 153720
   tttaaatttt tctagtagtt acatttaaaa agtaaaaaga aataggtgaa aataaagtta 153780
   aatttattat gttttattta acccaatata tctaaaatat cacttcaacg tgtaattaac 153840
   ataaaaacta tagagttatt ttacattttt ttatactaag tcttcaaaat cctgtgtgta 153900
   ttttacactt agagcacact gcaatttgga ccagccacat ttcaagtgct caatagacag 153960
   cacagttgta gccacacaca aatctccttt acttctgtta cagcataaaa ctcttagaca 154020
   cacagcgttt agcatctaat taattatgta actgtattac actgttattg caataatcat 154080
   taaacaagtg tcattccaac cccctatctg ttttaatgca atcttacatg aaacagtttc 154140
   ttatattgag atataggcca gtcttgatgg gtatagagat gtcattcctc tttatccaaa 154200
   gatttcctta ttattcttta ctgaatgtca catttacagc tattagatgg gaagattata 154260
   atttattcta gaaacttcta ttttaaaact atgtattaac caaaattaca catgtatggg 154320
   gtacctgctt atttctagta agcccactag ctgatgtagc aaataggtag ccaggccaaa 154380
   agcaaaattt tgatagttgg ccttttgttt aaaaagtgtt ttatatctga ttagctgaat 154440
   tctaactatc ttaggaaata aatcaactaa cagtgtaaga gacagtctac tatggtggaa 154500
   agagataaac ttcagagtca aacaaactta accctatcac ttactagctt tgtaatggtg 154560
   gatatattca ttagcttctc acttgtagaa tgagaataat aatatattat gtctgagact 154620
   gctagtttta tcccaatatc cattctcccc ttcttcatca gtaaataacc accaaaagtt 154680
   agctagacac agatgcccag aaaaaagtcc atgttttcag ccctttcttg cagctagatg 154740
   tgtttatgtg gttaaatact ggacactgag gtcaaaataa aagcggtgtc tgtaacttcc 154800
   aggaagtatc cttttaaaaa gcagggagaa tgctattttc ttccttatgg ctagaattca 154860
   gacatgatgg gtggagctag agaagtcatc atgaaccatg aaatgtaaac tgtgttgaga 154920
   acagtagaac aagagcagag ccctgggtct ctcctgatca tggaatcgcc accaaagccc 154980
   agggttttag aaaagaaaga aatgcatttc tatttttttg agccattgtt gttacttatt 155040
   ttctctaact tgctgctgaa cctaacacat gcttaatccg tagcatcttt ttgaggatta 155100
   aatgggatgg catatgtagt ccactttgta cagggcttgg cacctgggaa tgcaggtgct 155160
   taatatagga ctgtttttat tactagcata aaatatctcc ttaaaagggt cactataagg 155220
   gtcagatgaa acctataagt gagcttgctt ttcaatctgt aaatctgaga atcactgtat 155280
   aaatgttggt cataattatc atcatcacca ttaaattatt acaaggaact ggctaactgc 155340
   cagcatgctc tactatgatc aatctatatt taggtcaatt aaaaatagat agatacagac 155400
   ttctttgctt ttgttaggtt tacttaactt tagtcaactg tgaatgattg attataggct 155460
   tgccattaca ttgttgcttc agagcttaga tgaaacactc atttgggtag tataaagaaa 155520
   gcttagaact tgatgaggct atgacatctt ttattcattt acttttacct taaaaagttc 155580
   tctattacaa aaatcaatat agtgtttgtt gttaaaataa attatagaga catgagtaaa 155640
   gtgaaagaag agagttcacc cccttccttc ttttccatcc cattctttag aattaaccat 155700
   attacaatgt aaccaattat tccttgtatg taaatatcat ttttatataa tgggatatac 155760
   acatgtataa tatacatttt ctatttccaa atgaaaaaca aaatcttttt tttgcagaaa 155820
   gttcaaagtt gttgaaaaga aaatcttttg caacagcttt tttgatttgt tcgtttccca 155880
   atttaaaatg tctcctgggc atctttccat gtaaatacat attcatcaat cccattttaa 155940
   aaaccatata cagagtcatt taaacatttt tactaattaa ttattggaaa attgtttgta 156000
   aaataaagga gactgccctt aaaataaaat agcaacttag ctaggattaa attaggcaat 156060
   tcagaaaaat gcttagcaaa tgtttggcag attgtaaatg attgataaat aaatattagc 156120
   cattatgata ttgatgatag tgatgattaa agatgactat ctctctaagc tcactctcaa 156180
   taaatttctg ttgtcagaga ggtggtttat tttatttaat tgcccaatat gtacatttat 156240
   atataatatt cccattttat attttcattt taaggacgta cttggctgtg ttatatggtg 156300
   gaggtgggtg ccgtgttctg gaaggactgt cagactcaag ttggttctct tcaggatctg 156360
   cccaacccat ggcgacctag tacagtgcca aaattgaaca aaaaaaatac aatccatcca 156420
   tctgtcagtt agtgaggcag caaagaaggt atttagcttc taggacattt aaaaacctaa 156480
   gttaattcta tctaaaagaa agacatctgt ggggtgggac tcgcctcctc tgagcctttg 156540
   agaccagtgg ggatttaaac aggtataaat aatgactctt gaatagccac cagacagcca 156600
   gtggtgcaac ctactctagg gagatgagtc acaggctgag ccgtgggtag taagtcccta 156660
   ggagaaggga agaggggtgt ctgggatccc aggaagagca ataaatgcag tctgtcagct 156720
   ccgcagcaga ggaggggctg tgacagcagc cctcacccag aaggggtgaa tggtaaatct 156780
   acctcaatag acagtggcct gtgcattgta aatctgatca ttttgctgtg cccaaaacat 156840
   cttaagtata attataccaa agcctgaaaa aagactggct aaagagaaaa ctaaaataga 156900
   ttttttaaaa tgcaaacaca caattctgcc tacaaaaatg ttaagtgcca aggaaaatat 156960
   tgttaaaatc caagaggacc tcctttccta agctgcagtg ttcaggaatt tatatgtata 157020
   taaggttggt agtgaccatg gacaaatcat tgcctacacc tctgccagaa catctgtcat 157080
   ttctcatgaa gccggaactt taaatgtcac aactagatca gaaataacat tgatatcaga 157140
   accatcatgt actttttttc tgctctgcat gtgagcatct ggggtgccta aaatccaacc 157200
   ctcacagctc ttgaaaagtc ctgtgtcttg cagaagggat gcctttcagt gatgcatccg 157260
   tgaggaggcg gcgccttttg ataattcaca caaaggtctt gctagtagag ggaggcctta 157320
   ccttgactgg aggagagacc tacgtttgga tctcggctaa agtatcttcc aagagcaaca 157380
   ttcttgttag cataacatgc cattttcatg tcctacagat tcatagattt atggagtttt 157440
   gaactagtta aatgccttag aaatgaccaa tacccaccca ttttgttgat aagggacctg 157500
   aagcatagag aagtcagata gtttgaatat ttgtccctac ccaaatctca tgttgaattg 157560
   taatctccag tgttagaggt ggagcctggt gggaggtgtt tggatcatgg gggcaaattc 157620
   ctcatgaatg gcttgggcca tccccttggt gataagtgag ctctgagtta acactgagat 157680
   cacatagttt aaaagtgtgt ggcacctccc tcacacctca actctctttc ttttgcttct 157740
   gtttttgcct catgatgtgc ctactctccc ttgac                           157775